# EUROPEAN PATENT SPECIFICATION

(11) **EP 1 472 375 B1**
(45) Date of publication and mention of the grant of the patent: **01.04.2009**
(21) Application number: 03729383.4
(22) Date of filing: 08.01.2003
(51) Int. Cl.: C12Q 1/68, A01N 43/04, C07H 21/04, A61K 31/07

(54) **GENE PRODUCTS DIFFERENTIALLY EXPRESSED IN CANCEROUS BREAST CELLS AND THEIR METHODS OF USE**
IN KANZERÖSEN MAMMAZELLEN DIFFERENTIELL EXPRIMIERTE GENPRODUKTE UND IHRE VERWENDUNGSVERFAHREN
PRODUITS GENIQUES A EXPRESSION DIFFERENCIEE DANS DES CELLULES CANCEREUSES DU SEIN ET METHODES D'UTILISATION ASSOCIEES

(30) Priority: 08.01.2002 US 345637 P
(43) Date of publication of application: 03.11.2004
(73) Proprietor: Novartis Vaccines and Diagnostics, Inc., Emeryville, CA 94608 (US)
(72) Inventor: HANSEN, Rhonda, Richmond, CA 94806 (US)
(74) Representative: UEXKÜLL & STOLBERG
(86) International application number: PCT/US2003/000657
(87) International publication number: WO 2003/057926

(56) References cited:
- WO-A-99/21994
- BERTUCCI F ET AL: "Gene expression profiling of primary breast carcinomas using arrays of cancidate genes" HUMAN MOLECULAR GENETICS, OXFORD UNIVERSITY PRESS, SURREY, GB, vol. 9, no. 20, 2000, pages 2981-2991, XP002225994 ISSN: 0964-6906
- MARTIN KATHERINE J ET AL: "Linking gene expression patterns to therapeutic groups in breast cancer" CANCER RESEARCH, vol. 60, no. 8, April 2000 (2000-04), pages 2232-2238, XP001026395 ISSN: 0008-5472
- VACARRO ET AL.: 'VMP1 expression correlates with acinar cell cytoplasmic vacuolization in arginine-induced acute pancreatitis' PANCREATOLOGY vol. 3, 2003, pages 69 - 74, XP002997349
- DUSETTI ET AL.: 'Cloning and expression of the rat vacuole membrane protein 1 (VMP1), a new gene activated in pancreas with acute pancreatitis' BIOCHEMICAL AND BIOPHYSICAL RESEARCH COMMUNICATIONS vol. 290, 2002, pages 641 - 649, XP002965339

### SEQUENCE LISTING

<110> Hansen, Rhonda
<120> GENE PRODUCTS DIFFERENTIALLY EXPRESSED IN CANCEROUS BREAST CELLS AND THEIR METHODS OF USE
<130> 2300-17767WO
<150> 60/345,637
   <151> 2002-01-08
<160> 516
<170> FastSEQ for Windows Version 4.0
<210> 1
   <211> 114
   <212> DNA
   <213> Homo sapiens
<220>
   <221> misc_feature
   <222> 70
   <223> n = A,T,C or G
<400> 1
   catcctcgga cgccagcaag gtgacctcta agggggcagg gctctcaaag gcctttgtgg 60
   gccagaaggn aaggttcctt cctggtggac tgcagcaaag ctggctccaa catg       114
<210> 2
   <211> 430
   <212> DNA
   <213> Homo sapiens
<400> 2
   gggactcgcc acctcctctt gcacccctgc caggcccagc agccaccaca gcgcctgctt 60
   cctcggccct gaaatcatgc ccctaggtct cctgtggctg ggcctagccc tgttgggggc 120
   tctgcatgcc caggcccagg actccacctc agacctgatc ccagccccac ctctgaacaa 180
   ggtccctctg cagcagaact tgcaggacaa ccaattccag gggaagtggt atgtggtacg 240
   cctggcaggg aatgcaattc tcagagaaga caaagacccg caaaagatgt atgccaccat 300
   ctatgagctg aaagaagaca agagctacaa tgtcacctcc gtcctgttta ggaaaaagaa 360
   gtgtgactac tggatcacga cttttgttcc aggttgccag cccggcgagt tcacgctggg 420
   caacattaag                                                        430
<210> 3
   <211> 527
   <212> DNA
   <213> Homo sapiens
<400> 3
   ctgctaatac agccctggct gtggaatcct tcaccgtctc agctggtatc agccccagcc 60
   tgccttgtgc catatctcag cttggatctc tgctagagtc cccccaacca tatatcatag 120
   agttgaatca caatgagacc gttggctttg aatttgagtc gttggttccc atggtgagat 180
   gcttgttaag actttatact tgggtcaatc tctcacttta ttttgtagaa ccatttgaaa 240
   tcctaggatg tgcttgttct ggaaggatga catgggccca gactgaacaa gtcagcttga 300
   tgatcttaaa tgatggaagt ataggacgtt gcttatttta aaacaaggga aggacacaaa 360
   atggaatgac tgcttagtcc tttctcagat actcttaaaa caatttttta ttgttaaatt 420
   tgtggtaata catggtcaca accgtggatc aaacaaggtc agtctaaagt ggcaggtcct 480
   aggtgtgacc tgataccacc accctttgtg gcagcaccgg gctggac               527
<210> 4
   <211> 262
   <212> DNA
   <213> Homo sapiens
<220>
   <221> misc_feature
   <222> 186, 188
   <223> n = A,T,C or G
<400> 4
   ccggcctcgt ggaccagcct gggctctcgc tggaggaagt ggcttgcaag gaggcttggg 60
   aggagtgtgg ctaccacttg gccccctctg atctgcgccg ggtcgccaca tactggtctg 120
   gagtgggact gactggctcc agacagacca tgttctacac agaggtgaca gatgcccagc 180
   gtacgntncc aggtgggggc ctggtggagg agggtgagct cattgaggtg gtgcacctgc 240
   ccctggaagg cgcccaggcc tt                                          262
<210> 5
   <211> 201
   <212> DNA
   <213> Homo sapiens
<400> 5
   gccactgaaa atccttgtta aaaaccagat cacaaatctg gggctcttgg tcccattgga 60
   gaaggaagga agagcctcaa aataagtgtg cacccatgca catattcagg aacagcttgt 120
   ttagtcttta cactttgcct gaaagttgct tctcctcgtc cctttgtgtg cctgggtggc 180
   ctcggccctg tgcgttggca a                                          201
<210> 6
   <211> 621
   <212> DNA
   <213> Homo sapiens
<400> 6
   tgagggtccc cgctcagctc ctggggctcc tgctactctg gctccgaggt gccagatgtg 60
   acatccagat gacccagtct ccatcctccc tgtctgcatc tgttggagac agagtcacca 120
   tcgcttgccg ggcaagtcag agcattggca tctatttaaa ttggtatcaa caaaaaccag 180
   ggaaagcccc taaactcctg atctatgatt catccagatt gcaaagtggg gtcccatcaa 240
   ggttcagtgg cagtggaggt gggacacact tcactctcac catcagcagt ctgcaacctg 300
   aagatttagc aacttactac tgtcaacaag ggtacagtac acctggcacc ttcggccaag 360
   ggacacgact ggaaattaaa cgaactgtgg ctgcaccatc tgtcttcatc ttcccgccat 420
   ctgatgagca gttgaaatct ggaactgcct ctgttgtgtg cctgctgaat aacttctatc 480
   ccagagaggc caaagtacag tggaaggtgg ataacgccct ccaatcgggt aactcccagg 540
   aggggtgtca cagagcagga cagcaaggac agcacctaca gcctcagcag caccctgacg 600
   ctgagcaaag cagactacga g                                          621
<210> 7
   <211> 548
   <212> DNA
   <213> Homo sapiens
<400> 7
   gacagcatgg acatgagggt ccccgctcag ctcctggggc tcctgctact ctggctccga 60
   ggtgccagat gtgacatcca gatgacccag tctccatcct ccctgtctgc atctgttgga 120
   gacagagtca ccatcgcttg ccgggcaagt cagagcattg gcatctattt aaattggtat 180
   caacaaaaac cagggaaagc ccctaaactc ctgatctatg attcatccag attgcaaagt 240
   ggggtcccat caaggttcag tggcagtgga ggtgggacac acttcactct caccatcagc 300
   agtctgcaac ctgaagattt agcaacttac tactgtcaac aagggtacag tacacctggc 360
   accttcggcc aagggacacg actggaaatt aaacgaactg tggctgcacc atctgtcttc 420
   atcttcccgc catctgatga gcagttgaaa tctggaactg cctctgttgt gtgcctgctg 480
   aataacttct atcccagaga ggccaaagta cagtggaagg tggataacgc cctccaatcg 540
   ggtaactc                                                         548
<210> 8
   <211> 430
   <212> DNA
   <213> Homo sapiens
<400> 8
   tatacacaac atttatttca aactattggg agggatgaga gtggcttaaa aacttccatc 60
   cctacttttc aagagtgcag ttgattctga atctgaaagc ccgcctctgt cctaaaatac 120
   aaacaagcac agacattaaa cctggatact atatgataaa gagggatgta actattgaat 180
   tggatacaag gatcagaatg gaaagaaact cacgatgaaa ttgaacctgg tttttgtata 240
   tttatcaaac ttgtgctgag aatagtgtct gattatacga cttttaagca aagttgggtg 300
   taattaggtg aaaacagccc aggtcctccc gggagcacag aggggctagg ggctggtcct 360
   tctcgtttgc tctagtcttg ctttgctgtc tggtgtagct cctctgctgc tcccatctgc 420
   actaattgac                                                        430
<210> 9
   <211> 493
   <212> DNA
   <213> Homo sapiens
<400> 9
   ctcactattt ggaatttggc cctcgaggcc aagaattcgg cacgaggcgg cacgaggtgt 60
   aactattgaa ttggatacaa ggatcagaat ggaaagaaac tcacgatgaa attgaacctg 120
   gtttttgtat atttatcaaa cttgtgctga gaatagtgtc tgattatacg acttttaagc 180
   aaagttgggt gtaattaggt gaaaacagcc caggtcctcc cgggagcaca gaggggctag 240
   gggctggtcc ttctcgtttg ctctagtctt gctttgctgt ctggtgtagc tcctctgctg 300
   ctcccatctg cactaattga cccaaaacgt gggtatttcc tgctacacaa aagccaaaag 360
   gtttcatgta gattttagtt cactaaaggg tgcccacaaa atagagatta attttaactt 420
   aaattttaag cttgaagatt aggtactatc tgtgaagtta cacttttttt ttttttttaa 480
   aaggaaaaaa tgt                                                    493
<210> 10
   <211> 472
   <212> DNA
   <213> Homo sapiens
<400> 10
   cggcacgagg tgtaactatt gaattggata caaggatcag aatggaaaga aactcacgat 60
   gaaattgaac ctggtttttg tatatttatc aaacttgtgc tgagaatagt gtctgattat 120
   acgactttta agcaaagttg ggtgtaatta ggtgaaaaca gcccaggtcc tcccgggagc 180
   acagaggggc taggggctgg tccttctcgt ttgctctagt cttgctttgc tgtctggtgt 240
   agctcctctg ctgctcccat ctgcactaat tgacccaaaa cgtgggtatt tcctgctaca 300
   caaaagccaa aaggtttcat gtagatttta gttcactaaa gggtgcccac aaaatagaga 360
   ttaattttaa cttaaatttt aagcttgaag attaggtact atctgtgaag ttacactttt 420
   ttattttttt ttaaaggtag agatgtgtgt gtgtgtaggt attaaagatg tg         472
<210> 11
   <211> 271
   <212> DNA
   <213> Homo sapiens
<400> 11
   gtttttcttt tttttataca caacatttat ttcaaactat tgggagggat gagagtggct 60
   taaaaacttc catccctact tttcaagagt gcagttgatt ctggggggga aagcccgcct 120
   ctgtcctaaa atacaaacaa gcacagacat taaacctgga tactatatga taaagaggga 180
   tgtaactatt gaattggata caaggatcag aatggaaaga aactcacgat gaaattgaac 240
   ctggtttttg tatatttatc aaacttgtgc t 271
<210> 12
   <211> 343
   <212> DNA
   <213> Homo sapiens
<400> 12
   gtttttcttt tttttataca caacatttat ttcaaactat tgggagggat gagagtggct 60
   taaaaacttc catccctact tttcacgagt gcagctgatt ctgaatctga aagcccgcct 120
   ctgtcctaaa atacaaacac gcacagacat tagacctgga tactatatga tacagaggga 180
   tgtaactatt gaattggata cacggatcac aatggaaaga aactcacgat gaaattgaac 240
   ctggcttttg tatatttatc aaacttgtgc tgagaatagc gcctgattat acgactttta 300
   agcaaagctg ggtgtaatta ggtgaaaaca gcccacgtcc tcc 343
<210> 13
   <211> 345
   <212> DNA
   <213> Homo sapiens
<400> 13
   agtggcgagc aggttcccac ttgccaaaga tcccttttaa ccaacactag cccttgtttt 60
   taacacacgc tccagccctt catcagcctg ggcagtctta ccaaaatgtt taaagtgatc 120
   tcagaggggc ccatggatta acgccctcat cccaaggtcc gtcccatgac ataacactcc 180
   acacccgccc cagccaactt catgggtcac tttttctgga aaataatgat ctgtacagac 240
   aggacagaat gaaactcctg cgggtctttg gcctgaaagt tgggaatggt tgggggagag 300
   aagggcagca gcttattggt ggtcttttca ccattggcag aaacg 345
<210> 14
   <211> 401
   <212> DNA
   <213> Homo sapiens
<400> 14
   ttttccaagt ccgtttcagt cccttccttg gtctgaagaa attctgcagt ggcgagcagt 60
   ttcccacttg ccaaagatcc cttttaacca acactagccc ttgtttttaa cacacgctcc 120
   agcccttcat cagcctgggc agtcttacca aaatgtttaa agtgatctca gaggggccca 180
   tggattaacg ccctcatccc aaggtccgtc ccatgacata acactccaca cccgccccag 240
   ccaacttcat gggtcacttt ttctggaaaa taatgatctg tacagacagg acagaatgaa 300
   actcctgcgg ctctttggcc tgaaagttgg gaatggttgg gggagagaag ggcagcagct 360
   tattggtggt cttttcacca ttggcagaaa cagtgagagc t 401
<210> 15
   <211> 442
   <212> DNA
   <213> Homo sapiens
<400> 15
   ggcagccggc ccacatgtct ctcaagtacc tgtcccctcg ctctggtgat tatttcttgc 60
   agaatcacca cacgagacca tcccggcagt catggttttg ctttagtttt ccaagtccgt 120
   ttcagtccct tccttggtct gaagaaattc tgcagtggcg agcagtttcc cacttgccaa 180
   agatcccttt taaccaacac tagcccttgt ttttaacaca cgctccagcc cttcatcagc 240
   ctgggcagtc ttaccaaaat gtttaaagtg atctcagagg ggcccatgga ttaacgccct 300
   catcccaagg tccgtcccat gacataacac tccacacccg ccccagccaa cttcatgggt 360
   cactttttct ggaaaataat gatctgtaca gacaggacag aatgaaactc ctgcggctct 420
   ttggcctgaa agtgggaatg gt 442
<210> 16
   <211> 256
   <212> DNA
   <213> Homo sapiens
<220>
   <221> misc_feature
   <222> 96
   <223> n = A,T,C or G
<400> 16
   gaatatgtag atttgcttct taatcctgag cgctacactg gttacaaggg accagatgct 60
   tggaaaatat ggaatgtcat ctacgaagaa aactgnttta agccacagac cattaaaaga 120
   ccttaaatcc tttggcttct ggtcaaggga caagtgaaga gaacactttt tacagttggc 180
   tagaaggtct ctgtgtagaa aaaagagctt ctacagactt atatctggcc tacatgcaag 240
   ccattaatgt gcattt 256
<210> 17
   <211> 405
   <212> DNA
   <213> Homo sapiens
<400> 17
   attctgtgat ttatttgaaa ctgtgaaacc atgtgccata atagaatttt gagaattttg 60
   cttttaccta aattcaagaa aatgaaatta cacttttaag ttagtggtgc ttaagcataa 120
   tttttcctat attaaccagt attaaaatct caagtaagat tttccagtgc cagaacatgt 180
   taggtggaat tttaaaagtg cctcggcatc ctgtattaca tgtcatagaa ttgtaaagtc 240
   aacatcaatt actagtaatc attctgcact cactgggtgc atagcatggt tagaggggct 300
   agagatggac agtcatcaac tggcggatat agcggtacat atgatcctta gccaccaggg 360
   cacaagctta ccagtagaca atacagacag agcttttgtt gagct 405
<210> 18
   <211> 447
   <212> DNA
   <213> Homo sapiens
<400> 18
   tgtgatttca tttgaaactg tgaaaccatg tgccataata gaattttgag aattttgctt 60
   ttacctaaat tcaagaaaat gaaattacac ttttaagtta gtggtgctta agcataattt 120
   ttcctatatt aaccagtatt aaaatctcaa gtaagatttt ccagtgccag aacatgttag 180
   gtggaatttt aaaagtgcct cggcatcctg tattacatgt catagaattg taaagtcaac 240
   atcaattact agtaatcatt ctgcactcac tgggtgcata gcatggttag aggggctaga 300
   gatggacagt catcaactgg cggatatagc ggtacatatg atccttagcc accagggcac 360
   aagcttacca gtagacaata cagacagagc ttttgttgag ctgtaactga gctatggaat 420
   agcttctttg atgtacctct ttgcctt                                     447
<210> 19
   <211> 294
   <212> DNA
   <213> Homo sapiens
<400> 19
   tgtgatttca tttgaaactg tgaaaccatg tgccataata gaattttgag aattttgctt 60
   ttacctaaat tcaagaaaat gaaattacac ttttaagtta gtggtgctta agcataattt 120
   ttcctatatt aaccagtatt aaaatctcaa gtaagatttt ccagtgccag aacatgttag 180
   gtggaatttt aaaagtgcct cggcatcctg tattacatgt catagaattg taaagtcaac 240
   atcaattact agtaatcatt ctgcactcac tgggtgcata gcatggttag aggg       294
<210> 20
   <211> 562
   <212> DNA
   <213> Homo sapiens
<400> 20
   aggagcaggt tggactggcc atccgaagca agattgcaga tggcagtgtg aagagagaag 60
   acatattcta cacttcaaag ctttggagca attcccatcg accagagttg gtccgaccag 120
   ccttggaaag gtcactgaaa aatcttcaat tggactatgt tgacctctat cttattcatt 180
   ttccagtgtc tgtaaagcca ggtgaggaag tgatcccaaa agatgaaaat ggaaaaatac 240
   tatttgacac agtggatctc tgtgccacat gggaggccat ggagaagtgt aaagatgcag 300
   gattggccaa gtccatcggg gtgtccaact tcaaccacag gctgctggag atgatcctca 360
   acaagccagg gctcaagtac aagcctgtct gcaaccaggt ggaatgtcat ccttacttca 420
   accagagaaa actgctggat ttctgcaagt caaaagacat tgttctggtt gcctatagtg 480
   ctctgggatc ccatcgagaa gaaccatggg tggacccgaa ctccccggtg ctcttggagg 540
   acccagtcct ttgtgccttg gc                                          562
<210> 21
   <211> 721
   <212> DNA
   <213> Homo sapiens
<220>
   <221> misc_feature
   <222> 626, 685, 696
   <223> n = A,T,C or G
<400> 21
   ggcacgagat gaggagcagg ttggactggc catccgaagc aagattgcag atggcagtgt 60
   gaagagagaa gacatattct acacttcaaa gctttggagc aattcccatc gaccagagtt 120
   ggtcccgacc agccttggaa aggtcactga aaaatcttca attggactat gttgacctct 180
   atcttattca ttttccagtg tctgtaaagc caggtgagga agtgatccca aaagatgaaa 240
   atggaaaaat actatttgac acagtggatc tctgtgccac atgggaggcc atggagaagt 300
   gtaaagatgc aggattggcc aagtccatcg ggtgtccaac cttcaaccac aggctgctgg 360
   agatgatcct caacaagcca gggctcaagt acaagcctgt ctgcaaccag gtggaatgtc 420
   atccttactt caaccagaga aaactgctgg atttctgcaa gtcaaaagac attgttctgg 480
   ttgcctatag tgctctggga tcccatcgag aagaaccatg ggtggacccg aactccccgg 540
   tgctcttgga ggacccagtc ctttgtgcct tggcaaaaaa gcacaagcga accccaccct 600
   gattgccctg cgctaccagc ttgcancgtg gggttgtggt cctggccaag agcttcaatg 660
   agcacgcatc agacagaacg tgcangtgtt tgaatncagt tgacttcaga aggagatgaa 720
   a                                                                 721
<210> 22
   <211> 496
   <212> DNA
   <213> Homo sapiens
<400> 22
   agatgataac cagaagtctg catttgaagt tcacaaaagt aatcaagctc aaacagttag 60
   tgagaggcag aagaacagac ctaaatcttg taaaaaagga aaaaatatta gggaagatga 120
   tcctgtaaga atgttgcaaa ctgttgcaaa gaaattcgac ttcagtaatt tgagtagtag 180
   gttagatgga gtcagatttg aaaatgaaaa aaattaatgt tattgccaag aacactggta 240
   ataaactgaa gctaagtcag aaaaaatggt tgtttgctag atcccaatgg agaaaagtgt 300
   gtaactgctc ctcgtcaggt ctctgctctt caccataaag acattgctct gtctttggtt 360
   gctgcaagtg atggagctac agtctgtgtt accacaaggg gagatattta cttacttgca 420
   gactatcagt gcaagaagat ggcttctaaa cagttgaact tgaaaaaagt tcttgtgtct 480
   gggggtcata tggaat                                                 496
<210> 23
   <211> 549
   <212> DNA
   <213> Homo sapiens
<400> 23
   ctgcatttga agttcacaaa agtaatcaag ctcaaacagt tagtgagagg cagaagagca 60
   gacctaaatc ttgtaaaaaa ggaaaaaata ttagggaaga tgatcctgta agaatgttgc 120
   aaactgttgc aaagaaattc gacttcagta atttgagtag taggttagat ggagtcagat 180
   ttgaaaatga aaaaaattaa tgttattgcc aagaacactg gtaataaact gaagctaagt 240
   cagaaaaaat ggttgtttgc tagatcccaa tggagaaaag tgtgtaactg ctcctcgtca 300
   ggtctctgct cttcaccata aagacattgc tctgtctttg gttgctgcaa gtgatggagc 360
   tacagtctgt gttaccacaa ggggagatat ttacttactt gcagactatc agtgcaagaa 420
   gatggcttct aaacagttga acttgaaaaa agttcttgtg tctgggggtc atatggaata 480
   caaggttgat cctgaacatt tgaaagaaaa tgggggtcaa aaaatttgca ttcttgcaat 540
   ggatggagc                                                         549
<210> 24
<211> 55
   <212> DNA
   <213> Homo sapiens
<400> 24
   gtgtctgcct tcacaaatgt cattgtctac tcctagaaga accaaatacc tcaat 55
<210> 25
   <211> 498
   <212> DNA
   <213> Homo sapiens
<400> 25
   tccttattta tttaacttca cccgagttcc tctgggtttc taagcagtta tggtgatgac 60
   ttagcgtcaa gacatttgct gaactcagca cattcgggac caatatatag tgggtacatc 120
   aagttcatct gacaaaatgg ggcagaagag aaaggactca gtgtgtgatc cggtttcttt 180
   ttgctcgccc ctgttttttg tagaatctct tcatgcttga catacctacc agtattattc 240
   ccgacgacac atatacatat gagaatatac cttatttatt tttgtgtagg tgtctgcctt 300
   cacaaatgtc attgtctact cctagaagaa ccaaatacct caatttttgt ttttgagtac 360
   tgtactatcc tgtaaatata tcttaagcag gtttgttttc agcactgatg gaaaatacca 420
   gtgttgggtt tttttttagt tgccacagtt gtatgtttgc tgattattta tgacccgaaa 480
   aatatatttc ttctccta                                               498
<210> 26
   <211> 325
   <212> DNA
   <213> Homo sapiens
<400> 26
   gtcgctgcct ctgggggcgc tgtacaccgc ggccgtcgcg gctttagtgc tgtacaagtg 60
   tgtggggggg ggagatgaaa ctgcggttct ccaccaggag gcaagcaagc agcagccact 120
   gcagtcagag caacagctgg cccagttgac acaacagctg gcccagacag agcagcacct 180
   gaacaacctg atggcccagc tggaccccct ttttgagccg tgtgactact ctggctggag 240
   cccagcagga gcttctgaac atgaagctat ggaccatcca cgagctgctg caagatagca 300
   agccggacaa ggatatggag gcttc                                       325
<210> 27
   <211> 166
   <212> DNA
   <213> Homo sapiens
<400> 27
   gaatccagca tcttaaagtt gcatatgtgt agcactaatg tttcttttta aatagttggg 60
   ggaaaatgac ctagaaaacc aaattgcagt ttggtagcca aaattaactc ttggtttatt 120
   tgtcctttgt gtgtgaaaag tcctactatt ccgtgcgtca gacttc                166
<210> 28
   <211> 501
   <212> DNA
   <213> Homo sapiens
<400> 28
   tttttttttt tttttttttt tttttcgcag ctgaattaca tttactgtac aaagaacggt 60
   tcggagagaa ccaggaatgg cggagtgtct aacagcagcg cgggtagtgt tgatgccgtg 120
   aatgcaggac catccaggtc ctcaaagtct gcgaggtttg ttcataatcc caaacaaggg 180
   ccctgctggc agcaacagga caggtggggc caggacaggg aagctggagc aggaggccag 240
   tgtctttggg ggctgtggca gggccgcctg cctggggttc ccttactcat ctggtagttc 300
   atgcaggcca cggccctcat ctcccaggaa cgggccatgg ggcgagtcca ctggtgccca 360
   gtaacaccct ccgtgggacc accttgggaa gcatgtgccg cggagtccac cacggggggt 420
   cctgggtccc gggagggctc cttctgcgtg ctggccatgt cgtgccgcac ggcctgagga 480
   caggaggtag aggtgagcac c                                          501
<210> 29
   <211> 149
   <212> DNA
   <213> Homo sapiens
<400> 29
   cgtcccggag gtgcggtgtg gggcaccggg cggggccgcg ggaaccggcg ccccacggag 60
   ctgctgctgt cagaccaacc ccgggccccc atcatcactg cgccgcgctt tcaggcgccg 120
   agaactaccg ttcccggcat gccatgaaa                                  149
<210> 30
   <211> 475
   <212> DNA
   <213> Homo sapiens
<400> 30
   agcagtaaac agggctgcta tgcctgctct gtagtggtgg acggcgaagt aaagcattgt 60
   gtcataaaca aaacagcaac tggctatggc tttgccgagc cctataactt gtacagctct 120
   ctgaaagaac tggtgctaca ttaccaacac acctcccttg tgcagcacaa cgactccctc 180
   aatgtcacac tagcctaccc agtatatgca cagcagaggc gatgaagcgc ttactctttg 240
   atccttctcc tgaagttcag ccaccctgag gcctctggaa agcaaagggc tcctctccag 300
   tctgatctgt gaattgagct gcagaaacga agccatcttt ctttggatgg gactagagct 360
   ttctttcaca aaaaagaagt aggggaagac atgcagccta aggctgtatg atgaccacac 420
   gttcctaagc tggagtgctt atcccttctt tttctttttt tctttggttt aattt      475
<210> 31
   <211> 570
   <212> DNA
   <213> Homo sapiens
<400> 31
   cttttttttt tttttttttt tactggcatc ctgtacattt acttttaaaa aaggataaca 60
   aaaatgaata ttaacaaaaa tccgggacaa caatattttc aagcaacaaa aactggggtg 120
   gggaagctta ttctgaaggt acatttaaaa ctgaaataac aacttaatga aaattaagaa 180
   ttgcatagcg ctgtgaattt agccttcagc aaaacaaaac agaagctatt tggtattgat 240
   acaaatccat ctatttgata gttagtcatc caatattatg tacatatttt atatactgaa 300
   tgtcatttta agtcctgttt tccaaactcc atttttctgt tgctgggttt ttgttttttg 360
   acaagttaaa cactttctgg cactttctat gacagaattt cttctgaaca tacatgaact 420
   gacattctcc caaagcgtcc cttgtgagtg gacgcgcctt tctgctacat atcgttcatt 480
   tgttacaaaa tgaaataatc cacagtgcga tgtgtctggg tccaccgtgc acagcaacat 540
   ccaggctaaa ccaggctgga ccaaaccttc                                 570
<210> 32
   <211> 645
   <212> DNA
   <213> Homo sapiens
<400> 32
   tccgagcgtc gggagcctgt ggaagagaag agcgcgcggg cgacagttaa acaggcccga 60
   ggcagagaaa ccgccctagc agctctcgcg cgcccggtgc aggcggcggt tgctgcggag 120
   gtccgtgcac agactgcttt gcctgttgtt gctcttcgga ggcggcgatc cccgaaggcg 180
   agctgaaata cggctgcagg ctacaatttg cagccgacga ttaaggaaga cgacgagcgg 240
   gagaggtggc ccaccctcat ggagcgcttg tgctcggatg gcttcgcatt tccccattac 300
   tacattaaac cgtatcatct gaagaggatc cacagagctg tcttacgtgg taatctggag 360
   aaactgaagt accttctgct cacgtattat gacgccaata agagagacag gaaggaaagg 420
   actgccctac atttggcctg tgccactggc caaccggaaa tggtacatct cctggtgtcc 480
   agaagatgtg agcttaacct ctgcgaccgt gaagacagga cacctctgat caaggctgta 540
   caactgaggc aggaggcttg tgcaactctt ctgctgcaaa tggcgccgat ccaaatatta 600
   cggatgtctt tggaaggact gctctgcact acgctgtgta taatg 645
<210> 33
   <211> 572
   <212> DNA
   <213> Homo sapiens
<400> 33
   ctaactgagt aacattcatg aaatgaggct ttctgtggcg gcgtagtgtt tggaattaga 60
   aggtaattca gtagagtgta acttagagaa tattgcaagt gacacattga atcctgcccg 120
   tcagggcacc ttttcctcag agcaatccgg ccacacgaat agaaggctgt cgtgaatcac 180
   atcagatgta aaatcattcc ttctgtttac tcttttaatt ttcatccttt gcaggtagtg 240
   caaattcaac ttcaaatatg gtgtaggttt tgctagattc catatttttt tcttggattt 300
   ttgctaatta tttttagcaa aaaatttttg ctcagtggca ccctccctag tgtccatggg 360
   ttagggccat gctggggaaa acgggccggt atttacacac gcgcaaaaca cccagagacg 420
   gcacaaggag gttgaactca tgtttcagtt cgcgaacatt gactccttac gaaagtcact 480
   tcattctaac tagatgcgcc cacttctggt cattatttcg tttgcatgat gtattgcttc 540
   ttcacgtttt gtttttattg agcacggagt ag 572
<210> 34
   <211> 701
   <212> DNA
   <213> Homo sapiens
<220>
   <221> misc_feature
   <222> 34, 41, 43, 52, 58, 72, 180, 204, 205, 211, 214, 228, 243, 253, 269, 271, 295, 315, 343, 429, 439, 457, 483, 517, 529, 546, 554, 555, 557, 560, 561, 565, 627, 632, 637, 644, 655, 659, 662, 672, 680, 689, 690, 698
   <223> n = A,T,C or G
<400> 34
   ggcacgaggc taactgtgta acatttatga aatntgctct ntntggcggc gnaggggncg 60
   gaatgagaag gnaattcagt agagtgtaac ttagagaata ttgcaaggga cacattgaat 120
   cctgcccgtc agggcacctt ttcctcagag caatccggcc acacgaatag aaggctgcgn 180
   gaatcacatc agatgtaaaa tcannccttc nggngactct tttaattntc atcctttgca 240
   ggnagggcaa atncaacttc aaatatggng naggttttgc tagattccat atttntttct 300
   tggatttttg ctaantattt ttagcaaaaa atttttgctc agnggcaccc tccctagtgt 360
   ccatgggtta gggccatgct ggggaaaacg ggccggtatt tacacacgcg caaaacaccc 420
   agagacggna caaggaggnt gaactcatgt ttcagtncgc gaacattgac tccttacgaa 480
   agncacttca ttctaactag atgcgcccac ttctggncat tattacgant gcatgaagga 540
   ttgctncttc acgnntnggn nttantgagc acgggagtag aaattccagg gctggcttga 600
   catcttccct gcatgctccc tcccagngga cngtccntcc cttncacatg agganctgnc 660
   gnccatggtg gntttctccn ttgggcctnn tgggactngg a                     701
<210> 35
   <211> 300
   <212> DNA
   <213> Homo sapiens
<400> 35
   gctaactgag taacattcat gaaatgaggc tttctgtggc ggcgtagtgt ttggaattag 60
   aaggtaattc agtagagtgt aacttagaga atattgcaag tgacacattg aatcctgccc 120
   gtcagggcac cttttcctca gagcaatccg gccacacgaa tagaaggctg tcgtgaatca 180
   catcagatgt aaaatcattc cttctgttta ctcttttaat tttcatcctt tgcaggtagt 240
   gcaaattcaa cttcaaatat ggtgtaggtt ttgctagatt ccatattttt ttcttggatt 300
<210> 36
   <211> 374
   <212> DNA
   <213> Homo sapiens
<400> 36
   tggtacgcct gcaggtaccg gtccggaatt cccgggtcga cccacgcgtc cggaggggtc 60
   ctggagaatg ggttacccca gttgtcttat ttaaatggtt acccatcaga ttttaatttt 120
   atcttctctt tgagagcttg gtaataagaa gcacttaaat cactccaaag aagactttaa 180
   aaagggagca gtgaaaaggt cttaataatt tattgattga attaagaaat actagctaat 240
   taagaatctg agtctaaaca gcacagattt tttctttctg cttttaaatt gtgttttaaa 300
   aaaagagaca gggggctggg cgtggtggct cacgcctgta atcctagcac tttgggaggc 360
   cgaggcgggt ggat                                                   374
<210> 37
   <211> 290
   <212> DNA
   <213> Homo sapiens
<400> 37
   gaggggtcct ggagaaatgg gttaccccag ttgtcttatt taaatggtta cccatcagat 60
   tttaatttta tcttctcttt gagagcttgg taataagaag cacttaaatc actccaaaga 120
   agactttaaa aagggagcag tgaaaaggtc ttaataattt attgattgaa ttaagaaata 180
   ctagctaatt aagaatctga gtctaaacag cacagatttt ttctttctgc ttttaaattg 240
   tgttttaaaa aaagagacag ggggctgggc gtggtggctc acgcctgtaa           290
<210> 38
   <211> 405
   <212> DNA
   <213> Homo sapiens

<400> 38
   gccctttcga gcggccgccc gggcaggtac ctgggattac aggcacccac caccacgcct 60
   ggctaatttt tttttgtatc tttagtaggg ttttgccatg ttggccaggc tggtctttaa 120
   ctcctacctc gtgatccacc cgcctcggcc ccccaaagtg ctaggaccac aggcgtgagc 180
   caccacgccc agccccctgt ctcttttttt aaaacacaat ttaaaagcag aaagaaaaaa 240
   tctgtgctgt ttagactcag attcttaatt agctagtatt tcttaattca atcaataaat 300
   tattaagacc ttttcactgc tcccttttta aagtcttctt tggagtgatt taagtgcttc 360
   ttattaccaa gctctcaaag agaagataaa attaaaatct gatgg                405
<210> 39
   <211> 736
   <212> DNA
   <213> Homo sapiens
<220>
   <221> misc_feature
   <222> 2, 3, 4, 5, 6, 7, 8, 9, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65
   <223> n = A,T,C or G
<221> misc_feature
   <222> 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118
   <223> n = A,T,C or G
<221> misc_feature
   <222> 119, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671
   <223> n = A,T,C or G
<221> misc_feature
   <222> 672, 673, 674, 675, 676, 677, 678, 679, 680, 681, 682, 683, 684, 685, 686, 687, 688, 689, 690, 691, 692, 693, 694, 695, 696, 697, 698, 699, 700, 701, 702, 703, 704, 705, 706, 707, 708, 709, 710, 711, 712, 713, 714, 729, 736
   <223> n = A,T,C or G
<400> 39
   gnnnnnnnna gacnnnnnnn nnnnnnnnnn nnnnnnnnnn nnnnnnnnnn nnnnnnnnnn 60
   nnnnnnnnnn nnnnnnnnnn nnnnnnnnnn nnnnnnnnnn nnnnnnnnnn nnnnnnnnna 120
   cctgggatta caggcaccca ccaccacgcc tggctaattt ttttttgtat ctttagtagg 180
   gttttgccat gttggccagg ctggtcttta actcctacct cgtgatccac ccgcctcggc 240
   cccccaaagt gctaggacca caggcgtgag ccaccacgcc cagccccctg tctctttttt 300
   taaaacacaa tttaaaagca gaaagaaaaa atctgtgctg tttagactca gattcttaat 360
   tagctagtat ttcttaattc aatcaataaa ttattaagac cttttcactg ctcccttttt 420
   aaagtcttct ttggagtgat ttaagtgctt cttattacca agctctcaaa gagaagataa 480
   aattaaaatc tgatgggtaa ccatttaaat aagacaactg ggtaacccca tttctccagg 540
   acccctctct gcaacagaga gctattctct ttctttggcc tagtaaacct ctgctcttaa 600
   cctttaaaaa aaaaaaaaaa gtaccnnnnn nnnnnnnnnn nnnnnnnnnn nnnnnnnnnn 660
   nnnnnnnnnn nnnnnnnnnn nnnnnnnnnn nnnnnnnnnn nnnnnnnnnn nnnncatagt 720
   ggttcctgng tgaaan                                                 736
<210> 40
   <211> 725
   <212> DNA
   <213> Homo sapiens
<220>
   <221> misc_feature
   <222> 2, 3, 9, 5, 6, 7, 8, 9, 10, 12, 13, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63
   <223> n = A,T,C or G
<221> misc_feature
   <222> 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 605, 606, 607, 608, 609, 610, 611, 612, 613, 614, 615, 616, 617, 618, 619, 620, 621, 622, 623
   <223> n = A,T,C or G
<221> misc_feature
   <222> 624, 625, 626, 627, 628, 629, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670
   <223> n = A,T,C or G
<221> misc_feature
   <222> 671, 672, 673, 674, 675, 676, 677, 678, 679, 680, 681, 682, 683, 684, 685, 686, 687, 688, 689, 690, 691, 692, 693, 694, 695, 696, 697, 698, 699, 700, 701, 702, 703, 704, 705, 706, 707, 708, 709, 710, 711, 712, 713, 714, 715, 716, 717
   <223> n = A,T,C or G
<400> 40
   gnnnnnnnnn anngnnnnnn nnnnnnnnnn nnnnnnnnnn nnnnnnnnnn nnnnnnnnnn 60
   nnnnnnnnnn nnnnnnnnnn nnnnnnnnnn nnnnnnnnac ctgggattac aggcacccac 120
   caccacgcct ggctaatttt tttttgtatc tttagtaggg ttttgccatg ttggccaggc 180
   tggtctttaa ctcctacctc gtgatccacc cgcctcggcc ccccaaagtg ctaggaccac 240
   aggcgtgagc caccacgccc agccccctgt ctcttttttt aaaacacaat ttaaaagcag 300
   aaagaaaaaa tctgtgctgt ttagactcag attcttaatt agctagtatt tcttaattca 360
   atcaataaat tattaagacc ttttcactgc tcccttttta aagtcttctt tggagtgatt 420
   taagtgcttc ttattaccaa gctctcaaag agaagataaa attaaaatct gatgggtaac 480
   catttaaata agacaactgg ggtaacccat ttctccagga cccctctctg caacagagag 540
   ctattctctt tctttggcct agtaaacctc tgctcttaac ctttaaaaaa aaaaaaaaag 600
   taccnnnnnn nnnnnnnnnn nnnnnnnnnn nnnnnnnnnn nnnnnnnnnn nnnnnnnnnn 660
   nnnnnnnnnn nnnnnnnnnn nnnnnnnnnn nnnnnnnnnn nnnnnnnnnn nnnnnnnggt 720
   atccg                                                             725
<210> 41
   <211> 474
   <212> DNA
   <213> Homo sapiens
<220>
   <221> misc_feature
   <222> 243, 267
   <223> n = A,T,C or G
<400> 41
   ccggaaaaaa agaaccattt ggatacatag gtatggtctg agctatgata tcaattggct 60
   tcctagggtt tatcgtgtga gcacaccata tatttacagt aggaatagac gtagacacac 120
   gagcatattt cacctccgct accataatca tcgctatccc caccggcgtc aaagtattta 180
   gctgactcgc cacactccac ggaagcaata tgaaatgatc tgctgcagtg ctctgagccc 240
   tangattcat ctttcttttc accgtangtg gcctgactgg cattgtatta gcaaactcat 300
   cactagacat cgtactacac gacacgtact acgttgtagc ccacttccac tatgtcctat 360
   caataggagc tgtatttgcc atcataggag gcttcattca ctgatttccc ctattctcag 420
   gctacaccct agaccaaacc tacgccaaaa tccatttcac tatcatattc atcg       474
<210> 42
   <211> 540
   <212> DNA
   <213> Homo sapiens
<400> 42
   cataggtatg gtctgagcta tgatatcaat tggcttccta gggtttatcg tgtgagcaca 60
   ccatatattt acagtaggaa tagacgtaga cacacgagca tatttcacct ccgctaccat 120
   aatcatcgct atccccaccg gcgtcaaagt atttagctga ctcgccacac tccacggaag 180
   caatatgaaa tgatctgctg cagtgctctg agccctagga ttcatctttc ttttcaccgt 240
   aggtggcctg actggcattg tattagcaaa ctcatcacta gacatcgtac tacacgacac 300
   gtactacgtt gtagcccact tccactatgt cctatcaata ggagctgtat ttgccatcat 360
   aggaggcttc attcactgat ttcccctatt ctcaggctac accctagacc aaacctacgc 420
   caaaatccat ttcactatca tattcatcgg cgtaaatcta actttcttcc cacaacactt 480
   tctcggccta tccggaatgc cccgacgtta ctcggactac cccgatgcat acaccacatg 540
<210> 43
   <211> 587
   <212> DNA
   <213> Homo sapiens
<400> 43
   gaccatgagt catttagaat agtgataaat agaatacaca gaatagtgat gaaattcaat 60
   ttaaaaaatc acgttagcct ccaaaccatt taattcaaat gaacccatca actggatgcc 120
   aactctggcg aatgtaggac ctctgagtgg ctgtataatt gttaattcaa atgaaattca 180
   tttaaacagt tgacaaactg tcattcaaca attagctcca gtaaataaca gttatttcat 240
   cataaaacag tcccttcaaa cacacaattg ttctgctgaa gagttgtcat caacaatcca 300
   atgctcacct attcagttgc tctgtggtca gtgtggctgc atagcagtgg attccatgaa 360
   aggagtcatt ttagtgatga gctgccagtc cattcccagg ccaggctgtc gctggccatc 420
   cattcagtcg attcagtcat aggcgaatct gttctgcccg aggcttgtgg tcaagcaaaa 480
   attcagccct gaaatcaggc acatctgttc gttggactaa acccacaggt tagttcagtc 540
   aaagcaggca acccccttgt gggcactgac cctgccactg gggtcat 587
<210> 44
   <211> 622
   <212> DNA
   <213> Homo sapiens
<220>
   <221> misc_feature
   <222> 491, 541, 556, 561, 568, 578, 585
   <223> n = A,T,C or G
<400> 44
   accatgagtc atttagaata gtgataaata gaatacacag aatagtgatg aaattcaatt 60
   taaaaaatca cgttagcctc caaaccattt aattcaaatg aacccatcaa ctggatgcca 120
   actctggcga atgtaggacc tctgagtggc tgtataattg ttaattcaaa tgaaattcat 180
   ttaaacagtt gacaaactgt cattcaacaa ttagctccag taaataacag ttatttcatc 240
   ataaaacagt cccttcaaac acacaattgt tctgctgaag agttgtcatc aacaatccaa 300
   tgctcaccta ttcagttgct ctgtggtcag tgtggctgca tagcgtggga ttccatgaaa 360
   ggagtcattt tagtggtgga gctgccagtc cttcccgggc cgggtgtcgc tgggccatcc 420
   ttcagtcgtt tcgtcatagg cgatctgttc tgcccgaggg ttgtggtcag gcaaaattca 480
   gccctgaatt ngggcactct gttcgttggg ctaaaccccc ggttagttca gtcaaggcgg 540
   naaccccctt gtgggnactg ncctggcntt ggggtctngg cggtntggcc gttggggagg 600
   tttggcccca cggcctctgt gg 622
<210> 45
   <211> 340
   <212> DNA
   <213> Homo sapiens
<400> 45
   aaggcaggaa tgtcaggcct ctgagcccaa gccaagccat cgcatcccct gtgacttgca 60
   cgtatacacc cagatggcct gaagtaactg aagaatcaca aaagaagtga aaaggccctg 120
   ccccgcctca actgatgaca ttccaccatg gtgatttgtt cctgccccac cttaactgag 180
   tgattaaccc tgtgaatttc cttctcctgg ctcagaagct cccccactga gcaccttgtg 240
   acccccgccc ctgcccacca gagaacaacc ccctttgact gtaatttccc atcaccttcc 300
   caaatcctat aaaacggccc cacccctatc tccctttgct 340
<210> 46
   <211> 394
   <212> DNA
   <213> Homo sapiens
<400> 46
   aaggcaggaa tgtcaggcct ctgagcccaa gccaagccat cgcatcccct gtgacttgca 60
   cgtatacacc cagatggcct gaagtaactg aagaatcaca aaagaagtga aaaggccctg 120
   ccccgcctca actgatgaca ttccaccatg gtgatttgtt cctgccccac cttaactgag 180
   tgattaaccc tgtgaatttc cttctcctgg ctcagaagct cccccactga gcaccttgtg 240
   acccccgccc ctgcccacca gagaacaacc ccctttgact gtaatttccc atcaccttcc 300
   caaatcctat aaaacggccc cacccctatc tccctttgct gactctcttt ttggactcag 360
   cccgcctgca cccaggtgaa ataaacagcc atgt                             394
<210> 47
   <211> 246
   <212> DNA
   <213> Homo sapiens
<400> 47
   tagccctgat aggcgctatt ttcctcctgg ttttgtattt gaaccgcaag gggataaaaa 60
   agtggatgca taacatcaga gatgcctgca gggatcacat ggaagggtat cattacagat 120
   atgaaatcaa tgcggacccg gggattaaca aacctcagtt ctaactcgga tgtctgagaa 180
   atattagagg acagaccaag gacaactctg catgagatgt agacttaagc tttatcccta 240
   ctaggc                                                            246
<210> 48
   <211> 336
   <212> DNA
   <213> Homo sapiens
<400> 48
   acatatttcc ttttcctcca ttggccacaa tgggctccaa acaaccacat gcagatttta 60
   caaaaagaaa gttccaaaac tgctcaatca aaagaaagat tcaactctgt gagatgaata 120
   cacacatcac aacgaagttt ctcagaatgc ttctgtgttg tttttatgtg aagatatttc 180
   cttttccatc ataggcctct aagtgcatca actatccact tgcagattct acaaaaagag 240
   tgtttcaaaa ctgctcaatc aaaagaaagt atcaactctg tgaggaaatg cacacatcac 300
   aaagaagttt ctcagaatga ttctgtgtag ttttta                            336
<210> 49
   <211> 518
   <212> DNA
   <213> Homo sapiens
<400> 49
   cagaagggtc tgcaagatgc tgttcttggc cactttcttt cccacctggg aaggcggcat 60
   ctatgacttc attggggagt tcatgaaggc cagcgtggat gtgccagacc tgataggtct 120
   aaaccttgtc atgtcccgga atgccggcaa gggagagtac aagatcatgg ttgctgccct 180
   gggctgggcc actgctgagc ttattatgtc ccgctgcatt cccctatggg tcggagcccg 240
   gggcattgag tttgactgga agtacatcca gatgagcata gactccaaca tcagtctggt 300
   ccattacatc gtcgcgtctg ctcaggtctg gatgataaca cgctatgatc tgtaccacac 360
   cttccggcca gctgtcctcc tgctgatgtt cctcagtgtc tacaaggcct ttgttatgga 420
   gaccttcgtc cacctctgct cgctgggcag ttgggcagct ctactggccc gagcagtggt 480
   aacggggctg ctggccctca acactttggc cctgtatg                          518
<210> 50
   <211> 326
   <212> DNA
   <213> Homo sapiens
<400> 50
   tctgcaagat gctgttcttg gccactttct ttcccacctg ggaaggcggc atctatgact 60
   tcattgggga gttcatgaag gccagcgtgg atgtgccaga cctgataggt ctaaaccttg 120
   tcatgtcccg gaatgccggc aagggagagt acaagatcat ggttgctgcc ctgggctggg 180
   ccactgctga gcttattatg tcccgctgca ttcccctatg ggtcggagcc cggggcattg 240
   agtttgactg gaagtacatc cagatgagca tagactccaa catcagtctg gtccattaca 300
   tcgtcgcgtc tgctcaggtc tggatg                                     326
<210> 51
   <211> 331
   <212> DNA
   <213> Homo sapiens
<400> 51
   acattgaaaa aagtctagac aaactgaaag gcaataaatc ctatgtgaac atggacctct 60
   ctccggtggt agagtgcatg gaccacgctc taacaagtct cttccctaag actcattatg 120
   ccgctggaaa agatgccaaa attttctgga tacctctgtc tcacatgcca gcagctttgc 180
   aagacttttt attgttgaaa cagaaagcag agctggctaa tcccaaggca gtgtgactca 240
   gctaaccaca aatgtctcct ccaggctatg aaattggccg atttcaagaa cacatctcct 300
   tttcaacccc attccttatc tgctccaacc g                                331
<210> 52
   <211> 253
   <212> DNA
   <213> Homo sapiens
<400> 52
   acagaaggga tcgaagacaa attgaaggga gagatgatcg atctccaaca tggcagcctt 60
   ttccttagaa caccaaagat tgtctctggc aaagactcta atgtaactgc aaactccaag 120
   ctggtcatta tcacggctgg ggcacgtcag caagagggag aaagccgtct taatttggtc 180
   cagcgtaacg tgaacatatt taaattcatc attcctaatg ttgtaaaata cagcccgaac 240
   tgcaagttgc tta                                                    253
<210> 53
   <211> 356
   <212> DNA
   <213> Homo sapiens
<400> 53
   atcgaagaca aattgaaggg agagatgatg gatctccaac atggcagcct tttccttaca 60
   acaccaaaga ttgtctctgg caaagactat aatgtaactg caaactccaa gctggtcatt 120
   atcacggctg gggcacgtca gcaagaggga gaaagccgtc ttaatttggt ccagcgtaac 180
   gtgaacatat ttaaattcat cattcctaaa gttgtaaaat acagcccgaa ctgcaagttg 240
   cttattgttt caaatccagt ggatatcttg acctacgtgg cttggaagat aagtggtttt 300
   cccaaaaacc gtgttattgg aagaggttgc aatctggatt caacccgatt ccgcta     356
<210> 54
   <211> 570
   <212> DNA
   <213> Homo sapiens
<400> 54
   ccgctgccgc cgattccgga tctcattgcc acgcgccccc gacgaccgcc cgacgtgcat 60
   tcccgattcc ttttggttcc aagtccaata tggcaactct aaaggatcag ctgatttata 120
   atcttctaaa ggaagaacag accccccaga ataagattac agttgttggg gttggtgctg 180
   ttggcatggc ctgtgccatc agtatcttaa tgaaggactt ggcagatgaa cttgctcttg 240
   ttgatgtcat cgaagacaaa ttgaagggag agatgatgga tctccaacat ggcagccttt 300
   tccttagaac accaaagatt gtctctggca aagactataa tgtaactgca aactccaagc 360
   tggtcattat cacggctggg gcacgtcagc aagagggaga aagccgtctt aatttggtcc 420
   agcgtaacgt gaacatattt aaattcatca ttcctaatgt tgtaaaatac agcccgaact 480
   gcaagttgct tattgtttca aatccagtgg atatcttgac ctacgtggct tggaagataa 540
   gtggttttcc caaaaaccgt gttattggaa 570
<210> 55
   <211> 223
   <212> DNA
   <213> Homo sapiens
<400> 55
   gccgctgccg ccgattccgg atctcattgc cacgcgcccc cgacgaccgc ccgacgtgca 60
   ttcccgattc cttttggttc caagtccaat atggcaactc taaaggatca gctgatttat 120
   aatcttctaa aggaagaaca gaccccccag aataagatta cagttgttgg ggttggtgct 180
   gttggcatgg cctgtgccat cagtatctta atgaaggact tgg 223
<210> 56
   <211> 337
   <212> DNA
   <213> Homo sapiens
<400> 56
   gatgcccata agatatggga agctatgtta tcaagccata ttagatatca agcattaata 60
   tggaaataaa ccagcctgtt tggtgggctc ttcacatgga cgcgcatgaa atttggtgcc 120
   gtgactagga tcgggggacc tcccttggga gatcaatccc ctgtcctcct gctctttgct 180
   ccgtgagaaa catgcaccta tggcctcatg ttctcaaacc gaccaaacca agaaacatct 240
   caccaatttt aaatccgcct ggcttgtgag gccttttgac cccaattcaa gtcttttgat 300
   accctgtgaa ttgcacccat actgcccaga tggctag 337
<210> 57
   <211> 473
   <212> DNA
   <213> Homo sapiens
<400> 57
   aaagatcaaa gtgctgggct ccggtgcgtt cggcacggtg tataagggac tctggatccc 60
   agaaggtgag aaagttaaaa ttcccgtcgc tatcaaggaa ttaagagaag caacatctcc 120
   gaaagccaac aaggaaatcc tcgatgaagc ctacgtgatg gccagcgtgg acaaccccca 180
   cgtgtgccgc ctgctgggca tctgcctcac ctccaccgtg caactcatca cgcagctcat 240
   gcccttcggc tgcctcctgg actatgtccg ggaacacaaa gacaatattg gctcccagta 300
   cctgctcaac tggtgtgtgc agatcgcaaa gggcatgaac tacttggagg accgtcgctt 360
   ggtgcaccgc gacctggcag ccaggaacgt actggtgaaa acaccgcagc atgtcaagat 420
   cacagatttt gggctggcca aactgctggg tgcggaagag aaagaatacc atg       473
<210> 58
   <211> 487
   <212> DNA
   <213> Homo sapiens
<220>
   <221> misc_feature
   <222> 7
   <223> n = A,T,C or G
<400> 58
   actatcnccc aggacatggg accatgctca gctggttgct atcaagacct tgaaagacta 60
   taacaacccc cagcaatgga tggaatttca acaagaagcc tccctaatgg cagaactgca 120
   ccaccccaat attgtctgcc ttctaggtgc cgtcactcag gaacaacctg tgtgcatgct 180
   ttttgagtat attaatcagg gggatctcca tgagttcctc atcatgagat ccccacactc 240
   tgatgttggc tgcagcagtg atgaagatgg gactgtgaaa tccagcctgg accacggaga 300
   ttttctgcac attgcaattc agattgcagc tggcatggaa tacctgtcta gtcacttctt 360
   tgtccacaag gaccttggca gctcgcaata ttttaatcgg agaggcaact ttcatgttaa 420
   aggttttcag gacttggggg ctttccagag gaaattttac tccgctgatt tactacaggg 480
   tacccaa                                                          487
<210> 59
   <211> 532
   <212> DNA
   <213> Homo sapiens
<400> 59
   atagaagtct gggaaaaaaa taaaaacaga atttgagaac cttggaccac tcctgtccct 60
   gtagctcagt catcaaagca gaagtctggc tttgctctat taagattgga aatgtacact 120
   accaaacact cagtccactg ttgagcccca gtgctggaag ggaggaaggc ctttcttctg 180
   tgttaattgc gtaaaggcta caggggttag cctggactaa aggcatcctt gtcttttgag 240
   ctattcacct cagtagaaaa ggatctaagg gaagatcact gtagtttagt tctgttgacc 300
   tgtgcaccta ccccttggaa atgtctgctg gtatttctaa ttccacaggt catcagatgc 360
   ctgcttgata atatataaac aataaaaaca accttcactt cttcctattg taatcgtgtg 420
   ccatggatct gatctgtacc atgaccctac ataaggctgg atggcacccc aggctgaggg 480
   ccccaatgta tgtgtggctg tgggtgtggg tgggagtgtg tctgctgagt aa         532
<210> 60
   <211> 608
   <212> DNA
   <213> Homo sapiens
<400> 60
   tacggccggg atagagtctg gaaaaaataa aaacagaatt tgagaacctt ggaccactcc 60
   tgtccctgta gctcagtcat caaagcagaa gtctggcttt gctctattaa gattggaaat 120
   gtacactacc aaacactcag tccactgttg agccccagtg ctggaaggga ggaaggcctt 180
   tcttctgtgt taattgcgta gaggctacag gggttagcct ggactaaagg catccttgtc 240
   ttttgagcta ttcacctcag tagaaaagga tctaagggaa gatcactgta gtttagttct 300
   gttgacctgt gcacctaccc cttggaaatg tctgctggta tttctaattc cacaggtcat 360
   cagatgcctg cttgataata tataaacaat aaaaacaacc ttcacttctt cctattgtaa 420
   tcgtgtgcca tggatctgat ctgtaccatg accctacata aggctggatg gcaccccagg 480
   ctgagggccc caatgtatgt gtggctgtgg gtgtgggtgg gagtgtgtct gctgagtaag 540
   gaacacgatt ttcaagattc taaagctcaa ttcaagtgac acattaatga taaactcaga 600
   tctgatca 608
<210> 61
   <211> 480
   <212> DNA
   <213> Homo sapiens
<400> 61
   tagatgacac tgatgattct caccagtctt atgagtctca ccattctgat gaatctgatg 60
   aactggtcac tgattttccc acggacctgc cagcaaccga agttttcact ccagttgtcc 120
   ccacagtaga cacatatgat ggccgaggtg atagtgtggt ttatggactg aggtcaaaat 180
   ctaagaagtt tcgcagacct gacatccagt accctgatgc tacagacgag gacatcacct 240
   cacacatgga aagcgaggag ttgaatggtg catacaaggc catccccgtt gcccaggacc 300
   tgaacgcgcc ttctgattgg gacagccgtg ggaaggacag ttatgaaacg agtcagctgg 360
   atgaccagag tgctgaaacc cacagccaca agcagtccag attatataag cggaaagcca 420
   atgatgagag caatgagcat tccgatgtga ttgatagtca ggaactttcc aaagtcagcc 480
<210> 62
   <211> 440
   <212> DNA
   <213> Homo sapiens
<400> 62
   aggagatccg gcagatgggc actgagtgcc attacttcat ctgtgatgtg ggcaaccggg 60
   aggaggtgta ccagacggcc aaggccgtcc gggagaaggt gggtgacatc accatcctgg 120
   tgaacaatgc cgccgtggtc catgggaagg gcctaatgga cagtgatgat gatgccctcc 180
   tcaagtccca acacatcaac accctgggcc agttctggac caccaaggcc ttcctgccgc 240
   gtatgctgga gctgcagaat ggccacatcg tgtgcctcaa ctccgtgctg gcactgtctg 300
   ccatccccgg tgccatcgac taccgcacat ccaaagcgtc agccttcgcc ttcatggaga 360
   gcctgaccct ggggctgctg gactgtccgg gagtcagcgc caccacagtg ctgcccttcc 420
   acaccagcac cgagatgttc 440
<210> 63
   <211> 589
   <212> DNA
   <213> Homo sapiens
<400> 63
   ggcactgagt gccattactt catctgtgat gtgggcaacc gggaggaggt gtaccagacg 60
   gccaaggccg tccgggagaa ggtgggtgac atcaccatcc tggtgaacaa tgccgccgtg 120
   gtccatggga agggcctaat ggacagtgat gatgatgccc tcctcaagtc ccaacacatc 180
   aacaccctgg gccagttctg gaccaccaag gccttcctgc cgcgtatgct ggagctgcag 240
   aatggccaca tcgtgtgcct caactccgtg ctggcactgt ctgccatccc cggtgccatc 300
   gactaccgca catccaaagc gtcagccttc gccttcatgg agagcctgac cctggggctg 360
   ctggactgtc cgggagtcag cgccaccaca gtgctgccct tccacaccag caccgagatg 420
   ttccagggca tgagagtcag gtttcccaac ctctttcccc cactgaagcc ggagacggtg 480
   gcccggagga cagtggaagc tgtgcagctc aaccaggccc tcctcctcct cccatggaca 540
   atgcatgccc tcgttatctt gaaaagcata cttccacagg ctgcactcg 589
<210> 64
   <211> 313
   <212> DNA
   <213> Homo sapiens
<400> 64
   gcatattgtg ctcggggaag ggttcttgtc attgtgggaa gtgcatttgt tctgctgaag 60
   agtggtatat ttctggggag ttctgtgact gtgatgacag agactgcgac aaacatgatg 120
   gtctcatttg tacagggaat ggaatatgta gctgtggaaa ctgtgaatgc tgggatggat 180
   ggaatggaaa tgcatgtgaa atctggcttg gctcagaata tccttaacaa ttacatgaga 240
   gaggtctgga ttcttatttt ttctgggcca ttagaacata taaatgcgaa ggaaaccatg 300
   tatattcacc act 313
<210> 65
   <211> 223
   <212> DNA
   <213> Homo sapiens
<400> 65
   tgtgaatcag cagatggcat attgtgctcg gggaagggtt cttgtcattg tgggaagtgc 60
   atttgttctg ctgaagagtg gtatatttct ggggagttct gtgactgtga tgacagagac 120
   tgcgacaaac atgatggtct catttgtaca gggaatggaa tatgtagctg tggaaactgt 180
   gaatgctggg atggatggaa tggaaatgca tgtgaaatct ggc 223
<210> 66
   <211> 424
   <212> DNA
   <213> Homo sapiens
<400> 66
   ggtacagatt tagagcctgt aatcccagct acttgggagt ctaaggcaag agaatccctt 60
   gaacctggga ggtggagatt gcagtgagct gagatcacac cattgcccta cagcctgggt 120
   gacagtgaga ctgccccaag aaaaaacaaa agagacagcc ctagtgatct tgtaagttgc 180
   ctttggtggg tcagtctttc cttttcttaa agaatagtac acattgacag ccaggtagct 240
   ctatgatcct gttctataga attcaaaaag tcgacaacct tcctttgttc ctttctgttt 300
   tctctgccta cgttagttta aattggcagt gtctctgctg gaataatccc atctctcttc 360
   ctggcttctg ctgagatggc tgattaaatc cttgggtcac acccattatc tctttatcaa 420
   atgg                                                              424
<210> 67
   <211> 487
   <212> DNA
   <213> Homo sapiens
<400> 67
   ctgtaatccc agctacttgg gagtctaagg caagagaatc ccttgaacct gggaggtgga 60
   gattgcagtg agctgagatc acaccattgc cctacagcct gggtgacagt gagactgccc 120
   caagaaaaaa caaaagagac agccctagtg atcttgtaag ttgcctttgg tgggtcagtc 180
   tttccttttc ttaaagaata gtacacattg acagccaggt agctctatga tcctgttcta 240
   tagaattcaa aaagtcgaca accttccttt gttcctttct gttttctctg cctacgttag 300
   tttaaattgg cagtgtctct gctggaataa tcccatctct cttcctggct tctgctgaga 360
   tggctgatta aatccttggg tcacacccat tatctcttta tcaaatggtt gttcaggcta 420
   ggctcagtgt ttcacgcctg taatcccaac actttgggag actgaggagg gcagatcact 480
   tgagctc                                                           487
<210> 68
   <211> 492
   <212> DNA
   <213> Homo sapiens
<400> 68
   agtcgcgcac cgacgctcaa acgcgcgctc caacccgcag cctcctcctg cctcaccgcc 60
   cgaagatggc ggctctcaaa ctcctctcct ccgggcttcg gctctgcgcc tctgcccgcg 120
   gatctggggc aacctggtac aagggatgtg tttgttcctt ttccaccagt gctcatcgcc 180
   ataccaagtt ttatacagat ccagtagaag ctgtaaaaga catccctgat ggtgccacgg 240
   ttttggttgg tggttttggg ctatgtggaa ttccagagaa tcttatagat gctttactga 300
   aaactggagt aaaaggacta actgcagtca gcaacaatgc aggggttgac aattttggtt 360
   tggggctttt gcttcggtcc aagcagataa aacgcatggt ctcttcatat gtgggagaaa 420
   atgcagaatt tgaacgacag tacttatctg gtgaattaga agtggagctg acaccacagg 480
   gcacacttgc tg                                                     492
<210> 69
   <211> 494
   <212> DNA
   <213> Homo sapiens
<400> 69
   tttttttttt tttttttttc tccctttata aggcgatgta cataaatctg aggaatatgg 60
   atgtcttctg gagcaaatgc tccaatatcc acaatttctt caacctctac cactgtggtt 120
   tctgcagctt tgcacattgg caagttgaaa ttccttgcac ttttcctgaa aatcacgttt 180
   cctgctcggt ccgccttcca ggctttcacc aaagcaaaat cccctgtaat tgcttcctcc 240
   aaaataaagt gctgaccatt gaactccctc acctctcttg gcttattggc aatggcaaca 300
   ctgccatctt tgttgtattt gatgggcgat cctccttctt gtaccagggt cccataccct 360
   gttggggtgt aaaatgcagg aactccagcc ccgcctgcac ggatcctctc agcaagtgtg 420
   ccctgtggtg tcagctccac ttctaattca ccagataagt actgtcgttc aaattctgca 480
   ttttctccca cata                                                   494
<210> 70
   <211> 462
   <212> DNA
   <213> Homo sapiens
<400> 70
   catgatgtat tacaaggagg ccttctggaa gaagaaggat tactgtggct gcatgatcat 60
   tgaagatgaa gatgctccaa tttcaataac cttggatgac accaagccag atgggtcact 120
   gcctgccatc atgggcttta ttcttgcccg gaaagctggt cgacttgcta agctacataa 180
   ggaaataagg aagaagaaaa tctgtgagct ctatgccaaa gtgctgggat cccaagaagc 240
   tttacatcca gtgcattatg aagagaagaa ctggtgtgag gagcagtact ctgggggctg 300
   ctacacggcc tacttccctc ctgggatcat gactcaatat ggaagggtga ttcgtcaacc 360
   cgtgggcagg attttctttg cgggcacaga gactgccaca aagtggagcg gctacatgga 420
   aggggcagtt gaggctggag aacgagcagc tagggaggtc tt                     462
<210> 71
   <211> 626
   <212> DNA
   <213> Homo sapiens
<400> 71
<210> 72
   <211> 348
   <212> DNA
   <213> Homo sapiens
<400> 72
   tggtgaactg gtcatccatg aaaaagggtt ttactacatc tattcccaaa catactttcg 60
   atttcaggag gaaataaaag aaaacacaaa gaacgacaaa caaatggtcc aatatattta 120
   caaatacaca agttatcctg accctatatt gttgatgaaa agtgctagaa atagttgttg 180
   gtctaaagat gcagaatatg gactctattc catctatcaa gggggaatat ttgagcttaa 240
   ggaaaatgac agaatttttg tttctgtaac aaatgagcac ttgatagaca tggaccatga 300
   agccagtttt ttcggggcct ttttagttgg ctaactgacc tggaaaga 348
<210> 73
   <211> 207
   <212> DNA
   <213> Homo sapiens
<220>
   <221> misc_feature
   <222> 122, 123
   <223> n = A,T,C or G

<400> 73
   tcaactcagt ggaacacggt tctcccaaac agattttgta attccgaaaa ccacgcatgc 60
   gcaaacatac gcatacactc ccatgttcct ggacagttta tagctaccat aacctggcat 120
   tnnccaaaac ataccatggt agactcttgg atacacaagg taattttaga gccacattag 180
   gatgaacctt ctgaaaaagt tatgcat 207
<210> 74
   <211> 497
   <212> DNA
   <213> Homo sapiens
<400> 74
   gagcttaagg aaaatgacag aatttttgtt tctgtaacaa atgagcactt gatagacatg 60
   gaccatgaag ccagtttttt cggggccttt ttagttggct aactgacctg gaaagaaaaa 120
   gcaataacct caaagtgact attcagtttt caggatgata cactatgaag atgtttcaaa 180
   aaatctgacc aaaacaaaca aacagaaaac agaaaacaaa aaaacctcta tgcaatctga 240
   gtagagcagc cacaaccaaa aaattctaca acacacactg ttctgaaagt gactcactta 300
   tcccaagaaa atgaaattgc tgaaagatct ttcaggactc tacctcatat cagtttgcta 360
   gcagaaatct agaagactgt cagcttccaa acattaatgc aatggttaac atcttctgtc 420
   tttataatct actccttgta aagactgtag aagaaagcgc aacaatccat ctctcaagta 480
   gtgtatcaca gtagtag 497
<210> 75
   <211> 275
   <212> DNA
   <213> Homo sapiens
<220>
   <221> misc_feature
   <222> 96
   <223> n = A,T,C or G
<400> 75
   tgagcttaag gaaaatgaca gaatttttgt ttctgtaaca aatgagcact tgatagacat 60
   ggaccatgaa gccagttttt tcggggcctt tttagntggc taactgaccc tggaaagaaa 120
   aagcaataac ctcaaagtga ctattcagtt ttcaggatga tacactatga agatgtttca 180
   aaaaatctga ccaaaacaaa caaacagaaa acagaaaaca aaaaaacctc tatgcaatct 240
   gagtagagca gccacaacca aaaaattcta caaca                            275
<210> 76
   <211> 530
   <212> DNA
   <213> Homo sapiens
<400> 76
   gacagaaggg gcctctccgc cccgcgtcca gctcgcccag ctcgcccagc gtccgccgcg 60
   cctcggccaa ggcttcaacg gaccacacca aaatgccatc tcaaatggaa cacgccatgg 120
   aaaccatgat gtttacattt cacaaattcg ctggggataa aggctactta acaaaggagg 180
   acctgagagt actcatggaa aaggagttcc ctggattttt ggaaaatcaa aaagaccctc 240
   tggctgtgga caaaataatg aaggacctgg accagtgtag agatggcaaa gtgggcttcc 300
   agagcttctt ttccctaatt gcgggcctca ccattgcatg taatgactat tttgtagtac 360
   acatgaagca gaagggaaag aagtaggcag aaatgagcag ttcgctcctc cttgataaga 420
   gttgtcccaa agggtcgctt aaggaatctg ccccacagct tcccccatag aaggatttca 480
   tgagcagatc aggacactta gcaaatgtaa aaataaaatc taactctcat            530
<210> 77
   <211> 341
   <212> DNA
   <213> Homo sapiens
<400> 77
   gcctctccgc cccgcgtcca gctcgcccag ctcgcccagc gtccgccgcg cctcggccaa 60
   ggcttcaacg gaccacacca aaatgccatc tcaaatggaa cacgccatgg aaaccatgat 120
   gtttacattt cacaaattcg ctggggataa aggctactta acaaaggagg acctgagagt 180
   actcatggaa aaggagttcc ctggattttt ggaaaatcaa aaagaccctc tggctgtgga 240
   caaaataatg aaggacctgg accagtgtag agatggcaaa gtgggcttcc agagcttctt 300
   ttccctaatt gcgggcctca ccattgcatg taatgactat t                     341
<210> 78
   <211> 350
   <212> DNA
   <213> Homo sapiens
<400> 78
   ggcctctccg ccccgcgtgc agctcgccca gctcgcccag cgtccgccgc gcctcggcca 60
   aggcttcaac ggaccacacc aaaatgccat ctcaaatgga acacgccatg gaaaccatga 120
   tgtttacatt tcacaaattc gctggggata aaggctactt aacaaaggag gacctgagag 180
   tactcatgga aaaggagttc cctggatttt tggaaaatca aaaagaccct ctggctgtgg 240
   acaaaataat gaaggacctg gaccagtgta gagatggcaa agtgggcttc cagagcttct 300
   tttccctaat tgcgggcctc accattgcat gcaatgacta ttttgtagta            350
<210> 79
   <211> 171
   <212> DNA
   <213> Homo sapiens
<400> 79
   acagaaggga caaagagatc tggacagaat cgccggacag gtggcagctg ccaacaagaa 60
   gcattagaac aaaccatgct gggttaataa attgcctcat tcgtaaacaa aaaaaaaaaa 120
   aaaaaaaaaa agtttttttt ttttcccccc atttttattt ttttttcccc c         171
<210> 80
   <211> 389
   <212> DNA
   <213> Homo sapiens
<400> 80
   tggcgctgtg ttctatggag gaaaacaaag caggagaggg gagagtgact gctgggtaag 60
   gtcttcctcc acctcctttg catctttgct cacatgccag cttctcctgg gcttcacaga 120
   ccaccaattt ataatttcca tttaaaactt ccattttatt tttttaattt ttatttattt 180
   atttatttat tacgagatgg ggtttcgctc ttgttgccca agattgcacc actgcactgc 240
   agcctgggtg acagagcgag actttgtcaa aaagaaagaa agaaagaagg aaaggaagga 300
   aggaaggaag gaaggaagga aaagaaaaga aagggaaaga aaaaagaaaa agaaagaaag 360
   aaagaaaaaa aaaaaaaagg ggggccccc                                  389
<210> 81
   <211> 430
   <212> DNA
   <213> Homo sapiens
<400> 81
   tgcagataca gtggtggagt ggaagtttgc gttggtagag aatgggggag ttacccgctg 60
   ggaagaatgc agcaatagat tcctagaaac tggccatgag gataaagtgg ttcacgcatg 120
   gtgggggatt cactgattca gtttgcatag taatggagaa gctgtagaac aatgtggaag 180
   aagctgaggt tgtggaacac actgaataaa ataaaggcag tgtgactcca aattcagcca 240
   tctgaattgt ttaaatttgc tagtggattt tgtctactgt gcagaaatat atatgtctaa 300
   tgtgcagaaa tatatatgtg tgtatgtgtg tatatatatg cacacacaca cagataatgc 360
   ttccagtgaa tgtgaacttc ttttccctgt ggcactgatt gacagacttg tgctgatcca 420
   ttattacttt                                                       430
<210> 82
   <211> 556
   <212> DNA
   <213> Homo sapiens
<400> 82
   tttttttttt tttttttttt ttttttttaa gatattaaaa ttcaggtttt attatttgtt 60
   cagttataat aatttaagtt aatatttgct gtattctcag agcaaagatg tatttctgta 120
   ccactgtcct gtataaattt gttacccaag atagtgactg gtatgaaagg agagggaaga 180
   gggtgacaga tggaaacgat tgctgtagga cagtccatct ggccagatgc ggtgggggag 240
   gggagaagaa gtgggagaga gatggtccta cagatgctcc catgggtaaa tgatgggtgc 300
   atccctccct gcagtcgggc tgtgcctgaa cttcacagtc ctctaagagg tgtcattcag 360
   gccacctcac tcagcctatg cccaacccca ctcactttcc ctttccttat gggctgcccc 420
   cgcaactgac ttccatggtg attggttctc attaggccct ttgtttctac accagcctta 480
   gatcattaag acaaagacgt acttgctacc ctcatagcac ataacaacgc ctggcagatg 540
   aaaatcaaac aaaaag 556
<210> 83
   <211> 543
   <212> DNA
   <213> Homo sapiens
<400> 83
   tgcagtggac atgtcgggcg ggacggtcac agtccttgaa aagtccctgt atcaaaaggc 60
   caactgaagc aatacttcta cgagaccaag tgcaatccca tgggttacac aaaagaaggc 120
   tgcaggggca tagacaaaag gcattggaac tcccagtgcc gaactaccca gtcgtacgtg 180
   cgggccctta ccatggatag caaaaagaga attggctggc gattcataag gatagacact 240
   tcttgtgtat gtacattgac cattaaaagg ggaagatagt ggatttatgt tgtatagatt 300
   agattatatt gagacaaaaa ttatctattt gtatatatac ataacagggt aaattattca 360
   gttaagaaaa aaataatttt atgaactgca tgtataaatg aagtttatac agtacagtgg 420
   ttctacaatc tatttattgg acatgtccat gaccagaagg gaaacagtca tttgcgcaca 480
   acttaaaaag tctgcattac attccttgat aatgttgtgg tttgttgccg ttgccaagaa 540
   ctg 543
<210> 84
   <211> 242
   <212> DNA
   <213> Homo sapiens
<400> 84
   cggcggcaga caaaaagact gcagtggaca tgtcgggcgg gacggtcaca gtccttgaaa 60
   aggtccctgt atcaaaaggc caactgaagc aatacttcta cgagaccaag tgcaatccca 120
   tgggttacac aaaagaaggc tgcaggggca tagacaaaag gcattggaac tcccagtgcc 180
   gaactaccca gtcgtacgtg cgggccctta ccatggatag caaaaagaga attggctggc 240
   ga 242
<210> 85
   <211> 350
   <212> DNA
   <213> Homo sapiens
<400> 85
   tttttttttt tttttttttt tctttttttt tttttttttt tttattatta attatcttct 60
   ttattaatac tcacatgtaa cctttgcttt ttacacaaaa gtctgcttta gaagaatgcc 120
   tcctcggctt atcatgccca atggggcttt ttgtttctgg accacttccc ctttctccac 180
   ccccaccccc acatccaaat tactcttaac atgttcacag ataccacgaa tattttgtaa 240
   acaagatttg ggttactgga acttgatttc attaacatcc cacttcaaaa tggaaggcag 300
   gtggaggaca gggtaagaaa taggagaaag aggacaagag aaggcaaaga 350
<210> 86
   <211> 448
   <212> DNA
   <213> Homo sapiens
<400> 86
   acagtttaag aagtggtgac attttgcatg atgaatgacc tgacttttag ccaccaggta 60
   ctctttaaac agttttcctt atcagaggcc ctcctgtgct ggtgacccag catctgagtt 120
   aggttccagc atgtaaagag ctgggagggc ggagaattct tagcatacat tcagacgttt 180
   tttctgcaca ataataagtc catctgtcac ttgcattcca ctttttgtta catagaaaga 240
   gtctgaccct ttaatccaaa aggtcttttt acattgtgaa tgctgtggga aggcaatttc 300
   tctgcacaca agaggctacg ttttggaagt gatgtatgtt atttgatgac tgaaaatgaa 360
   ctgtaaatgc tcctagagta tattcctctg ctgaacaaaa ttaaacttca aaaaaatcta 420
   acagtaacac acccctgctt gggaccct 448
<210> 87
   <211> 586
   <212> DNA
   <213> Homo sapiens
<400> 87
   aatttacaga acagtttaag aagtggtgac attttgcatg atgaatgacc tgacttttag 60
   ccaccaggta ctctttaaac agttttcctt atcagaggcc ctcctgtgct ggtgacccag 120
   catctgagtt aggttccagc atgtaaagag ctgggagggc ggagaattct tagcatacat 180
   tcagacgttt tttctgcaca ataataagtc catctgtcac ttgcattcca ctttttgtta 240
   catagaaaga gtctgaccct ttaatccaaa aggtcttttt acattgtgaa tgctgtggga 300
   aggcaatttc tctgcacaca agaggctacg ttttggaagt gatgtatgtt atttgatgac 360
   tgaaaatgaa ctgtaaatgc tcctagagta tattcctctg ctgaacaaaa ttaaacttca 420
   aaaaaatcta acagtaacac acccctgctt gggaccctag ctatatgcat tttatgtgac 480
   cttgccatgc ttcagtgaac atactaattc tatgtctagc acatgttgat ttcctatgta 540
   ttctgggtat tctattaaag gaaactttga actatgtcaa aaaaaa 586
<210> 88
   <211> 203
   <212> DNA
   <213> Homo sapiens
<400> 88
   aatgaattta cagaacagtt taagaagtgg tgacattttg catgatgaat gacctgactt 60
   ttagccacca ggtactcttt aaacagtttt ccttatcaga ggccctcctg tgctggtgac 120
   ccagcatctg agttaggttc cagcatgtaa agagctggga gggcggagaa ttcttagcat 180
   acattcagac gttttttctg cac 203
<210> 89
   <211> 548
   <212> DNA
   <213> Homo sapiens
<400> 89
   tgctggaagg cattcgcatc tgccggcgag ggcttccgca accggatcgt cttccaggag 60
   ttccgccaac gctacgagat cctggcggcg aatgccatcc ccaaaggctt catggacggg 120
   aagcaggcct gcattctcat gatcaaagcc ctggaacttg accccaactt atacaggata 180
   gggcagagca aaatcttctt ccgaactggc gtcctggccc acctagagga ggagcgagat 240
   ttgaagatca ccgatgtcat catggccttc caggcgatgt gtcgtggcta cttggccaga 300
   aaggcttttg ccaagaggca gcagcagctg accgccatga aggtgattca gaggaactgc 360
   gctgcctacc tcaagctgcg gaactggcag tggtggaggc ttttcaccaa agtgaagcca 420
   ctgctgcagg tgacacggca ggaggaggag atgcaagcca aggaggatga actgcagaag 480
   accaaggagc ggcagcagaa ggcagagaat gagcttaagg agctggaaca gaagcactcg 540
   cagctgac 548
<210> 90
   <211> 595
   <212> DNA
   <213> Homo sapiens
<400> 90
   tgcaatgggg tgctggaagg cattcgcatc tgccggcagg gcttccccaa ccggatcgtc 60
   ttccaggagt tccgccaacg ctacgagatc ctggcggcga atgccatccc caaaggcttc 120
   atggacggga agcaggcctg cattctcatg atcaaagccc tggaacttga ccccaactta 180
   tacaggatag ggcagagcaa aatcttcttc cgaactggcg tcctggccca cctagaggag 240
   gagcgagatt tgaagatcac cgatgtcatc atggccttcc aggcgatgtg tcgtggctac 300
   ttggccagaa aggcttttgc caagaggcag cagcagctga ccgccatgaa ggtgattcag 360
   aggaactgcg ctgcctacct caagctgcgg aactggcagt ggtggaggct tttcaccaaa 420
   gtgaagccac tgctgcaggt gacacggcag gaggaggaga tgcaggccaa ggaggatgaa 480
   ctgcagaaga ccaaggagcg gcagcagaag gcagagaatg agcttaagga gctggaacag 540
   aagcactcgc agctgaccga ggagaagaac ctgctacagg aacagctgca ggcag 595
<210> 91
   <211> 498
   <212> DNA
   <213> Homo sapiens
<400> 91
   tgacagagca agacttggtt tcaaaaaaga gaaacacagt tggccctcca tatctgagtt 60
   tcacagacga aaaatattca gaagaaaaaa aaatcaatgg ctgtatttgt actaaacatg 120
   cccaggcttt ttttcttatt gttatcccct aaacaataca acaactattt ttatagcatt 180
   tacattgtat tagatgttat aactactcta aagaggattt aaagtatatg gaatgatgtg 240
   cataggttat atgcaaatac tatactattt atatcaggga cttgagcatc cttggatttt 300
   ggtatgtgtg ggaggtcctg aaaccaatgt cctgtggata ctgaaggata actgtactaa 360
   tttggagatt tctctctact atgatcaaga ttttcaaaca ttacattgct gattacatta 420
   catcgttaca ttgtgattct ttccaagact tgagataaag tttgggaaga agtaccactt 480
   gtttcagttt atgaaata 498
<210> 92
   <211> 510
   <212> DNA
   <213> Homo sapiens
<400> 92
   aaacacagtt ggccctccat atctgagttt cacagacgaa aaatattcag aagaaaaaaa 60
   aatcaatggc tgtatttgta ctaaacatgc ccaggctttt tttcttattg ttatccccta 120
   aacaatacaa caactatttt tatagcattt acattgtatt agatgttata actactctaa 180
   agaggattta aagtatatgg aatgatgtgc ataggttata tgcaaatact atactattta 240
   tatcagggac ttgagcatcc ttggattttg gtatgtgtgg gaggtcctga aaccaatgtc 300
   ctgtggatac tgaaggataa ctgtactaat ttggagattt ctctctacta tgatcaagat 360
   tttcaaacat tacattgctg attacattac atcgttacat tgtgattctt tccaagactt 420
   gagataaagt ttgggaagaa gttaccactt gtttcagttt atgaaataga aaaaaaaaaa 480
   aggggtaaag catgaaataa aaacctaaac                                  510
<210> 93
   <211> 299
   <212> DNA
   <213> Homo sapiens
<400> 93
   tggatccccc gggctgcagg aattcggcac gagcagaagt gcctgagacg cggagacatg 60
   gctggtgtta aatggagcta ttcaatagca gtgacgcgct ctcctcagcc accaaatgtc 120
   cctgacaccc tccccagccc ccacagataa catcagctga ggtttttttc agtatgaacc 180
   tgtcctaaat caattcctca aagtgtgcac aaaactaaag aatataaata aacaaaagaa 240
   aggtgaaaaa aaaaaaaaaa aaaaaaactc gggggggggc ccgggcccca attccccct  299
<210> 94
   <211> 234
   <212> DNA
   <213> Homo sapiens
<220>
   <221> misc_feature
   <222> 163, 189, 219, 222, 225, 226, 228, 233
   <223> n = A, T, C or G
<400> 94
   cagaagtgcc tgagacgcgg agacatggct ggtgttaaat ggagctattc aatagcagtg 60
   acgcgctctc ctcagccacc aaatgtccct gacaccctcc ccagccccca cagataacat 120
   cagctgaggt ttttttcagt atgaacctgt cctaaatcaa ttnctcaaag tgtgcacaaa 180
   actaaagant ataaataaac aaaagaaagg tgaaaaaana anaannanaa aana       234
<210> 95
   <211> 534
   <212> DNA
   <213> Homo sapiens
<400> 95
   tgaagcagaa gtacctggac tatgccagag tccccaatag caatccccct gaatatgagt 60
   tcttctgggg cctgcgctct tactatgaga ccagcaagat gaaagtcctc aagtttgcct 120
   gcaaggtaca aaagaaggat cccaaggaat gggcagctca gtaccgagag gcgatggaag 180
   cagatttgag ggctgcagct gaggctgcag ctgaagccaa ggctagggcc gagattagag 240
   ctcgaatggg cattgggctc ggctcggaga atgctgccgg gccctgcaac tgggacgaag 300
   ctgatatcgg accctgggcc aaagcccgga tccaggcggg agcagaagct aaagccaaag 360
   cccaagagag tggcagtgcc agcactggtg ccagtaccag taccaataac agtgccagtg 420
   ccagtgccag caccagtggt ggcttcagtg ctggtgccag cctgaccgcc actctcacat 480
   ttgggctctt cgctggcctt ggtggagctg gtgccagcac cagtggcagc tctg 534
<210> 96
   <211> 351
   <212> DNA
   <213> Homo sapiens
<400> 96
   tttttttttt tttttttttt tttctgaaat ggcaaataga tttaatgcag agtgtcaact 60
   tcaattgatt gatagtggct gcctagagtg ctgtgttgag taggtttctg aggatgcacc 120
   ctggcttgaa gagaaagact ggcaggatta acaatatcta aaatctcact tgtaggagaa 180
   accacaggca ccagagctgc cactggtgct ggcaccagct ccaccaaggc cagcgaagag 240
   cccaaatgtg agagtggcgg tcaggctggc accagcactg aagccaccac tggtgctggc 300
   actggcactg gcactgttat tggtactggt actggcacca gtgctggcac t 351
<210> 97
   <211> 610
   <212> DNA
   <213> Homo sapiens
<400> 97
   tttatgaatg ataaagatgt ttccggaaag atgaacaggt cacaatttga agaactctgt 60
   gctgaacttc tgcaaaagat agaagtaccc ctttattcac tgttggaaca aactcatctc 120
   aaagtagaag atgtgagtgc agttgagatt gttggaggca ctacacgaat tccagctgtg 180
   aaggaaagaa ttgccaaatt ctttggaaaa gatattagca caacactcaa tgcagatgaa 240
   gcagtagcca gaggatgtgc attacagtgt gcaatacttt ccccggcatt taaagttaga 300
   gaattttccg tcacagatgc agttcctttt ccaatatctc tgatctggaa ccatgattca 360
   gaagatactg aaggtgttca tgaagtcttt agtcgaaacc atgctgctcc tttctccaaa 420
   gttctcacct ttctgagaag gggccctttt gagctagaag ctttctattc tgatccccaa 480
   ggagttccat atccagaagc aaaaataggc cgctttgtag ttcagaatgt ttctgcacag 540
   aaagatggag aaaaatctag agtaaaagtc aaagtgcgag tcaacaccca tggcattttc 600
   accatctcta 610
<210> 98
   <211> 551
   <212> DNA
   <213> Homo sapiens
<400> 98
   tttttttttt tttttttttt tagcattatc atcttaccct ctgtctcaat atacatgtta 60
   agaaggtctt tccctaactg ccagaccaag ttggcttcaa taggcagctc aacattcacc 120
   acctttattt tgggcttttt agcttctgga ggctggtcaa cttttttttc atttgctttg 180
   tcagcatctg ggattttgtt ttcttctgag gtaagttcag gtgaaggggg agactgtgag 240
   gtttgttgag catcagtttg tacctggggc tgtgttccag cttcactgtt gtcttgctgg 300
   acatttttat cagtgtctgg gttttctggt ggtctctgat tcagacactc catgtcagct 360
   tcagaagaca tttcattctc ctcagttggg actttctcca ccatagatgc cgtagagatg 420
   gtgaaaatgc catgggtgtt gactcgcact ttgactttta ctctagattt ttctccatct 480
   ttctgtgcag aaacattctg aactacaaag cggcctattt ttgcttctgg atatggaact 540
   ccttggggat c                                                      551
<210> 99
   <211> 550
   <212> DNA
   <213> Homo sapiens
<400> 99
   tgtggggctc tatttttgct ttggctttct ggtgagagag tgaggaagca ttctttcctt 60
   cactaagttt gtctttcttg tcttctggat agattgattt taagagacta agggaattta 120
   caaactaaag attttagtca tctggtggaa aaggagactt taagattgtt tagggctggg 180
   cggggtgact cacatctgta atcccagcac tttgggaggc caaggcaggc agaacacttg 240
   aaggagttcg agaccagcgt ggccaacgtg gtgaaaccct gtctctacta aaaatacaaa 300
   aattgtttag ctctgttttt cataatagaa atagaaaagg taaaattgct tttcttctga 360
   aaagaacaag tattgttcat ccaagaaggg tttttgtgac tgaatcagca gtgcctgccc 420
   tagtcatagc tgtgcttcaa aaacctcagc atgattagtg ttggagcaaa acaaggaagc 480
   aaagcaaata ctgtttttga aattctatct gttgcttgaa ctattttgta ataattaaac 540
   tttgatgttg                                                        550
<210> 100
   <211> 300
   <212> DNA
   <213> Homo sapiens
<400> 100
   ctaagcttta agatttaaaa aatgttcaat gttgaaattt ctgtggggct ctatttttgc 60
   tttggctttc tggtgagaga gtgaggaagc attctttcct tcactaagtt tgtctttctt 120
   gtcttctgga tagattgatt ttaagagact aagggaattt acaaactaaa gattttagtc 180
   atctggtgga aaaggagact ttaagattgt ttagggctgg gcggggtgac tcacatctgt 240
   aatcccagca ctttgggagg ccaaggcagg cagaacactt gaaggagttc aagaccagcg 300
<210> 101
   <211> 583
   <212> DNA
   <213> Homo sapiens
<400> 101
   gtttgagtca tgagcatgct gttgtctaga gtgggcgggg atgacgtggt tggagtgggt 60
   gcgctgctct gtacttgatt tttttgagtc tgaaattagc tttccaggct ggggcaggga 120
   ggggagcaca ggtggatcag tactgccccc aagcggtgga gctttggtgg tggatcaaat 180
   actgctgccg cctgtctgca caaacatatt tctctcttcc agcccttcag aagtgtattg 240
   gaatatgtcg ataacaataa tgatggtggt gaagatgatg atgatgtggg taattctggc 300
   taccttattg ggtccaagct ccccacaatt cgttgcacaa agcactctac atacattctc 360
   tttagtcctg atcaaaccac ctttcagagt aggatttagt gtcctatttt aaagatgaag 420
   gagctcgggc tcagagagag atcgtttaga cacacacaca actttggaat gaaacattta 480
   cagccgggcg cggtggcgcg tgcctgtagt cccagctact tgggaggctg aggctggagg 540
   atcgcttgag tccaggagtt ctgggctgta gtgcgctatg ccg 583
<210> 102
   <211> 517
   <212> DNA
   <213> Homo sapiens
<400> 102
   cccggaaggc gacgggaagg agccgagctt gggtcatggc ggcgccgggc gcgctgctgg 60
   tgatgggcgt gagcggctcg gggaaatcca ccgtgggcgc cctgctggca tctgagctgg 120
   gatggaaatt ctatgatgct gatgattatc acccggagga aaatcgaagg aagatgggaa 180
   aaggcatacc gctcaatgac caggaccgga ttccatggct ctgtaacttg catgacattt 240
   tactaagaga tgtagcctcg ggacagcgtg tggttctagc ctgttcagcc ctgaagaaaa 300
   cgtacagaga catattaaca caaggaaaag atggtgtagc tctgaagtgt gaggagtcgg 360
   gaaaggaagc aaagcaggct gagatgcagc tcctggtggt ccatctgagc gggtcgtttg 420
   aggtcatctc tggacgctta ctcaaaagag agggacattt tatgccccct gaattattgc 480
   agtcccagtt tgagactctg gagcccccag cagctcc 517
<210> 103
   <211> 590
   <212> DNA
   <213> Homo sapiens
<400> 103
   tttttttttt tttttttttt ttttttacta gcgaagtttc atttatttgt gcaaatacag 60
   gcatgagcaa gaatgttcta aacaatgtaa cgatttccag cattgattac agaatttcct 120
   ctgatcattt gatttggtta tagatgaatt taaacttcaa tttaagcttg acttttaaaa 180
   ctccccctct gcttcctgat gaaccagcat aattcctaaa attacaccta aacaagtctg 240
   tcttgacaca ttggggtttg cctttagaaa catttagaat ctattatggg caaggcggct 300
   ggaacgaggt ttgggatggc acaatgattt atgcttagtt ctgtttggac cactgataca 360
   aaatcattgt catttcattt ttagggtttc cataattgta gcaattatct ctgaaacatt 420
   tttgtccaca cttatttgga taaagttttc tggagctgct gggggctcca gagtctcaaa 480
   ctgggactgc aataattcag ggggcataaa atgtccctct cttttgagta agcgtccaga 540
   gatgacctca aacgacccgc tcagatggac caccaggagc tgcatctcag 590
<210> 104
   <211> 116
   <212> DNA
   <213> Homo sapiens
<400> 104
   gacacttaca aattgctgct tgtccaaatc aggatccact gcaaggaaca acaggcctta 60
   ttccactgct ggggattggt gtgtgggagc acgcttacta ccttcagtat aaaaat 116
<210> 105
   <211> 574
   <212> DNA
   <213> Homo sapiens
<400> 105
   ttcttttttt tttttttttt tttgcacaaa gcatttacta ttttcaatca cttgcccaat 60
   aacaaaatgt ttagtaagaa attattcaga acattaagtt gtttatgaaa taagtgacta 120
   agcaacatca agaaatgcta caatagagca gcttactgta ttctgcagta ctctatacca 180
   ctacaaaaac agtcataaag agcttaacat actcagcata acgatcgtgg tctacttttt 240
   gcaagccatg tatctttcag ttacattctc ccagttgatt acattccaaa tagcttttag 300
   ataatcaggc ctgacatttt tatactgaag gtagtaagcg tgctcccaca caccaatccc 360
   cagcagtgga ataaggcctg ttgttccttg cagtggatcc tgatttggac aagcagcaat 420
   ttgtaagtgt ccccgttcct tattgaaacc aagccaaccc caacctgagc cttggacacc 480
   aacagatgca gccgtcagct tctccttaaa cttgtcaaag gaaccaaagt cacgtttgat 540
   ggcttccagc aactcccctt tgggttctcc acca 574
<210> 106
   <211> 474
   <212> DNA
   <213> Homo sapiens
<400> 106
   tttttttttt tttttttttt ttgggggggt gacagattct tttattaaca gtcaaaaact 60
   tcacacaatt ggaaaataaa tgtttcttca atgaataatc aaacaaaaat tatccaggac 120
   cttatagggt tttcagtatg taccaggctt gatgcacatc ttagaagaca ggacattatc 180
   ttgctgggat cattagggta tgatcagcat aacgatcgtg gtttactttt tgcaagccat 240
   gtatctttca gttacattct cccagttgat tacattccaa atagctttta gataatcagg 300
   cctgacattt ttatactgaa ggtagtaagc gtgctcccac acatcaatcc ccagcagtgg 360
   aataaggcct gttgttcctt gcagtggatc ctgatttgga caagcagcaa tttgtaagtg 420
   tccccgttcc ttattgaaac caagccaacc ccaacctgag ccttggacac caac 474
<210> 107
   <211> 526
   <212> DNA
   <213> Homo sapiens
<400> 107
   gggaacccgg ggcgcggcgc actgcgcagg cggccggact ccgctcagtt tccggtgcgg 60
   cgaacaccaa agtccgggaa cttaagcatt ttcggtttct agggttgtta cgaagctgca 120
   ggagcgagat ggaggtggac gcaccgggtg ttgatggtcg agatggtctc cgggagcggc 180
   gaggctttag cgagggaggg aggcagaact tcgatgtgag gcctcagtct ggggcaaatg 240
   ggcttcccaa acactcctac tggttggacc tctggctttt catccttttc gatgtggtgg 300
   tgtttctctt tgtgtatttt ttgccatgac ttgttcgctg atatctaaat taagaagttg 360
   gttcttgagt gaattctgaa aatggctaca aacttcttga ataaagaaga caggactctc 420
   aatagaagaa tttcacatct ccaagggacc cttcctttca ttttacactt tgttactaat 480
   ttgcagaact ctattaattg ggtaggattt cacccattcc tagcta 526
<210> 108
   <211> 344
   <212> DNA
   <213> Homo sapiens
<400> 108
   gaacccgggg cgcggcgcac tgcgcatgcg gccggactcc gctcagtttc cggtgcggcg 60
   aacaccaaag tccgggaact taagcatttt cggtttctag ggttgttacg aagctgcagg 120
   agcgagatgg aggtggacgc accgggtgtt gatggtcgag atggtctccg ggagcggcga 180
   ggctttagcg agggagggag gcagaacttc gatgtgaggc ctcagtctgg ggcaaatggg 240
   cttcccaaac actcctactg gttggacctc tggcttttca tccttttcga tgggggggag 300
   cttctctctg tgtatttctc gccatgacct gttcagtgac accc                  344
<210> 109
   <211> 332
   <212> DNA
   <213> Homo sapiens
<400> 109
   gaacccgggg cgcggcgcac tgcgcaggcg gccggactcc gctcagtttc cggtgcggcg 60
   aacaccaaag tccgggaact taagcatttt cggtttctag ggttgttacg aagctgcagg 120
   agcgagatgg aggtggacgc accgggtgtt gatggtcgag atggtctccg ggagcggcga 180
   ggctttagcg agggagggag gcagaacttc gatgtgaggc ctcagtctgg ggcaaatggg 240
   cttcccaaac actcctactg gttggacctc tggcttttca tccttttcga tgtggaggag 300
   attctctttg tgtatttttt gccatgactt gt                              332
<210> 110
   <211> 545
   <212> DNA
   <213> Homo sapiens
<400> 110
   cggctgcgag aagacgacag aaggggagtt tccggtgcgg cgaacaccaa agtccgggaa 60
   cttaagcatt ttcggtttct agggttgtta cgaagctgca ggagcgagat ggaggtggac 120
   gcaccgggtg ttgatggtcg agatggtctc cgggagcggc gaggctttag cgagggaggg 180
   aggcagaact tcgatgtgag gcctcagtct ggggcaaatg ggcttcccaa acactcctac 240
   tggttggacc tctggctttt catccttttc gatgtggtgg tgtttctctt tgtgtatttt 300
   ttgccatgac ttgttcgctg atatctaaat taagaagttg gttcttgagt gaattctgaa 360
   aatggctaca aacttcttga ataaagaaga caggactctc aatagaagaa tttcacatct 420
   ccaagggacc cttcctttca ttttacactt tgttactaat ttgcagaact ctattaattg 480
   ggtaggattt cacccattcc tagctaagtt cttaaaatta aaccctttgg ttcgtgttta 540
   aaaac                                                             545
<210> 111
   <211> 329
   <212> DNA
   <213> Homo sapiens
<400> 111
   gagtttccgg tgcggcgaac accaaagtcc gggaacttaa gcattttcgg tttctagggt 60
   tgttacgaag ctgcaggagc gagatggagg tggacgcacc gggtgttgat ggtcgagatg 120
   gtctccggga gcggcgaggc tttagcgagg gagggaggca gaacttcgat gtgaggcctc 180
   agtctggggc aaatgggctt cccaaacact cctactggtt ggacctctgg cttttcatcc 240
   ttttcgatga ggaggtgttt ctctttgtgt attttttgcc atgacttgtt cgctgatatc 300
   taaatttaca agttggatct tgagtgaaa                                   329
<210> 112
   <211> 284
   <212> DNA
   <213> Homo sapiens
<400> 112
   gcgcggcgcc tcgcctcggc cggcgcctat cagccgactt agaactggtg cggaccaggg 60
   gaatccgact gtttaattaa aacaaagcat cgcgaaggcc cgcggcgggt gttgacgcga 120
   tgtgatttct gcccagtgct ctgaatgcca tattaaaaat aaactttaaa atttaaaagg 180
   gggccgtttt tctctgattc ccaccccgtt aaaaaccctt ttgggggggg ggcccccccc 240
   ccctcatggg gcggggaaaa aaggcctttt ttgggaaatt tggg                  284
<210> 113
   <211> 522
   <212> DNA
   <213> Homo sapiens
<400> 113
   gttgcaggtc actgtagcgg gacttctttt ggttttcttt ctctttgggg cacctctgga 60
   ctcactcccc agcatgaagg cgctgagccc ggtgcgcggc tgctacgagg cggtgtgctg 120
   cctgtcggaa cgcagtctgg ccatcgcccg gggccgaggg aagggcccgg cagctgagga 180
   gccgctgagc ttgctggacg acatgaacca ctgctactcc cgcctgcggg aactggtacc 240
   cggagtcccg agaggcactc agcttagcca ggtggaaatc ctacagcgcg tcatcgacta 300
   cattctcgac ctgcaggtag tcctggccga gccagcccct ggaccccctg atggccccca 360
   ccttcccatc cagacagccg agcccgctcc ggaacttgtc atctccaacg acaaaaggag 420
   cttttgccac tgactccggc cgtgtcctga cacctccaga acgcaggtgc tggcgcccgt 480
   tctgcctggg accccgggaa cctctcctgc cggaagccgg ac                    522
<210> 114
   <211> 510
   <212> DNA
   <213> Homo sapiens
<400> 114
   gttgcaggtc actgtagcgg gacttctttt ggttttcttt ctctttgggg cacctctgga 60
   ctcactcccc agcatgaagg cgctgagccc ggtgcgcggc tgctacgagg cggtgtgctg 120
   cctgtcggaa cgcagtctgg ccatcgcccg gggccgaggg aagggcccgg cagctgagga 180
   gccgctgagc ttgctggacg acatgaacca ctgctactcc cgcctgcggg aactggtacc 240
   cggagtcccg agaggcactc agcttagcca ggtggaaatc ctacagcgcg tcatcgacta 300
   cattctcgac ctgcaggtag tcctggccga gccagcccct ggaccccctg atggccccca 360
   ccttcccatc cagacagccg agcccgctcc ggaacttgtc atctccaacg acaaaaggag 420
   cttttgccac tgactcggcc gtgtcctgac acctccagaa cgcaggtgct ggcgcccgtt 480
   ctgcctggga ccccgggaac ctctcctgcc                                   510
<210> 115
   <211> 385
   <212> DNA
   <213> Homo sapiens
<400> 115
   aatagtctgt gtccaagaaa ataagaatca cgtcatctag ctgtggacac tgagcaaaaa 60
   ggagcagcat gctattaaga tggttgagac acacgagtga acaaagatgg gacaaactgt 120
   gcttcgttca agaagtttca tcaagacccc taccgccccc cgtccttcag ctctgtacag 180
   taactttagc tttacataga gctgagataa aaataaagct ttcttacaaa ttacattttt 240
   ttccagtgaa ttacttttgc agtaaaaata gctgctacat aaatccctcc tgatctctga 300
   aaaggagttg catatttcca aaaataatat tcttatttta atcacacaga agaacgtgga 360
   gcacaggaag gaaatggctg gctgg                                        385
<210> 116
   <211> 645
   <212> DNA
   <213> Homo sapiens

<400> 116
   tacggccggg tcttttaaag aggccgggaa tacacatgac tcaggtgctc ttttgaaacg 60
   actacaaaag tctccatttt gatcaaaacg ttttctccga atgaatggct ccgatgcttt 120
   ctctttccca tcttaagtcc ccgctctgtg cctcagaata gtctgtgtcc aagaaaataa 180
   gaatcacgtc atctagctgt ggacactgag caaaaaggag cagcatgcta ttaagatggt 240
   tgagacacac gagtgaacaa agatgggaca aactgtgctt cgttcaagag gtttcatcaa 300
   gacccctacc gccccccgtc cttcagctct gtacagtaac tttagcttta catagagctg 360
   agataaaaat aaagctttct tacaaattac atttttttcc agtgaattac ttttgcagta 420
   aaaatagctg ctacataaat ccctcctgat ctctgaaaag gagttgcata tttccaaaaa 480
   taatattctt attttaatca cacagaagaa cgtggagcac aggaaggaaa tggctggctg 540
   gtcagggaga ggtgagctgt cggagaaaca cagtaaaact aaaaaataaa atccattttg 600
   tgtataaact gacttaaacg catgcaaaga agtggaaaac atatg                  645
<210> 117
   <211> 500
   <212> DNA
   <213> Homo sapiens
<400> 117
   atgtcgaggg aatgcagaaa gagttaagga aggcaggttg tccttctatt caggccactc 60
   ttcgttttcc atgtactgca tgctgtttgt ggcactttat cttcaagcca ggatgaaggg 120
   agactgggca agactcttac gccccacact gcaatttggt cttgttgccg tatccattta 180
   tgtgggcctt tctcgagttt ctgattataa acaccactgg agcgatgtgt tgactggact 240
   cattcaggga gctctggttg caatattagt tgctgtatat gtatcggatt tcttcaaaga 300
   aagaacttct tttaaagaaa gaaaagagga ggactctcat acaactctgc atgaaacacc 360
   aacaactggg aatcactatc cgagcaatca ccagccttga aaggcagcag ggtgcccagg 420
   tgaggctggc ctgttttcta aaggaagatg attgccacaa ggcaagaaga tgcatctttc 480
   ttcctggtgt acaagccttt                                             500
<210> 118
   <211> 592
   <212> DNA
   <213> Homo sapiens
<400> 118
   taaggaaggc aggttgtcct tctattcagg ccactcttcg ttttccatgt actgcatgct 60
   gtttgtggca ctttatcttc aagccaggat gaagggagac tgggcaagac tcttacgccc 120
   cacactgcaa tttggtcttg ttgccgtatc catttatgtg ggcctttctc gagtttctga 180
   ttataaacac cactggagcg atgtgttgac tggactcatt cagggagctc tggttgcaat 240
   attagttgct gtatatgtat cggatttctt caaagaaaga acttctttta aagaaagaaa 300
   agaggaggac tctcatacaa ctctgcatga aacaccaaca actgggaatc actatccgag 360
   caatcaccag ccttgaaagg cagcagggtg cccaggtgag gctggcctgt tttctaaagg 420
   aagatgattg ccacaaggca agaggatgca tctttcttcc tggtgtacaa gcctttaaag 480
   acttctgctg ctgctatgcc tcttggatgc acactttgtg tgtacatagt tacctttaac 540
   tcagtggtta tctaatagct ctaaactcat taaaaaaact ccaagccttc ca         592
<210> 119
   <211> 197
   <212> DNA
   <213> Homo sapiens
<400> 119
   ggccgccctt tttttttttt tttttttttt tttttttggg ggaaaagggg gtcttttttg 60
   ggtccccccc ccccttttaa aaaacccccc taaaaaatgc ccccaaaaaa aaaaattttt 120
   tttttggggg ggggggaaaa aaagggggaa aaaacccccc ccccccgggg ggggaaaaaa 180
   acccccccaa aaccccc                                                197
<210> 120
   <211> 493
   <212> DNA
   <213> Homo sapiens
<400> 120
   tttttttttt ttaatggtaa aaactttatt tactatttat aaatacattg caagacaaac 60
   ttctcaaaaa tacttttccc cccaaaaagt taaaaaaata aagaaaagct aataggtagg 120
   cagaatgtct tgagacccct ctgttttcaa ggagagctct atgcagcgtg tgtccacacc 180
   gaggtctgca gcagggcaga gtctccctga gcctgacttt gccagacctt cttgggtttg 240
   gcctccggga gagcagccca gtctctgggt cgacgtcctt tcctcagtca tggccacagt 300
   tgtatcatat agcatctcta acatttcatc taggattatc tagtatagat cttactatat 360
   ttggggctat gttgtataca atgttaacaa gaacatatct tctctgcata tatgtgtgaa 420
   ttataaagaa aagcatgaga atgactctaa gttcaacaaa catgggtgaa tctctatgtg 480
   ctcccagtgt cct                                                    493
<210> 121
   <211> 265
   <212> DNA
   <213> Homo sapiens
<400> 121
   tggtacgcct gcagtaccgg tccggaattc ccgggtcgac ccacgcgtcc gcttcctgtt 60
   ttctgttgtc aaatgatgat aatgtgccat gatgttttat atatatcatt cagaaaaagt 120
   tttatttttt aataacattc tattaacatt attttgcttg ccgctggcat gcctgaggaa 180
   tgtatttggc tttgattaca cactaagttt ttgtaataaa tttgactcat taaaaacctt 240
   tttttttaaa aaaaaaaaaa aaaaa                                        265
<210> 122
   <211> 186
   <212> DNA
   <213> Homo sapiens
<400> 122
   tttctgtttt ctgttgtcaa atgatgataa tgtgccatga tgttttatat atatcattca 60
   gaaaaagttt tattttttaa taacattcta ttaacattat tttgcttgcc gctggcatgc 120
   ctgaggaatg tatttggctt tgattacaca ctaagttttt gtaataaatt tgactcatta 180
   aaaacc                                                            186
<210> 123
   <211> 475
   <212> DNA
   <213> Homo sapiens
<400> 123
   cagcccgtcc gcggcctctc cagccccggg ttcgcgctct cgactccccc gacccagtcc 60
   gcggtgcccc ggcgggtgat gccaaataca gccatgaaga aaaaggtgct gctgatgggg 120
   aagagcgggt cggggaagac cagcatgagg tcgataatct tcgccaatta cattgctcgc 180
   gacacccggc gcctgggggc caccattgac gtggaacact cccacgtccg attcctaggg 240
   aacctggtgc tgaacctgtg ggactgtggc ggtcaggaca ccttcatgga aaattacttc 300
   accagccagc gagacaatat cttccgtaac gtggaagttt tgatttacgt gtttgacgtg 360
   gagagccgcg aactggaaaa ggacatgcat tattaccagt cgtgtctgga ggccatcctc 420
   cagaactctc ctgacgccaa aatcttctgc ctggtgcaca aaatggatct ggttc      475
<210> 124
   <211> 122
   <212> DNA
   <213> Homo sapiens
<400> 124
   agaagggttg ctggagccta ggacgtcgag gctgcagtga gatatgatca caccactgca 60
   ctccagcatg actgagtgag accctgtctc aaaaaaaaaa aaaaaaaagt tttttttttt 120
   tc                                                                122
<210> 125
   <211> 147
   <212> DNA
   <213> Homo sapiens
<400> 125
   ggaggggaag gttggtaggt aagctgtaac agattgctcc agttgcctta aactacgcac 60
   atagctaagt gaccaaactt cttgttttga tttgaaaaag tgcattgttt tcttgtccct 120
   ccctttgatg aaacgttacc ctttgac                                     147
<210> 126
   <211> 607
   <212> DNA
   <213> Homo sapiens
<400> 126
   cagtgaagac ttgcatgttg ttttcactac tgtacacttg acctgcacat gcgagaaaaa 60
   ggtggaatgt ttaaaacacc ataatcagct cagggtattt gccaatctga aataaaagtg 120
   ggatgggaga gtgtgtcctt cagatcaagg gtactaaagt ccctttcgct gcagtgagtg 180
   agaggtatgt tgtgtgtgaa tgtacggatg tgtgtttgcg tgcatgtttg tgcatgtgtg 240
   actgtgcatg ttatgtttct ccatgtgggc aaagatttga aatgtaagct tttatttatt 300
   attttagaat gtgacataat gagcagccac actcggggga ggggaaggtt ggtaggtaag 360
   ctgtaacaga ttgctccagt tgccttaaac tacgcacata gctaagtgac caaacttctt 420
   gttttgattt gaaaaaagtg cattgttttc ttgtccctcc ctttgatgaa acgttaccct 480
   ttgacgggcc ttttgatgtg aacagatgtt ttctaggaca aactataagg actaatttta 540
   aacttcaaac attccacttt tgtaatttgt tttaaattgt tttatgtata gtaagcacaa 600
   ctgtaat                                                           607
<210> 127
   <211> 463
   <212> DNA
   <213> Homo sapiens
<400> 127
   attccaatta gccaggaatg gaaggatgag aagcgggatt tgctgactga aggacaaagt 60
   tttagcagcc ttgatgaaga agccctggga tcccgacaca ggccagacct ggtccctagc 120
   actccatcac tgtttgaagc tgcttccttg gcaaccacaa tttcatcttc ttccttatac 180
   gtcaatgagc actatccaca cgacaggcct acactctatt caaacagcaa agggttacct 240
   tccagttcaa catttacctt ggaagagggg accatctact tgaccgctga gcccaacact 300
   ctggaagtgc aggatgacaa tgcttctgtg cttgacgtct atttataagt gaaaatggtg 360
   atcacctaag cacatggatg agacgtgagc acagttatgg cagagaagtt tctccgcacc 420
   agaattatcc acagcaactt ggctgagccc cactacacac aga                   463
<210> 128
   <211> 592
   <212> DNA
   <213> Homo sapiens
<400> 128
   ccaattagcc aggaatggaa ggatgagaag cgggatttgc tgactgaagg acaaagtttt 60
   agcagccttg atgaagaagc cctgggatcc cgacacaggc cagacctggt ccctagcact 120
   ccatcactgt ttgaagctgc ttccttggca accacaattt catcttcttc cttatacgtc 180
   aatgagcact atccacacga caggcctaca ctctattcaa acagcaaagg gttaccttcc 240
   agttcaacat ttaccttgga agaggggacc atctacttga ccgctgagcc caacactctg 300
   gaagtgcagg atgacaatgc ttctgtgctt gacgtctatt tataagtgaa aatggtgatc 360
   acctaagcac atggatgaga cgtgagcaca gttatggcag agaagtttct ccgcaccaga 420
   attatccaca gcaacttggc tgagccccac tacacacaga gaaatcatca acctgactta 480
   agagttttca agatgtcaac ttcaggctga tcagcagatg ggatgtgaaa aatactaccc 540
   tattctatca tttgctgttg cttgctgaac tgtgaagaac tgcatgaact at         592
<210> 129
   <211> 251
   <212> DNA
   <213> Homo sapiens
<400> 129
   caattagcca ggaatggaag gatgagaagc gggatttgct gactgaagga caaagtttta 60
   gcagccttga tgaagaagcc ctgggatccc gacacaggcc agacctggtc cctagcactc 120
   catcactgtt tgaagctgct tccttggcaa ccacaatttc atcttcttcc ttatacgtca 180
   atgagcacta tccacacgac aggcctacac tctattcaaa cagcaaaggg ttaccttcca 240
   gttcaacatt t                                                      251
<210> 130
   <211> 229
   <212> DNA
   <213> Homo sapiens
<400> 130
   gtagcagaag cctcattcca gaacccatct ggccagagaa gcagcagcat cctgggggat 60
   ggccgtgcat ggggtgtaca ctcgctatag gcataggccc ggcatggctg tcgctggacg 120
   ccagctgtgc acacccagcc acacctgctg cacgccgcgt tagtgtgcgg ctccgggcct 180
   gagcattcgc aaagctcgct tctccaggga gcctcctctt ggctttgga              229
<210> 131
   <211> 316
   <212> DNA
   <213> Homo sapiens
<400> 131
   cgccataacc tggtcagaag tgtgcctgtc ggcggggaga gaggcaatat caaggtttta 60
   aatctcggag aaatggcttt cgtttgcttg gctatcggat gcttatatac ctttctgata 120
   agcacaacat ttggctgtac ttcatcttca gacaccgaga taaaagttaa ccctcctcag 180
   gattttgaga tagtggatcc cggatactta ggttatctct atttgcaatg gcaaccccca 240
   ctgtctctgg atcattttaa ggaatgcaca gtggaatatg aactaaaata ccgaaacatt 300
   ggtagtgaaa catgga                                                  316
<210> 132
   <211> 270
   <212> DNA
   <213> Homo sapiens
<220>
   <221> misc_feature
   <222> 37
   <223> n = A,T,C or G
<400> 132
   agtcgccata acctggtcag aagtgtgcct gtcggcnggg agagaggcaa tatcaaggtt 60
   ttaaatctcg gagaaatggc tttcgtttgc ttggctatcg gatgcttata tacctttctg 120
   ataagcacaa catttggctg tacttcatct tcagacaccg agataaaagt taaccctcct 180
   caggattttg agatagtgga tcccggatac ttaggttatc tctatttgca atggcaaccc 240
   ccactgtctc tggatcattt taaggaatgc                                   270
<210> 133
   <211> 341
   <212> DNA
   <213> Homo sapiens
<400> 133
   ttacatacgt ttttattact cgggggggac ctgtacgtca ccaatgccca gcttcacggg 60
   ggcatgtagt gtgactcacg gctgaacaca aaatcactgt gaagcctgtg ctacagaagg 120
   atgtccagtc gctgaggcca ggagagaggt gggcaggcct gggtctggca gtggagacgg 180
   tcctccaggg agccgttggg caggaagccg tacaccaggc agtagaagcc gttctgagca 240
   cagtagccag caaagtccac aatgtttggg tgacgaaacc tggacagctg ctccacctcg 300
   gtcaggaagc tctgcttcac tgcagtccac tccaggtcag c                      341
<210> 134
   <211> 466
   <212> DNA
   <213> Homo sapiens
<400> 134
   attatgtgat taatgatttg acagccgttc caatctccac gtctccagaa gagattccac 60
   atgggagttt ctcagactga ttcttgacct ctcaatgaaa gtgttgaaac aggatgggaa 120
   atattttaca caggggaact gtgtcaatct gacagaagca ctgtcgctct atgaagaaca 180
   gctggggcgc ctgtattgtc ctgtggaatt ttcaaaggag atcgtctgtg tcccttcata 240
   cttggaattg tgggtatttt acactgtttg gaagaaagct aaaccctgaa gatcagtagc 300
   ccctaatcac atgtgctgca aatagccttc ctgacctcca tatgctgtac atgacatcaa 360
   aatgagtcag gcaattgatt gtgaattcct taaagttttc ctttttttaa taattatttt 420
   taatttaaaa aagcaaatgg aaaatgtata ttttgatgag cttagg                466
<210> 135
   <211> 70
   <212> DNA
   <213> Homo sapiens
<400> 135
   agttttcctt tttttaataa ttatttttaa tttaaaaaag caaatggaaa atgtatattt 60
   tgatgagctt                                                        70
<210> 136
   <211> 442
   <212> DNA
   <213> Homo sapiens
<400> 136
   tttttttttt tttttttcgg ctcagtataa agcttccttt tcttagggac catgcaaaga 60
   ttctttgatt ctagaagtgc catttcatta tttctgtgac tcctgtctga atcatctgcc 120
   aggtaactat cttgattttg tcttagcaat cgacttagca gaccattctt ggagaaagaa 180
   aaatcctgag gtgaaacagg ctccgattta aagtcttcgg acactggtaa ggcaggtgcg 240
   cttctctgca cagcaggagc catacccaag aatggggcac tcttagcatc atggctcaag 300
   tgcacatttg tgttaggaat ttgtaagtca tcacaaggct cagattttat tttcaccatc 360
   agtatttgtt cacttaaagc tctctctgag tgttcctgag tactttcatc tcttaaggga 420
   gtttctcttt ttttttcact ct                                           442
<210> 137
   <211> 275
   <212> DNA
   <213> Homo sapiens
<220>
   <221> misc_feature
   <222> 244
   <223> n = A,T,C or G
<400> 137
   agaaaaatac aaaaaatctg cattaaaaat attaatcctg catgctggac atgtatggta 60
   ataatttcta ttttgtacca ttttctgttt aactttagca tgttgttgat catggatcat 120
   actcccctgt ttcttgggtg agaagggatc gccagtttgg aaactccggc ggctgcgtgc 180
   ggggtttcag tcccactgta ggcttgtaaa taccgccccg ccaaaccgca tagagacgtg 240
   gcancactga gggctttgtt gggttatata cgtat                            275
<210> 138
   <211> 353
   <212> DNA
   <213> Homo sapiens
<400> 138
   taagctcgga attcgggtcg aggaaaaata caaaaaatct gcattaaaaa tattaatcct 60
   gcatgctgga catgtatggt aataatttct attttgtacc attttcttgt ttaactttag 120
   catgttgttg atcatggatc atactcccct tgtttctttg ggtgagaagg gatcgcagtt 180
   tggaaactcc ggcggctgcg tgcggggttt cagtcccagc tgtaggcttg taaatacccg 240
   ccccgccaaa ccgcatagag aacgtggcag caagctgagg gtctttgttt gggtttatta 300
   ttacggtatt tttgtttgta agttaaaaaa aaaaaaaaaa gggggggccc cca       353
<210> 139
   <211> 559
   <212> DNA
   <213> Homo sapiens
<400> 139
   gaatttggcc ctcgaggcca agaattcggc actagggcgc agaaggacca gcagaaagat 60
   gccgaggcgg aagggctgag cggcacgacc ctgctgccga agctgattcc ctccggtgca 120
   ggccgggagt ggctggagcg gcgccgcgcg accatccggc cctggagcac cttcgtggac 180
   cagcagcgct tctcacggcc ccgcaacctg ggagagctgt gccagcgcct cgtacgcaac 240
   gtggagtact accagagcaa ctatgtgttc gtgttcctgg gcctcatcct gtactgtgtg 300
   gtgacgtccc ctatgttgct ggtggctctg gctgtctttt tcggcgcctg ttacattctc 360
   tatctgcgca ccttggagtc caagcttgtg ctctttggcc gagaggtgag cccagcgcat 420
   cagtatgctc tggctggagg catctccttc cccttcttct ggctggctgg tgcgggctcg 480
   gccgtcttct gggtgctggg agccaccctg gtggtcatcg gctcccacgc tgccttccac 540
   cagattgagg ctgtggacg                                              559
<210> 140
   <211> 711
   <212> DNA
   <213> Homo sapiens
<220>
   <221> misc_feature
   <222> 444
   <223> n = A,T,C or G
<400> 140
   tttttttttt tttttttttg acacccataa cagctttatt ttcaaaggcg ggatccctcc 60
   ccgggcttgt gatgggacgg cgctgtgggc ccgagcagca aagccgtgca ggacaggcat 120
   gggcaggggt ggggcagctg gcccgggagg ccggcaggtc ccaaaagaca cctcacacgg 180
   gttccatctg cagctcctcc ccgtccacag cctcaatctg gtggaaggca gcgtgggagc 240
   cgatgaccac cagggtggct cccagcaccc agaagacggc cgagcccgca ccagccagcc 300
   agaagaaggg gaaggagatg cctccagcca gagcatactg atgcgctggg ctcacctctc 360
   ggccaaagag cacaagcttg gactccaagg tgcgcagata gagaatgtaa caggcgccga 420
   aaaagaccag ccagagccac cagncacata ggggacgtca ccacacagta caggatgagg 480
   cccaggaaca cgaacacata gttgctctgg tagtactcca cgttgcgtac gaggcgctgg 540
   cacagctctc ccaggttgcg gggccgtgag aagcgctgct ggtccacgaa ggtgctccag 600
   gggccggatg gtcgcgcggc gccgctccag ccactcccgg cctgcacccg gaggaatcag 660
   cttcggcagc aaggtcgtgc cggtcagccc ttccgcctcg gcattctttc t          711
<210> 141
   <211> 468
   <212> DNA
   <213> Homo sapiens
<400> 141
   actcgcagtc cttcttctct ggcctctttg gaggctcatc caaaatagag gaagcatgcg 60
   aaatctacgc cagagcagca aacatgttca aaatggccaa aaactggagt gctgctggaa 120
   acgcgttctg ccaggctgca cagctgcacc tgcagctcca gagcaagcac gacgcagcca 180
   cctgctttgt ggacgctggc aacgcattca agaaagccga cccccaagag gccattaact 240
   gtttgatgcg agcaatcgag atctacacag acatgggccg attcacgatt gcggccaagc 300
   accacatctc cattgctgag atctatgaga cagagttggt ggacatcgag aaggccattg 360
   cccactacga gcagtctgca gactactaca aaggcgagga gtccaacagc tcagccaaca 420
   agtgtctgct gaaggtggct ggttacgctg cgctgctgga gcagtatc                468
<210> 142
   <211> 203
   <212> DNA
   <213> Homo sapiens
<400> 142
   cgcaaagtga agaactcgca gtccttcttc tctggcctct ttggaggctc atccaaaata 60
   gaggaagcat gcgaaatcta cgccagagca gcaaacatgt tcaaaatggc caaaaactgg 120
   agtgctgctg gaaacgcgtt ctgccaggct gcacagctgc acctgcagct ccagagcaag 180
   cacgacgcag ccacctgctt tgt                                         203
<210> 143
   <211> 212
   <212> DNA
   <213> Homo sapiens
<400> 143
   tctgcgggga acagaacatg atcggcatga cgcccacggt catcgctgag cattacctgg 60
   ctgaaacgga gcagcgggag aagttcgggc taaagaagcg ggagggggcc tgggagctca 120
   tgaagaaggg gtacacccag caactggcct tcatacaacc cagctctgcc tttgcggcct 180
   tcgtgaaacg ggcacccagc acctggctga cc                               212
<210> 144
   <211> 226
   <212> DNA
   <213> Homo sapiens
<220>
   <221> misc_feature
   <222> 109, 128, 153, 161, 167, 174, 175, 178, 196, 202, 206, 211, 213
   <223> n = A, T, C or G
<400> 144
   gaagcacctc attgtgaccc cctcgggctg cggggaacag aacatgatcg gcatgacgcc 60
   cacggtcatc gctgtgcatt acctggatga aacggagcag tgggagaant tcggcctaga 120
   gaagcggnag ggggccttgg agctcatcaa ganggggtac ncccagnagc tggnnttnag 180
   acaacccagc tctgcntttg cnggcnttcg nanaaagggc ccccac 226
<210> 145
   <211> 97
   <212> DNA
   <213> Homo sapiens
<400> 145
   ctgggctgcg gctgatgcgc atccgttttc ctgccctggg catgtgtctc tgaaaccgta 60
   tggcgggcgg tgggcaacgg gcactgctaa gggaggc 97
<210> 146
   <211> 120
   <212> DNA
   <213> Homo sapiens
<400> 146
   ggcacgagct catctgtttg cggatcagaa cccgagctgt gcttgtggct gcggctgcta 60
   actggctgcg cacagggagc tgtcaccatg cctcactcgt acccagccct ttctgctgag 120
<210> 147
   <211> 273
   <212> DNA
   <213> Homo sapiens
<400> 147
   ggccgccctt tttttttttt ttttttttcc cccctttttt ttggtggggg ggtttttcca 60
   aggggttgaa tgggggtttt ttttcccccc ttttacccca gaaaaagggg gaggaaaaaa 120
   ggaacccccg gggaaaattt tccttttttt ggaaaatttg ggggacccga aaaagggggg 180
   gggaaccccc cccctttttt ttttctttta aaaaattttt ttgcccccaa aaaaaggggg 240
   gccccctttc ccccccttct tgggccccgg ggg 273
<210> 148
   <211> 90
   <212> DNA
   <213> Homo sapiens
<400> 148
   cacttcatgc aaggcacatg tgctgtcctg caggtctgca gggaaccgac ccagagagcc 60
   cagcggcagg ccctggaaca cccgcctctg                                  90
<210> 149
   <211> 463
   <212> DNA
   <213> Homo sapiens
<400> 149
   gacttgtccg ggaatccggt gcttcggatc tactacacct cgaggcctgc tctgttcacc 60
   ttgtgtgctg ggaatgagct cttctactgc ctcctctacc tgttccattt ctctgaggga 120
   cctttagttg gctctgtggg actgttccgg atgggcctct gggtcactgc ccccatcgcc 180
   ttgctgaagt cgctcatcag cgtcatccac ctgatcacgg ccgcccgcaa catggctgcc 240
   ctggacgcag cagaccgcgc caagaagaag tgacgctgga gccccgggtc ctggctgccc 300
   acctgccctg ggagtcttgc tgtgccacac agctccccac cccctgctag gaggtcccag 360
   tctcacgcct tcctcatgtg ttgttctacc tgctgggatg ggggtcagcc tctctttggt 420
   gacgtcacgt tctctgggat cctgaggacc cgggcctcaa atc                  463
<210> 150
   <211> 693
   <212> DNA
   <213> Homo sapiens
<220>
   <221> misc_feature
   <222> 285, 455, 597, 606, 636, 667, 686
   <223> n = A,T,C or G
<400> 150
   ggcacgagga gagagagagt cacaagatga tcgacttgtt cgggaatccg gtgcttcgga 60
   tctactacac ctcgaggcct gctctgttca ccttgtgtgc tgggaatgag ctcttctact 120
   gcctcctcta cctgttccat ttctctgagg gacctttagt tggctctgtg ggactgttcc 180
   ggatgggcct ctgggtcact gcccccatcg ccttgctgaa gtcgctcatc agcgtcatcc 240
   acctgatcac ggccgcccgc aacatggctg ccctggacgc agcanaccgc gccaagaaga 300
   agtgacgctg gagccccggg tcctggctgc cacctgccct gggagtcttg ctgtgccaca 360
   cagctcccca ccccctgcta ggaggtccca gtctcacgcc ttcctcatgt gttgttctac 420
   ctgctgggat gggggtcagc ctctctttgg tgacntcacg ttcttctggg atcctgagga 480
   ccgggcctca aatcagggag gatacccggg agggcccctt catccaagcg gtgcttctgg 540
   ggtgccggga ccgggcagtg tcacaccctg cctgctagtc ctggggtcca gatctangga 600
   ccttantgaa ggagtggtgt gaggcagttc tgaagnggat aactcgccca caacaagttg 660
   ggacatncag aggaaactca actctnacgt ctt                              693
<210> 151
   <211> 300
   <212> DNA
   <213> Homo sapiens
<400> 151
   gagagagaga gtcacaagat gatcgacttg tccgggaatc cggtgcttcg gatctactac 60
   acctcgaggc ctgctctgtt caccttgtgt gctgggaatg agctcttcta ctgcctcctc 120
   tacctgttcc atttctctga gggaccttta gttggctctg tgggactgtt ccggatgggc 180
   ctctgggtca ctgcccccat cgccttgctg aagtcgctca tcagcgtcat ccacctgatc 240
   acggccgccc gcaacatggc tgccctggac gcagcagacc gcgccaagaa gaagtgacgc 300
<210> 152
   <211> 300
   <212> DNA
   <213> Homo sapiens
<220>
   <221> misc_feature
   <222> 37, 41
   <223> n = A,T,C or G
<400> 152
   gacttgtccg ggaatccggt gcttcggatc tactacncct ngaggcctgc tctgttcacc 60
   ttgtgtgctg ggaatgagct cttctactgc ctcctctacc tgttccattt ctctgaggga 120
   cctttagttg gctctgtggg actgttccgg atgggcctct gggtcactgc ccccatcgcc 180
   ttgctgaagt cgctcatcag cgtcatccac ctgatcacgg ccgcccgcaa catggctgcc 240
   ctggacgcag cagaccgcgc caagaagaag tgacgctgga gccccgggtc ctggctgccc 300
<210> 153
   <211> 239
   <212> DNA
   <213> Homo sapiens
<220>
   <221> misc_feature
   <222> 168, 190, 203, 229
   <223> n = A,T,C or G
<400> 153
   gttgccctgc ctctggctcc agaacagaaa gggagcctca cgctggctca cacaaaacag 60
   ctgacactga ctaaggaact gcagcatttg cacaggggag gggggtgcct ccttcctaga 120
   ggccctgggg gccaggctga ttggggggca gattgacata ggccccantc atcagatgtc 180
   tgaaattcan cacgggggta acntgggggg ttagggacta tttttaaant aggggtggc 239
<210> 154
   <211> 113
   <212> DNA
   <213> Homo sapiens
<400> 154
   gacacatttg ttacttcgtg agcaagcccg gaggctcgga gccccctgcc gtgttcacag 60
   gtgacacctt gtttgtggct ggctgcggga agttctatga agggactgcg gat 113
<210> 155
   <211> 294
   <212> DNA
   <213> Homo sapiens
<400> 155
   tttttttttt tttttttttt ttttggcggg aataaatact tgttaaactt ctcttataaa 60
   tatgcattaa aacgtccgat aacacaagcc aagggctgta aaattaaggt taaatcaaga 120
   ctgaatttcc cgcacggacc agcaggaaag ccagttacct aaaagagcct aatccccaaa 180
   tccgctgaag gtgcagggcg gcctcagtcc cggggcatct tgaactggtc cttctccctg 240
   cgcacggccc gcatggtggt caccgggtcc gtctcacctg cgtgctgctg cacc       294
<210> 156
   <211> 419
   <212> DNA
   <213> Homo sapiens
<400> 156
   tagccatggc aggacagctc ctggaccagg tctcataatg catgtggcac ttaggtccaa 60
   gctctccaga gggtgaaagc tggagtctgt caatgtccta ctgagacagc acagccaacc 120
   tagctagcaa catttgtttt agtctgaaca atatatactt atagaattca gtcaaagata 180
   cacaatctga aacagcttca tggggtggac tctaacagta gttgcaatgt tttagaatga 240
   gacttacttc tctgctatct agatctgaac tccttggctt ctttacttag ttcaagcccc 300
   agcctaggaa agccagttac ataaaagttg gctcaggagt cttagagctt tacctaaata 360
   gagcccagaa aacggaggat gggggtgggg cgccttcctg gaggtgacac ttgatgggg  419
<210> 157
   <211> 357
   <212> DNA
   <213> Homo sapiens
<400> 157
   cgtattgctg tcaagccgtg agctagccat ggcaggacag ctcctggacc aggtctcata 60
   atgcatgtgg cacttaggtc caagctctcc agagggtgaa agctggagtc tgtcaatgtc 120
   ctactgagac agcacagcca acctagctag caacatttgt tttagtctga acaatatata 180
   cttatagaat tcagtcaaag atacacaatc tgaaacagct tcatggggtg gactctaaca 240
   gtagttgcaa tgttttagaa tgagacttac ttctctgcta tctagatctg aactccttgg 300
   cttctttact tagttcaagc cccagcctag gaaagccagt tacataaaag ttggctc    357
<210> 158
   <211> 408
   <212> DNA
   <213> Homo sapiens
<400> 158
   actttgtatc actgcagcgc ttcacacctt catcctgaag atatctggaa cattcgtagt 60
   atctgcagca ccaccaatat ccaatgcaag aacggcaaga tgaactgcca tgagggtgta 120
   gtgaaggtca cagattgcag ggacacagga agttccaggg cacccaactg cagatatcgg 180
   gccatagcga gcactagacg tgttgtcatt gcctgtgagg gtaacccaca ggtgcctgtg 240
   cactttgacg gttagatgcc accatgtagg gattatcgcg agtggttgac cttacactta 300
   ctccttaaat agcagtgagt aatgcatttg agctgcccca ggctctgtct cctcagctca 360
   tttcttactc tttttctcta tataactcat tctattaaat acattgca 408
<210> 159
   <211> 550
   <212> DNA
   <213> Homo sapiens
<400> 159
   acaaggacgc caaccccacc tagatgcaaa gcaggattca aaagaacatc tttgcgtttt 60
   ctaccggctc cccatcatcg tactagggag gaagaagcgg gtgagaaaca aaacttcttt 120
   ccattgtcct gccctttctt gcggacttgt tctgaggccg aggcacctct aagatactga 180
   tggctctgca gaggacccat tcattgcttc tgcttttgct gctgaccctg ctggggctgg 240
   ggctggtcca gccctcctat ggccaggatg gcatgtacca gcgattcctg cggcaacacg 300
   tgcaccctga ggagacaggt ggcagtgatc gctactgcaa cttgatgatg caaagacgga 360
   agatgacttt gtatcactgc aagcgcttca acaccttcat ccatgaagat atctggaaca 420
   ttcgtagtat ctgcagcacc accaatatcc aatgcaagaa cggcaagatg aactgccatg 480
   agggtgtagt gaaggtcaca gattgcaggg acacaggaag ttccagggca cccaactgca 540
   gatatcgggc 550
<210> 160
   <211> 554
   <212> DNA
   <213> Homo sapiens

<400> 160
   ccaaccccac ctagatgcaa agcaggattc aaaagaacat ctttgcgttt tctaccggct 60
   ccccatcatc gtactaggga ggaagaagcg ggtgagaaac aaaacttctt tccattgtcc 120
   tgccctttct tgcggacttg ttctgaggcc gaggcacctc taagatactg atggctctgc 180
   agaggaccca ttcattgctt ctgcttttgc tgctgaccct gctggggctg gggctggtcc 240
   agccctccta tggccaggat ggcatgtacc agcgattcct gcggcaacac gtgcaccctg 300
   aggagacagg tggcagtgat cgctactgca acttgatgat gcaaagacgg aagatgactt 360
   tgtatcactg caagcgcttc aacaccttca tccatgaaga tatctggaac attcgtagta 420
   tctgcagcac caccaatatc caatgcaaga acggcaagat gaactgccat gagggtgtag 480
   tgaaggtcac agattgcagg gacacaggaa gttccagggc acccaactgc agatatcggg 540
   ccatagcgag cact 554
<210> 161
   <211> 313
   <212> DNA
   <213> Homo sapiens
<400> 161
   aattacatct tctttaaagc caaatgggag atgccctttg acccccaaga tactcatcag 60
   tcaaggggcg tacttgagca ggaaaaagtg ggtaatggtg cccatgatga gtttgcatca 120
   cctgactata ccttacttcc gggacgagga gctgtcctgc accgtggtgg agctgaagta 180
   cacaggcaat gccagcgcac tcttcatcct ccctgatcaa gacaagatgg aggaagtgga 240
   agccatgctg ctcccagaga ccctgaagcg gtggagagac tctctggagt tcagagagat 300
   aggtgagctc tac                                                   313
<210> 162
   <211> 519
   <212> DNA
   <213> Homo sapiens
<400> 162
   cggccgccct tttttttttt tttggccccc cggggccccc ttattttaaa aacccccccc 60
   ccccctgggg ggggggcccc gaccttttaa gttttttttt tttcccccgg gggaaaaaaa 120
   ggggggaaaa aaaaaaaaaa ttcccccccc tttttccccc ccccaaaaaa gggggggacc 180
   ccccgggggg gggggggttt ccccgggggg gaaaaaaaaa acccccgggg gccccccccc 240
   aattttttcc ccccccccct tggggggggg gggggggggg gggggggggg gggggccccc 300
   cccccccccc ccccccccat tttggggggt tgggttgggg gaaatttttt tttaaaaaaa 360
   aaaaaaaaaa atttgggggt cccccccccc ctttttttcc cccccctttt ttccaaaagg 420
   ggaccccccc cccccccccc caaaaaaacc cccccccccc ccccaaaaaa aacccccccc 480
   cgggggggga aaaaaaaggg gggggggggg ggccccccc                        519
<210> 163
   <211> 422
   <212> DNA
   <213> Homo sapiens
<400> 163
   aactaaaaac tacagtggaa gaaaggaagt cttcagaagc ctcccccact gcgcaaagaa 60
   gtaaagatca cagtaaggaa tgcataaacg ctgccccaga ttctccgtcc aaacagcttc 120
   cagaccagat ttcattcttc agtggaaatc catcagttga aatagttcat ggtattatgc 180
   acctatataa gacaaataag atgacctcct taaaagaaga tgtgcggcgc agtgccatgc 240
   tgtgtattct cacagtccct gctgcaatga ccagtcatga ccttatgaag tttgttgccc 300
   catttaacga agtaattgaa caaatgaaaa ttatcagaga ctctactccc aaccaatata 360
   tggtgctgat aaagtttcgt gcacaggctg atgcggatag tttttatatg acatgcaatg 420
   gc                                                               422
<210> 164
   <211> 626
   <212> DNA
   <213> Homo sapiens
<400> 164
   tacggccggg tgcgagctct gcgggaagcg gttcctggat agtttgcggc tgagaatgca 60
   cttactggct cattcagcgg gtgccaaagc ctttgtctgt gatcagtgcg gtgcacagtt 120
   ttcgaaggag gatgccctgg agacacacag gcagacccat actggcactg acatggccgt 180
   cttctgtctg ctgtgtggga agcgcatcca ggcgcagagc gcactgcagc agcacatgga 240
   ggtccacgcg ggcgtgcgca gctacatctg cagtgagtgc aaccgcacct tccccagcca 300
   cacggctctc aaacgccacc tgcgctcaca tacaggcgac cacccctacg agtgtgagtt 360
   ctgtggcagc tgcttccggg atgagagcac actcaagagc cacaaacgca tccacacggg 420
   tgagaaaccc tacgagtgca atggctgtgg caagaagttc agcctcaagc atcagctgga 480
   gacgcactat agggtgcaca caggtgagaa gccctttgag tgtaggctct gccaccagcg 540
   ctcccgggac tactcggcca tgatcaagca cctgagaacg cacaacggcg cctcgcccta 600
   ccagtgcacc atctgcacag agtact                                     626
<210> 165
   <211> 515
   <212> DNA
   <213> Homo sapiens
<400> 165
   gatagtttgc ggctgagaat gcacttactg gctcattcag cgggtgccaa agcctttgtc 60
   tgtgatcagt gcggtgcaca gttttcgaag gaggatgccc tggagacaca caggcagacc 120
   catactggca ctgacatggc cgtcttctgt ctgctgtgtg ggaagcgcat ccaggcgcag 180
   agcgcactgc agcagcacat ggaggtccac gcgggcgtgc gcagctacat ctgcagtgag 240
   tgcaaccgca ccttccccag ccacacggct ctcaaacgcc acctgcgctc acatacaggc 300
   gaccacccct acgagtgtga gttctgtggc agctgcttcc gggatgagag cacactcaag 360
   agccacaaac gcatccacac gggtgagaaa ccctacgagt gcaatggctg tggcaagaag 420
   ttcagcctca agcatcagct ggagacgcac tatagggtgc acacaggtga gaagcccttt 480
   gagtgtaggc tctgccacca gcgctcccgg gacta                           515
<210> 166
   <211> 615
   <212> DNA
   <213> Homo sapiens
<400> 166
   actgttcaag gtttattggg ggttttagtt ggtataacac ttggatagtt ggttgcattg 60
   tttgtatgta gatcttttta cattatatgg taatgtacac tactgatata gttcacaaaa 120
   taagatcctt tggaagaatt atgcacaaga catgatattg gatttataca ctggatccca 180
   ggatgtgact cactgggaaa aaatgttgga ctaggcatgt tcagtgaagg agccaggaag 240
   ttatataaca cacggtaaac atccacctgg ctcaaggggc aaatgcagta cgtacagcat 300
   tggcagtggt gcgtcagagg tggcagaact atttcacact aaccagttga agactacaca 360
   agattaatac catccagcat caggatatag ctgtggattt tacaaaccat tcttatttct 420
   aacttcagga gttgatgttt ttcccagtcc atcttaaaat attactgctt taatcacaga 480
   tcagataaaa aggacaacat gcacaacctc cacctagaat cctgttgtag cctagacagt 540
   gaaatgatat gacatcagaa gactttaaaa ttgcagctcc ttttggatcc cccaaagtgt 600
   atctgcactc ttctt                                                 615
<210> 167
   <211> 99
   <212> DNA
   <213> Homo sapiens
<400> 167
   tttttttttt ccactgttca aggtttattg ggggttttag ttggtataac acttggatag 60
   tgggttgcat tgtttgtatg taaatctttt tacattata                       99
<210> 168
   <211> 612
   <212> DNA
   <213> Homo sapiens
<400> 168
   tacggccggg acatgaagga gctaggagtg ggaatagctt tgcgaaaaat gggcgcaatg 60
   gccaagccag attgtatcat cacttgtgat ggtaaaaacc tcaccataaa aactgagagc 120
   actttgaaaa caacacagtt ttcttgtacc ctgggagaga agtttgaaga aaccacagct 180
   gatggcagaa aaactcagac tgtctgcaac tttacagatg gtgcattggt tcagcatcag 240
   gagtgggatg ggaaggaaag cacaataaca agaaaattga aagatgggaa attagtggtg 300
   gagtgtgtca tgaacaatgt cacctgtact cggatctatg aaaaagtaga ataaaaattc 360
   catcatcact ttggacagga gttaattaag agaatgtcca agctcagttc aatgagcaaa 420
   tctccatact gtttctttct ttttttttca ttactgtgtt caattatctt tatcataaac 480
   attttacatg cagctatttc aaagtgtgct ggattaatta ggatcatccc tttggttaat 540
   aaataaatgg gtttgtgcta atatatcttg tatgcattct ttaaacctta caggaaatta 600
   gtgatgagtt tt                                                    612
<210> 169
   <211> 410
   <212> DNA
   <213> Homo sapiens
<400> 169
   gaaaacaaca cagttttctt gtaccctggg agagaagttt gaagaaacca cagctgatgg 60
   cagaaaaact cagactgtct gcaactttac agatggtgca ttggttcagc atcaggagtg 120
   ggatgggaag gaaagcacaa taacaagaaa attgaaagat gggaaattag tggtggagtg 180
   tgtcatgaac aatgtcacct gtactcggat ctatgaaaaa gtagaataaa aattccatca 240
   tcactttgga caggagttaa ttaagagaat gtccaagctc agttcaatga gcaaatctcc 300
   atactgtttc tttctttttt tttcattact gtgttcaatt atctttatca taaacatttt 360
   acatgcagct atttcaaagt gtgctggatt aattaggatc atccctttgg           410
<210> 170
   <211> 310
   <212> DNA
   <213> Homo sapiens
<400> 170
   gctcgggaat tcgctcgagt gctgctcccc acccatggac aggagatcct gggttgggcc 60
   tccctctgat gaccccagcc agatgagcga gtggggctca gcgtggccca tggtgcctgt 120
   cactcagcat tcccatgcct gatgtttacc aagtgctgtg ttggacactg gctttctcca 180
   aacaggattt gcctcctcca cgctccctac acacctgaga tgtaaactgg cagtcagtgt 240
   tcactcagga cctaggatta gaaaatggca gagttggtgc tggatccacc ttgcacttct 300
   atcaagccct                                                       310
<210> 171
   <211> 257
   <212> DNA
   <213> Homo sapiens
<400> 171
   tgctgctccc cagcccatgg acaggagatc ctgggttggg cctccctctg atgaccccag 60
   ccagatgagc gagtggggct cagcgtggcc catggtgcct gtcactcagc attcccatgc 120
   ctgatgttta ccaagtgctg tgttggacac tgactttctc caaacaggat ttgcctcctc 180
   cacgctccct acacacctga gatgtaaact ggcagtcagt gttcactcag gacctaggat 240
   tagaaaatgg cagagtt                                                257
<210> 172
   <211> 593
   <212> DNA
   <213> Homo sapiens
<400> 172
   tgaagaacgg tgccacttac gaagccaaaa tcaaggatgt ggatgagaaa gcagacatcg 60
   cactcatcaa aattgaccac cagggcaagc tgcctgtcct gctgcttggc cgctcctcag 120
   agctgcggcc gggagagttc gtggtcgcca tcggaagccc gttttccctt caaaacacag 180
   tcaccaccgg gatcgtgagc accacccagc gaggcggcaa agagctgggg ctccgcaact 240
   cagacatgga ctacatccag accgacgcca tcatcaacta tggaaactcg ggaggcccgt 300
   tagtaaacct ggacggtgaa gtgattggaa ttaacacttt gaaagtgaca gctggaatct 360
   cctttgcaat cccatctgat aagattaaaa agttcctcac ggagtcccat gaccgacagg 420
   ccaaaggaaa agccatcacc aagaagaagt atattggtat ccgaatgatg tcactcacgt 480
   ccagcaaagc caaagagctg aaggaccggc accgggactt cccagacgtg atctcaggag 540
   cgtatataat tgaagtaatt cctgataccc cagcagaagc tggtggtctc aag        593
<210> 173
   <211> 304
   <212> DNA
   <213> Homo sapiens
<220>
   <221> misc_feature
   <222> 106, 113, 125, 137
   <223> n = A,T,C or G
<400> 173
   gggtcaaagt tgagctgaag aacggtgcca cttacgaagc caaaatcaag gatgtggatg 60
   agaaagcaga catcgcactc atcaaaattg accaccaggg caagcngcct gtnctgctgc 120
   ttggncgctc ctcaganctg cggccgggag agttcgtggt cgccatcgga agcccgtttt 180
   cccttcaaaa cacagtcacc accgggatcg tgagcaccac ccagcgaggc ggcaaagagc 240
   tggggctccg caactcagac atggactaca tccagaccga cgccatcatc aactatggaa 300
   actc                                                              304
<210> 174
   <211> 258
   <212> DNA
   <213> Homo sapiens
<400> 174
   ggtcagaaga gttgtgcacg cagattagca ggccaaggtc tgagccacag cagcattttt 60
   atttcagatt ttgataactg tttatatgtg ttgaaaacca aaatgacatc tttttaaagc 120
   ttatccataa aaaaaaatag atgtctttta tagtggaaaa acacatgggg aaaaaaatca 180
   tctattttga tgcagcattt gataatgata aaacacctca cacctcactc tttatagtgc 240
   acaaaatgaa tgaggtct                                               258
<210> 175
   <211> 442
   <212> DNA
   <213> Homo sapiens
<400> 175
   aagtagccgc tccgagtgga ggcgactggg ggctgaagag cgcgccgccc tctcgtccca 60
   ctttccaggt gtgtgatcct gtaaaattaa atcttccaag atgatctggt atatattaat 120
   tataggaatt ctgcttcccc agtctttggc tcatccaggc ttttttactt caattggtca 180
   gatgactgat ttgatccata ctgagaaaga tctggtgact tctctgaaag attatattaa 240
   ggcagaagag gacaagttag aacaaataaa aaaatgggca gagaagttag atcggctaac 300
   tagtacagcg acaaaagatc cagaaggatt tgttgggcat ccagtaaatg cattcaaatt 360
   aatgaaacgt ctgaatactg agtggagtga gttggagaat ctggtcctta agggtatgtc 420
   agatggcttt atctctaacc ta                                          442
<210> 176
   <211> 611
   <212> DNA
   <213> Homo sapiens
<400> 176
   gggctgaggt aggaagtagc cgctccgagt ggaggcgact gggggctgaa gagcgcgccg 60
   ccctctcgtc ccactttcca ggtgtgtgat cctgtaaaat taaatcttcc aagatgatct 120
   ggtatatatt aattatagga attctgcttc cccagtcttt ggctcatcca ggctttttta 180
   cttcaattgg tcagatgact gatttgatcc atactgagaa agatctggtg acttctctga 240
   aagattatat taaggcagaa gaggacaagt tagaacaaat aaaaaaatgg gcagagaagt 300
   tagatcggct aactagtaca gcgacaaaag atccagaagg atttgttggg catccagtaa 360
   atgcattcaa attaatgaaa cgtctgaata ctgagtggag tgagttggag aatctggtcc 420
   ttaagggtat gtcagatggc tttatctcta acctaaccat tcagagacag tactttccta 480
   atgatgaaga tcaggttggg gcagccaaag ctctgttacg tctccaggat acctacaatt 540
   tggatacaga taccatctca aagggtaatc ttccaggagt gaaacacaaa tcttttctac 600
   ggctgaggac t                                                      611
<210> 177
   <211> 416
   <212> DNA
   <213> Homo sapiens
<400> 177
   ttacaaactc ctgaaccata atattctcgt ctccacagac acatactcca taatttaaaa 60
   ccaaatgctt gtgagaaagc ttgctcatca tacttgctgc ttcaaagaaa gactctgaat 120
   agtttctgtg tgctttatcc agaactttta aaagaacttc tgtttcatgc agttgaccgt 180
   agtctcctac ttctcttcgt acgcctttaa aaatctttgt aaaagtgcct tggccaaggc 240
   tttcattaaa tatcaaatct tcatttctga ttttgtgaaa caccatttgg ttcatatgag 300
   taggcctctg taatgttggt gaggttggta catcagaaac accattcgtt ctgaagacta 360
   gaaggtttga tttatctttt cggctttggg ggacagcatt tagtacacgg gaaaat     416
<210> 178
   <211> 163
   <212> DNA
   <213> Homo sapiens
<400> 178
   gggctttttt tttttgcaaa gttccaaatt tatgggtcgg gaaataaatc caaatttctc 60
   attaaaaaac tcctttggaa aaacttgggc ccaaaagttt cccatccgaa ctcagccttt 120
   tttgccccga tccccgactt ttttactcaa ggcccgggaa ggc                   163
<210> 179
   <211> 285
   <212> DNA
   <213> Homo sapiens
<400> 179
   aaagttacaa atttattggt ctggaaataa atacaaatat ctcattaaga aactcctctg 60
   gaaagacttg tgcacaatag tttcccatcc gtactcagcc tctcttgccc cgatccccga 120
   cttttctact caaggccagg gaaaggcctc caaggtgatg ggcggcaggt aacgagtcat 180
   tgcctctcac gccacctgga aggctggact acttcctcct cccaactgcg gggtcccaga 240
   aatcctcggg tcccagtggc tgacttacaa tattcaattc actct                 285
<210> 180
   <211> 458
   <212> DNA
   <213> Homo sapiens
<400> 180
   tcgagccgcc gccgcccctg tacaacaaca acaacaactg cgaggaaaat gagcagtctc 60
   tgcccccgcc ggccggcctc aacagttcct gggtggagct acccatgaac agcagcaatg 120
   gcaatgataa tggcaatggg aaaaatgggg ggctggaaca cgtaccatcc tcatcctcca 180
   tccacaatgg agacatggag aagattcctt tggatgcaca acatgaatca ggacagagta 240
   gttccagagg cagttctcac tgtgacagcc cttcgccaca agaagatggg cagatcatgt 300
   ttgatgtgga aatgcacacc agcagggacc atagctctca gtcagaagaa gaagttgtag 360
   aaggagagaa ggaagtcgag gctttgaaga aaagtgcgga ctgggtatca gactggtcca 420
   gtagacccga aaacattcca cccaaggagt tccacttc                         458
<210> 181
   <211> 329
   <212> DNA
   <213> Homo sapiens
<400> 181
   tttttttttt tttttttttt tttcttttta ataactatca actcaaactt agggaaactt 60
   gcctttgtct tgggggaaaa aaacaactag acaataaagc ttcttttaca tcatttgcta 120
   acctgatctc gttttaagag agagatggta gttatgttgc aagagtaaaa tttataccat 180
   gaatgataca ggtctagtct ggtggcacta attagagata atagcattgc tgacaaaatt 240
   ataatctgct ggtggcattt gcggaaaaga ggcccttgca aatttctaaa caacagtaaa 300
   ctctgttagg aaattctaaa atgtcttca                                    329
<210> 182
   <211> 527
   <212> DNA
   <213> Homo sapiens
<400> 182
   atacatgtaa cttcattatt ttaaaaatat ttttagaact ccaatactca ccctgttatg 60
   tcttgctagt ttaaattttg ctaattaact gaaacatgct taccagattc acactgttcc 120
   agtgtctata aaagaaacac tttgaagtct ataaaaaata aaataattat aaatgtcatt 180
   gtacatagca tgtttatatc tgcaaaaaac ctaatagcta attaatctgg aatatgcaac 240
   attgtcctta attgatgcaa ataacacaaa tgctgcaaag aaatctacta tatcccttaa 300
   tgaaatacat cattcttcat atatttctcc ttcagtccat tcccttaggc aatttttaat 360
   ttttaaaaat tattatcagg ggagaaaaat tggcaacgct attatatgta agggaaatat 420
   atacaaaaag aaaattaatc atagtcacct gactaagaaa ttctgactgc tagttgccat 480
   aaataactca atggaaatat tcctatggga taatgtattt taagtga                527
<210> 183
   <211> 530
   <212> DNA
   <213> Homo sapiens
<400> 183
   atacatacat gtaacttcat tattttaaaa atatttttag aactccaata ctcaccctgt 60
   tatgtcttgc taatttaaat tttgctaatt aactgaaaca tgcttaccag attcacactg 120
   ttccagtgtc tataaaagaa acactttgaa gtctataaaa aataaaataa ttataaatat 180
   cattgtacat agcatgttta tatctgcaaa aaacctaata gctaattaat ctggaatatg 240
   caacattgtc cttaattgat gcaaataaca caaatgctca aagaaatcta ctatatccct 300
   taatgaaata catcattctt catatatttc tccttcagtc cattccctta ggcaattttt 360
   aatttttaaa aattattatc aggggagaaa aattggcaaa actattatat gtaagggaaa 420
   tatatacaaa aagaaaatta atcatagtca cctgactaag aaattctgac tgctagttgc 480
   cataaataac tcaatggaaa tattcctatg ggataatgta ttttaagtga             530
<210> 184
   <211> 253
   <212> DNA
   <213> Homo sapiens
<220>
   <221> misc_feature
   <222> 98, 141, 162, 213
   <223> n = A,T,C or G
<400> 184
   tatacataca tgtaacttca ttattttaaa aatattttta gaactccaat actcaccctg 60
   ttatgtcttg ctaatttaaa ttttgctaat taactganac atgcttacca gattcacact 120
   gttccagtgt ctataaaaga nacactttga agtctataaa anataaaata attataaata 180
   tcattgtaca tagcatgttt atatctgcaa aanacctaat agctaattaa tctggaatat 240
   gcaacattgt cct                                                    253
<210> 185
   <211> 421
   <212> DNA
   <213> Homo sapiens
<400> 185
   ccgttgctgt cgatcccagc tccttgggag gctgaggcgg gagaattgcg ggaaggcggg 60
   gacggaggtt gcagtgagcc gagatcgcac tgctgtaccc agcctgggcc acagtgcaag 120
   actccatctc aaaaaaaaaa gaaaagaaaa agcctgttta atgcacaggt gtgagtggat 180
   tgcttatggc tatgagatag gttgatctcg cccttacccc ggggtctggt gtatgctgtg 240
   ctttcctcag cagtatggct ctgacatctc ttaaatgtcc caacttcagc tgttgggaga 300
   tggtgatatt ttcaacccta cttcctaaac atctgtctgg ggttccttta gtcttgaatg 360
   tcttatgctc aattatttgg tgttgagcct ctcttccaca agagctcctc catgtttgga 420
   t                                                                 421
<210> 186
   <211> 377
   <212> DNA
   <213> Homo sapiens
<400> 186
   cagctccttg ggaggctgag gcgggagaat tgcttgaacc cggggacgga ggttgcagtg 60
   agccgagatc gcactgctgt acccagcctg ggccacagtg caagactcca tctcaaaaaa 120
   aaaagaaaag aaaaagcctg tttaatgcac aggtgtgagt ggattgctta tggctatgag 180
   ataggttgat ctcgccctta ccccggggtc tggtgtatgc tgtgctttcc tcagcagtat 240
   ggctctgaca tctcttagat gtcccaactt cagctgttgg gagatggtga tattttcaac 300
   cctacttcct aaacatctgt ctggggttcc tttagtcttg aatgtcttat gctcaattat 360
   ttggtgttga gcctctc                                                377
<210> 187
   <211> 243
   <212> DNA
   <213> Homo sapiens
<220>
   <221> misc_feature
   <222> 228
   <223> n = A,T,C or G
<400> 187
   gaggtattcc acctcctacc ggaatataat taaagggaga aatacactgt atgaagtata 60
   tgttgatact atgacatgtt gccaacacct tgagaagcat tatttgtttc taataaaagt 120
   aatggctttg tcaatatatt ggtgggttta aaactttgct gcttttttac ataaagcctg 180
   tgcctttcct agaaagttaa gatgtaaatg tattctcaca tgtaaatntg aaagttcagg 240
   ggt                                                               243
<210> 188
   <211> 544
   <212> DNA
   <213> Homo sapiens
<400> 188
   tattccacct cctaccggaa tataattaaa gggagaaata cactgtatga agtatatgtt 60
   gatactatga catgttgcca acaccttgag aagcattatt tgtttctaat aaaagtaatg 120
   gctttgtcaa tatattggtg ggtttaaaac tttgctgctt ttttacataa agcctgtgcc 180
   tttcctagaa agttaagatg taaatgtatt ctcacatgta aatttgaaag ttcaggggtc 240
   tattatgaaa tgatacacat ttttaaatga accataattt ttttcactaa gctgtttgcc 300
   ttccaaagtg tttacacctt aagccttaac atgtatcttc attcagaaaa cagttatatt 360
   gtcataccat agtaggaaga aaaaccttta tttggaatat acactactgt aagtttgtac 420
   agatcatata cctaccacct gtctttgctt aaagagcctt gattacataa atatgtagga 480
   aaaaacatat tgagttcaaa atttatatct aacattgttt atgttatgat ttttttttaa 540
   ttgc                                                              544
<210> 189
   <211> 244
   <212> DNA
   <213> Homo sapiens
<220>
   <221> misc_feature
   <222> 210
   <223> n = A,T,C or G
<400> 189
   cacaaaaggt atgatcagca acttgcttgg gaaaggagcc gtggaccagc tgacacggct 60
   ggtgctggtg aatgccctct acttcaacgg ccagtggaag actcccttcc ccgactccag 120
   cacccaccgc cgcctcttcc acaaatcaga cggcagcact gtctctgtgc ccatgatggc 180
   tcagaccaac aagttcaact atactgagtn caccacgccc gatggccatt atacgacatc 240
   ctgg                                                              244
<210> 190
   <211> 209
   <212> DNA
   <213> Homo sapiens
<220>
   <221> misc_feature
   <222> 140
   <223> n = A,T,C or G
<400> 190
   gaacactgtt gctcttggtg gacgggccca gaggaattca gagttaaacc ttgagtgcct 60
   gcgtccgtga gaattcagca tggaatgtct ctactatttc ctgggatttc tgctcctggc 120
   tgcaagattg ccacttgatn ccgccaaacg atttcatgat gtgctgggca atgaaagacc 180
   ttctgcttac atgagggagc acaatcaat                                   209
<210> 191
   <211> 254
   <212> DNA
   <213> Homo sapiens
<220>
   <221> misc_feature
   <222> 85, 100, 143, 155, 182, 203, 229, 245, 254
   <223> n = A,T,C or G
<400> 191
   ctcccaacca agctctcttg aggatcttga aggaaactga attcaaaaag atcaaagtcc 60
   tgggctccgg tgcgttcggc acggngtata agggactctn gatcccagaa ggtgagaaag 120
   ttaaaattcc cgtcgctatc aangaattaa gagangcaac atctccgaaa gccaacaagg 180
   anatcctcga tgaagcctac gtnatggcca gcgtggacaa cccccacgng tgccgcctgc 240
   tgggnatctg tctn                                                   254
<210> 192
   <211> 484
   <212> DNA
   <213> Homo sapiens
<400> 192
   tttttttttt tttttttttc aaatatacct ctttgaaaga taaatttctg ctcaaaggga 60
   caatattctt gctggatgcg ttcctgtaaa tgcttcacag tttgaagaca aaggaatgca 120
   acttcccaaa atgtgcccga ggtggaagta cttcctggct agtcggtgta aacgttgcaa 180
   aaccagtctg tgggtctaag agctaatgcg ggcatggctg ttgggatgga ggacctgctg 240
   tggcttggtc ctgggtatcg aaagagtctg gatttttagg gctcatacta tcctccgtgg 300
   tcatactcca ataaattcac tgctttgtgg cgcgaccctt aggtattctg cattttcagc 360
   tgtggagccc ttaaagatgc catttggctt ggcttccttg ggaaagaagt cctgctggta 420
   gtcagggttg tccaggctaa tttggtggct gcctttctgg gcccagtggg cagggctgtc 480
   gaat                                                              484
<210> 193
   <211> 660
   <212> DNA
   <213> Homo sapiens
<400> 193
   tttaatcata tccaggagtt tgcaagaaac aggtgcttaa cactaattca cctcctgaac 60
   aagaaaaatg ggctgtgacc ggaactgtgg gctcatcgct ggggctgtca ttggtgctgt 120
   cctggctgtg tttggaggta ttctaatgcc agttggagac ctgcttatcc agaagacaat 180
   taaaaagcaa gttgtcctcg aagaaggtac aattgctttt aaaaattggg ttaaaacagg 240
   cacagaagtt tacagacagt tttggatctt tgatgtgcaa aatccacagg aagtgatgat 300
   gaacagcagc aacattcaag ttaagcaaag aggtccttat acgtacagag ttcgttttct 360
   agccaaggaa aatgtaaccc aggacgctga ggacaacaca gtctctttcc tgcagcccaa 420
   tggtgccatc ttcgaacctt cactatcagt tggaacagag gctgacaact tcacagttct 480
   caatctggct gtggcagctg catcccatat ctatcaaaat caatttgttc aaatgatcct 540
   caattcactt attaacaagt caaaatcttc tatgttccaa gtcagaactt tgagagaact 600
   gttatggggc tatagggatc catttttgag tttggttccg taccctgtta ctaccacagt 660
<210> 194
   <211> 277
   <212> DNA
   <213> Homo sapiens
<400> 194
   ctttaatcat atccaggagt ttgcaagaaa caggtgctta acactaattc acctcctgaa 60
   caagaaaaat gggctgtgac cggaactgtg ggctcatcgc tggggctgtc attggtgctg 120
   tcctggctgt gtttggaggt attctaatgc cagttggaga cctgcttatc cagaagacaa 180
   ttaaaaagca agttgtcctc gaagaaggta caattgcttt taaaaattgg gttaaaacag 240
   gcacagaagt ttacagacag ttttggatct ttgatgt                         277
<210> 195
   <211> 457
   <212> DNA
   <213> Homo sapiens
<400> 195
   gactgggttt gggtgcagac gttgttgctt gggcgcttct ccgctgcgtg taggtgaagg 60
   gggcttcctg accgagacat ggatttaggt gctattacaa aatactcagc attacacgcc 120
   aagcccaatg gactgatcct tcaatacggg actgctggat ttcgaacgaa ggcagaacat 180
   cttgatcatg tcatgtttcg catgggatta ttagctgtcc tgaggtcaaa acagacaaaa 240
   tccactatag gagtcatggt aacagcgtcc cacaatcctg aggaagacaa tggtgtaaaa 300
   ttggttgatc ctttgggtga aatgttggca ccatcctggg aggaacatgc cacctgttta 360
   gcaaatgctg aggaacaaga tatgcagaga gtgcttattg acatcagcga gaaagaagct 420
   gtgaatctgc aacaagatgc ctttgtagtt attggta                         457
<210> 196
   <211> 361
   <212> DNA
   <213> Homo sapiens
<400> 196
   tttttttttt tttttttttt tttgggcagg agaccatgtt actttattca tttgtttaac 60
   tttaaccatg ttcaataaac ttttcacctg tttggtgagt tccacaaaag ccttagagag 120
   tttctggtag taaccttcta tagttgcctt tccatatcgg ccacccgtgt ttcgacaata 180
   caccatgtag tgcagctggg gtgttgttaa caagccataa tcatggaatt gacctcctag 240
   aacagtcaca ccatctatta cagattgtga aagtttctca ctgctgggcc tggtatctct 300
   accaataact acaaaggcat cttgttgcag attcacagct tctttctcgc tgatgtcaat 360
   a                                                                 361
<210> 197
   <211> 551
   <212> DNA
   <213> Homo sapiens
<400> 197
   gagccgagct gatttgatcg aggagcgcgg ttaccggacg ggctgggtct atggtcgctc 60
   cgcgggccgc tccgccggct ggtgcttttt tatcagggca agctgtgttc catggcaggg 120
   aacttttggc agatctccca ctatttgcaa tggattttgg ataaacaaga tctgttgaag 180
   gagcgccaaa aggatttaaa gtttctctca gaggaagaat attggaagtt acaaatattt 240
   tttacaaatg ttatccaagc attaggtgaa catcttaaat taagacaaca agttattgcc 300
   actgctacgg tatatttcaa gagattctat gccaggtatt ctctgaaaag tatagatcct 360
   gtattaatgg ctcctacatg tgtgtttttg gcatccaaag tagaggaatt tggagtagtt 420
   tcaaatacaa gattgattgc tgctgctact tctgtattaa aaactagatt ttcatatgcc 480
   tttccaaagg aatttcctta taggatgaat catatattag aatgtgaatt ctatctgtta 540
   gaactaatgg a 551
<210> 198
   <211> 637
   <212> DNA
   <213> Homo sapiens
<400> 198
   tacggccggg agtcgagccg agctgatttg atcgaggagc gcggttaccg gacgggctgg 60
   gtctatggtc gctccgcggg ccgctccgcc ggctggtgct tttttatcag ggcaagctgt 120
   gttccatggc agggaacttt tggcagagct cccactattt gcaatggatt ttggataaac 180
   aagatctgtt gaaggagcgc caaaaggatt taaagtttct ctcagaggaa gaatattgga 240
   agttacaaat attttttaca aatgttatcc aagcattagg tgaacatctt aaattaagac 300
   aacaagttat tgccactgct acggtatatt tcaagagatt ctatgccagg tattctctga 360
   aaagtataga tcctgtatta atggctccta catgtgtgtt tttggcatcc aaagtagagg 420
   aatttggagt agtttcaaat acaagattga ttgctgctgc tacttctgta ttaaaaacta 480
   gattttcata tgcctttcca aaggaatttc cttataggat gaatcatata ttagaatgtg 540
   aattctatct gttagaacta atggattgtt gcttgatagt gtatcatcct tatagacctt 600
   tgctccagta tgtgcaggac atgggccaag aagacat 637
<210> 199
   <211> 130
   <212> DNA
   <213> Homo sapiens
<400> 199
   tagaaagcct ccacctggag tacaatgccc tcaaggtcct tcacaatggc accctggctg 60
   agttgcaagg tctaccccac attagggttt tcctggacaa caatccctgg gtctgcgact 120
   gccacatggc 130
<210> 200
   <211> 372
   <212> DNA
   <213> Homo sapiens
<220>
   <221> misc_feature
   <222> 29, 100, 297, 298, 353, 357
   <223> n = A,T,C or G

<400> 200
   gtgctgtttg accaatggtc atgtggccna gattggggac ttcgggctgg ctagggacat 60
   catgaatgac tccaactaca ttgtcaaggg caatgccgcn ctgcctgtga agtggatggc 120
   cccagagagc atctttgact gtgtctacac ggttcagagc gacgtctggt cctatggcat 180
   cctcctctgg gagatcttct cacttgggct gaatccctac cctggcatcc tggtgaacag 240
   caagttctat aaactggtga aggatggata ccaaatggcc cagcctgcat ttgcccnnaa 300
   gaatatatac agcatcatgc aggcctgctg ggcttgggag cccacccaca ganccanctt 360
   ccagcagatc tg 372
<210> 201
   <211> 478
   <212> DNA
   <213> Homo sapiens
<220>
   <221> misc_feature
   <222> 3, 10, 11, 78, 112, 130, 150, 231, 457
   <223> n = A,T,C or G
<400> 201
   gancacctgn nacaaggagg atggacggcc cctggagctc cgggacctgc ttcacttctc 60
   cagccaagta gcccaggnat ggccttcctc gcttccaaga attgcatcca cngggacgtg 120
   gcagcgcgtn acgtgctgtt gaccaatggn catgtggcca agattgggga cttcgggctg 180
   gctagggaca tcatgaatga ctccaactac attgtcaagg gcaatgccgc nctgcctgtg 240
   aagtggatgg ccccagagag catctttgac tgtgtctaca cggttcagag cgacgtctgg 300
   tcctatggca tcctcctctg ggagatcttc tcacttgggc tgaatcccta ccctggcatc 360
   ctggtgaaca gcaagttcta taaactgggt gaaaggatgg ataccaaatg gcccagcctg 420
   cattttgccc ccaaagaata tatacaagca tccatgnagg cccttctggg ccttggag 478
<210> 202
   <211> 218
   <212> DNA
   <213> Homo sapiens
<400> 202
   gcgagcaagg ggatatcgcc cagcccttgc tgcagcccaa caactatcag ttctgctgag 60
   gagttgacga cagggagtac cactctcccc tcccacaaac ttcaactcct ccatggatgg 120
   ggcgacacgg ggagaacata caaactctgc cttcggtcat ttcactcaac agctcggccc 180
   agctctgaaa cttgggaagg tgagggattc aggggagg 218
<210> 203
   <211> 556
   <212> DNA
   <213> Homo sapiens
<400> 203
   taagctcgga attcggctcg aggcgagcaa ggggatatcg cccagccctt gctgcagccc 60
   aacaactatc agttctgctg aggagttgac gacagggagt accactctcc cctcccacaa 120
   acttcaactc ctccatggat ggggcgacac ggggagaaca tacaaactct gccttcggtc 180
   atttcactca acagctcggc ccagctctga aacttgggaa ggtgagggat tcaggggagg 240
   tcagaggatc ccacttcctg agcatgggcc atcactgcca gtcaggggct gggggctgag 300
   ccctcacccc cccctcccct actgttctca tggtgttggc ctcgtgtttg ctatgccaac 360
   tagtagaacc ttctttccta atccccttat cttcatggaa atggactgac tttatgccta 420
   tgaagtcccc aggagctaca ctgatactga gaaaaccagg ctctttgggg ctagacagac 480
   tggcagagag tgagatctcc ctctctgaga ggagcagcag atgctcacag accacactca 540
   gctcaggccc cttgga                                                  556
<210> 204
   <211> 319
   <212> DNA
   <213> Homo sapiens
<400> 204
   tccttattta tttaacttca cccgagttcc tctgggtttc taagcagtta tggtgatgac 60
   ttagcgtcaa gacatttgct gaactcagca cattcgggac caatatatag tgggtacatc 120
   aagtccatct gacaaaatgg ggcagaagag aaaggactca gtgtgtgatc cggtttcttt 180
   ttgctcgccc ctgttttttg tagaatctct tcatgcttga catacctacc agtattattc 240
   ccgacgacac atatacatat gagaatatac cttatttatt tttgtgtagg tgtctgcctt 300
   cacaaatgtc atgtctact                                               319
<210> 205
   <211> 456
   <212> DNA
   <213> Homo sapiens
<400> 205
   attccgttgc tgtcgagggt cactaccagt acaagagcat ccctgtggag gacaaccaca 60
   aggcagacat cagctcctgg ttcaacgagg ccattgactt catagactcc atcaagaatg 120
   ctggaggaag ggtgtttgtc cactgccagg caggcatttc ccggtcagcc accatctgcc 180
   tggcttacct tatgaggact aatcgagtca agctggacga ggcctttgag tttgtgaagc 240
   agaggcgaag catcatctct cccaacttca gcttcatggg ccagctgctg cagtttgagt 300
   cccaggtgct ggctccgcac tgttcggcag aggctgggag ccccgccatg gctgtgctcg 360
   accgaggcac ctccaccacc accgtgttca acttccccgt ctccatccct gtccactcca 420
   cgaacagtgc gctgagctac cttcagagcc ccatta                            456
<210> 206
   <211> 533
   <212> DNA
   <213> Homo sapiens
<400> 206
   agtttttaaa taatgaatat tatttaatac cacaacagaa ttatccccaa tttccaataa 60
   gtcctatcat tgaaaattca aatataagtg aagaaaaaat tagtagatca acaatctaaa 120
   caaatccctc ggttctaaga tacaatggat tccccatact ggaaggactc tgaggcttta 180
   ttcccccact atgcatatct tatcatttta ttattataca cacatccatc ctaaactata 240
   ctaaagccct tttcccatgc atggatggaa atggaagatt tttttttaac ttgttctaaa 300
   agtcttaata tgggctgttg ccatgaaggc ttgcagaatt gagtccattt tctagctgcc 360
   tttattcaca tagtggacgg ggtacctaaa agtactgggg ttgactcaga gagtcgctgt 420
   cattctgtca ttgctgctac tctaacactg agcaacactc tcccagtggc agatcccctg 480
   tatcattcca agaggagcat tcatcccttt gctctaatga tcaggaatga tgc 533
<210> 207
   <211> 246
   <212> DNA
   <213> Homo sapiens
<400> 207
   aatgcactaa ctcaatacca agatgagttt ttaaataatg aatattattt aataccacaa 60
   cagaattatc cccaatttcc aataagtcct atcattgaaa attcaaatat aagtgaagaa 120
   aaaattagta gatcaacaat ctaaacaaat ccctcggttc taagatacaa tggattcccc 180
   atactggaag gactctgagg ctttattccc ccactatgca tatcttatca ttttattatt 240
   atacac 246
<210> 208
   <211> 407
   <212> DNA
   <213> Homo sapiens
<400> 208
   ggccgccctt tttttttttt tttttttttt ttttttttgg gcaaaaaggg gctttttttt 60
   ttttcccccc cccttttttt aacccttccc ctaatatttc ccccaaaaaa aaaaattttt 120
   tttttttggg ggggggaaaa aaaagggaaa aaaaaccccc ccccccgggg ggggaaaaaa 180
   accccccaaa aacccccctt ttgggggggt ccccccccat gggggttccc cccccaattt 240
   ttttcccccc cccaaaaaaa tttttaaccc ccccccaagg gggtggaaaa ccttaaaaaa 300
   aaccccccgg aaaaaccaaa accccttttt taaaaaaaaa aaaaaaattt ttggggggca 360
   aaaccccccc cccccaaaaa accccccccc ccccccttaa aaaaaaa 407
<210> 209
   <211> 359
   <212> DNA
   <213> Homo sapiens
<220>
   <221> misc_feature
   <222> 1, 53, 121, 123, 128, 133, 142, 150, 174, 179, 183, 186, 196, 200, 201, 204, 207, 212, 215, 218, 224, 229, 230, 231, 243, 244, 249, 260, 261, 267, 268, 270, 273, 279, 289, 291, 295, 301, 303, 305, 312, 315, 337, 345, 357
   <223> n = A,T,C or G
<400> 209
   ncggggactg cgcggcggtg cagagccggg cgtgggcgag aacgaacggg ctncctgcgg 60
   ctgagagcgt cgagtgtcac catgggtatc acgcttggag cttcctaaag gacttcctgg 120
   ncngggcntc gcnctgcccg tntccaagan cccggtcggc cccaatcgag aggncaaanc 180
   tgntgntgaa ggtgcnagan nccnagnaac angtnaantc ttangaagnn ntacaaaggg 240
   gtnnattant tttttggtan nattccnnan gancaaggnt ttcctttcnt nttgnagggt 300
   nancntggca angtnattcc ttaatttccc aaccaangtt ttaantttgg ctttaangg  359
<210> 210
   <211> 394
   <212> DNA
   <213> Homo sapiens
<400> 210
   tttttttttt gcattaagtg gtctttattg atgtttcaca ttcagttatt atcaattctt 60
   cagttaattg tacaagtatg ataaattatt ttctatttgc tgtgggaatt taaatgtaaa 120
   ataaatacaa aatacatgtg tggtttaatg aacactcaat gaagcatctc ttctgaggta 180
   ttcctttcag tctggtttta tcccaggatc tttttacttc ccctaggaat agtctattaa 240
   accacacaat ggatctgtga acttgtagat caagttcact gtaaatctgt gaacttgtgt 300
   tttaattaca ttagacatat tttttgatct catcatacaa caccaataca aaaggcaccg 360
   cccatgcctc tcaggcacat tgggaccggg cacc                             394
<210> 211
   <211> 292
   <212> DNA
   <213> Homo sapiens
<400> 211
   gggagcccac cagcaagaat gagttggagc aatcttttca tgtgacctcc ttaacagata 60
   tttactgaag gaatctaggt tgtattttca gtggacaatg ggaataaagc atttctaaag 120
   caccgactgg agaggaaggc aacagagaca aggagagaag ccgagagaca tgtctgcgtg 180
   ctgccacgca tttgagcgat tgctctgtga agagttgtac actgaacact ttcaggggag 240
   gctgtttacc caggcaatgt cctcaaacaa gcctgtgccg gggtgtcctg ga         292
<210> 212
   <211> 495
   <212> DNA
   <213> Homo sapiens
<400> 212
   aattccgttg ctgtcgctgc gcccaggtaa tttgagcaaa ggccacagtg aactccggcg 60
   tggctgagga aggaggaggc acccacaggc tgctgggagg agagcataag gctcaaaatg 120
   gaaaatcata aatccaataa taaggaaaac ataacaattg ttgatatatc cagaaaaatt 180
   aaccagcttc cagaagcaga aaggaatcta cttgaaaatg gatcggttta tgttggatta 240
   aatgctgctc tttgtggcct catagcaaac agtctttttc gacgcatctt gaatgtgaca 300
   aaggctcgca tagctgctgg cttaccaatg gcagggatac cttttcttac aacagactta 360
   acttacagat gttttgtaag ttttcctttg aatacaggtg atttggattg tgaaacctgt 420
   accataacac ggagtggact gactggtctt gttattggtg gtctataccc tgttttcttg 480
   gctatacctg taaat                                                  495
<210> 213
   <211> 358
   <212> DNA
   <213> Homo sapiens
<400> 213
   tgcgaccgcg atctcctgca gctggtgcac cacctcggcg atggacagcc gctcctccgg 60
   gttcacctgc agcatggcga ggatgaggct gtggaagacc gtgtactgcg tgtcgtgcgg 120
   ggggatcgag tacttcccat tgactattcg aagtttcgct ccatcctcaa aagggtgctg 180
   ccggaagcac agcaggtaca agatgcagcc cagggcccag atatcctgct tctcgccgat 240
   cgggaagttg gaatacaagt ctatgatttc tggtgttcta tacattggtg ttgtattcct 300
   cgtgatctga aaaaatacaa acatttcaaa ggaaaagttg catcccacaa acagtatt   358
<210> 214
   <211> 406
   <212> DNA
   <213> Homo sapiens
<400> 214
   tggtacgcct gcaggtaccg gtccggaatt cccgggtcga cccacgcgtc cgaggacatc 60
   tggaatgtca ctggtgccca ggtgtacttg agctgtgagg tcatcggaat cccgacacct 120
   gtcctcatct ggaacaaggt aaaaaggggt cactatggag ttcaaaggac agaactcctg 180
   cctggtgacc gggacaacct ggccattcag acccggggtg gcccagaaaa gcatgaagta 240
   actggctggg tgctggtatc tcctctaagt aaggaagatg ctggagaata tgagtgccat 300
   gcatccaatt cccaaggaca ggcttcagca tcagcaaaaa ttacagtggt tgatgcctta 360
   catgaaatac cagtgaaaaa aggtgaaggt gccgagctat aaacct                406
<210> 215
   <211> 300
   <212> DNA
   <213> Homo sapiens
<220>
   <221> misc_feature
   <222> 66, 71, 259
   <223> n = A,T,C or G
<400> 215
   aggacatctg gaatgtcact ggtgcccagg tgtacttgag ctgtgaggtc atcggaatcc 60
   cgacanctgt nctcatctgg aacaaggtaa aaaggggtca ctatggagtt caaaggacag 120
   aacttctgcc tggtgaccgg gacaacctgg ccattcagac ccggggtggc ccagaaaagc 180
   atgaagtaac tggctgggtg ctggtatctc ctctaagtaa ggaagatgct ggagaatatg 240
   agtgccatgc atccaattnc caaggacagg cttcagcatc agcaaaaatt acagtggttg 300
<210> 216
   <211> 232
   <212> DNA
   <213> Homo sapiens
<400> 216
   ttcaaaagct tagagagaat aagcttcttg gtggtgaaat acaactctca cgtgtgctcc 60
   agttctaaaa ttaacctgtg cctggtctct gaagcccttt cttgctctgt gcctttcagc 120
   cacatcctta ggtgctaacg gccatgagct ccgactctcc aaagtgagct ccactttggg 180
   tctgaggagc ccctggcaga gtccacgctg cctcaggtat catgggcgta at         232
<210> 217
   <211> 453
   <212> DNA
   <213> Homo sapiens
<400> 217
   ataagcttct tggtggtgaa actacaactc tcacgtgtgc tccagttcta aaattaacct 60
   gtgcctggtc tctgaagccc tttcttgctc tgtgcctttc agccacatcc ttaggtgcta 120
   acggccatga gctccgactc tccaaagtga gctccacttt gggtctgagg agcccctggc 180
   agagtccacg ctgcctcagg tatcatgggc gtaatgatca cccaggctcc gggagatctc 240
   atggatgatt actgtatgag acagagggga cttcagtctt tccagggcct tggtggaatt 300
   tttggctctg gtgttttcgc cagacaataa acttacactg gaagctttga ttcaccctcc 360
   acagtactcc agaaaggact gtcctataag ttgtacactt taaaaggtca tgtagaggtt 420
   gtagtagaat ggcttttcac cctggtgact ttg                              453
<210> 218
   <211> 520
   <212> DNA
   <213> Homo sapiens
<400> 218
   agatgtgtga gaagtgcccc acctgcccgg atgcatgcag caccaagaga gattgcgtcg 60
   agtgcctgct gctccactct gggaaacctg acaaccagac ctgccacagc ctatgcaggg 120
   atgaggtgat cacatgggtg gacaccatcg tgaaagatga ccaggaggct gtgctatgtt 180
   tctacaaaac cgccaaggac tgcgtcatga tgttcaccta tgtggagctc cccagtggga 240
   agtccaacct gaccgtcctc agggagccag agtgtggaaa cacccccaac gccatgacca 300
   tcctcctggc tgtggtcggt agcctcctcc ttgttgggct tgcactcctg gctatctgga 360
   agctgcttgt caccatccac gaccggaggg agtttgcaaa gtttcagagc gagcgatcca 420
   gggcccgcta tgaaatggct tcaaatctat tatacagaaa gcctatctcc acgcacactg 480
   tggacttcac cttcaacaag ttcaacaaat cctacaatgg                       520
<210> 219
   <211> 404
   <212> DNA
   <213> Homo sapiens
<400> 219
   agatgtgtga gaagtgcccc acctgcccgg atgcatgcag caccaagaga gattgcgtcg 60
   agtgcctgct gctccactct gggaaacctg acaaccagac ctgccacagc ctatgcaggg 120
   atgaggtgat cacatgggtg gacaccatcg tgaaagatga ccaggaggct gtgctatgtt 180
   tctacaaaac cgccaaggac tgcgtcatga tgttcaccta tgtggagctc cccagtggga 240
   agtccaacct gaccgtcctc agggagccag agtgtggaaa cacccccaac gccatgacca 300
   tcctcctggc tgtggtcggt agcctcctcc ttgttgggct tgcactcctg gctatctgga 360
   agctgcttgt caccatccac gaccggaggg agtttgcaaa gttt                  404
<210> 220
   <211> 80
   <212> DNA
   <213> Homo sapiens
<400> 220
   atggcttcaa atccattata cagaaagcct atctccacgc acactgtgga cttcaccttc 60
   aacaagttca acaaatccta                                             80
<210> 221
   <211> 607
   <212> DNA
   <213> Homo sapiens
<400> 221
   tgccccacct gcccggatgc atgcagcacc aagagagatt gcgtcgagtg cctgctgctc 60
   cactctggga aacctgacaa ccagacctgc cacagcctat gcagggatga ggtgatcaca 120
   tgggtggaca ccatcgtgaa agatgaccag gaggctgtgc tatgtttcta caaaaccgcc 180
   aaggactgcg tcatgatgtt cacctatgtg gagctcccca gtgggaagtc caacctgacc 240
   gtcctcaggg agccagagtg tggaaacacc cccaacgcca tgaccatcct cctggctgtg 300
   gtcggtagca tcctccttgt tgggcttgca ctcctggcta tctggaagct gcttgtcacc 360
   atccacgacc ggagggagtt tgcaaagttt cagagcgagc gatccagggc ccgctatgaa 420
   atggcttcaa atccattata cagaaagcct atctccacgc acactgtgga cttcaccttc 480
   aacaagttca acaaatccta caatggcact gtggactgat gtttccttct ccgaggggct 540
   ggagcgggga tctgatgaaa aggtcagact gaaacgcctt gcacggctgc tcggcttgat 600
   cacaact                                                           607
<210> 222
   <211> 583
   <212> DNA
   <213> Homo sapiens
<400> 222
   ggtatgtgcc atcacaagca gatgtggcag tatttgaagc cgtgtccagc ccaccgcctg 60
   ccgacttgtg tcatgcccta cgttggtata atcacatcaa gtcttacgaa aaggaaaagg 120
   ccagcctgcc aggagtgaag aaagctttgg gcaaatatgg tcctgccgat gtggaagaca 180
   ctacaggaag tggagctaca gatagtaaag atgatgatga cattgacctc tttggatctg 240
   atgatgagga ggaaagtgaa gaagcaaaga ggctaaggga agaacgtctt gcacaatatg 300
   aatcaaagaa agccaaaaaa cctgcacttg ttgccaagtc ttccatctta ctagatgtga 360
   aaccttggga tgatgagaca gatatggcga aattagagga gtgcgtcaga agcattcaag 420
   cagacggctt agtctggggc tcatctaaac tagttccagt gggatacgga attaagaaac 480
   ttcaaataca gtgtgtagtt gaagatgata aagttggaac agatatgctg gaggagcaga 540
   tcactgcttt tgaggactat gtgcagtcca tggatgtggc tgc                   583
<210> 223
   <211> 296
   <212> DNA
   <213> Homo sapiens
<400> 223
   tacatcgagg ggtatgtgcc atcacaagca gatgtggcag tatttgaagc cgtgtccagc 60
   ccaccgcctg ccgacttgtg tcatgcccta cgttggtata atcacatcaa gtcttacgaa 120
   aaggaaaagg ccagcctgcc aggagtgaag aaagctttgg gcaaatatgg tcctgccgat 180
   gtggaagaca ctacaggaag tggagctaca gatagtaaag atgatgatga cattgacctc 240
   tttggatctg atgatgagga ggaaagtgaa aaagcaaaga ggctaaggga agaacg     296
<210> 224
   <211> 208
   <212> DNA
   <213> Homo sapiens
<220>
   <221> misc_feature
   <222> 97
   <223> n = A,T,C or G
<400> 224
   gactacatct tggacctgca gatcgccctg gactcgcatc ccactattgt cagcctgcat 60
   caccagagac ccgggcagaa ccaggcgtcc aggacgncgc tgaccaccct caacacggat 120
   atcagcatcc tgtccttgca ggcttctgaa ttcccttctg agttaatgtc aaatgacagc 180
   aaagcactgt gtggctgaat aagcggtg                                   208
<210> 225
   <211> 274
   <212> DNA
   <213> Homo sapiens
<220>
   <221> misc_feature
   <222> 133
   <223> n = A,T,C or G
<400> 225
   gcagcggctg gagcggcaga tcagccagga tgtcaagctg gagccagaca tcctgcttcg 60
   ggccaagcaa gatttcctga agacggacag tgactcggac ctacagctct acaaggaaca 120
   gggtgagggg canggtgacc ggagcctgcg ggagcgtgat gtgctggaac gggagtttca 180
   gcgggtcacc atctctgggg aggagaagtg tggggtgccg ttcacagacc tgctggatgc 240
   agccaagatg tggtgcgggc gtcttcatcc ggga                             274
<210> 226
   <211> 330
   <212> DNA
   <213> Homo sapiens
<400> 226
   ggccgccctt tttttttttt tttttttttg ggcccagggg gggccccctt gggaaaaaca 60
   cccgggaaac ttcccaaagg ggccttgggg gaattttttt taaaaaaaaa ccttttttta 120
   aaaaaaactt tgggatttaa attttttttc cggccccttt tttgggccgg gtaccccaat 180
   ttaaaaaagg ggggcttttt aaaggttggg aaaaaaaaaa aattgggggg gcccaaaaaa 240
   ttggggggcc cccaaaaaaa aagcggggtt tggaaaaatt ttgggggggt ttggaaattt 300
   gggccccaaa acgggggacc cctttccccc                                  330
<210> 227
   <211> 525
   <212> DNA
   <213> Homo sapiens
<400> 227
   gaatttggcc ctcgaggcca agaattcggc acgagggttc acatagcaat ttaatcaagt 60
   aatggttaat tagttacccc ctatatataa atatatgtaa tcaatttctt caaatagctt 120
   gcttacatga taatcaatta gccaaccatg agtcatttag aatagtgata aatagaatac 180
   acagaatagt gatgaaattc aatttaaaaa atcacgttag cctccaaacc atttaattca 240
   aatgaaccca tcaactggat gccaactctg gcgaatgtag gacctctgag tggctgtata 300
   attgttaatt caaatgaaat tcatttaaac agttgacaaa ctgtcattca acaattagct 360
   ccaggaaata acagttattt catcataaaa cagtcccttc aaacacacaa ttgttctgct 420
   gaagagttgt catcaacaat ccaatgctca cctattcagt tgctctgtgg tcagtgtggc 480
   tgcataacag tggattccat gaaaggagtc attttagtga tgagc                 525
<210> 228
   <211> 788
   <212> DNA
   <213> Homo sapiens
<220>
   <221> misc_feature
   <222> 42, 44, 48, 49, 51, 52, 53, 54, 55, 57, 59, 61, 62, 63, 64, 68, 69, 70, 71, 73, 74, 75, 76, 77, 79, 80, 83, 87, 89, 92, 93, 94, 95, 97, 98, 107, 112, 113, 117, 122, 125, 127, 130, 131, 133, 671, 677, 685, 706, 713, 718, 725, 757, 771
   <223> n = A,T,C or G
<221> misc_feature
   <222> 783
   <223> n = A,T,C or G
<400> 228
   gttcacatag caatttaatc aagtaatcat taattagggg gngngggnng nnnnngngnt 60
   nnnngtgnnn ngnnnnngnn ggngtgngng tnnnngnngg gaggtgngga anngttnttt 120
   tntgngngan nantagaata cacagaatag tgatgaaatt caatttaaaa aatcacgtta 180
   gcctccaaac catttaattc aaatgaaccc atcaactgga tgccaactct ggcgaatgta 240
   ggacctctga gtggctgtat aattgttaat tcaaatgaaa ttcatttaaa cagttgacaa 300
   actgtcattc aacaattagc tccaggaaat aacagttatt tcatcataaa acagtccctt 360
   caaacacaca attgttctgc tgaagagttg tcatcaacaa tccaatgctc acctattcag 420
   ttgctctgtg gtcagtgtgg ctgcataaca gtggattcca tgaaaggagt cattttagtg 480
   atgagctgcc agtccattcc caggccaggc tgtcgctggc catccattca gtcgattcag 540
   tcataggcga atctgttctg cccgaagctt gtggtcaagc aaaaattcag ccctgaaaat 600
   cagcacatct gttcggtgga ctaaaccaca gttagttcgt caagcagcaa cccctgtggc 660
   atgaccgcca ntgggtncat gcgtntgcac tgggagttgg ccaaanctcc ggnggtcncg 720
   gggtnttttt tgtgggtttt ttttttttag tcttgtnttt gggtaagtgg nttttttttt 780
   tcnttggg                                                          788
<210> 229
   <211> 156
   <212> DNA
   <213> Homo sapiens
<400> 229
   gccgagggaa gggcccggca gctgaggagc cgctgagctt gctggacgac atgaaccact 60
   gctactcccg cctgcgggaa ctggtacccg gagtcccgag aggcactcag cttagccagg 120
   tggaaatcct acagcgcgtc atcgactaca ttctcg                           156
<210> 230
   <211> 538
   <212> DNA
   <213> Homo sapiens
<400> 230
   tacgactcct atagggaatt tggccctcga ggccaagaat tcggcacgag ggtgactttg 60
   gctttgctcg catcatcggc gagaagtcgt tccgccgctc agtggtgggc acgccggcct 120
   acctggcacc cgaggtgctg ctcaaccagg gctacaaccg ctcgctggac atgtggtcag 180
   tgggcgtgat catgtacgtc agcctcagcg gcaccttccc tttcaacgag gatgaggaca 240
   tcaatgacca gatccagaac gccgccttca tgtacccggc cagcccctgg agccacatct 300
   cagctggagc cattgacctc atcaacaacc tgctgcaggt gaagatgcgc aaacgctaca 360
   gcgtggacaa atctctcagc cacccctggt tacaggagta ccagacgtgg ctggacctcc 420
   gagagctgga ggggaagatg ggagagcgat acatcacgca tgagagtgac gacgcgcgct 480
   gggagcagtt tgcagcagag catccgctgc ctgggtctgg gctgcccacg gacaggga   538
<210> 231
   <211> 232
   <212> DNA
   <213> Homo sapiens
<220>
   <221> misc_feature
   <222> 18, 56, 94, 103, 117, 128, 145, 184, 204, 219
   <223> n = A,T,C or G
<400> 231
   tggctttgct cgcatcancg gcgagaagtc gtcccgccgc tcagtggtgg gcacgncggc 60
   ctacctggca cccgaggtct tgctcaacca gggntacaac cgntcgctcg acatgtngtc 120
   agtgggcntg atcatgtacg tcagnctcag cggcaccttc cctttcaacg aggatgagga 180
   catnaatgac cagatccaga acgncgactt catgtaccng gccagaccct gg         232
<210> 232
   <211> 420
   <212> DNA
   <213> Homo sapiens
<400> 232
   taccggtccg gaattcccgg gtcgacccac gcgtccggcg tctctgctcc accaaggtgc 60
   cctggacatg ctgaccaagg tgatggccct agagctcggg ccccacaaga tccgagtgaa 120
   tgcagtaaac ccccacagtg tgatgacgtc catgggccag gccacctgga gtgaccccca 180
   caaggccaag actatgctga accgaatccc acttggcaag tttgctgagg tagagcacgt 240
   ggtgaacgcc atcctctttc tgctgagtga ccgaagtggc atgaccacgg gttccacttt 300
   gccggtggaa gggggcttct gggcctgctg agctccctcc acacacctca agccccatgc 360
   cgtgctcatc ctacccccaa tccctccaat aaacctgatt ctgctgccca aaaaaaaaaa 420
<210> 233
   <211> 294
   <212> DNA
   <213> Homo sapiens
<220>
   <221> misc_feature
   <222> 2, 170
   <223> n = A,T,C or G
<400> 233
   gngtctactg ctccaccaag ggtgccctgg acatgctgac caaggtgatg gccctagagc 60
   tcgggcccca caagatccga gtgaatgcag taaaccccac agtggtgatg acgtccatgg 120
   gccaggccac ctggagtgac ccccacaagg ccaagactat gctgaaccgn atcccacttg 180
   gcaagtttgc tgaggtagag cacgtggtga acgccatcct ctttctgctg agtgaccgaa 240
   gtggcatgac cacgggttcc actttgccgg tggaaggggg ttctggggct gctg 294
<210> 234
   <211> 55
   <212> DNA
   <213> Homo sapiens
<220>
   <221> misc_feature
   <222> 42
   <223> n = A,T,C or G
<400> 234
   gtctcggtcc atgactctgg agatccgaga aggaagaggc tntggcctga gaaag 55
<210> 235
   <211> 394
   <212> DNA
   <213> Homo sapiens
<220>
   <221> misc_feature
   <222> 22, 335, 365, 377, 383, 391
   <223> n = A,T,C or G
<400> 235
   ttttttgttc atttatattt tntttaagag ctgtgcccag ttttatcatc tcacaagaat 60
   gaagcaaggg acaaaggtaa gtgccacgct ccctggccac tgggttcctg gcaagctccc 120
   agccactagg tgccaatctc ccttcaatgt actccttctt ccccagagtg cagaagcgta 180
   tgaagacagt tatgacatgg acacatgcat gagctattat acataattac aaaagctgat 240
   tctgtcatca ccacatcttg tctcatcagt aggagcgaat ggctggcggg acggtggcac 300
   agtcagcctt gttcaaagtt ttgtcgatca cgggncctat attccagagt gacctttccc 360
   agtgnccaac gttccanata ggncagggtc ntgc 394
<210> 236
   <211> 468
   <212> DNA
   <213> Homo sapiens
<400> 236
   agctcgggat tcggctcgag gacctggaaa ttccaggtgg tgagctgcat cgaaggggag 60
   cctgggcccg tcaggagcgt cctcttcaac ccagacggct gctgcctgta cagcggctgc 120
   caggactcac tgcgtgtcta cggctgggaa cctgagcggt gctttgatgt ggtcctcgtc 180
   aactggggca aggtggccga cctggccatc tgcaatgacc agttgatagg tgtggccttc 240
   tcccagagca acgtctcctc ctacgtggtg gatctgacgc gtgtcaccag gactggcacg 300
   gtggcccggg accctgtgca ggaccaccgg cccctggcac agccactgcc caaccccagc 360
   gcccccctcc ggcgcatcta tgagcggccc agcacaacct gcagcaagcc tcagagggtg 420
   aagcagaact cagagagcga gcgccgcacc cccagcagcg aggatgac                 468
<210> 237
   <211> 254
   <212> DNA
   <213> Homo sapiens
<220>
   <221> misc_feature
   <222> 48, 85, 97
   <223> n = A,T,C or G
<400> 237
   gacctggaga agttccaggt ggtgagctgc atcgaagggg agcctggncc cgtcaggagc 60
   gtcctcttca acccagacgg ctgcngcctg tacagcngct gccaggactc actgcgtgtc 120
   tacggctggg aacctgagcg gtgctttgat gtggtcctcg tcaactgggg caaggtggcc 180
   gacctggcca tctgcaatga ccagttgata ggtgtggcct tctcccagag caacgtctcc 240
   tcctacgtgg tgga                                                   254
<210> 238
   <211> 419
   <212> DNA
   <213> Homo sapiens
<400> 238
   gacccacgcg tccgtcttca acttctttag tcctcctgag attcctatga ttgggaagct 60
   ggaaccacga gaagatgcta tcctggatga ggactttgaa attgggcaga ttttacatga 120
   taatgtcatc ctgaaatcaa tctattacta tactggagaa gtcaatggta cctactatca 180
   atttggcaaa cattatggaa acaagaaata cagaaaataa gtcaatctga aagatttttc 240
   aagaatctta aaatctcaag aagtgaagca gattcataca gccttgaaaa aagtaaaacc 300
   ctgacctgta acctgaacac tattattcct tatagtcaag tttttgtggt ttcttggtag 360
   tctatatttt aaaaatagtc ctaaaaagtg tctaagtgcc agtttattct atctaggct  419
<210> 239
   <211> 228
   <212> DNA
   <213> Homo sapiens
<220>
   <221> misc_feature
   <222> 190
   <223> n = A,T,C or G
<400> 239
   gaaccccgcc cgcggccaca gcgtctgctc cacctccagc ttgtacctgc aggatctgag 60
   cgccgccgcc tcagagtgca tcgacccctc ggtggtcttc ccctaccctc tcaacgacag 120
   cagctcgccc aagtcctgcg cctcgcaaga ctccagcgcc ttctctccgt cctcggattc 180
   tctgcactcn tcgacggagt cctccccgca gggcagcccc gagcccct              228
<210> 240
   <211> 525
   <212> DNA
   <213> Homo sapiens

<400> 240
   aaccccgccc gcggccacag cgtctgctcc acctccagct tgtacctgca ggatctgagc 60
   gccgccgcct cagagtgcat cgacccctcg gtggtcttcc cctaccctct caacgacagc 120
   agctcgccca agtcctgcgc ctcgcaagac tccagcgcct tctctccgtc ctcggattct 180
   ctgctctcct cgacggagtc ctccccgcag ggcagccccg agcccctggt gctccatgag 240
   gagacaccgc ccaccaccag cagcgactct gaggaggaac aagaagatga ggaagaaatc 300
   gatgttgttt ctgtggaaaa gaggcaggct cctggcaaaa ggtcagagtc tggatcacct 360
   tctgctggag gccacagcaa acctcctcac agcccactgg tcctcaagag gtgccacgtc 420
   tccacacatc agcacaacta cgcagcgcct ccctccactc ggaaggacta tcctgctgcc 480
   aagagggtca agttggacag tgtcagagtc ctgagacaga tcagc                525
<210> 241
   <211> 552
   <212> DNA
   <213> Homo sapiens
<400> 241
   tggaaggaac tggtctgctc acacttgctg gcttgcgcat caggactggc tttatctcct 60
   gactcacggt gcaaaggtgc actctgcgaa cgttaagtcc gtcccagcgc ttggaatcct 120
   acggccccca cagccggatc ccctcagcct tccaggtcct caactcccgc ggacgctgaa 180
   caatggcctc catggggcta caggtaatgg gcatcgcgct ggccgtcctg ggctggctgg 240
   ccgtcatgct gtgctgcgcg ctgcccatgt ggcgcgtgac ggccttcatc ggcagcaaca 300
   ttgtcacctc gcagaccatc tgggagggcc tatggatgaa ctgcgtggtg cagagcaccg 360
   gccagatgca gtgcaaggtg tacgacttgc tgctggcact gccgcaggac ctgcaggcgg 420
   cccgcgccct cgtcatcatc agcatcatcg tggctgctct gggcgtgctg ctgtccgtgg 480
   tggggggcaa gtgtaccaac tgcctggagg atgaaagcgc caaggccaag accatgatcg 540
   tggcgggcgt gg                                                     552
<210> 242
   <211> 519
   <212> DNA
   <213> Homo sapiens
<400> 242
   tggaaggaac tggtctgctc acacttgctg gcttgcgcat caggactggc tttatctcct 60
   gactcacggt gcaaaggtgc actctgcgaa cgttaagtcc gtcccagcgc ttggaatcct 120
   acggccccca cagccggatc ccctcagcct tccaggtcct caactcccgc ggacgctgaa 180
   caatggcctc catggggcta caggtaatgg gcatcgcgct ggccgtcctg ggctggctgg 240
   ccgtcatgct gtgctgcgcg ctgcccatgt ggcgcgtgac ggccttcatc ggcagcaaca 300
   ttgtcacctc gcagaccatc tgggagggcc tatggatgaa ctgcgtggtg cagagcaccg 360
   gccagatgca gtgcaaggtg tacgacttgc tgctggcact gccgcaggac ctgcaggcgg 420
   cccgcgccct cgtcatcatc agcatcatcg tggctgctct gggcgtgctg ctgtccgtgg 480
   tggggggcaa gtgtaccaac tgcctggagg atgaaagcg 519
<210> 243
   <211> 296
   <212> DNA
   <213> Homo sapiens
<220>
   <221> misc_feature
   <222> 64, 187, 195
   <223> n = A,T,C or G
<400> 243
   aggttcctca tctgctcgcg aggatgcctt ttctcttctg ccttgcgaaa taacagcagc 60
   ctanctgttg cccgtgacca gtgagaaagg cagcgtcacg ggctgattag gtttcaccca 120
   aagggtgccg gcgccgaatt ggtttctaac gagaactttt aaaatgatcc gttccaaaaa 180
   agggtangag ccgcnagacc ctccaactgc ccagagaaaa caagtctcgt ctggcaaaat 240
   tctcggccca cgcggtccgc ggccaagggg caaaggtcct cgccccacgt tgccga 296
<210> 244
   <211> 273
   <212> DNA
   <213> Homo sapiens
<400> 244
   cttgcccatg gcgaattgtg gatgactgtg gtggggcctt tacgatgggt accattggtg 60
   gtggtatctt tcaagcaatc aaaggttttc gcaattctcc agtgggagta aaccacagac 120
   tacgagggag tttgacagct attaaaacca gggctccaca gttaggaggt agctttgcag 180
   tttggggagg gctgttttcc atgattgact gtagtatggt tcaagtcaga ggaaaggaag 240
   atccctggaa ctccatcaca agtggtgcct taa 273
<210> 245
   <211> 386
   <212> DNA
   <213> Homo sapiens
<400> 245
   ttcgaattcg gcacgaggct cgatgtacgt cccggaggac ctccttcccg tctacaaaga 60
   aaaagtggtg ccgcttgcag acattatcac gcccaaccag tttgaggccg agttactgag 120
   tggccggaag atccacagcc aggaggaagc cttgcgggtg atggacatgc tgcactctat 180
   gggccccgac accgtggtca tcaccagctc cgacctgccc tccccgcagg gcagcaacta 240
   cctgattgtg ctggggagtc agaggaggag gaatcccgct ggctccgtgg tgatggaacg 300
   catccggatg gacattcgca aagtggacgc cgtctttgtg ggcactgggg acctgtttgc 360
   tggcatgctc ctggcgtgga cacaca 386
<210> 246
   <211> 239
   <212> DNA
   <213> Homo sapiens
<400> 246
   tttttttttt caaaaaagtc atggaggcca tggggttggc ttgaaaccag ctttgggggg 60
   ttcgattcct tccttttttg cctaaatttt atgtatacgg gttcttcaaa tgtgtggtag 120
   ggtggggggc atccatatag tcactccagg tttatggagg gttcttctac tattaggact 180
   tttcgcttca aagcgaaggc ttctcaaatc atgaaaatta ttaatattac tgctgttaa 239
<210> 247
   <211> 623
   <212> DNA
   <213> Homo sapiens
<400> 247
   aaaaagtcat ggaggccatg gggttggctt gaaaccagct ttggggggtt cgattccttc 60
   cttttttgtc tagattttat gtatacgggt tcttcgaatg tgtggtaggg tggggggcat 120
   ccatatagtc actccaggtt tatggagggt tcttctacta ttaggacttt tcgcttcgaa 180
   gcgaaggctt ctcaaatcat gaaaattatt aatattactg ctgttagaga aatgaatgag 240
   cctacagatg ataggatgtt tcatgtggtg tatgcatcgg ggtagtccga gtaacgtcgg 300
   ggcattccgg ataggccgag aaagtgttgt gggaagaaag ttagatttac gccgatgaat 360
   atgatagtga aatggatttt ggcgtaggtt tggtctaggg tgtagcctga gaatagggga 420
   aatcagtgaa tgaagcctcc tatgatggca aatacagctc ctattgatag gacatagtgg 480
   aagtgagcta caacgtagta cgtgtcgtgt agtacgatgt ctagtgatga gtttgctaat 540
   acaatgccag tcaggccacc tacggtgaaa agaaagatga atcctagggc tcagagcact 600
   gcagcagatc atttcatatt gct 623
<210> 248
   <211> 265
   <212> DNA
   <213> Homo sapiens
<400> 248
   ggcttagcgg ataacaattt cacacaggag ttgcaccata atcatcgcta tccccaccgg 60
   cgtcaaagta tttagctgac tcgccacact ccacggaagc aatatgaaat gatctgctgc 120
   agtgctctga gccctaggat tcatctttct tttcaccgta ggtggcctga ctggcattgt 180
   attagcaaac tcatcactag acatcgtact acacgacacg tactacgttg tagctcactt 240
   ccactatgtc ctatcaatag gagct 265
<210> 249
   <211> 625
   <212> DNA
   <213> Homo sapiens
<400> 249
   aatcatcgct atccccaccg gcgtcaaagt atttagctga ctcgccacac tccacggaag 60
   caatatgaaa tgatctgctg cagtgctctg agccctagga ttcatctttc ttttcaccgt 120
   aggtggcctg actggcattg tattagcaaa ctcatcacta gacatcgtac tacacgacac 180
   gtactacgtt gtagctcact tccactatgt cctatcaata ggagctgtat ttgccatcat 240
   aggaggcttc attcactgat ttcccctatt ctcaggctac accctagacc aaacctacgc 300
   caaaatccat ttcactatca tattcatcgg cgtaaatcta actttcttcc cacaacactt 360
   tctcggccta tccggaatgc cccgacgtta ctcggactac cccgatgcat acaccacatg 420
   aaacatccta tcatctgtag gctcattcat ttctctaaca gcagtaatat taataatttt 480
   catgatttga gaagccttcg cttcgaagcg aaaagtccta atagtagaag aaccctccat 540
   aaacctggag tgactatatg gatgcccccc accctaccac acattcgaag aacccgtata 600
   cataaaatct agacaaaaaa ggaag                                       625
<210> 250
   <211> 253
   <212> DNA
   <213> Homo sapiens
<400> 250
   ggcttgtaat acgactcact atagggcttt ttttttttca aaaaagtcat ggaggccatg 60
   gggttggctt gaaaccagct ttggggggtt cgattccttc cttttttgtc taaattttat 120
   gtatacgggt tcttcaaatg tgtggtaggg tggggggcat ccatatagtc actccaggtt 180
   tatggagggt tcttctacta ttaggacttt tcgcttcaaa gcgaaggctt ctcaaatcat 240
   gaaaattatt aat                                                    253
<210> 251
   <211> 290
   <212> DNA
   <213> Homo sapiens
<400> 251
   caaactcatc actagacatc gtactacacg acacgtacta cgttgtagct cacttccact 60
   atgtcctatc aataggagct gtatttgcca tcataggagg cgtcattcac tgatttcccc 120
   tattctcagg ctacacccta gaccaaacct acgccaaaat ccatttcact atcatattca 180
   tcggcgtaaa tctaactttc ttcccacaac actttctcgg cctatccgga atgccccgac 240
   gttattcgga ctaccccgat gcatacacca catgaaacat cctatcatct           290
<210> 252
   <211> 638
   <212> DNA
   <213> Homo sapiens
<220>
   <221> misc_feature
   <222> 522, 634, 636
   <223> n = A,T,C or G
<400> 252
   atatttacag taggaataga cgtagacaca cgagcatatt tcacctccgc taccataatc 60
   atcgctatcc ccaccggcgt caaagtattt agctgactcg ccacactcca cggaagcaat 120
   atgaaatgat ctgctgcagt gctctgagcc ctaggattca tctttctttt caccgtaggt 180
   ggcctgactg gcattgtatt agcaaactca tcactagaca tcgtactaca cgacacgtac 240
   tacgttgtag ctcacttcca ctatgtccta tcaataggag ctgtatttgc catcatagga 300
   ggcttcattc actgatttcc cctattctca ggctacaccc tagaccaaac ctacgccaaa 360
   atccatttca ctatcatatt catcggcgta aatctaactt tcttcccaca acactttctc 420
   ggcctatccg gaatgccccg acgttattcg gactaccccg atgcatacac cacatgaaac 480
   atcctatcat ctgtaggctc attcatttct ctaacagcag tnatattaat aattttcatg 540
   atttgagaag ccttcgcttc gaagcgaaaa gtcctaatag tagaagaacc cttcataaac 600
   ctggagtgac tatatggatg ccccccaccc tacnanca 638
<210> 253
   <211> 531
   <212> DNA
   <213> Homo sapiens
<400> 253
   ggcttagcgg ataacaattt cacacaggag ttgcaccata tatttacagt aggaatagac 60
   gtagacacac gagcatattt cacctccgct accataatca tcgctatccc caccggcgtc 120
   aaagtattta gctgactcgc cacactccac ggaagcaata tgaaatgatc tgctgcagtg 180
   ctctgagccc taggattcat ctttcttttc accgtaggtg gcctgactgg cattgtatta 240
   gcaaactcat cactagacat cgtactacac gacacgtact acgttgtagc tcacttccac 300
   tatgtcctat caataggagc tgtatttgcc atcataggag gcttcattca ctgatttccc 360
   ctattctcag gctacaccct agaccaaacc tacgccaaaa tccatttcac tatcatattc 420
   atcggcgtaa atctaacttt cttcccacaa cactttctcg gcctatccgg aatgccccga 480
   cgttactcgg actaccccga tgcatacacc acatgaaaca tcctatcatc t 531
<210> 254
   <211> 625
   <212> DNA
   <213> Homo sapiens
<400> 254
   atatttacag taggaataga cgtagacaca cgagcatatt tcacctccgc taccataatc 60
   atcgctatcc ccaccggcgt caaagtattt agctgactcg ccacactcca cggaagcaat 120
   atgaaatgat ctgctgcagt gctctgagcc ctaggattca tctttctttt caccgtaggt 180
   ggcctgactg gcattgtatt agcaaactca tcactagaca tcgtactaca cgacacgtac 240
   tacgttgtag ctcacttcca ctatgtccta tcaataggag ctgtatttgc catcatagga 300
   ggcttcattc actgatttcc cctattctca ggctacaccc tagaccaaac ctacgccaaa 360
   atccatttca ctatcatatt catcggcgta aatctaactt tcttcccaca acactttctc 420
   ggcctatccg gaatgccccg acgttactcg gactaccccg atgcatacac cacatgaaac 480
   atcctatcat ctgtaggctc attcatttct ctaacagcag taatattaat aattttcatg 540
   atttgagaag tcttcgcttc gaagcgaaaa gtcctaatag tagaagaacc cttcataaac 600
   ctggagtgac tatatggatg ccccc 625
<210> 255
   <211> 217
   <212> DNA
   <213> Homo sapiens
<400> 255
   tttttttttt taaaaagtca tggaggccat ggggttggct tgaaaccacc tttggggggt 60
   tcaatccctt ccttctttgt ctaaatttta tgtatacggg ttcttcaaat gtgtggtagg 120
   ggggggggca tccatatagc ccctccaggt ttatggaggg ttcttctact attagaactt 180
   ttcccttcaa agcaaaggct tctcaaatca tgaaaat 217
<210> 256
   <211> 636
   <212> DNA
   <213> Homo sapiens
<220>
   <221> misc_feature
   <222> 496, 562, 564, 605, 635
   <223> n = A,T,C or G
<400> 256
   aaagtcatgg aggccatggg gttggcttga aaccagcttt ggggggttcg attccttcct 60
   tctttgtcta gattttatgt atacgggttc ttcgaatgtg tggtagggtg gggggcatcc 120
   atatagtcac tccaggttta tggagggttc ttctactatt aggacttttc gcttcgaagc 180
   gaaggcttct caaatcatga aaattattaa tattactgct gttagagaaa tgaatgagcc 240
   tacagatgat aggatgtttc atgtggtgta tgcatcgggg tagtccgagt aacgtcgggg 300
   cattccggat aggccgagaa agtgttgtgg gaagaaagtt agatttacgc cgatgaatat 360
   gatagtgaaa tggattttgg cgtaggtttg gtctagggtg tagcctgaga ataggggaaa 420
   tcagtgaatg aagcctccta tgatggcaaa tacagctcct attgatagga catagtggaa 480
   gtgagctaca acgtantacg tgtcgtgtag tacgatgtct agtgatgagt ttgctaatac 540
   aatgccagtc aggccaccta cngngaaaaa gaaagatgaa tcctagggct caaaacacct 600
   gcacnagatc atttcatatt ggcttccgtg gagtnc 636
<210> 257
   <211> 279
   <212> DNA
   <213> Homo sapiens
<400> 257
   ggcttagcgg ataacaattt cacacaggag ttgcaccata atcatcgcta tccccaccgg 60
   cgtcaaagta tttagctgac tcgccacact ccacggaagc aatatgaaat gatctgctgc 120
   agtgctctga gccctaggat tcatctttct tttcaccgta ggtggcctga ctggcattgt 180
   attagcaaac tcatcactag acatcgtact acacgacacg tactacgttg tagctcactt 240
   ccactatgtc ctatcaatag gagctgtatt tgccatcat 279
<210> 258
   <211> 623
   <212> DNA
   <213> Homo sapiens
<220>
   <221> misc_feature
   <222> 537
   <223> n = A,T,C or G
<400> 258
   aatcatcgct atccccaccg gcgtcaaagt atttagctga ctcgccacac tccacggaag 60
   caatatgaaa tgatctgctg cagtgctctg agccctagga ttcatctttc ttttcaccgt 120
   aggtggcctg actggcattg tattagcaaa ctcatcacta gacatcgtac tacacgacac 180
   gtactacgtt gtagctcact tccactatgt cctatcaata ggagctgtat ttgccatcat 240
   aggaggcttc attcactgat ttcccctatt ctcaggctac accctagacc aaacctacgc 300
   caaaatccat ttcactatca tattcatcgg cgtaaatcta actttcttcc cacaacactt 360
   tctcggccta tccggaatgc cccgacgtta ctcggactac cccgatgcat acaccacatg 420
   aaacatccta tcatctgtag gctcattcat ttctctaaca gcagtaatat taataatttt 480
   catgatttga gaagccttcg cttcgaagcg aaaagtccta atagtagaag aaccctncat 540
   aaacctggag tgactatatg gatgcccccc accctaccac acattcgaag aacccgtata 600
   cataaaatct agacaaaaaa gga 623
<210> 259
   <211> 189
   <212> DNA
   <213> Homo sapiens
<220>
   <221> misc_feature
   <222> 170; 173
   <223> n = A,T,C or G
<400> 259
   tggcctttcc cccttcatgg gagacaacga taacgaaacc ttggccaacg ttacctcagc 60
   cacctgggac ttcgacgacg aggcattcga tgagatctcc gacgatgcca aggatttcat 120
   cagcaatctg ctgaagaaag atatgaaaaa ccgcctggac tgcacgcagn ctntcagcat 180
   ccatggcta 189
<210> 260
   <211> 507
   <212> DNA
   <213> Homo sapiens
<400> 260
   cctttccccc ttcatgggag acaacgataa cgaaaccttg gccaacgtta cctcagccac 60
   ctgggacttc gacgacgagg cattcgatga gatctccgac gatgccaagg atttcatcag 120
   caatctgctg aagaaagata tgaaaaaccg cctggactgc acgcagtgcc ttcagcatcc 180
   atggctaatg aaagatacca agaacatgga ggccaagaaa ctctccaagg accggatgaa 240
   gaagtacatg gcaagaagga aatggcagaa aacgggcaat gctgtgagag ccattggaag 300
   actgtcctct atggcaatga tctcagggct cagtggcagg aaatcctcaa cagggtcacc 360
   aaccagcccg ctcaatgcag aaaaactaga atctgaagaa gatgtgtccc aagctttcct 420
   tgaggctgtt gctgaggaaa agcctcatgt aaaaccctat ttctctaaga ccattcgcga 480
   tttagaagtt gtggagggaa gtgctgc 507
<210> 261
   <211> 193
   <212> DNA
   <213> Homo sapiens
<400> 261
   tttttttttt tttttttttt ttttttggcc gagactccaa gactattatt tttatttccg 60
   gacaaaaaca tctgcttcac acagtgcacg gcatcaaatg aagaggaaag aacttgtatc 120
   ccaaagcctg gctttctgta tcatccacaa attaagacag catctgctga gcccatgctg 180
   agcctgtcac agt                                                    193
<210> 262
   <211> 235
   <212> DNA
   <213> Homo sapiens
<220>
   <221> misc_feature
   <222> 183, 184, 185, 193
   <223> n = A,T,C or G
<400> 262
   cccacttccc caggagcagg ccacagaccc ccttgtggac agcctgggca gtggcattgt 60
   ctactcagcc cttacctgcc acctgtgcgg ccacctgaaa cagtgtcatg gccaggagga 120
   tggtggccag acccctgtca tggccagtcc ttgctgtggc tgctgctgtg gagacaggtc 180
   ctnnncccct acnacccccc tgagggcccc agacccctct ccaggtgggg ttcca      235
<210> 263
   <211> 493
   <212> DNA
   <213> Homo sapiens
<400> 263
   agaatttcag cagttctctg atttttatat tttattcctc ttcctatcca atccctgcct 60
   tttgagtcca ggtggtaagt acattttctt taacgttttt cctgcttttc ttcccaaatg 120
   tgtctttttc tttgggctac tgtaccctgc ttccagtgct gtccccggca taggtccatc 180
   tctgcagaag ccatttcagg agtacctgga ggctcaacgg cagaagcttc accacaaaag 240
   cgaaatgggc acaccacagg gagaaaactg cttgtcctgg atgtttgaaa agtcggtcga 300
   tgtcatggtg tgttacttca tcctatctat cattaactcc atggcacaaa gttatgccaa 360
   acgaatccag cagcggttga actcagagga gaaaactaaa taagtagaga aagttttaaa 420
   ctgcagaaat tggagtggat gggttctgcc ttatattggg aggactccaa gccgggaagg 480
   aaaattccct ttt                                                    493
<210> 264
   <211> 345
   <212> DNA
   <213> Homo sapiens
<400> 264
   agaatttcag cagttctctg atttttatat tttattcctc ttcctatcca atccctgcct 60
   tttgagtcca ggtggtaagt acattttctt taacgttttt cctgcttttc ttcccaaatg 120
   tgtctttttc tttgggctac tgtaccctgc ttccagtgct gtccccggca taggtccatc 180
   tctgcagaag ccatttcagg agtacctgga ggctcaacgg cagaagcttc accacaaaag 240
   cgaaatgggc acaccacagg gagaaaactg gttgtcctgg atgtttgaaa agtcggtcgt 300
   tgtcatggtg tgttacttca tcctatctat cattaactcc atggt                 345
<210> 265
   <211> 374
   <212> DNA
   <213> Homo sapiens
<400> 265
   tagaagagct aacctcacac tcatcccact ctaaactatg tgattcaaca ctgattttac 60
   atccaacaaa gtgaaatctt gatagttggg tgtaaaaagg agagtaatgg agatttcaga 120
   gtagttgggg ttgcttactt ttcattttta attctttagg ttttgtaagt tacacacttc 180
   aagcattata gatgatcctc tttttactac tgaactaatg aagccttttt cattgcattg 240
   ttctgcattt atttctacag ggagaaaact ggttgtcctg gatgtttgaa aagttggtcg 300
   ttgtcatggt gtgttacttc atcctatcta tcattaactc catggcacaa agttatgcca 360
   aacgaatcca gcag                                                   374
<210> 266
   <211> 360
   <212> DNA
   <213> Homo sapiens
<400> 266
   tttttttttt tttttttttg tgcggtggga attctctaat tgtatcatgt gggccttttg 60
   aaagtaacaa acagaaggcc agtctgctgc aagtttgctg ctgaacatca cattccaccc 120
   taagaaaaca caaggtggat tgcatcgagg gtggatacct taccttagca cggaaggaaa 180
   aagtatgtca gtgcaaagta tggactaaac tgctttcagg aaaaaagttg taaaaattga 240
   tacaggttgg aaaagggaat tttccttccc ggcttggagt cctcccaatt taaggcagaa 300
   cccatccact ccaatttctg cagtttaaaa ctttctctac ttatttagtt ttctcctctg 360
<210> 267
   <211> 247
   <212> DNA
   <213> Homo sapiens
<400> 267
   ctggaattgt catctttgga acagtgattg caacagcact tatgggattg acagagaaac 60
   tgattttttc cctgagagat cctgcataca gtacattccc gccagaaggt gttttcgtaa 120
   atacgcttgg ccttctgatc ctggtgttcg gggccctcat tttttggata gtcaccagac 180
   cgcaatggaa acgtcctaag gagccaaatt ctaccattct tcatccaaat ggaggcatga 240
   acaggga                                                           247
<210> 268
   <211> 350
   <212> DNA
   <213> Homo sapiens
<400> 268
   taatggattt gtttggagat ggcatgttgg tagacgactg aatatggaaa ggatatcaag 60
   ttatctattt tgttaatttt atttttgttt tttatcatct agatttttat catggattag 120
   tctgaaattt aaagttctgg ccagtcggtt ttctttcatc ttgtagtttt tacagtattt 180
   ccactgtgca tatgcaaaat gggtattaca taactgtatc atatttggta ttgataattt 240
   tttttttttt ttggaaacgg gtttttgttt tggcccagcc caaaaacatc ccttggttac 300
   cccttccggg gaaaaaaaac caaacccctt tttcggggaa aaaaaaaggg 350
<210> 269
   <211> 455
   <212> DNA
   <213> Homo sapiens
<220>
   <221> misc_feature
   <222> 81, 195, 231, 247, 298, 307, 317, 395, 427, 446, 451
   <223> n = A,T,C or G
<400> 269
   ttttttttaa atcaaagagt agtttattaa aaaaggaatc aaacaggaaa ctctaagtac 60
   cagtgtgtac attgtacaat nttaaatgac tcacgagaat gaagtttttt tcaaatatat 120
   taagatcaca ccaccttgtt gtttatcgaa agatattcaa ggagaaagat ctgactctcc 180
   aaactgcatc tgagnattgc cactttaaac aggacctcat ttcaaacatg ncaacaacgc 240
   cactggntaa taaaggcttt gggaatgggg tgctcattct attatttcac tacaaacngc 300
   atagganagg caggagnagt tggggaattt attctaaaat aggaatggga gggttgtcca 360
   tctacagcag gcactccttc acttcctctg tttgnccttt ttaggcagta ctccttggtc 420
   ggtcttngaa cggttttcca accctnttca ntggg 455
<210> 270
   <211> 444
   <212> DNA
   <213> Homo sapiens
<220>
   <221> misc_feature
   <222> 17, 20, 391, 430
   <223> n = A,T,C or G
<400> 270
   ttttctgacg tctgttnctn aggctggaag aaatgagcag aaaacaaggg atgagtactt 60
   tttagagtat gtgcatgtta cgtaatacct gtttctgggc aatgctgctt cttctgactc 120
   aacaaatggg gagagcaaat tgaaaatgcg taaattggaa ggcaagttct gaaattaaac 180
   gttgtacttt ggcctgatgt tctgaccttt aaggaagcaa gagtttgtaa acttccaaat 240
   atttactatt ctgaactgcc gtgtaaacct gacgtattcc caagtcaaca taccagtata 300
   ccaataggat gtgaataatg tttgtgttga gtttaaaacc atagcagttt tgctctggca 360
   agtaatggaa agcgttctcg cttcctgagt ntgagctcca gcagactgca gagtggccag 420
   tgccacagtn gtagcctgac tttc 444
<210> 271
   <211> 502
   <212> DNA
   <213> Homo sapiens
<400> 271
   ggttctgcgc tggtcggcgg agtagcaagt ggccatgggg agcctcagcg gtctgcgcct 60
   ggcagcagga agctgtttta ggttatgtga aagagatgtt tcctcatctc taaggcttac 120
   cagaagctct gatttgaaga gaataaatgg attttgcaca aaaccacagg aaagtcccgg 180
   agctccatcc cgcacttaca acagagtgcc tttacacaaa cctacggatt ggcagaaaaa 240
   gatcctcata tggtcaggtc gcttcaaaaa ggaaggtgaa atcccagaga ctgtctcgtt 300
   ggagatgctt gatgctgcaa agaacaagat gcgagtgaag atcagctatc taatgattgc 360
   cctgacggtg gtaggatgca tcttcatggt tattgagggc aagaaggctg cccaaagaca 420
   cgagacttta acaagcttga acttagaaaa gaaagctcgt ctgaaagagg aagcagctat 480
   gaaggccaaa acagagtagc ag                                          502
<210> 272
   <211> 377
   <212> DNA
   <213> Homo sapiens
<400> 272
   ggttctgcgc tggtcggcgg agtagcaagt ggccatgggg agcctcagcg gtctgcgcct 60
   ggcagcagga agctgtttta ggttatgtga aagagatgtt tcctcatctc taaggcttac 120
   cagaagctct gatttgaaga gaataaatgg attttgcaca aaaccacagg aaagtcccgg 180
   agctccatcc cgcacttaca acagagtgcc tttacacaaa cctacggatt ggcagaaaaa 240
   gatcctcata tggtcaggtc gcttcaaaaa ggaaggtgaa atcccagaga ctgtctcgtt 300
   ggagatgctt gatgctgcaa agaacaagat gcgagtgaag atcagctatc taatgattgc 360
   cctgacggtg gtaggaa                                                377
<210> 273
   <211> 552
   <212> DNA
   <213> Homo sapiens
<400> 273
   agctcggaat tcggctcgag tctgctcagc ctggtgaacc cacaggcccg agtttcaccc 60
   agtccccact ccacggtgca gctgcggctt atctctcagc ccagcgagat gccagccttc 120
   ctgtcccggg ccagcgctct gacatgcaga aggtgaccct gggcctgctt gtgttcctgg 180
   caggctttcc tgtcctggac gccaatgacc tagaagataa aaacagtcct ttctactatg 240
   actggcacag cctccaggtt ggcgggctca tctgcgctgg ggttctgtgc gccatgggca 300
   tcatcatcgt catgagtgca aaatgcaaat gcaagtttgg ccagaagtcc ggtcaccatc 360
   caggggagac tccacctctc atcaccccag gctcagccca aagctgatga ggacagacca 420
   gctgaaattg ggtggaggac cgttctctgt ccccaggtcc tgtctctgca cagaaacttg 480
   aactccagga tggaattctt cctcctctgc tgggactcct ttgcatggca gggcctcatc 540
   tcacctctcg ca                                                     552
<210> 274
   <211> 186
   <212> DNA
   <213> Homo sapiens
<400> 274
   ctgctcagcc tggtgaacac acagcccgat ttacccagtc cccactccag gtgcagctgc 60
   ggcttatctc tcagcccagc gagatgccag ccttcctgtc ccgggccagc gctctgacat 120
   gcagaaggtg accctgggcc tgcttgtgtt cctggcaggc tttcctgtcc tggacgccaa 180
   tgacct                                                            186
<210> 275
   <211> 121
   <212> DNA
   <213> Homo sapiens
<400> 275
   tctgctcagc ctggtgaacc acacaggccc gagtttcacc cagtccccac tccacggtgc 60
   agctgcggct tatctctcag cccagcgaga tgccagcctt cctgtcccgg gccagcgctc 120
   t 121
<210> 276
   <211> 336
   <212> DNA
   <213> Homo sapiens
<220>
   <221> misc_feature
   <222> 336
   <223> n = A,T,C or G
<400> 276
   agggacccgc agctcagcta cagcacagat cagcaccatg aagcttctca cgggcctggt 60
   tttctgctcc ttggtcctga gtgtcagcag ccgaagcttc ttttcgttcc ttggcgaggc 120
   ttttgatggg gctcgggaca tgtggagagc ctactctgac atgagagaag ccaattacat 180
   cggctcagac aaatacttcc atgctcgggg gaactatgat gctgccaaaa ggggacctgg 240
   gggtgcctgg gccgcagaag tgatcagcaa tgccagagag aatatccaga gactcacagg 300
   ccatggtgcg gaggactcgc tggccgatca ggctgn 336
<210> 277
   <211> 460
   <212> DNA
   <213> Homo sapiens
<400> 277
   tgcagacgga ggtcaggtct tcctctttcc tgagactgga tctgttcaaa cagcaaacgc 60
   ccacagatgg cccagaggtg gtggtagtca gggtgtgtgg gtgtttttag ggttctttag 120
   tgttgtttct ttcacccagg ggtggtggtc ccagccagtt tggtgctgac ggtgagagga 180
   aattagaatc tgtttgcaaa ttgtccaacc caccccctca acatgagggg cttccatttt 240
   ctgtgttttg taagggaact gtttccttca tgccgccatg ttcctgatat tagttctgat 300
   ttctttttaa caaatgttat catgattaag aaaatttcca gcactttaat ggccaattaa 360
   ctgagaatgt aagaaaattg atgctgtaca aggcaaataa agctgtttat taaccttgaa 420
   aaaaaaaaaa aaaaaaaaaa aaaaaaaaaa tttttggggg 460
<210> 278
   <211> 432
   <212> DNA
   <213> Homo sapiens
<220>
   <221> misc_feature
   <222> 46, 151, 350, 362, 383, 403, 417
   <223> n = A,T,C or G
<400> 278
   ggggttgcag acggaggtca ggtcttcctc tttcctgaga ctgganctgt tcaaacagca 60
   aacgcccaca gatggcccag aggtggtggt agtcagggtg tgtgggtgtt tttagggttc 120
   tttagtgttg tttctttcac ccaggggtgg ntggtcccag ccagtttggt gctgacggtg 180
   agaggaaatt agaatctgtt tgcaaattgt ccaacccacc ccctcaacat gaggggcttc 240
   cattttctgt gttttgtaag ggaactgttt ccttcatgcc gccatgttcc tgatattagt 300
   tctgatttct ttttaacaaa tgttatcatg attaagaaaa tttccagcan ttaatgggcc 360
   anttaactga gaatgtaaga aantgatgct gttacaaggc aantaaagcc gttttantta 420
   accctgaaaa aa                                                     432
<210> 279
   <211> 467
   <212> DNA
   <213> Homo sapiens
<400> 279
   acgtgacgcg gggccaggcg gccgtacagc agctgcaggc ggagggcctg agcccgcgct 60
   tccaccagct ggacatcgac gatctgcaga gcatccgcgc cctgcgcgac ttcctgcgca 120
   aggagtacgg gggcctggac gtgctggtca acaacacggg catcgccttc aaggttgctg 180
   atcccacacc ctttcatatt caagctgaag tgacgatgaa aacaaatttc tctggtaccc 240
   gagatgtgtg cacagaatta ctccctctaa taaaacccca agggagagtg gtgaacgtac 300
   ctagcatcat gagcgtcaga gcccttaaaa gctgcagccc agagctgcag cagaagttcc 360
   gcagtgagac catcactgag gaggagctgg tggggctcat gaacaagttt gtggaggata 420
   caaagaaggg agtgcaccag aaggagggct ggcccagcag cgcatac               467
<210> 280
   <211> 626
   <212> DNA
   <213> Homo sapiens

<400> 280
   tacggccggg acgtgacgcg gggccaggcg gccgtacagc agctgcaggc ggagggcctg 60
   agcccgcgct tccaccagct ggacatcgac gatctgcaga gcatccgcgc cctgcgcgac 120
   ttcctgcgca aggagtacgg gggcctggac gtgctggtca acaacacggg catcgccttc 180
   aaggttgctg atcccacacc ctttcatatt caagctgaag tgacgatgaa aacaaatttc 240
   tctggtaccc gagatgtgtg cacagaatta ctccctctaa taaaacccca agggagagtg 300
   gtgaacgtac ctagcatcat gagcgtcaga gcccttaaaa gctgcagccc agagctgcag 360
   cagaagttcc gcagtgagac catcactgag gaggagctgg tggggctcat gaacaagttt 420
   gtggaggata caaagaaggg agtgcaccag aaggagggct ggcccagcag cgcatacggg 480
   gtgacgaaga ttggcgtcac cgttctgtcc aggatccacg ccaggaaact gagtgagcag 540
   aggaaagggg acaagatcct cctgaatgcc tgctgcccag ggtgggtgag aactgacatg 600
   gcgggaccca aggccaccaa gagccc                                      626
<210> 281
   <211> 487
   <212> DNA
   <213> Homo sapiens
<400> 281
   tggcctgttc ctcagcgagg gcctgaagct agtggataag tttttggagg atgttaaaaa 60
   gttgtaccac tcagaagcct tcactgtcaa cttcggggac accgaagagg ccaagaaaca 120
   gatcaacgat tacgtggaga agggtactca agggaaaatt gtggatttgg tcaaggagct 180
   tgacagagac acagtttttg ccctggtgaa ttacatcttc tttaaaggca aatgggagag 240
   accctttgaa gtcaaggaca ccgaggaaga ggacttccac gtggaccagg cgaccaccgt 300
   gaaggtgcct atgatgaagc gtttaggcat gtttaacatc cagcactgta agaagctgtc 360
   cagctgggtg ctgctgatga aatacctggg caatgccacc gccatcttct tcctgcctga 420
   tgaggggaaa ctacagcacc tggaaaatga actcacccac gatatcatca ccaagttcct 480
   ggaaaat                                                           487
<210> 282
   <211> 345
   <212> DNA
   <213> Homo sapiens
<400> 282
   tggcctgttc ctcagcgagg gcctgaagct agtggataag tttttggagg atgttaaaaa 60
   gttgtaccac tcagaagcct tcactgtcaa cttcggggac accgaagagg ccaagaaaca 120
   gatcaacgat tacgtggaga agggtactca agggaaaatt gtggatttgg tcaaggagct 180
   tgacagagac acagtttttg ccctggtgaa ttacatcttc tttaaaggca aatgggagag 240
   accctttgaa gtcaaggaca ccgaggaaga ggacttccac gtggaccagg cgaccaccgt 300
   gaaggtgcct atgatgaagc gtttaggcat gtttaacatc cagca                 345
<210> 283
   <211> 495
   <212> DNA
   <213> Homo sapiens
<400> 283
   cggccgccct tttttttttt tttttttttt tttttttttt tttttttttt tttttttttt 60
   tttttttttc aaaaaaaaat ttttttgggt tttttttttt aaaacttttt tttttttttt 120
   ttttgggggg ggccaaattc ccccccaaaa aaaaaaaaaa aggggggggt ttcccccccc 180
   cccctttttt tttttggggg ggtttttttt tttggggggg gccccccccc cctttttttt 240
   tttttggaaa aaaatccccc ccttgggggg ggtttctttt tcccaaaggg agtttttttt 300
   cccccccccc cggggggggg ggggggtttt ttttttttta aaaaaaaaac ccccggaaaa 360
   aaaaaaaccc cccccccccc cccccccccc aaaaaaaaaa aaggggggaa aaatgggggc 420
   cccccctttt tttttttttt tttttttggg gggggggaaa aaaaaccccc cccccctttt 480
   tgggggggtt ttttt                                                  495
<210> 284
   <211> 503
   <212> DNA
   <213> Homo sapiens
<400> 284
   attccgttgc tgtcgagcat gaccaagcag ctgggtgact tctggacacg gatggaggag 60
   ctccgccacc aagcccggca gcagggggca gaggcagtcc aggcccagca gcttgcggaa 120
   ggtgccagcg agcaggcatt gagtgcccaa gagggatttg agagaataaa acaaaagtat 180
   gctgagttga aggaccggtt gggtcagagt tccatgctgg gtgagcaggg tgcccggatc 240
   cagagtgtga agacagaggc agaggagctg tttggggaga ccatggagat gatggacagg 300
   atgaaagaca tggagttgga gctgctgcgg ggcagccagg ccatcatgct gcgctcagcg 360
   gacctgacag gactggagaa gcgtgtggag cagatccgtg accacatcaa tgggcgcgtg 420
   ctctactatg ccacctgcaa gtgatgctac agcttccagc ccgttgcccc actcatctgc 480
   cgcctttgct tttggttggg ggc 503
<210> 285
   <211> 581
   <212> DNA
   <213> Homo sapiens
<400> 285
   agtggcactg caggaagctc aggacaccat gcaaggcacc agccgctccc ttcggcttat 60
   ccaggacagg gttgctgagg ttcagcaggt actgcggcca gcagaaaagc tggtgacaag 120
   catgaccaag cagctgggtg acttctggac acggatggag gagctccgcc accaagcccg 180
   gcagcagggg gcagaggcag tccaggccca gcagcttgcg gaaggtgcca gcgagcaggc 240
   attgagtgcc caagagggat ttgagagaat aaaacaaaag tatgctgagt tgaaggaccg 300
   gttgggtcag agttccatgc tgggtgagca gggtgcccgg atccagagtg tgaagacaga 360
   ggcagaggag ctgtttgggg agaccatgga gatgatggac aggatgaaag acatggagtt 420
   ggagctgctg cggggcagcc aggccatcat gctgcgctca gcggacctga caggactgga 480
   gaagcgtgtg gagcagatcc gtgaccacat caatgggcgc gtgctctact atgccacctg 540
   caagtgatgc tacagcttcc agcccgttgc cccactcatc t 581
<210> 286
   <211> 598
   <212> DNA
   <213> Homo sapiens
<400> 286
   agtggcactg caggaagctc aggacaccat gcaaggcacc agccgctccc ttcggcttat 60
   ccaggacagg gttgctgagg ttcagcaggt actgcggcca gcagaaaagc tggtgacaag 120
   catgaccaag cagctgggtg acttctggac acggatggag gagctccgcc accaagcccg 180
   gcagcagggg gcagaggcag tccaggccca gcagcttgcg gaaggtgcca gcgagcaggc 240
   attgagtgcc caagagggat ttgagagaat aaaacaaaag tatgctgagt tgaaggaccg 300
   gttgggtcag agttccatgc tgggtgagca gggtgcccgg atccagagtg tgaagacaga 360
   ggcagaggag ctgtttgggg agaccatgga gatgatggac aggatgaaag acatggagtt 420
   ggagctgctg cggggcagcc aggccatcat gctgcgctca gcggacctga caggactgga 480
   gaagcgtgtg gagcagatcc gtgaccacat caatgggcgc gtgctctact atgccacctg 540
   caagtgatgc tacagcttcc agcccgttgc cccactcatc tgccgccttt gcttttgg 598
<210> 287
   <211> 316
   <212> DNA
   <213> Homo sapiens
<400> 287
   ctgcccttca cctcgcagtg gacctgcaaa atcctgacct ggtgtcactc ctgttgaagt 60
   gtggggctga tgtcaacaga gttacctacc agggctattc tccctaccag ctcacctggg 120
   gccgcccaag cacccggata cagcagcagc tgggccagct gacactagaa aaccttcaga 180
   tgctgccaga gagtgaggat gaggagagct atgacacaga gtcagagttc acggagttca 240
   cagaggacga gctgccctat gatgactgtg tgtttggagg ccagcgtctg acgttatgag 300
   cgcaaagggg ctgaaa                                                 316
<210> 288
   <211> 275
   <212> DNA
   <213> Homo sapiens
<400> 288
   atgattagga gaagtggtgg ccacagtcga aaaatcccaa ggcccaaacc tgcaccactg 60
   actgctgaaa tacagcaaaa gattttgcat ttgccaacat cttgggactg gagaaatgtt 120
   catggtatca attttgtcag tcctgttcga aaccaagcat cctgtggcag ctgctactca 180
   tttgcttcta tgggtatgct agaagcgaga atccgtatac taaccaacaa ttctcagacc 240
   ccaatcctaa gccctcagga ggttgtgtct tgtag                            275
<210> 289
   <211> 522
   <212> DNA
   <213> Homo sapiens
<400> 289
   cagaagggaa caccagagct ttgctaataa ttagtgtggt caagagccgt ctgagcctaa 60
   tgagtcccag ctgcattagg ttaagagact cttccagagc catcgccagg tcgggaatgg 120
   cacctctccc taggatacac agcctgcagg tccccaggac ctggatgaca cccgcctcac 180
   tgtggcagtg tattgcctgt taattgctgc taattctaat tctgatgatg actcctactc 240
   cattgtttac cccaaagcat cagctaggct ggagtgattt gttacaaatg agcaaaagat 300
   gagtccttgc ttccctcaga aataaaagga gctcagctgc agcgttgcat tggcttcttt 360
   ggcctcccaa ctcttcccac tcccagaatc cagaagtaag ctctgcatgt tccccttcct 420
   gggaggaaac cagttgtcag aaggatgtat gatgaccccc tcccctccca tccttcacct 480
   cctaagcagt cctggctttt cctcatcact cccctctaca gt                    522
<210> 290
   <211> 331
   <212> DNA
   <213> Homo sapiens
<400> 290
   aacaccagag ctttgctaat aattagtgtg gtcaagagcc gtctgagcct aatgagtccc 60
   agctgcatta ggttaagaga ctcttccaga gccatcgcca ggtcttgaat ggcacctctc 120
   cctaggatac acagcctgca ggtccccagg acctggatga cacccgcctc actgtggcag 180
   tgtattgcct gttaattgct gctaattcta attctgatga tgactcctac tccattgttt 240
   accccaaagc atcagctagg ctggagtgat ttgttacaaa tgagcaaaag atgagtcctt 300
   gcttccctca gaaataaaag gagctcagct g                                331
<210> 291
   <211> 228
   <212> DNA
   <213> Homo sapiens
<400> 291
   gagatgcaaa gcaggattca aaagaacatc tttgcgtttt ctaccggctc cccatcatcg 60
   tactagggag gaagaagcgg gtgagaaaca aaacttcttt ccattgtcct gcccgtttct 120
   gcggacttgt tctgaggccg aggcacctct aagatactga tggctctgca gaggacccat 180
   tcattgcttc tgcttttgct gctgaccctg ctggggctgg ggctggtc             228
<210> 292
   <211> 342
   <212> DNA
   <213> Homo sapiens
<400> 292
   ggagctgtcc tgcaccgtgg tggagctgaa gtacacaggc aatgccagcg cactcttcat 60
   cctccctgat caagacaaga tggaggaagt ggaagccatg ctgctcccag agaccctgaa 120
   gcggtggaga gactctctgg agttcagaga gataggtgag ctctacctgc caaagttttc 180
   catctcgagg gactataacc tgaacgacat acttctccag ctgggcattg aggaagcctt 240
   caccagcaag gctgacctgt cagggatcac aggggccagg aaccctacag tctcccaggt 300
   ggtccataag gctgtgcttg atgtatttga ggagggcaca ga                   342
<210> 293
   <211> 311
   <212> DNA
   <213> Homo sapiens
<400> 293
   ggagctgtcc tgcaccgtgg tggagctgaa gtacacaggc aatgccagcg cactcttcat 60
   cctccctgat caagacaaga tggaggaagt ggaagccatg ctgctcccag agaccctgaa 120
   gcggtggaga gactctctgg agttcacaga gataggtgag ctctacctgc caaagttttc 180
   catctcgagg gactataacc tgaacgacat acttctccag ctgggcattg aggaagcctt 240
   caccagcaag gctgacctgt cagggatcac aggggccagg aacctagcag tctcccaggt 300
   ggtccataag g                                                     311
<210> 294
   <211> 402
   <212> DNA
   <213> Homo sapiens
<400> 294
   cggctgcgag aagacgacag aagggaagat ggaggaagtg gaagccatgc tgctcccaga 60
   gaccctgaag cggtggagag actctctgga gttcagagag ataggtgagc tctacctgcc 120
   aaagttttcc atctcgaggg actataacct gaacgacgac ttctccagct gggcattgag 180
   gaagccttca ccagcaaggc tgacctgtca gggatcacag gggccaggaa cctagcagtc 240
   tcccaggtgg tccataaggc tgtgcttgat gtatttgagg agggcacaga agcatctgct 300
   gccacagcag tcaaaatcac cctcctttct gcattagtgg agacaaggac cattgtgcgt 360
   ttcaacaggc ccttcctgat gatcattgtg cctacagaca cc                   402
<210> 295
   <211> 232
   <212> DNA
   <213> Homo sapiens
<400> 295
   ttccatctcg agggactata acctgaacga cgacttctcc agctgggcat tgaggaagcc 60
   ttcaccagca aggctgacct gtcagggatc acaggggcca ggaacctagc agtctcccag 120
   gtggtccata aggctgtgct tgatgtattt gaggagggca cagaagcatc tgctgccaca 180
   gcagtcaaaa tcaccctcct ttctgcatta gtggagacaa ggaccattgt gc         232
<210> 296
   <211> 435
   <212> DNA
   <213> Homo sapiens
<400> 296
   tgactctgac ttctgaggaa gaggcccgtt tgaagaagag tgcacatcac tttgggggat 60
   ccaaaaggag ctgcaatttt aaagtcttct gatgtcatat catttcactg tctaggctac 120
   aacaggattc taggtggagg ttgtgcatgt tgtccttttt atctgatctg cgattaaagc 180
   agtaatattt taagatggac tgggaaaaac atcaactcct gaagttagaa ataagaatgg 240
   tttgtaaaat ccacagctat atcctgatgc tggatggtat taatcttgtg tagtcttcaa 300
   ctggttagtg tgaaatagtt ctgccacctc tgacgcacca ctgccaatgc tgtacgtact 360
   gcatttgccc cttgagccag gtggatgttt accgtgtgtt atataactta ctggctcctt 420
   cactgaacat gccta                                                  435
<210> 297
   <211> 309
   <212> DNA
   <213> Homo sapiens
<400> 297
   atcatttcac tgtctaggct acaacaggat tctaagggga cgttgtgcat gttggccttt 60
   gtatctgatc tgtgattaaa gcagtaatat tttaagatgg actgggaaaa acatcaactc 120
   ctgaagttag aaataagaat ggtttgtaaa atccacagct gtatgctgaa gctggatggt 180
   attaatcttg cgtagtcttc aactggttag gtgaaatagt tctgccacct ctgacgcacc 240
   actgccaatg ctgtacgtac tggatttggc ccttgagcca ggtggatgtt caccgggcgt 300
   gatataact                                                         309
<210> 298
   <211> 342
   <212> DNA
   <213> Homo sapiens
<220>
   <221> misc_feature
   <222> 342
   <223> n = A,T,C or G
<400> 298
   atcatttgac tgtctaggct acaacaggat tctaggtgga ggttgtgcat gttgtccttt 60
   ttatctgatc tgtgattaaa gcagtaatat tttaacatgg actgggaaaa acatcaactc 120
   ctgaagttag aaataagaat ggtttgtaaa atccacagct atatcctgat gctggatggt 180
   attaatcttg tgtagtcttc aactggttag ttgaaatagt tctgccacct ctgacgcacc 240
   actgccaatg ctgtacgtac tgcatttgcc ccttgagcca ggtggatgtt taccgtgtgt 300
   tatataactt cctggctcct tcactgaaca tgcctagtcc an 342
<210> 299
   <211> 266
   <212> DNA
   <213> Homo sapiens
<400> 299
   gggacagaat ggctatctcg gaccttgtga aggtgactct gacttctgag gaagacgccc 60
   gcttgaagaa gagagcccat acactttggg ggatccaaaa cgagctgcga ttttcaagtc 120
   ttctgatgtc atatcattcc actgtctagg ctacaacagg attctagggg gacgttgtgc 180
   atgttggcct ttttatctga tctgtgacta aagcactaat attttaagat ggactgggaa 240
   aaacatcaac tcctgaagtt agaaat 266
<210> 300
   <211> 383
   <212> DNA
   <213> Homo sapiens
<400> 300
   ggacagaatg gaatctcaga ccttgtgaag gtgactctga cttctgagga agaggcccgt 60
   ttgaagaaga gtgcagatac actttggggg atccaaaagg agctgcaatt ttaaagtctt 120
   ctgatgtcat atcatttcac tgtctaggct acaacaggat tctaggtgga ggttgtgcat 180
   gttgaccttt ttatctgatc tgtgattaaa gcagtaatat tttaagatgg actgggaaaa 240
   acatcaactc ctgaagttag aaataagaat ggtttgtaaa atccacagct atatcctgat 300
   gctggatggt attaatcttg tgtagtcttc aactggttag tgtgaaatag ttctgccacc 360
   tctgacgcac cactgccaat gct 383
<210> 301
   <211> 453
   <212> DNA
   <213> Homo sapiens
<400> 301
   aaccgcttct ccgttgaaca acatactaga tggggacaga atggaatctc agaccttgtg 60
   aaggtgactc tgacttctga ggaagaggcc cgtttgaaga agagtgcaga tacactttgg 120
   gggatccaaa aggagctgca attttaaagt cttctgatgt catatcattt cactgtctag 180
   gctacaacag gattctaggt ggaggttgtg catgttgtcc tttttatctg atctgtgatt 240
   aaagcagtaa tattttaaga tggactggga aaaacatcaa ctcctgaagt tagaaataag 300
   aatggtttgt aaaatccaca gctatatcct gatgctggat ggtattaatc ttgtgtagtc 360
   ttcaactggt tagtgtgaaa tagttctgcc acctctgacg caccactgcc aatgctgtac 420
   gtactgcatt tgccccttga gccaggtgga tgt 453
<210> 302
   <211> 383
   <212> DNA
   <213> Homo sapiens
<400> 302
   ggacagaatg gaatctcaga ccttgtgaag gtgactctga cttctgagga agaggcccgt 60
   ttgaagaaga gtgcagatac actttggggg atccaaaagg agctgcaatt ttaaagtctt 120
   ctgatgtcat atcatttcac tgtctaggct acaacaggat tctaggtgga ggttgtgcat 180
   gttgaccttt ttatctgatc tgtgattaaa gcagtaatat tttaagatgg actgggaaaa 240
   acatcaactc ctgaagttag aaataagaat ggtttgtaaa atccacagct atatcctgat 300
   gctggatggt attaatcttg tgtagtcttc aactggttag tgtgaaatag ttctgccacc 360
   tctgacgcac cactgccaat gct 383
<210> 303
   <211> 97
   <212> DNA
   <213> Homo sapiens
<400> 303
   gttgccttgg agatgatcaa agtaactggt ggctatccat ttgaagctta caaaaattgt 60
   tttcttaact tagccattcc aattgtagta tttacag 97
<210> 304
   <211> 442
   <212> DNA
   <213> Homo sapiens
<400> 304
   gccctagtta ttataccata ttacatcatt actctatgta attatctatg aagctatgta 60
   gttatttacc cctgtattaa gtgattttag actgttgtta ttttttgagt tacagcatgt 120
   gctttcaaaa tagggagact gtatggttga attaatattt ttttaaataa ctgttaacat 180
   gtatagagta ggttgaaagt ttgaaagtat aaaatatact aaaagtatac agacctgtaa 240
   taagaaattt atattactat agtcccatag ctgcttttac tatccacaga gaaatgcttg 300
   aaaacgtgaa agttgaatag atgcaattaa aatcacggat agttttaggc tgtttatatt 360
   atcagatcac cttcttttat ctaggttgcc ttggagatga tcaaagtaac tggtggctat 420
   ccatttgaag cttacaaaaa tt 442
<210> 305
   <211> 380
   <212> DNA
   <213> Homo sapiens
<400> 305
   gagacgttcg cacacctggg tgccagcgcc ccagaggtcc cgggacagcc cgaggcgccg 60
   cgcccgccgc cccgagctcc ccaagccttc gagagcggcg cacactcccg gtctccactc 120
   gctcttccaa cacccgctcg ttttggcggc agctcgtgtc ccagagaccg agttgcccca 180
   gagaccgaga cgccgccgct gcgaaggacc aatgagagcc ccgctgctac cgccggcgcc 240
   ggtggtgctg tcgctcttga tactcggctc aggccattat gctgctggat tggacctcaa 300
   tgacacctac tctgggaagc gtgaaccatt ttctggggac cacagggctg atggatttga 360
   ggttacctcc agaagggagg                                              380
<210> 306
   <211> 133
   <212> DNA
   <213> Homo sapiens
<400> 306
   ccagtactgc ctcctgtgct cgtgccaaga cacagtgaat ataaccccca gctcagcctc 60
   ctggccaagt tccgcagcgc ctccctgcac agtgagccac tcatgccaca caacgccacc 120
   tatcctgact ctt                                                     133
<210> 307
   <211> 428
   <212> DNA
   <213> Homo sapiens
<400> 307
   tccagtactg cctcctgtgc tcgtgccaag acacagtgaa tataaccccc agctcagcct 60
   cctggccaag ttccgcagcg cctccctgca cagtgagcca ctcatgccac acaacgccac 120
   ctatcctgac tctttccagc agcctccgtg ctctgcactc cctccctcac ccagccacgc 180
   gttctcccag tccccgtgca cggccagcta ccctcactcc ccaggaagtc cttctgagcc 240
   agagagtccc tatcaacact cagactttcg accagtttgt tacgaggagc ccccactggt 300
   gctcggtcgc ctactatgaa ctgaacaacc gagttgggga gacattccag gcttcctccc 360
   gaagtgtgct catagatggg ttcaccgacc cttcaaataa caggaacaga ttctgtcttg 420
   gacttctt                                                           428
<210> 308
   <211> 497
   <212> DNA
   <213> Homo sapiens
<400> 308
   cggctgcgag aagacgacag aaggggggaa tgtgtctggc ccttcagcag tttctcttgg 60
   cagcatcagc tgggctgctt tctttgtgtg tggccccagg tgtcaaaatg acaccagctg 120
   tctgtactag acaaggttac caagtgcgga attggttaat actaacagag agatttgctc 180
   cattctcttt ggaataacag gacatgctgt atagatacag gcagtaggtt tgctctgtac 240
   ccatgtgtac agcctaccca tgcagggact gggattcgag gacttccagg cgcatagggt 300
   agaaccaaat gatagggtag gagcatgtgt tctttagggc cttgtaaggc tgtttccttt 360
   tgcatctgga actgactata taattgtctt caatgaagac taattcaatt ttgcatatag 420
   aggagccaaa gagagatttc agctctgtat ttgtggtatc agtttggaaa aaaaaaatct 480
   gatactccat ttgatta                                                 497
<210> 309
   <211> 356
   <212> DNA
   <213> Homo sapiens
<400> 309
   gggaatgtgt ctggcccttc agcagtttct cttggcagca tcagctgggc tgctttcttt 60
   gtgtgtggcc ccaggtgtca aaatgacacc agctgtctgt actagacaag gttaccaagt 120
   gcggaattgg ttaatactaa cagagagatt tgctccattc tctttggaat aacaggacat 180
   gctgtataga tacaggcagt aggtttgctc tgtacccatg tgtacagcct acccatgcag 240
   ggactgggat tcgaggactt ccaggcgcat agggtagaac caaatgatag ggtaggagca 300
   tgtgttcttt aaggccttgt aaggctgttt ccttttgcat ctggaactga ctatat     356
<210> 310
   <211> 348
   <212> DNA
   <213> Homo sapiens
<400> 310
   gggaatgtgt ctggcccttc agcagtttct cttggcagca tcagctgggc tgctttcttt 60
   gtgtgtggcc ccaggtgtca aaatgacacc agctgtctgt actagacaag gttaccaagt 120
   gcggaattgg ttaatactaa cagagagatt tgctccattc tctttggaat aacaggacat 180
   gctgtataga tacaggcagt aggtttgctc tgtacccatg tgtacagcct acccatgcag 240
   ggactgggat tcgaggactt ccaggcgcat agggtagaac caaatgatag ggtaggagca 300
   tgtgttcttt agggccttgt aaggctgttt ccttttgcat ctggaact              348
<210> 311
   <211> 337
   <212> DNA
   <213> Homo sapiens
<400> 311
   aagttgtggt ctgacacaca ctgctgtggt tcccctggat ttagtgaaat gccgtatgca 60
   ggtggacccc caaaagtaca agggcatatt taacggattc tcagttacac ttaaagagga 120
   tggtgttcgt ggtttggcta aaggatgggc tccgactttc cttggctact ccatgcaggg 180
   actctgcaag tttggctttt atgaagtctt taaagtcttg tatagcaata tgcttggaga 240
   ggagaatact tatctctggc gcacatcact atatttggct gcctctgcca gtgctgaatt 300
   ctttgctgac attgccctgg ctcctatgga agctgct                          337
<210> 312
   <211> 252
   <212> DNA
   <213> Homo sapiens
<220>
   <221> misc_feature
   <222> 144
   <223> n = A,T,C or G
<400> 312
   agcccaagcc ctcagtggaa cctgtcaaga gcatcagcag catggagctg aagaccgagc 60
   cctttgatga cttcctgttc ccagtgacac ttcagagagc tggtagttag tagcatgttg 120
   agccaggcct gggtctgtgt ctcntttctc tttctcctta gtcttctcat agcattaact 180
   aatctattgg gttcattatt ggaattaacc tggtgctgga tattttcaaa ttgtatctag 240
   tgcagctgat tt                                                     252
<210> 313
   <211> 51
   <212> DNA
   <213> Homo sapiens
<400> 313
   actcccagct gcactggtta cacgtcttcc ttcgtcttca cctaccccga g 51
<210> 314
   <211> 348
   <212> DNA
   <213> Homo sapiens
<400> 314
   atggccacag agctggagcc cctgtgcact ccggtggtca cctgtactcc cagctgcact 60
   gcttacacgt cttccttcgt cttcacctac cccgaggctg actccttccc cagctgtgca 120
   gctgcccacc gcaagggcag cagcagcaat gagccttcct ctgactcgct cagctcaccc 180
   acgctgctgg ccctgtgagg gggcagggaa ggggaggcag ccggcaccca caagtgccac 240
   tgcccgagct ggtgcattac agagaggaga aacacatctt ccctagaggg ttcctgtaga 300
   cctagggagg accttatctg tgcgtgaaac acaccaggct gtgggccc 348
<210> 315
   <211> 507
   <212> DNA
   <213> Homo sapiens
<400> 315
   ccggtggtca cctgtactcc cagctgcact gcttacacgt cttccttcgt cttcacctac 60
   cccgaggctg actccttccc cagctgtgca gctgcccacc gcaagggcag cagcagcaat 120
   gagccttcct ctgactcgct cagctcaccc acgctgctgg ccctgtgagg gggcagggaa 180
   ggggaggcag ccggcaccca caagtgccac tgcccgagct ggtgcattac agagaggaga 240
   aacacatctt ccctagaggg ttcctgtaga cctagggagg accttatctg tgcgtgaaac 300
   acaccaggct gtgggcctca aggacttgaa agcatccatg tgtggactca agtccttacc 360
   tcttccggag atgtagcaaa acgcatggag tgtgtattgt tcccagtgac acttcagaga 420
   gctggtagtt agtagcatgt tgagccaggc ctgggtctgt gtctcttttc tctttctcct 480
   tagtcttctc atagcattaa ctaatct 507
<210> 316
   <211> 239
   <212> DNA
   <213> Homo sapiens
<220>
   <221> misc_feature
   <222> 223
   <223> n = A,T,C or G
<400> 316
   agactccaag ccctactggg aggcacggag ggtggcgagg caggctcagc tggaagctca 60
   gaaagccacg caggacttcc agagggccac agaggtgctc cgcgccgcca aggagaccat 120
   ctccctggcc gagcagcggc tgctggagga tgacaagcgg cagttcgact ccgcctggca 180
   ggagatgctg aatcacgcca ctcagagggt catggaggcg ganagaccaa gaccaggag 239
<210> 317
   <211> 313
   <212> DNA
   <213> Homo sapiens
<400> 317
   catcagtgat agggatgatt cacaaacaca aagctggtct tttcaaaatg ggaagaaaaa 60
   agatgcaatt gatcccttac tattcaagta taaagtgcaa cccactaaaa aagaattaca 120
   tgagtctgct attgttaaag caacacaaat cagccggaga aaacacctat tttctcgtga 180
   taaactaaag ctttttctga agcaacactg ggaaccacaa gatggagtca ttaaaataaa 240
   ggcatcatct ctttcaacgg ataaaatagc cgaacaagat tttttcttat ttcttccctg 300
   atgattccac ccc                                                    313
<210> 318
   <211> 574
   <212> DNA
   <213> Homo sapiens
<400> 318
   aaataacatc aacagaacag cttcactttg ggccaaacat ttgaaaaact ttttataaaa 60
   aattgtttga tatttcttaa tgtctgctct gagccttaaa acacagattg aagaagaaaa 120
   gaaagaaaaa acttaaatat ttatttctat gctttgttgc ctctgagaat aatgacaatt 180
   tatgaatttg tgtttcaaat tgataaaata tttaggtaca aataacaaga ctaataatat 240
   tttcttattt aaaaaaagca tgggaagatt tttatttatc aaaatataga ggaaatgtag 300
   acaaaatgga tataaatgaa aattaccatg ttgtaaaacc ttgaaaatca gattctaact 360
   ggatttgtat gcaactaagt atttttctga acacctatgc aggtcttatt tacagtagtt 420
   actaagggaa cacacaaaga attacacaac gttttcctca agaaaatggt acaaaacaca 480
   accgaggagc gtatacagtt gaaaacattt ttgttttgat tggaaggcag attattttat 540
   attagtatta aaaatcaaac cctatgtttc tttc                             574
<210> 319
   <211> 518
   <212> DNA
   <213> Homo sapiens
<400> 319
   gaagggaaat aacatcaaca gaacaacttc actttgggcc aaacatttga aaaacttttt 60
   ataaaaaatt gtttgatatt tcttaatgtc tgctctgagc cttaaaacac agattgaaga 120
   agaaaagaaa gaaaaaactt aaatatttat ttctatgctt tgttgcctct gagaataatg 180
   acaatttatg aatttgtgtt tcaaattgat aaaatattta ggtacaaata acaagactaa 240
   taatattttc ttatttaaaa aaagcatggg aagattttta tttatcaaaa tatagaggaa 300
   atgtagacaa aatggatata aatgaaaatt accatgttgt aaaaccttga aaatcagatt 360
   ctaactggat ttgtatgcaa ctaagtattt ttctgaacac ctatgcaggt cttatttaca 420
   gtagttacta agggaacaca caaagaatta cacaacgttt tcctcaagaa aatggtacaa 480
   aacacaaccg aggagcgtat acagttgaaa acattttt                         518
<210> 320
   <211> 353
   <212> DNA
   <213> Homo sapiens
<400> 320
   aaataacatc aacagaacaa cttcactttg ggccaaacat ttaaaaaact ttttataaaa 60
   aaatgtttga tatttcttaa tgtctgctct gagccttaca acacagattg aagaagaaaa 120
   gaaagaacaa acttagatat ttatttctat gctttgttgc ctctgagaat aatgacaatt 180
   tatgaatttg agtttcaaat tgataaaata tttaggtact aataacaaga ctaataatat 240
   tttcttattt ataaaaagca tgggaagatt tttatttatc aaaatataca ggaagtgtag 300
   acaaaatgga tataaatgaa aattaccatg ttgtaaaacc ttgaaaatca gag        353
<210> 321
   <211> 401
   <212> DNA
   <213> Homo sapiens
<400> 321
   gacctgcaca cagagactcc ctcctgggct cctggcacca tggccccctg aagagctggc 60
   cctggtcacc ctcctcctgg gggcttctct gcagcacatc cacgcagctc gagggaccaa 120
   tgtgggccgg gagtgctgcc tggagtactt caagggagcc attcccctta gaaagctgaa 180
   gacgtggtac cagacatctg aggactgctc cagggatgcc atcgtttttg taactgtgca 240
   gggcagggcc atctgttcgg accccaacaa caagagagtg aagaatgcag ttaaatacct 300
   gcaaagcctt gagaggtctt gaagcctcct caccccagac tcctgactgt ctcccgggac 360
   tacctgggac ctccaccggt ggtgttcacc gcccccaccc t                     401
<210> 322
   <211> 547
   <212> DNA
   <213> Homo sapiens
<400> 322
   gacctgcaca cagagactcc ctcctgggct cctggcacca tggccccact gaagatgctg 60
   gccctggtca ccctcctcct gggggcttct ctgcagcaca tccacgcagc tcgagggacc 120
   aatgtgggcc gggagtgctg cctggagtac ttcaagggag ccattcccct tagaaagctg 180
   aagacgtggt accagacatc tgaggactgc tccagggatg ccatcgtttt tgtaactgtg 240
   cagggcaggg ccatctgttc ggaccccaac aacaagagag tgaagaatgc agttaaatac 300
   ctgcaaagcc ttgagaggtc ttgaagcctc ctcaccccag actcctgact gtctcccggg 360
   actacctggg acctccaccg ttggtgttca ccgcccccac cctgagcgcc tgggtccagg 420
   ggaggccttc cagggacgaa gaagagccac agtgagggag atcccatccc cttgtctgaa 480
   ctggagccat gggcacaaag ggcccagatt aaagtcttta tcctcaaaaa aaaaaaaaaa 540
   aaaaaaa                                                           547
<210> 323
   <211> 283
   <212> DNA
   <213> Homo sapiens

<400> 323
   ctgagcagag ggacctgcac acagagactc cctcctgggc tcctggcacc atggccccac 60
   tgaagatgct ggccctggtc accctcctcc tgggggcttc tctgcagcac atccacgcag 120
   ctcgagggac caatgtgggc cgggagtgct gcctggagta cttcaaggga gccattcccc 180
   ttagaaagct gaagacgtgg taccagacat ctgaggactg ctccagggat gccatcgttt 240
   ttgtaactgt gcagggcagg gccatctgtt cggaccccaa caa                   283
<210> 324
   <211> 160
   <212> DNA
   <213> Homo sapiens
<400> 324
   gcggtgacga cggggaccat tttaccatca ccacccaccc tgagagcaac cagggcatcc 60
   tgacaaccag gaagggtttg gattttgagg ccaaaaacca gcacaccctg tacgttgaag 120
   tgaccaacga ggcccctttt gtgctgaagc tcccaacctc                       160
<210> 325
   <211> 300
   <212> DNA
   <213> Homo sapiens
<400> 325
   tttttttttg gggccaattc tttaatttaa ctaaattagg aacgcagctt ttacagaaca 60
   ataaacacaa gggacggggc caccccagga tctaacagct tttcagggac ctatgttgca 120
   agctcaaaag taatccacta acgaaccaag tcaaactcca gtttttaata aaaaggggct 180
   gggggaggtt gtcaaacccc ttccaatata aatccccaat ccgagggcca ccaaatgaaa 240
   aagcaccaaa aatggaagga aaactttcaa aaattctgca aaaaatatgc cccctttttt 300
<210> 326
   <211> 394
   <212> DNA
   <213> Homo sapiens
<400> 326
   gtctattctt ttattttact aaattaggaa cgcagcattt acagaacaat aaacacaagt 60
   gacgtggcca ccccaggatc taacagctct tcagtgagct atgttgcaag ctcagaagta 120
   atccactaac gaaccaagtc agactccagt tcttcatcaa aaggtgctgg tggaggttgt 180
   cagacgcctt ccaatataga tccccaatcc gatggccagc aaatgagaga gcagcagaga 240
   tggaaggaaa acttccagaa attctgcaga gaatatgccc cctttcttca tgacgctcgt 300
   gttcccccat gctgaaggtg gccgtgcgct tccggtgttt aaagaagaac ccttgggggg 360
   aatatttccc ggccatttga ccaatcccat tcca                             394
<210> 327
   <211> 524
   <212> DNA
   <213> Homo sapiens
<400> 327
   gtctattctt ttattttact aaattaggaa cgcagcattt acagaacaaa taaacacaag 60
   tgacgtggcc accccaggat ctaacagctc ttcagtgagc tatgttgcaa gctcagaagt 120
   aatccactaa cgaaccaagt cagactccag ttcttcatca aaaggtgctg gtggaggttg 180
   tcagacgcct tccaatatag atccccaatc cgatggccag caaatgagag agcagcagag 240
   atggaaggaa aactttcaga aattctgcag agaatatgcc ccctttcttc atgacgctcg 300
   tgttcctcat gctgaggtgg ccgtgcgctt ccggtgttta aagaagaacc cttgggggga 360
   atatttccgg ccgacttgac caatcccata tccatctgat ttttcttcca gaagctttca 420
   cttccttcct ccttcaatat cactccctca actgtgactg ttttcccccc aatgctatgg 480
   tttctgttca aaaccccgtt ggttctgttg ggtcgctact ccgt 524
<210> 328
   <211> 55
   <212> DNA
   <213> Homo sapiens
<400> 328
   ggccgccctt tttttttttt ttttttcggg ggcgtttttt gatttttaaa attgg 55
<210> 329
   <211> 463
   <212> DNA
   <213> Homo sapiens
<400> 329
   tcactatagg gaaagctggt acgcctgcag gtaccggtcc ggaattcccg ggtcgaccca 60
   cgcgtccgcc gcccccgaga cctgtgaaga aaaccatctt gtgaggggct gcctggactg 120
   gtctggcagg ttgggcctgg atggggaggc tctagcatct ctcataggtg caacctgaga 180
   gtgggggagc taagccatga ggtaggggca ggcaagagag aggattcaga cgctctggga 240
   gccagttcct agtcctcaac tccagccacc tgccccagct cgacggcact gggccagttc 300
   cccctctgct ctgcagctcg gtttcctttt ctagaatgga aatagtgagg gccaatgccc 360
   agggttggag ggaggagggc gttcatagaa gaacacacat gcgggcacct tcatcgtgtg 420
   tggcccactg tcagaactta ataaaagtca actcatttgc tgg 463
<210> 330
   <211> 274
   <212> DNA
   <213> Homo sapiens
<220>
   <221> misc_feature
   <222> 144, 218, 268
   <223> n = A,T,C or G
<400> 330
   ccgcccccga gaccatgtga agaaaaccat cttgtgaggg gctgcctgga ctggtctggc 60
   aggttgggcc tggatgggga ggctctagca tctctcatag gtgcaacctg agagtggggg 120
   agctaagcca tgaggtaggg gcangcaaga gagaggattc agacgctctg ggagccagtt 180
   cctagtcctc aactccagcc acctgcccca gctcgacngc actgggccag ttccccctct 240
   gctctgcagt cggtttcctt ttctagantg gaaa 274
<210> 331
   <211> 232
   <212> DNA
   <213> Homo sapiens
<400> 331
   cggctgtgag aatacgacag aagggtccgg ctgcgagaag acgacagaag ggggatctca 60
   gcggggagcc acgtctcttg cactgtggtc tctgcatgga ccccagggct gtggggactt 120
   gggggacagt aatcaagtaa tccccttttc cagaatgcat taacccactc ccctgacctc 180
   acgctggggc aggtccccaa gtgtgcaagc tcagtattca tgatggtggg gg         232
<210> 332
   <211> 321
   <212> DNA
   <213> Homo sapiens
<400> 332
   gttgtgttga gatccagtgc agttgtgatt tctgtggatc ccagcttggt tccaggaatt 60
   ttgtgtgatt ggtttaaatc cagttttcaa tcttcgacag ctgggctgga acgtgaactc 120
   agtagctgaa cctgtctgac ccggtcacgt tcttggatcc tcagaactct ttgctcttgt 180
   cggggtgggg gtgggaactc tcgtgaggag cgccagctgt gtaaatgcca cgactccgta 240
   attcttattc ggtgggacct tgcttccctc tgggagctgg ctcgttttgt tggtgtctaa 300
   cctttcgccg aatcgttaaa g                                           321
<210> 333
   <211> 344
   <212> DNA
   <213> Homo sapiens
<220>
   <221> misc_feature
   <222> 265, 267, 272, 337
   <223> n = A,T,C or G
<400> 333
   gtcctatttc tcattttgtt gataatttct gcatttaatg gtctgtgctt taaatggtaa 60
   cgctacggcc ccaggtcact gcgaggcact taccatgtag atacgggctc aaaagtcacc 120
   tctcagagac ctacgtcatc cactcaggaa ttcgcgcctc tcatacttgc ctgtctcatt 180
   ttatcttcct tctagcagct gtctgaaatt ggttcgtctg ttttcttgtt tatggtattc 240
   tcaagccctt gacagaccgg ctagngnggt tntcccgtgc atcttcagcc tggcacatta 300
   tggacactta aatactacgt attgatctaa tattganggg ttaa                  344
<210> 334
   <211> 405
   <212> DNA
   <213> Homo sapiens
<400> 334
   ggcacgaggg atgaagggtg ctgctcattt tcattagatg tatgtgaagg cacagtgaaa 60
   atggaaatgt tcttggagct acttcctcaa aatgtatcct tagtcacctc agtgcaacag 120
   ctgggagggg gccgtgttaa gatttttttt gctacaaaga ggaggtggca atggtagatc 180
   cacccttatg cttctcagtt tagcataacc tcttatggat tttcatcaaa ttcagcgtgt 240
   tggtcactgg aaagagcctt ttccttctcc ttttcttact ctcccctcat ggggttcccc 300
   tcttaaagga gaggagcttt taatttacac ttaccacctc atttgctttt ttggaggcca 360
   tgccatataa gcgggactac cgagttaatc tcctttttac aaaag 405
<210> 335
   <211> 227
   <212> DNA
   <213> Homo sapiens
<400> 335
   ggatgaacta ttcagatgct atcgtttggc taaaagaaca tgatgtaaag aaagaagatg 60
   gaactttcta tgaatttgga gaagatatcc cagaagctcc tgagagactg atgacagaca 120
   ccattaatga accaatcttg ctgtgtcgat ttcctgtgga gatcaagtcc ttctacatgc 180
   agcgatgtcc tgaggattcc cgtcttactg aatctgtcga cgtgttg 227
<210> 336
   <211> 521
   <212> DNA
   <213> Homo sapiens
<400> 336
   tcgaattcgg atgaactatt cagatgctat cgtttggcta aaagaacatg atgtaaagaa 60
   agaagatgga actttctatg aatttggaga agatatccca gaagctcctg agagactgat 120
   gacagacacc attaatgaac caatcttgct gtgtcgattt cctgtggaga tcaagtcctt 180
   ctacatgcag cgatgtcctg aggattcccg tcttactgaa tctgtcgacg tgttgatgcc 240
   caatgttggt gagattgtgg gaggctcaat gcgtatcttt gatagtgaag aaatactggc 300
   aggttataaa agggaaggga ttgaccccac tccctattac tggtatacgg atcagagaaa 360
   atacggtaca tgtccccatg gaggatatgg cttgggcttg gaacgattct taacgtggat 420
   tctgaatagg tatcacatcc gagacgtgtg cttataccct cgatttgtcc agcgttgcac 480
   gccataacca ttttctccag aagcgtggag gaaagattat g 521
<210> 337
   <211> 325
   <212> DNA
   <213> Homo sapiens
<400> 337
   ggactttccc gatcgccagg caggagtttc tctcggtgac tactatcgct gtcatgtctg 60
   gtcgtggcaa gcaaggaggc aaggcccgcg ccaaggccaa gtcgcgctcg tcccgcgcgg 120
   gccttcagtt cccggtaggg cgagtgcatc gcttgctgcg caaaggcaac tacgcggagc 180
   gagtgggggc cggcgcgccc gtctacatgg ctgcgttcct cgagtatctg accgctgaga 240
   tcctggagct ggcgggcaac gcggctcggg acaacaagaa gacgcgcatc atccctcgtc 300
   acctccagct ggccatccgc aacga 325
<210> 338
   <211> 401
   <212> DNA
   <213> Homo sapiens
<220>
   <221> misc_feature
   <222> 264
   <223> n = A,T,C or G
<400> 338
   cgttgctgtc ggttttagga aacctggcat ggtgctttca ggtctggggc ttttagagcc 60
   ccccgtgtgg cttacaaatt ctacagcata cagagcaggc cacgctcagg cccggcatgc 120
   gggccaccaa gttctggaaa ccacgtggtg tccctgcgaa tggggcgatc aagtccagag 180
   ccggggcact ttcagagttt gaaggtaact gagagcagat ggtcctccat ttcaactcca 240
   gaagtggggc tctgggaggg atgntctaac cctccctggc atgtcacaac caggctctgg 300
   ctggaggatc cctccatccg gctcctgtca tcccctacac tttggcctag caagaggtgg 360
   aataaccact tgtgtgctca ttactgttgg gaggaacaaa g                     401
<210> 339
   <211> 460
   <212> DNA
   <213> Homo sapiens
<400> 339
   catgcgggcc accaagttct ggaaaccacg tggtgtccct gcgaatgggg cgatcaagtc 60
   cagagccggg gcactttcag agtttgaagg taactgagag cagatggtcc tccatttcaa 120
   ctccagaagt ggggctctgg gagggatgtt ctagccctcc ctggcatgtc agaaccaggc 180
   tctgcctgga ggatccctcc atccggctcc tgtcatcccc tacactttgg ccaagcaaga 240
   agtggtagaa ccacttggct gctccttcct tctggaggac acacagtctc agtccagatg 300
   ccttcctgtc tttctggccc tttctggacc agatcctact cttcctttct aaatctgaga 360
   tctccctcca gggaatccgc ctgcagagga cagagctggc tgtcttcccc cacccctaac 420
   ctggcttatt cccaactgct ctgcccactg tgaaaccact                       460
<210> 340
   <211> 496
   <212> DNA
   <213> Homo sapiens
<400> 340
   tttttttttt tttttttttt tttttgggat tcttaaatat agatgtattt ttttcatctc 60
   atctccggac acactccaat cacacccctc ctgccctccc ctctcaactg caaaccaagc 120
   ggtgcagaca cagcacagca cacatgaggg gccctccctt tcaccaaagc tgaaggcagg 180
   gcacagtttg gggatggaag agcctcgagg taaatgtggg ggttctagaa cccagtgacc 240
   tcagttctgg atcatgggaa agggatcagt atgcagtaac gtggtaaggt tccagatcta 300
   gaagccagga cctagaacct agtggtttca cagtgggcag agcagttggg aataagccag 360
   gttaggggtg ggggaagaca gccagctctg tcctctgcag gcggattccc tggagggaga 420
   tctcagattt agaaaggaag agtaggatct ggtccagaaa gggccagaaa gacaggaagg 480
   catctggact gagact                                                 496
<210> 341
   <211> 283
   <212> DNA
   <213> Homo sapiens
<400> 341
   tttttttttt tttttttttt ttttttttag gatttgaata catttattgt gacaagaatg 60
   ctgttataaa tattcataag caaaggccat ctttttatct aggaattgtc aaagagaaga 120
   ttccaaattg gaaggataca tcttttgtaa aatctgccac caattcctgc tttgagaata 180
   agcacctatt gtaaaatttc tactaacatt ataaatggtc acagcacatg ccacttgata 240
   caatccaaac tttgaaatgt ttgacttctc agtgggctgt ccc                   283
<210> 342
   <211> 335
   <212> DNA
   <213> Homo sapiens
<400> 342
   tgtcgggcag caggcgcagc ccagcctcga aatgcagaac gacgccggcg agttcgtgga 60
   cctgtacgtg ccgcggaaat gctccgctag caatcgcatc atcggtgcca aggaccacgc 120
   atccatccag atgaacgtgg ccgaggttga caaggtcaca ggcaggttta atggccagtt 180
   taaaacttat gctatctgcg gggccattcg taggatgggt gagtcagatg attccattct 240
   ccgattggcc aaggccgatg gcatcgtctc aaagaacttt tgactggaga gaatcacaga 300
   tgtggaatat ttgtcataaa taaataatga aaacc                            335
<210> 343
   <211> 75
   <212> DNA
   <213> Homo sapiens
<400> 343
   gggtagagtt cttaaatcga gatctggagg tagatggacg ctttgtaacc ctccagatct 60
   gggacactgc agggc                                                  75
<210> 344
   <211> 611
   <212> DNA
   <213> Homo sapiens
<400> 344
   gccggggggc agcggcgggc gcgagcggca gctgtcaggc caccgaggtc caagccgcac 60
   ttgctgcccc attgaggacg aggaggcagc aggagcagtg acggtgactc taaggagccg 120
   gattcccggc acgcagagct gacctgcctg gcacccgcgg ccctctcctg tttccttccc 180
   attgtgttgg caccctaaaa agaaagaata aaacaacaac aggaaaaaaa ggaaaatatt 240
   taaattgtga caaaaaccca ctgggttctc ttggttacaa actccttccc ttctggtgct 300
   acaaaaatga gtgggaaatc cctgctctta aaggtcattc tcttgggtga tggtggagtt 360
   gggaaaagtt cgcttatgaa ccgttacgta accaacaaat ttgactccca ggcttttcac 420
   accatagggg tagagttctt aaatcgagat ctggaggtag atggacgctt tgtaaccctc 480
   cagatctggg acactgcagg gcaggaacgt ttcaagagcc ttaggacacc cttctacagg 540
   ggagcagact gctgcctctt gaccttcagc gtggatgatc ggcagagctt cgagaatctt 600
   ggtaactggc a                                                      611
<210> 345
   <211> 441
   <212> DNA
   <213> Homo sapiens
<400> 345
   ggcctttgca agcctcaccg gcgatgcaag gatagtcatc aacagggccc gggtggagtg 60
   ccagagccac cggctgactg tggaggaccc ggtcactgtg gagtacatca cccgctacat 120
   cgccagtctg aagcagcgtt atacgcatag cactgggcgc aggcgtttgg catctctgcc 180
   ctcatcgtgg gtttctactt tgatggcact cctaggctct atcagactga cccctctgtc 240
   acataccatg cctggaaggc caatgccata cgccggggtg ccaactcagt gcgtgagttc 300
   ctggagaaga actatactga cgaagccatt gtaacatatg atctgaccat taagctggtg 360
   atcaacgcac tcctggaagt ggttcactca ggtggcaaaa acattgaact tgctgtcatg 420
   aggcgagatc aatccctcaa g                                           441
<210> 346
   <211> 323
   <212> DNA
   <213> Homo sapiens
<400> 346
   ggcctttgca ggcctcaccg ccgatgcaag gatagtcatc aacagggccc gggtggagtg 60
   ccagagccac cggctgactg tggaggaccc ggtcactgtg gagtacatca cccgctacat 120
   cgccagtctg aagcagcgtt atacgcacag caatgggcgc aggcgtttgg catctctgcc 180
   ctcatcgtgg gtttcgactt tgatggcact cctaggctct atcagactga cccctcgggc 240
   acataccatg cctggaaggc caatgccata tgccggggtg ccaagtcagt gcgtgagttc 300
   ctggagaaga actatactga cga                                         323
<210> 347
   <211> 567
   <212> DNA
   <213> Homo sapiens
<400> 347
   ccagcggcct cttccccttc ctggtgctgc ttgccctggg aactctggca ccttgggctg 60
   tggaaggctc tggaaagtcc ttcaaagctg gagtctgtcc tcctaagaaa tctgcccagt 120
   gccttagata caagaaacct gagtgccaga gtgactggca gtgtccaggg aagaagaaat 180
   gttgtcctga cacttgtggc atcaaatgcc tggatcctgt tgacacccca aacccaacaa 240
   ggaggaagcc tgggaagtgc ccagtgactt atggccaatg tttgatgctt aaccccccca 300
   atttctgtga gatggatggc cagtgcaagc gtgacttgaa gtgttgcatg ggcatgtgtg 360
   ggaaatcctg cgtttcccct gtgaaagctt gattcctgcc atatggagga ggctctggag 420
   tcctgctctg tgtggtccag gtcctttcca ccctgagact tggctccacc actgatatcc 480
   tcctttgggg aaaggcttgg cacacagcag gctttcaaga agtgccagtt gatcaatgaa 540
   taaataaacg agcctatttc tctttgc                                     567
<210> 348
   <211> 314
   <212> DNA
   <213> Homo sapiens
<220>
   <221> misc_feature
   <222> 48
   <223> n = A,T,C or G
<400> 348
   atgaagtcca gcggcctctt ccccttcctg gtgctgcttg ccctgggnac tctggcacct 60
   tgggctgtgg aaggctctgg aaagtccttc aaagctggag tctgtcctcc taagaaatct 120
   gcccagtgcc ttagatacaa gaaacctgag tgccagagtg actggcagtg tccagggaag 180
   aagagatgtt gtcctgacac ttgtggcatc aaatgcctgg atcctgttga caccccaaac 240
   ccaacaagga ggaagcctgg gaagtgccca gtgacttatg gccaatgttt gatgcttaac 300
   ccccccaatt tctg                                                  314
<210> 349
   <211> 611
   <212> DNA
   <213> Homo sapiens
<400> 349
   ggctctgctc tgcagcacac ccgtgggtga cccctcaccc cagaagcagc agtggcagct 60
   tgggaaatgt gaggaaggga aggagggaga gacgggaggg aggagagaga ggagaaggga 120
   ggcaggggag gggcagcaga accaaggcaa atatttcagc tgggctatac ccctctcccc 180
   atccctgtta tagaagctta gagagccagc cagcaatgga accttctggt tcctgcgcca 240
   atcgccacca gtatcaattg tgtgagcttg ggtgcgagtg cacgcgtgcg tgagtacgga 300
   gagtatatat agatctctat ctcttagcaa aggtgaatgc cagatgtaaa tggcgcctct 360
   gggcaaagga ggcttgtatt ttgcacattt tataaaaact tgagagaatg agatttctgc 420
   ttgtatattt ctaaaaagag gaaggagccc aaaccatcct ctccttacca ctcccatccc 480
   tgtgagccct accttacccc tctgccccta gccaaggagt gtgaatttat agatctaact 540
   ttcataggca aaacaaaagc ttcgagctgt tgcgtgtgtg agtctgttgt gtggatgtgc 600
   gtgtgtggtc c                                                     611
<210> 350
   <211> 370
   <212> DNA
   <213> Homo sapiens
<400> 350
   tggctggatg ggcttggact gtggtcctga aagcagcaag aagtatgctg aggctgtcac 60
   tcgggctaag cagattgtgt ggaatggtcc tgtgggggta tttgaatggg aagcttttgc 120
   ccggggaacc aaagctctca tggatgaggt ggtgaaagcc acttctaggg gctgcatcac 180
   catcataggt ggtggagaca ctgccacttg ctgtgccaaa tggaacacgg aggataaagt 240
   cagccatgtg agcactgggg gtggtgccag tttggagctc ctggaaggta aagtccttcc 300
   tggggtggat gctctcagca atatttagta ctttcctgcc ttttagttcc tgtgcacagc 360
   ccctaagtca                                                       370
<210> 351
   <211> 177
   <212> DNA
   <213> Homo sapiens
<400> 351
   gggctgcatc accatcatag gtggtggaga cactgccact tgctgtgcca aatggaacac 60
   ggaggataaa gtcagccatg tgagcactgg gggtggtgcc agtttggagc tcctggaagg 120
   gaaagtcctt cctggggtgg atgctctcag caatatttag tactttcctg cctttta    177
<210> 352
   <211> 204
   <212> DNA
   <213> Homo sapiens
<220>
   <221> misc_feature
   <222> 53, 55, 76, 86, 137
   <223> n = A,T,C or G
<400> 352
   atggctttta ccttccttaa ggtgctcaac aacatggaga ttgggcactt tcncnggttg 60
   atgaagaagg aagccnagat ttgtcnaaga cctaatgtcc aaaagctgag aagaatggtg 120
   tgaagattac cttgccntgt tgacttgtca ctgctgacaa gtttgatgag aatgcccaag 180
   actggcccag ccactggtgg cttc                                        204
<210> 353
   <211> 489
   <212> DNA
   <213> Homo sapiens
<400> 353
   cttttacctt ccttaaggtg ctcaggacat ggagattggc acttctctgt ttgatgaaga 60
   gggagccaag attgtcaaag acctaatgtc caaagctgag aagaatggtg tgaagattac 120
   cttgcctgtt gactttgtca ctgctgacaa gtttgatgag aatgccaaga ctggccaagc 180
   cactgtggct tctggcatac ctgctggctg gatgggcttg gactgtggtc ctgaaagcag 240
   caagaagtat gctgaggctg tcactcgggc taagcagatt gtgtggaatg gtcctgtggg 300
   ggtatttgaa tgggaagctt ttgcccgggg aaccaaagct ctcatggatg aggtggtgaa 360
   agccacttct aggggctgca tcaccatcat aggtggtgga gacactgcca cttgctgtgc 420
   caaatggaac acggaggata aagtcagcca tgtgagcact gggggtggtg ccagtttgga 480
   gctcctgga                                                         489
<210> 354
   <211> 885
   <212> DNA
   <213> Homo sapiens
<400> 354
   tttttttttt tcacggtttc aatggacact tttattgttt acttaatgga tcatcaattt 60
   tgtctcacta cctacaaatg gaatttcatc ttgtttccat gctgagtagt gaaacagtga 120
   caaagctaat cataataacc tacatcaaaa gagaactaag ctaacactgc tcactttctt 180
   tttaacaggc aaaatataaa tatatgcact ctaaaatgca caatggttta gtcactaaaa 240
   aattcaaatg ggatcttgaa gaatgtatgc aaatccaggg tgcagtgaaa atgagctgag 300
   atgctgtgca actgtttaag ggttcctggc actgcatctc ttggccacta gctgaatctt 360
   gacatggaag gttttagcta atgcccaggg gaaatgcaaa aaatgctaat ttgacttagg 420
   gcctgtgcac aggaactaaa aggcaggaaa gtactaaata ttgctgagag catccacccc 480
   aggaaggact ttaccttcca ggagctccaa actggcacca cccccagtgc tcacatggct 540
   gactttatcc tccgtgttcc atttggcaca gcaagtggca gtgtctccac cacctatgat 600
   ggtgatgcag ccccctaaaa gtggctttca ccacctcatc catgagagct ttggttcccc 660
   gggcaaaagc ttcccattca aataccccca caggaccatt ccacacaatc tgcttaaccc 720
   gagtgacagc ctcagcatac ttcttgctgg tttcaggacc acagtccaag ccccatccca 780
   ccagcaggta tgcaagaagg cccagtgggc ttgccagtct tggcatttct catcaacttg 840
   tcagcagtga caaagtcaac cgggaaggaa tcttcacacc atctt 885
<210> 355
   <211> 434
   <212> DNA
   <213> Homo sapiens
<400> 355
   cggctgcgag aagacgacag aaggggggag tggttgctat accttgactt catttatatg 60
   aatttccact ttattaaata atagaaaaga aaatcccggt gcttgcagta gagtgatagg 120
   acattctatg cttacagaaa atatagccat gattgaaatc aaatagtaaa ggctgttctg 180
   gctttttatc ttcttagctc atcttaaata agcagtacac ttggatgcag tgcgtctgaa 240
   gtgctaatca gttgtaacaa tagcacaaat cgaacttagg atttgtttct tctcttctgt 300
   gtttcgattt ttgatcaatt ctttaatttt ggaagcctat aatacagttt tctattcttg 360
   gagataaaaa ttaaatggat cactgatatt ttagtcattc tgcttctcat ctaaatattt 420
   ccatattctg tatt 434
<210> 356
   <211> 318
   <212> DNA
   <213> Homo sapiens
<400> 356
   gggagtgggt gctatacctt gacttcattt atatgaattt ccactttatt aaataataga 60
   aaagaaaatc ccggtgcttg cagtagagtg ataggacatt ctatgcttac agaaaatata 120
   gccatgattg aaatcaaata gtaaaggctg ttctggcttt ttatcttctt agctcatctt 180
   aaataagcag tacacttgga tgcagtgcgt ctgaagtgct aatcagttgt aacaatagca 240
   caaatcgaac ttaggatttg cttcttctct tctgtgttgc gatttttgat caattcttta 300
   attttggaag cctataat 318
<210> 357
   <211> 231
   <212> DNA
   <213> Homo sapiens
<400> 357
   cggctgcgag aagacaacag aagggggctc ccgctcggga tctcgctccg gatctcgctc 60
   cgggtcccgc agtgggtccc ggagaggaag ctttgacgcc acaaggaatt cttcctactc 120
   ttattcctac tcatttagca gtagttctat tgggcactat tagtcagttg ggagtgggtg 180
   ctataccttg acttcattta tatgaatttc cactttatta aataatagaa a          231
<210> 358
   <211> 446
   <212> DNA
   <213> Homo sapiens
<400> 358
   atttgctgta tgccgagaat ggaaaaattg gaccacctaa actggatatc agaaaggagg 60
   agaagcaaat catgattgac atatttcacc cttcagtttt tgtaaatgga gacgagcagg 120
   aagtcgatta tgatcccgaa actacctgtt acattagggt gtacaatgtg tatgtgagaa 180
   tgaacggaag tgagatccag tataaaatac tcacgcagaa ggaagatgat tgtgacgaga 240
   ttcagtgcca gttagcgatt ccagtatcct cactgaattc tcagtactgt gtttcagcag 300
   aaggagtctt acatgtgtgg ggtgttacaa ctgaaaagtc aaaagaagtt tgtattacca 360
   ttttcaatag cagtataaaa ggttctcttt ggattccagt tgttgctgct ttactactct 420
   ttctagtgct tagcctggta ttcatc                                      446
<210> 359
   <211> 209
   <212> DNA
   <213> Homo sapiens
<220>
   <221> misc_feature
   <222> 19, 185, 193
   <223> n = A,T,C or G
<400> 359
   gagaatttgc tgtatgccng agatggaaaa attggaccac ctaaactgga tatcagaaag 60
   gaggagaagc aaatcatgat tgacatattt cacccttcaa gtttttgtaa atggagacga 120
   gcaggaagtc gattatgatc ccgaaactac ctgttacatt agggtgtaca atgtgtatgt 180
   gagantgaac ggnagtgaga tccagtata                                   209
<210> 360
   <211> 521
   <212> DNA
   <213> Homo sapiens
<400> 360
   tgctgtcggt gactactgaa gaaatattcc tgacgtggtc ccgggcagcc atctgactcc 60
   aatagagaga gagagttctt cacctttaag tagtaaccag tctgaacctg gcagcatcgc 120
   tttaaactcg tatcactcca gaaattgttc tgagagtgat cactccagaa atggttttga 180
   tactgattcc agctgtctgg aatcacatag ctccttatct gactcagaat ttcccccaaa 240
   taataaaggt gaaataaaaa cagaaggaca agagctcata accgtaataa aagcccccac 300
   ctcctttggt tatgataaac cacatgtgct agtggatcta cttgtggatg atagcggtaa 360
   agagtccttg attggttata gaccaacaga agattccaaa gaattttcat gagatcagct 420
   aagttgcacc aactttgaag tctgattttc ctggacagtt ttctgcttta atttcatgaa 480
   aagattatga tctcagaaat tgtatcttag ttggtatcaa c                     521
<210> 361
   <211> 522
   <212> DNA
   <213> Homo sapiens
<400> 361
   tggccctcga ggccaagaat tcggcactag gggagaggag cttgaatttc tgacacacat 60
   aacatgtaaa aagtatttgg catttcataa ggatttgggg tggggtaaac gcaaggttag 120
   tctgttttaa aaaatgtttt cattaacgag cacataactg gtggttccta atgggaatac 180
   ttgacccagg cagaaactag aaaagtagca agtaggaaac ttccatttct ctcccctaaa 240
   caacccctta aggcactgtg agctggagac aggagaggtg ttgcccaacc tttgttcata 300
   tactcggtga cgatgtagat gggctcctca gacaccactg catagagctg gaccagcttg 360
   tcgtgcttca gcttcttcat gatctgcgct tcctcaagga atgattcggg ggacattgtg 420
   cctggtttaa gagtctttat ggctactttt gtgtttccat tccaggtacc tacaaacatc 480
   ccagaatatg aagtcaaacc aaagatcttc ttttgatgga aa                   522
<210> 362
   <211> 421
   <212> DNA
   <213> Homo sapiens
<220>
   <221> misc_feature
   <222> 12, 331, 372
   <223> n = A,T,C or G
<400> 362
   ttaatgagtt anaaatctta atatagccat cttagccata accacaaata aactcatttt 60
   ttctgttaaa atacttgaca gagtccttgc aattgaatgt ctttgttcaa caaaaactgt 120
   attaagtgtt ttaaatttaa aatctaatct tatgcaaata gctggtggtc aaaacctttt 180
   tccatcaaaa gaagatcttt ggtttgactt catattctgg gatgtttgta ggtacctgga 240
   atggaaacac aaaagtagcc ataaagactc ttaaaccagg cacaatgtcc cccgaatcat 300
   tccttgagga agcgcagatc atgaagaagc ngaagcacga caagctggtc cagctctatg 360
   gcagtggtgt cngaggagcc catctacatc gtcaccgagt atatgaacaa aggttgggca 420
   a                                                                 421
<210> 363
   <211> 503
   <212> DNA
   <213> Homo sapiens
<400> 363
   cagaaggggt ttccgaatgt tttagttagc cttttggtgg agccgccagc tgacaggaca 60
   tcttacaaga gaatttgcac atctctggaa gcttagcaat cttattgcac actgttcgct 120
   ggaagctttt tgaagagcac attctcctca gtgagctcat gaggttttca tttttattct 180
   tccttccaac gtggtgctat ctctgaaacg agcgttagag tgccgcctta gacggaggca 240
   ggagtttcgt tagaaagcgg acgctgttct aaaaaaggtc tcctgcagat ctgtctgggc 300
   tgtgatgacg aatattatga aatgtgcctt ttctgaagag attgtgttag ctccaaagct 360
   tttcctgtcg cagtgtttca gttctttatt ttcccttgtg gatatgctgt gtgaaccgtc 420
   gtgtgagtgt ggtatgcctg atcacagatg gattttgtta taagcatcaa tgtgacactt 480
   gcaggacact acaacgtggg aca                                        503
<210> 364
   <211> 365
   <212> DNA
   <213> Homo sapiens
<400> 364
   ggccgccctt tttttttttt ttggggggga aaaaattttt ttttaaaaaa aaaaaaactt 60
   cccccctggg gaaaaaaaaa ggttttttaa aaaaaaaacc aaacaaaatt ttcccgggcc 120
   ctttaggggt tttaaatttt cccccgggtt gaaccccttt taaaaaaaaa ggaatttttt 180
   tggggggaaa taatggggga aaaaccaaaa aaaaaggggg gttttttttt taaaaccctt 240
   ttttttttaa aaaaccttcc cccaggggaa aaattcccaa aaccttttaa aaaaaaaggg 300
   ccgaaatttt taatccaaag gggaaaaacc ccccccccaa caaaaaaccc ccaaagggga 360
   aaaag                                                             365
<210> 365
   <211> 680
   <212> DNA
   <213> Homo sapiens
<220>
   <221> misc_feature
   <222> 172, 173, 176, 186, 199, 200, 591, 625, 659, 670
   <223> n = A,T,C or G
<400> 365
   aggacacaga caaggaactt gctgaaaggc caaccatttc aggatcagtc aaaggcagca 60
   agcagataga ctcaaggtgt gtgaaagatg ttatacacca ggagctgcca cttcatgtcc 120
   caaccagact gtgtctgtct gtgtctgcat gtaagagtga gggagggaag gnnggnacta 180
   caaganagtc ggagatgann cagcacacac acaattcccc agcccacgtg atgcttgtgt 240
   tgaccagatg ttcctgagtc tggagcaagc acccaggcca gaataacaga gctttcttag 300
   ttggtgaaga cttaaacatc tgcctgaggt caggaggcaa tttgcctgcc ttgtacaaaa 360
   gctcaggtga aagactgaga tgaatgtctt tcctctccct gcctcccacc agacttcctc 420
   ctggaaaacg ctttggtaga tttggccagg agctttcttt tatgtaattg gataaataca 480
   cacaccatac actatccaca gatatagcca agtagatttg ggtagaggat actatttcca 540
   gaatagtgtt tagctcacct agggggatat gttgtatcac atttgcatat nccacatggg 600
   gacataagct aattttttac agacncgatt ctgtcatgct gttaatagcc atggttaanc 660
   ccccattggn ggggccggtg                                             680
<210> 366
   <211> 570
   <212> DNA
   <213> Homo sapiens
<400> 366
   taagctcggg attcggctcg agcggctcga gtcaagagaa aacacaagaa ggacatcagc 60
   cagaacaagc gagccgtgag gcggctgcgc accgcctgcg agagggccaa gaggaccctg 120
   tcgtccagca cccaggccag cctggagatc gactccctgt ttgagggcat cgacttctac 180
   acgtccatca ccagggcgag gttcgaggag ctgtgctccg acctgttccg aagcaccctg 240
   gagcccgtgg agaaggctct gcgcgacgcc aagctggaca aggcccagat tcacgacctg 300
   gtcctggtcg ggggctccac ccgcatcccc aaggtgcaga agctgctgca ggacttcttc 360
   aacgggcgcg acctgaacaa gagcatcaac cccgacgagg ctgtggccta cggggcggcg 420
   gtgcaggcgg ccatcctgat gggggacaag tccgagaacg tgcaggacct gctgctgctg 480
   gacgtggctc ccctgtcgct ggggctggag acggccggag gcgtgatgac tgccctgatc 540
   aagcgcaact ccaccatccc caccaagcag 570
<210> 367
   <211> 454
   <212> DNA
   <213> Homo sapiens

<400> 367
   gccgcccttt tttttttttt tttttttttt tttttttttg tttttttttt tttttcaaaa 60
   aaaaaaaatc tttttagaaa aaaaaacccc cccccaacaa aaaatggggg ggggggggga 120
   ttttccctcc cgggggaagg agaaaaagcc gcagtaataa aaaggggggg aaccaaaaaa 180
   tttttttttt tttttaaaaa aggttttttt gggggccccc ccccccaaaa aaaaaaaagg 240
   tcccccccct ttttttcccc cctttttggg ggggaaaaaa aaaaaagggg ggggaaaaaa 300
   acagaaaatt ttccccaaaa atttaaaaaa aaaagggggg ggggggggaa aaaaaaggtt 360
   tttttacccc cctggggggg aaaaaaaaaa aatttggggc caccaaaaag gggggggggc 420
   cccccaaaaa agggggtttt ttttaaaaaa aaaa 454
<210> 368
   <211> 651
   <212> DNA
   <213> Homo sapiens
<400> 368
   taagctcggg attcggctcg agtggtcttc gtctactccg ggtctttcag gaggccaaaa 60
   ggcagctcca gaagattgac aaatctgagg gccgcttcca tgtccagaac cttagccagg 120
   tggagcagga tgggcggacg gggcatggac tccgcagatc ttccaagttc tgcttgaagg 180
   agcacaaagc cctcaagacg ttaggcatca tcatgggcac tttcaccctc tgctggctgc 240
   ccttcttcat cgttaacatt gtgcatgtga tccaggataa cctcatccgt aaggaagttt 300
   acatcctcct aaattggata ggctatgtca attctggttt caatcccctt atctactgcc 360
   ggagcccaga tttcaggatt gccttccagg agcttctgtg cctgcgcagg tcttctttga 420
   aggcctatgg gaatggctac tccagcaacg gcaacacagg ggagcagagt ggatatcacg 480
   tggaacagga gaaagaaaat aaactgctgt gtgaagacct cccaggcacg gaagactttg 540
   tgggccatca aggtactgtg cctagcgata acattgattc acaagggagg aattgtagta 600
   caaatgactc actgctgtaa agcagttttt ctacttttaa agaccccccc c 651
<210> 369
   <211> 280
   <212> DNA
   <213> Homo sapiens
<220>
   <221> misc_feature
   <222> 112
   <223> n = A,T,C or G
<400> 369
   tggtcttcgt ctactccagg gtctttcagg aggccaaaag gcagctccag aagattgaca 60
   aatctgaggg ccgcttccat gtccagaacc ttagccaggt ggagcaggat gngcggacgg 120
   ggcatggact ccgcagatct tccaagttct gcttgaagga gcacaaagcc ctcaagacgt 180
   taggcatcat catgggcact ttcaccctct gctggctgcc cttcttcatc gttaacattg 240
   tgcatgtgat ccaggataac ctcatccgta agaagtttac                       280
<210> 370
   <211> 418
   <212> DNA
   <213> Homo sapiens
<400> 370
   ggccgccctt tttttttttt ttttttcccg ggcttttttg ggaaaaaccc ccctttccca 60
   taaaaaaatt tttttggggg tttcccaatt tttttttcca atttcaaata atttttttcc 120
   aaaaaaaacc caaacccttg gggccttttt tttttttttt aaagggcctt tttacttttc 180
   cccaaggagg ccttggggaa ataaaaaaaa cccggttggg gggccccaaa aaaggggtgg 240
   gcccccttga atcccccatt ggtttggggg taaaaaaggc ccccccatgg gcccccttcc 300
   cccggggggg ggaacccccc cccaagaccc ccccggggga aaccgggccc aaaaaaaaaa 360
   ccctttaaaa ttttaaaaaa cgggcccccc cctaaaaaaa ctttttttta aaaagggg   418
<210> 371
   <211> 292
   <212> DNA
   <213> Homo sapiens
<400> 371
   ttagggtata agttgctgta aaatttgtgt aaatttgtat ccacacaaat tcagtctctg 60
   aatacacagt attcagagtc tctgatacac agtaattgtg acaatagggc taaatgttta 120
   aagaaatcaa aagaatctat tagattttag aaaaacattt aaacttttta aaatacttat 180
   taaaaaattt gtataagcca cttgtcttga aaactgtgca actttttaaa gtaaattatt 240
   aagcagactg gaaaagtgat gtattttcat agtgacctgt gtttcactta at         292
<210> 372
   <211> 415
   <212> DNA
   <213> Homo sapiens
<400> 372
   tccttattta tttaacttca cccgagttcc tctgggtttc taagcagtta tggtgatgac 60
   ttagcgtcaa gacatttgct gaactcagca cattcgggac caatatatag tgggtacatc 120
   aagtccatct gacaaaatgg ggcagaagag aaaggactca gtgtgtgatc cggtttcttt 180
   ttgctcgccc ctgttttttg tagaatctct tcatgcttga catacctacc agtattattc 240
   ccgacgacac atatacatat gagaatatac cttatttatt tttgtgtagg tgtctgcctt 300
   cacaaatgtc attgtctact cctagaagaa ccaaatacct caatttttgt ttttgagtac 360
   tgtactatcc tgtaaatata tcttaagcag gtttgttttc agcactgatg gaaaa      415
<210> 373
   <211> 326
   <212> DNA
   <213> Homo sapiens
<400> 373
   tccttattta tttaacttca cccgagttcc tctgggtttc taagcagtta tggtgatgac 60
   ttagcgtcaa gacatttgct gaactcagca cattcgggac caatatatag tgggtacatc 120
   aagtccatct gacaaaatgg ggcagaagag aaaggactca gtgtgtgatc cggtttcttt 180
   ttgctcgccc ctgttttttg tagaatctct tcatgcttga catacctacc agtattattc 240
   ccgacgacac atatacatat gagaatatac cttatttatt tttgtgtagg ggtctgcctt 300
   cacaaatgtc attgtctact cctaca 326
<210> 374
   <211> 324
   <212> DNA
   <213> Homo sapiens
<400> 374
   tccttattta tttaacttca cccgagttcc tctgggtttc taagcagtta tggtgatgac 60
   ttagcgtcaa gacatttgct gaactcagca cattcgggac caatatatag tgggtacatc 120
   aagttcatct gacaaaatgg ggcagaagag aaaggactca gtgtgtgatc cggtttcttt 180
   ttgctcgccc ctgttttttg tagaatcttt tcatgcttga catacctacc agtattattc 240
   ccgacgacac atatacatat gagaatatac cttatttatt tttgagtagg tgtctgcctt 300
   cacaaatggc attggctact ccag 324
<210> 375
   <211> 466
   <212> DNA
   <213> Homo sapiens
<400> 375
   taactctggg aggggctcga gagggctggt ccttatttat ttaacttcac ccgagttcct 60
   ctgggtttct aagcagttat ggtgatgact tagcgtcaag acatttgctg aactcagcac 120
   attcgggacc aatatatagt gggtacatca agtccatctg acaaaatggg gcagaagaga 180
   aaggactcag tgtgtgatcc ggtttctttt tgctcgcccc tgttttttgt agaatctctt 240
   catgcttgac atacctacca gtattattcc cgacgacaca tatacatatg agaatatacc 300
   ttatttattt ttgtgtaggt gtctgccttc acaaatgtca ttgtctactc ctagaagaac 360
   caaatacctc aatttttgtt tttgagtact gtactatcct gtaaatatat cttaagcagg 420
   tttgttttca gcactgatgg aaaataccag tgttgggttt tttttt 466
<210> 376
   <211> 324
   <212> DNA
   <213> Homo sapiens
<400> 376
   tccttattta tttaacttca cccgagttcc tctgggtttc taagcagtta tggtgatgac 60
   ttagcgtcaa gacatttgct gaactcagca cattcgggac caatatatag tgggtacatc 120
   aagttcatct gacaaaatgg ggcagaagag aaaggactca gtgtgtgatc cggtttcttt 180
   ttgctcgccc ctgttttttg tagaatcttt tcatgcttga catacctacc agtattattc 240
   ccgacgacac atatacatat gagaatatac cttatttatt tttgagtagg tgtctgcctt 300
   cacaaatggc attggctact ccag 324
<210> 377
   <211> 326
   <212> DNA
   <213> Homo sapiens
<400> 377
   tccttattta tttaacttca cccgagttcc tctgggtttc taagcagtta tggtgatgac 60
   ttagcgtcaa gacatttgct gaactcagca cattcgggac caatatatag tgggtacatc 120
   aagtccatct gacaaaatgg ggcagaagag aaaggactca gtgtgtgatc cggtttcttt 180
   ttgctcgccc ctgttttttg tagaatctct tcatgcttga catacctacc agtattattc 240
   ccgacgacac atatacatat gagaatatac cttatttatt tttgtgtagg ggtctgcctt 300
   cacaaatgtc attgtctact cctaca 326
<210> 378
   <211> 494
   <212> DNA
   <213> Homo sapiens
<400> 378
   atgccccgca tagatgcgga cctcaagctc gacttcaagg atgtcctgct ccgacctaag 60
   cggagcagcc tcaagagccg agccgaggtg gatcttgaac gcaccttcac gtttcgaaat 120
   tcaaagcaga cctactcagg gattcccatc atcgtggcca acatggacac tgtgggcacg 180
   tttgagatgg cagccgtgat gtcacagcac tccatgttta cagcaattca taagcattac 240
   tccctggatg actggaagct ctttgccaca aatcacccag aatgcctgca gaatgtagcc 300
   gtgagttcag gcagtgggca gaatgatctg gaaaagatga ccagcatcct ggaagctgtg 360
   ccacaggtta agtttatttg cctggatgtg gccaatgggt attcaaaaca ttttgtggaa 420
   ttcgtgaaac ttgtccgtgc caaatttcct gaacacacca ttatggcagg gaacgtggtg 480
   acaggagaaa tggt 494
<210> 379
   <211> 243
   <212> DNA
   <213> Homo sapiens
<400> 379
   gccgctgcac catgccccgc atagatgcgg acctcaagct cgacttcaag gatgtcctgc 60
   tccgacctaa gcggacagcc tcaagagccg agccgaggtg gatcttgaac gcaccttcac 120
   gtttcgaaat tcaaagcaga cctactcagg gattcccatc atcgtggcca acatggacac 180
   tgtgggcacg tttgagatgg cagccgtgat gtcacagcac tccatgttta cagcaattca 240
   taa 243
<210> 380
   <211> 804
   <212> DNA
   <213> Homo sapiens
<400> 380
   gcaaatgttt gattaattct gctcatatgc acatctgaaa gcatgagaca cactccacag 60
   acagcacgca ctggagctgg tggggcagat gggcactcgc cgattaggta ttaatgtcaa 120
   taatacgtgc ataaagtgct gataaaataa cttaagtgtt acaaaaacag acagtccacg 180
   gtggctgcag gcacatgcag gcgggactgg gtcagacact ccagggctgc acatgttcca 240
   gctggcctga gtccgacacg tcatagctgg ccttgtactt ggccaggatt ttcatgaggg 300
   gccgtagctt gagccaccac tgttctttgg gaatcctgtg ctcaaaatcc gtttgcttct 360
   tcagctctgc cacaggtttg aaaaataacg tttcttttgc ttattcccag cacacaaatg 420
   gaatcatcgg tggtaaattt ttttcctctg ccccgggcct ccttgagttt tgcagtgatc 480
   cactccatag ctctggcaga gattttggtt ccaaagtttc tatcaaatgg agagggtgcc 540
   ccaccctgct gcatgtgacc cagcacgttc ttcctgcagt caaacacgcc tttgccctct 600
   tctgaataca gctggtaaat gaagtcggtg gtgtagtttt cactgcagct ctcatttctg 660
   agcacaaggc ctctctggat ggtggtcttc attttctccg tcaggtgctc cacgttggac 720
   tgcagatcct gatgtcgaag ggctcttcga aatgtatgcg gcatcagtcc ggccgcagcc 780
   ccccatgttg gcaggtagca cagt                                        804
<210> 381
   <211> 624
   <212> DNA
   <213> Homo sapiens
<220>
   <221> misc_feature
   <222> 514
   <223> n = A,T,C or G
<400> 381
   tggagttgta ggcaaatgtt taattaattc tgctcatatg cacatctgaa agcatgagac 60
   acactccaca gacagcacgc actggggctg gtggggcaga tgggcactcg ccgattaggt 120
   attaatgtca ataatacgtg cataaagtgc tgataaaata acttaagtgt tacaaaaaca 180
   gacagtccac ggtggctgca ggcacatgca ggcgggactg ggtcagacac tccagggctg 240
   cacatgttcc agctggcctg agtccgacac gtcatagctg gccttgtact tggccaggat 300
   tttcatgagg ggccgtagct tgagccacca ctgttctttg ggaatcctgt gctcaaaatc 360
   cgtttgcttc ttcagctctg ccacaggttg aaaaataacg tttcttttgc ttattcccag 420
   cacacaaatg gaatcatcgg tggtaaattt ttttcctctg ccccgggcct ccttgagttt 480
   tgcagtgatc cactccatag ctctggcaga gatnttggtt ccaaagtttc tatcaaatgg 540
   agaggtgccc caccctgctg atgtgacccc acacgttctt cctgagtcaa acacgccttt 600
   gccctcttct gaatacaagc tggt                                        624
<210> 382
   <211> 507
   <212> DNA
   <213> Homo sapiens
<220>
   <221> misc_feature
   <222> 301, 460, 498
   <223> n = A,T,C or G
<400> 382
   ttttttggag ttgtaggaaa tgtttaattc tgctcatatg cacatctgaa agcatgagac 60
   acactccaca agacagcacg cactggggct ggtggggcag atgggcactc gcgattaggt 120
   attaatgtta ataatacgtg cataaagtgc tgataaaata acttaagtgt tacaaaaaca 180
   gacagtccac ggtggctgca ggcacatgca ggcgggactg ggtcagacac tccagggctg 240
   cacatgttcc agctggcctg agtcccgaca cgtcatagct ggccttgtac ttggccaggg 300
   nttttcatga ggggccctag ctttgagcca ccacttgttc tttggggaat cctgtgcttc 360
   aaaatcccgt tttgcttctt tcagctcttc ccacaggttt gaaaaataac gttttctttt 420
   tgcttatttc ccagcacaca aatgggattc atcggtgggn aatttttttc ctctgccccg 480
   gggcttcttg agtttttnca gtgattc 507
<210> 383
   <211> 224
   <212> DNA
   <213> Homo sapiens
<220>
   <221> misc_feature
   <222> 198, 219
   <223> n = A,T,C or G
<400> 383
   atcagatccc aaagaccaat tgcaacgtag ctgtcatcaa cgtgggggca cccgcggctg 60
   ggatgaacgc ggccgtacgc tcagctgtgc gcgtgggcat tgccgacggc acaggatgct 120
   cgccatctat gatggtttga cggcttcgca agggccagat caaagaaatc ggctggacag 180
   atgtcggggg ctggaccngc caaggaggct ccattcttng gaca 224
<210> 384
   <211> 507
   <212> DNA
   <213> Homo sapiens
<220>
   <221> misc_feature
   <222> 301, 460, 498
   <223> n = A,T,C or G
<400> 384
   ttttttggag ttgtaggaaa tgtttaattc tgctcatatg cacatctgaa agcatgagac 60
   acactccaca agacagcacg cactggggct ggtggggcag atgggcactc gcgattaggt 120
   attaatgtta ataatacgtg cataaagtgc tgataaaata acttaagtgt tacaaaaaca 180
   gacagtccac ggtggctgca ggcacatgca ggcgggactg ggtcagacac tccagggctg 240
   cacatgttcc agctggcctg agtcccgaca cgtcatagct ggccttgtac ttggccaggg 300
   nttttcatga ggggccctag ctttgagcca ccacttgttc tttggggaat cctgtgcttc 360
   aaaatcccgt tttgcttctt tcagctcttc ccacaggttt gaaaaataac gttttctttt 420
   tgcttatttc ccagcacaca aatgggattc atcggtgggn aatttttttc ctctgccccg 480
   gggcttcttg agtttttnca gtgattc 507
<210> 385
   <211> 224
   <212> DNA
   <213> Homo sapiens
<220>
   <221> misc_feature
   <222> 198, 219
   <223> n = A,T,C or G
<400> 385
   atcagatccc aaagaccaat tgcaacgtag ctgtcatcaa cgtgggggca cccgcggctg 60
   ggatgaacgc ggccgtacgc tcagctgtgc gcgtgggcat tgccgacggc acaggatgct 120
   cgccatctat gatggtttga cggcttcgca agggccagat caaagaaatc ggctggacag 180
   atgtcggggg ctggaccngc caaggaggct ccattcttng gaca                  224
<210> 386
   <211> 232
   <212> DNA
   <213> Homo sapiens
<400> 386
   acgacagaag ggtacggctg cgagaagacg acagatgggt acggctgtga gaagacgact 60
   gatgggaaca gctaaggact gctaaacccc actctgcatc aactgaacgc aaatcagcca 120
   ctttaattaa gctaagccct tactagacca atgggactta aacccacaaa cacttagtta 180
   acagctaagc accctaatca actggcttca atgtacttct cccgccgtcg gg         232
<210> 387
   <211> 339
   <212> DNA
   <213> Homo sapiens
<400> 387
   tactggtttt ggagaacttg tctacaacca gggattgatt ttaaagatgt ctttttttat 60
   tttacttttt tttaagcacc aaattttgtt gttttttttt ttttctccct tccccacaaa 120
   tcccttttaa aatatttttg ttaaccccct ttccaacggg ccgaggaaac ttaaaacccc 180
   tttttcctcg gcctggttcc tctttaattt ttaatttttc cccatcagtt taaaggtttt 240
   ggcatacttg gcatcttttt tcaaagggaa aacttttttt gccattcttt ggacttcccc 300
   ttttttaaag gaaatggggg ggccaaaagg ggatttcaa                        339
<210> 388
   <211> 456
   <212> DNA
   <213> Homo sapiens
<400> 388
   tttttttttt tttttttttt tttaaccatc aaattcacag ctatttttcg cttttagtgt 60
   gctcacagaa aattagaaca ccttaagcag gagtttaata gcattttttg taagcaaagt 120
   tacattccat ctctaagtca aattggtcaa agcttctcca gtatttacaa aacatgatag 180
   acaagatgct acacaaaacc attgcatctg aagattttgt tttcctttat tctcaaagac 240
   gactggaaaa gaaagcatta tctgctgtaa tcaaaaacat accacagtat aaacagttac 300
   cattccactt atcacagctt ggttgagttt agaattagtg ttttaaaaag tccaagatga 360
   ctgcagtttt acaaaaatgg gcagggtgga aagttgcaaa cttcatgtgc ttctggatat 420
   caagatttgt ttttatacaa tagtcacagt taaaaa                           456
<210> 389
   <211> 490
   <212> DNA
   <213> Homo sapiens
<400> 389
   ttacattgaa tactacatat gtcgagggaa tgcagaaaga gttaaggaag gcaggttgtc 60
   ctgctatgga ggccactctt cgttttccat gtactgcatg ctgtttgtgg cactttatct 120
   tcaagccagg atgaagggag actgggcaag actcttaccc cccacactgc aatttggtct 180
   tgttgccgta tccatttatg tgggcctttc tcgagttgct gattataaac accactggag 240
   cgatgtgttg actggactca ttcagggagc tctggttgca atattagttg ctgtatatgt 300
   atcggatttc ttcaaagaaa gaacttcttt taaagaaaga aaagaggagg actctcatac 360
   aactctgcat gaaacaccaa caactgggaa tcactatccg agcaatcacc agccttgaaa 420
   ggcagcaggg tgcccaggtg aagctggcct gttttctaaa ggaaaatgat tgccacaagg 480
   caagaggatg                                                        490
<210> 390
   <211> 334
   <212> DNA
   <213> Homo sapiens
<400> 390
   gaactcggtg gtggccactg cgcagaccag acttcgctcg tactcgtgcg cctcgcttcg 60
   cttttcctcc gcaaccatgt ctgacaaacc cgatatggct gagatcgaga aattcgataa 120
   gtcgaaactg aagaagacag agacgcaaga gaaaaatcca ctgccttcca aagaaacgat 180
   tgaacaggag aagcaagcag gcgaatcgta atgaggcgtg cgccgccaat atgcactgta 240
   cattccacaa gcattgcctt cttattttac ttcttttagc tgtttaactt tgtaagatgc 300
   aaagaggttg gatcaagatt aaatgactgt gctg                             334
<210> 391
   <211> 377
   <212> DNA
   <213> Homo sapiens
<220>
   <221> misc_feature
   <222> 349
   <223> n = A,T,C or G
<400> 391
   gaactcggtg gtggccactg cgcagaccag acttcgctcg tactcgtgcg cctcgcttcg 60
   cttttcctcc gcaaccatgt ctgacaaacc cgatatggct gaggtcgaga aattcgataa 120
   gtcgaaactg aagaagacag agacgcaaga gaaaaatcca ctgccttcca aagaaacgat 180
   tgaacaggag aagcaagcag gcgaatcgta atgaggcgtg cgccgccaat atgcactgta 240
   cattccacaa gcattgcctt cttattttac ttcttttagc tgtttaactt tgtaagacgc 300
   atagagggtg gatcaagttt aaatgactgt gctgcccctt tcacatcana gaactactga 360
   caacgaaggc cgcgcct 377
<210> 392
   <211> 555
   <212> DNA
   <213> Homo sapiens
<400> 392
   ctcggtggtg gccactgcgc agaccagact tcgctcgtac tcgtgcgcct cgctttgctt 60
   ttcctccgca accatgtctg acaaacccga tatggctgag atcgagaaat tcgataagtc 120
   gaaactgaag aagacagaga cgcaagagaa aaatccactg ccttccaaag aaacgattga 180
   acaggagaag caagcaggcg aatcgtaatg aggcgtgcgc cgccaatatg cactgtacat 240
   tccacaagca ttgccttctt attttacttc ttttagctgt ttaactttgt aagatgcaaa 300
   gaggttggat caagtttaaa tgactgtgct gcccctttca catcaaagaa ctactgacaa 360
   cgaaggccgc gcctgccttt cccatctgtc tatctatctg gctggcaggg aaggaaagaa 420
   cttgcatgtt ggtgaaggaa gaagtggggt ggaagaagtg gggtgggacg acagtgaaat 480
   ctagagtaaa accaagctgg cccaaggtgt cctgcaggct gtaatgcagt ttaatcagag 540
   tgccattttt ttttt 555
<210> 393
   <211> 300
   <212> DNA
   <213> Homo sapiens
<400> 393
   gctcaattgg actatgttga cctctatctt attcattctc caatgtctct aaagccaggt 60
   gaggaacttt caccaacaga tgaaaatgga aaagtaatat ttgacatagt ggatctctgt 120
   accacctggg aggccatgga gaagtgtaag gatgcatgat tggccaagtc cattggggtg 180
   tcaaacttca accgcaggca gctggagatg atcctcaaca agccaggact caagtacaag 240
   cctggctgca accaggtaga aagtcattcg tatttcaacc ggagtaaatt gctagaatcg 300
<210> 394
   <211> 344
   <212> DNA
   <213> Homo sapiens
<400> 394
   acagaagggt acggctgcga gaagacgaca gaagggtacg gctgcgagaa gacgacagaa 60
   gggtacggct gcgagaagac gacagaaggg taaaacactg aactgacaat taacagccca 120
   atatctacaa tcaaccgaca agtcattatt accctcactg tcaacccaac acaggcatgc 180
   tcataaggaa aggttaaaaa aagtaaaagg aactcggcaa atcttacccc gcctgtttac 240
   caaaaacatc acctgtagca tcaccagtat tagaggcacc gcctgcccag tgacacatgt 300
   ttaacggccg cggtacccta accgtgcaaa ggtagcataa tcac 344
<210> 395
   <211> 507
   <212> DNA
   <213> Homo sapiens
<400> 395
   tgctcggtcc ttccgaggaa gctaaggctg cgttggggtg aggccctcac ttcatccggc 60
   gactagcacc gcgtccggca gcgccagccc tacactcgcc cgcgccatgg cctctgtctc 120
   cgagctcgcc tgcatctact cggccctcat tctgcacgac gatgaggtga cagtcacgga 180
   ggataagatc aatgccctca ttaaagcagc cggtgtaaat gttgagcctt tttggcctgg 240
   cttgtttgca aaggccctgg ccaacgtcaa cattgggagc ctcatctgca atgtaggggc 300
   cggtggacct gctccagcag ctggtgctgc accagcagga ggtcctgccc cctccactgc 360
   tgctgctcca gttgaggaga agaaagtgga agcaaagaaa gaagaatccg aggagtctga 420
   tgatgacatg ggctttggtc tttttgacta aacctctttt ataacatgtt caataaaaag 480
   ctgaacttta aaaaaaaaaa aaaaaaa 507
<210> 396
   <211> 488
   <212> DNA
   <213> Homo sapiens
<400> 396
   gaggccctca cttcatccgg cgactagcac cgcgtccggc agcgccagcc ctacactcgc 60
   ccgcgccatg gcctctgtct ccgagctcgc ctgcatctac tcggccctca ttctgcacga 120
   cgatgaggtg acagtcacgg aggataagat caatgccctc attaaagcag ccggtgtaaa 180
   tgttgagcct ttttggcctg gcttgtttgc aaaggccctg gccaacgtca acattgggag 240
   cctcatctgc aatgtagggg ccggaggacc tgctccagca gctggtgctg caccagcagg 300
   aggtcctgcc ccctgcactg ctgctgctcc agttgaggag aagaaagtgg aagcatagaa 360
   agaagaatcc gacgagtctg atgatgacat gggctatggt ctttttgact aaacctcttt 420
   tataacatgt tcaataaaaa gctgaacttt aaaaagaaaa aaaaaaaact cgagcctcta 480
   gaactata 488
<210> 397
   <211> 180
   <212> DNA
   <213> Homo sapiens
<400> 397
   ctgcgttggg gtgaggccct cacttcatcc ggcgactagc accgcgtccg gcagcgccag 60
   ccctacactc gcccgcgcca tggcctctgt ctccgagctc gcctgcatct actcggccct 120
   cattctgcac gacgatgagg tgacagtcac ggaggataag atcaatgccc tcattaaagc 180
<210> 398
   <211> 491
   <212> DNA
   <213> Homo sapiens
<220>
   <221> misc_feature
   <222> 12, 154, 255, 348, 368, 402, 409, 450, 471
   <223> n = A,T,C or G
<400> 398
   tttttttttt tntttcactg ttcaaggttt attgggggtt ttagttggta taacacttgg 60
   atagttggtt gcattgtttg tatgtagatc tttttacatt atatggtaat gtacactact 120
   gatatagttc acaaaataag atcctttgga aganttatac acaagacatg atattggatt 180
   tatacactgg atcccaggga tgtgactcac tgggaaaaaa tgttggacta ggcatgttca 240
   gtgaaggagc caggnagtta tataacacac ggtaaacatc cacctggctc aaggggcaaa 300
   tgcagtacgt acagcattgg cagtggtgcg tcagaggtgg cagaactntt tcacactaac 360
   cagttganga ctacacaaga ttaataccat ccagcatcag gntatagcnt gtggatttta 420
   caaaccattt cttatttcta actttcaggn gttgatgttt ttcccagtcc ntcttaaaat 480
   ttttactgct t                                                      491
<210> 399
   <211> 235
   <212> DNA
   <213> Homo sapiens
<400> 399
   tgatttctgt ggatcccagc ttggttccag gaattttgtg tgattggctt aaatccagtt 60
   ttcaatcttc gacagctggg ctggaacgtg aactcagtag ctgaacctgt ctgacccggt 120
   cacgttcttg gatcctcaga actctttgct cttgtcgggg tgggggtggg aactcacgtg 180
   gggagcggtg gctgagaaaa tgtaaggatt ctggaataca tattccatgg gactt      235
<210> 400
   <211> 465
   <212> DNA
   <213> Homo sapiens
<400> 400
   tacggctgcg agaagacgac agaagggtac ggctgcgaga agacgacaga agggtacggc 60
   tgcgagaaga cgacagaagg gtacggctgc gagaagacga cagaagggtg atttctgtgg 120
   atcccagctt ggttccagga attttgtgtg attggcttaa atccagtttt caatcttcga 180
   cagctgggct ggaacgtgaa ctcagtagct gaacctgtct gacccggtca cgttcttgga 240
   tcctcagaac tctttgctct tgtcggggtg ggggtgggaa ctcacgtggg gagcggtggc 300
   tgagaaaatg taaggattct ggaatacata ttccatggga ctttccttcc ctctcctgct 360
   tcctcttttc ctgctcccta acctttcgcc gaatggggca gcaccactga cgtttctggg 420
   cggccagtgc ggctgccagg ttcctgtact actgccttgt acttt                 465
<210> 401
   <211> 243
   <212> DNA
   <213> Homo sapiens
<400> 401
   tgatttctgt ggatcccagc ttggttccag gaattttgtg tgattggctt aaatccagtt 60
   ttcaatcttc gacagctggg ctggaacgtg aactcagtag ctgaacctgt ctgacccggt 120
   cacgttcttg gatcctcaca actctttgct cttgtcgggg tgggggtggg aactcacgtg 180
   gggagcggtg gctgagaaaa tgtaaggatt ctggaataca tattccatgg gactttcctt 240
   ccc 243
<210> 402
   <211> 506
   <212> DNA
   <213> Homo sapiens
<400> 402
   ttctagcatc ctcttaacgt gcagcaaaag caggcgacaa aatctcctgg ctttacagac 60
   aaaaatattt cagcaaacgt tgggcatcat ggtttttgaa ggctttagtt ctgctttctg 120
   cctctcctcc acagccccaa cctcccaccc ctgatacatg agccagtgat tattcttgtt 180
   cagggagaag atcatttaga tttgttttgc attccttaga atggagggca acattccaca 240
   gctgccctgg ctgtgatgag tgtccttgca ggggccggag taggagcact ggggtggggg 300
   cggaattggg gttactcgat gtaagggatt ccttgttgtt gtgttgagat ccagtgcagt 360
   tgtgatttct gtggatccca gcttggttcc aggaattttg tgtgattggc ttaaatccag 420
   ttttcaatct tcgacagctg ggctggaacg tgaactcagt agctgaacct gtctgacccg 480
   gtcacgttct tggatcctca gaactc 506
<210> 403
   <211> 390
   <212> DNA
   <213> Homo sapiens
<400> 403
   gtagtcgcct ctctttcagc agttacccag ggtttttgga gtctctggat gatttttaca 60
   ttcttagcag tggattgata ttgctgcaga ccacaaacag tgtgtttaat aaaaccctgc 120
   taaagcaggt aatacccgag actctcctgt cctggcaaag agtccgtgtg gccaatatga 180
   tggcagatag tggcaagagg tgggcagaca tcttttcaaa atacaactct ggcacctata 240
   acaatcaata catggttctg gacctgaaga aagtaaagct gaaccacagt cttgacaaag 300
   gcactctgta cattgtggag caaattccta catatgtaga atattctgaa caaactgatg 360
   ttctacggaa aggatattgg ccctcctaca 390
<210> 404
   <211> 372
   <212> DNA
   <213> Homo sapiens
<400> 404
   aggagattca gaagcacaac cacagcaaga gcacctggct gatcctgcac cacaaggtgt 60
   acgatttgac caaatttctg gaagagcatc ctggtgggga agaagtttta agggaacaag 120
   ctggaggtga cgctactgag aactttgagg atgtcgggca ctctacaaat gccagggaaa 180
   tgtccaaaac attcatcatt ggggagctcc atccagatga cagaccaaag ttaaacaagc 240
   ctccggaaac tcttatcact actattgatt ctagttccag ttggtggacc aactgggtga 300
   tccctgccat ctctgcagtg gccgtcgcct tgatgtatcg cctatacatg gcagaggact 360
   gaacacctcc tc 372
<210> 405
   <211> 619
   <212> DNA
   <213> Homo sapiens
<400> 405
   tcccgggtgg agctggctga gtcgcgcgct ctgctccacc cgacggggct gtgtgtgctg 60
   ggcctggctc gcggcgaacc gagatggcag agcagtcgga cgaggccgtg aagtactaca 120
   ccctagagga gattcagaag cacaaccaca gcaagagcac ctggctgatc ctgcaccaca 180
   aggtgtacga tttgaccaaa tttctggaag agcatcctgg tggggaagaa gttttaaggg 240
   aacaagctgg aggtgacgct actgagaact ttgaggatgt cgggcactct acaaatgcca 300
   gggaaatgtc caaaacattc atcattgggg agctccatcc agatgacaga ccaaagttaa 360
   acaagcctcc ggaaactctt atcactacta ttgattctag ttccagttgg tggaccaact 420
   gggtgatccc tgccatctct gcagtggccg tcgccttgat gtatcgccta tacatggcag 480
   aggactgaac acctcctcag aagtcagcgc aggaagagcc tgctttggac acgggagaaa 540
   agaagccatt gctaactact tcaactgaca gaaaccttca cttgaaaaca atgattttaa 600
   tatatctctt tctttttct                                              619
<210> 406
   <211> 499
   <212> DNA
   <213> Homo sapiens

<400> 406
   taagctcgga attcggctcg agggctccag ctgagctcct gcttctactg aggacatacc 60
   tcccagatga ggtggggccc ccaaccccat tccctgagcc tggagcagag ccccctctca 120
   ctgtgggctt gctcaaagcc ctgctggagc agactggggc tcaaggatgg ctgtcgggcc 180
   cagttctaag cccatatgag gacatcctat gggaccccag cactccaccc ccgactccac 240
   ctcgggacct atgactaccc ttcaggcatc agaacactca gggcctggag gcttgcttgg 300
   gactggaggc ttgcttggac agttcctctg tgtcactgac acaggaaatc atttctagga 360
   cacagtgatc agggaagggt gcctgggact tggagggtcc catgtatgga cctgtgtatg 420
   caatactgtt ctgtcatctg gagctatttt taagatgtgt gtgttaaata tatacatagt 480
   ttaatatata aaaaaaaaa                                              499
<210> 407
   <211> 229
   <212> DNA
   <213> Homo sapiens
<400> 407
   ggctccagct gagctcctgc ttctactgag gacatacctc ccagatgagg tggggccccc 60
   aaccccattc cctgagcctg gagcagagcc ccctctcact gtgggcttgc tcaaagccct 120
   gctggagcag actggggctc aaggatggct gtcgggccca gttctaagcc catatgagga 180
   catcctatgg gaccccagca ctccaccccc gactccacct cgggaccta             229
<210> 408
   <211> 467
   <212> DNA
   <213> Homo sapiens
<400> 408
   ggaagttctg cgctggtcgg cggagtatca agtggccatg gggagcctca gcggtctgcg 60
   cctggcagca ggaagctgtt ttaggttatg tgaaagagat gttggcctca tctctaaggc 120
   ttaccagaag ctctgatttg aagagaataa atggattttg cacaaaacca caggaaagtc 180
   ccggagctcc atcccgcact tacaacagag tgcctttaca caaacctacg gattggcaga 240
   aaaagatcct catatggtca ggtcgcttca aaaaggaaga tgaaatccca gagactgtct 300
   cgttggagat gcttgatgct gcaaagaaca agatgcgagt gaagatcagc tatctaatga 360
   ttgccctgac ggtggtagga tgcatcttca tggttattga gggcaagaag gctgcccaaa 420
   gacacgagac tttaacaagc ttgaacttat aaaagaaagc tcgtctg              467
<210> 409
   <211> 338
   <212> DNA
   <213> Homo sapiens
<400> 409
   ggaagttctg cgctggtcgg cggagtagca agtggccatg gggagcctca gcggtctgcg 60
   cctggcagca ggaagctgtt ttaggttatg tgaaagagat gtttcctcat ctctaaggct 120
   taccagaagc tctgatttga agagaataaa tggattttgc acaaaaccac aggaaagtcc 180
   cggagctcca tcccgcactt acaacagagt gcctttacac aaacctacgg attggcagaa 240
   aaagatcctc atatggtcag gtcgcttcaa aaaggaagat gaaatcccag agactgtctc 300
   gttggagatg cttgatgctg cagagatcaa gatgcgag                         338
<210> 410
   <211> 601
   <212> DNA
   <213> Homo sapiens
<400> 410
   tttgcacgat gccttccaca tcccacggcg ctgctgctgg gggcagattg gcctggggag 60
   gcagcacttg ctctccagct catctgggtt gcttttcccc gcagtggata tcacaggcta 120
   aagggggggg cagtccccac catatttgag tctttctcca agttgcgccg gacaaccaag 180
   accaaaggac acagttaccc acctggcccc tctgaagtca gccggctcag acgatgcagg 240
   aagcgctgct ccgagggccg agggcccaca actccatttt ctccacctcc acctgctgat 300
   gtcacctgct ttcctgtgga agaggcctca gcacctgcca ctttgccggc ctccccagct 360
   gggaggctgg agcctggcct tagcagcccc ttttcagacc tactgggccc cttgggtgcc 420
   caggcagatg aagcaggctg cagcgcccag ccttcaccag agcggcagcc ctcccctctc 480
   gaaccacggc cagtctcccc ctcagcgtat atgctgcgcc tgcccccacc cgccggagcc 540
   tacatccaga atgaacacag ctaccaggtg ggcagcgcct tactctggaa gcggcgagcc 600
   g                                                                 601
<210> 411
   <211> 52
   <212> DNA
   <213> Homo sapiens
<400> 411
   gccccttggg tgcccaggca gatgaagcag gctgcagcgc ccagccttca cc 52
<210> 412
   <211> 525
   <212> DNA
   <213> Homo sapiens
<400> 412
   cgtttcggtt tctagggttg ttacgaagct gcaggagcga gatggaggtg gacgcaccgg 60
   gtgttgatgg tcgagatggt ctccgggagc ggcgaggctt tagcgaggga gggaggcaga 120
   acttcgatgt gaggcctcag tctggggcaa atgggcttcc caaacactcc tactggttgg 180
   acctctggct tttcatcctt ttcgatgtgg tggtgtttct ctttgtgtat tttttgccat 240
   gacttgttcg ctgatatcta aattaagaag ttggttcttg agtgaattct gaaaatggct 300
   acaaacttct tgaataaaga agacaggact ctcaatagaa gaatttcaca tctccaaggg 360
   acccttcctt tcattttaca ctttgttact aatttgcaga actctattaa ttgggtagga 420
   tttcacccat tcctagctaa gttcttaaaa ttaaaccctt tggttcgtgt ttaaaaactt 480
   tcaaacatct gatggcttta caggggctga atataaaagc atttg                 525
<210> 413
   <211> 604
   <212> DNA
   <213> Homo sapiens
<220>
   <221> misc_feature
   <222> 12, 14, 18, 20, 24, 27, 29, 31, 33, 35, 54, 594, 595
   <223> n = A,T,C or G
<400> 413
   ttcgaaccca tncntttncn atcnganana ngntnctagt tcttctgaag accncatcga 60
   ttcgtttcgg tttctagggt tgttacgaag ctgcaggagc gagatggagg tggacgcacc 120
   gggtgttgat ggtcgagatg gtctccggga gcggcgaggc tttagcgagg gagggaggca 180
   gaacttcgat gtgaggcctc agtctggggc aaatgggctt cccaaacact cctactggtt 240
   ggacctctgg cttttcatcc ttttcgatgt ggtggtgttt ctctttgtgt attttttgcc 300
   atgacttgtt cgctgatatc taaattaaga agttggttct tgagtgaatt ctgaaaatgg 360
   ctacaaactt cttgaataaa gaagacagga ctctcaatag aagaatttca catctccaag 420
   ggacccttcc tttcatttta cactttgtta ctaatttgca gaactctatt aattgggtag 480
   gatttcaccc attcctagct aagttcttaa aattaaaccc tttggttcgt gtttaaaaac 540
   tttcaaacat ctgatggctt tacaggggct gaatataaaa gcatttgtac ttannaaaaa 600
   aaaa                                                              604
<210> 414
   <211> 285
   <212> DNA
   <213> Homo sapiens
<400> 414
   ctctaacgtg ggcaacagag accctgtctc aaaaagaaaa tattcctgtt agccctaaag 60
   gctttacatg aggaatggta gaagtggtct tttgtttaaa ttagttgcat tcagcatata 120
   tgaattgtct taaatatttt ggggatactc cccgcctttt taaacagggc ataagatctg 180
   gtaaactctc tgtatatctt cctacctttc aaaatcgttc ttagggttag tcaagtctgg 240
   aatataattg ctgactataa agttagcaat tatgctttaa ggtga                 285
<210> 415
   <211> 241
   <212> DNA
   <213> Homo sapiens
<400> 415
   atttacactt gatggctaat aaagatggac agctaatgac agaattattt aatcgattag 60
   aaagtcagca tcatttccag atagaaaagg ctctagttga gaaacttcag caggattttg 120
   tagctgactg gtgctctgag ggagagtgcc tagcagctat taactccacc tataatactt 180
   cagggtatat tttggatcca cacactgctg ttgcaaaagt ggttgcagat agggtgcaag 240
   a                                                                 241
<210> 416
   <211> 315
   <212> DNA
   <213> Homo sapiens
<400> 416
   cggcttctgg aagagggggt gttgcggcag atccctgtag tgggcttcgt gctgaattgg 60
   ttttctccgg tccaggcttc acagtaggga agaactttta acttgacagc aggctctctg 120
   gagtccacag aacccatata tgtctacaaa gcacaaggtg caggagtcac gctgcctcca 180
   acgccctcgg gcagtcgcac caagcagagg cttccaggcc agaagccttt taaaaggtcc 240
   ctgcgaggtt cagatgcttt gagtgagacc agctcagtca gtcatattga agacttagaa 300
   aaggtggagc gccta                                                  315
<210> 417
   <211> 164
   <212> DNA
   <213> Homo sapiens
<400> 417
   tggatccccc gggctgcagg aattcgaatt ctgtgtgtgt gtgtgtgtat gaatggtata 60
   tttattacat tatttagaaa gagaatgagt gtgttatgag gataatgtta tatacagtct 120
   aagtggatgt ttctgtttgg cacagaatgt aggatttctg aaac                  164
<210> 418
   <211> 206
   <212> DNA
   <213> Homo sapiens
<400> 418
   tatatttatt acattatttt gaaagagaat tagtgtgtta tgtggataat gttatataca 60
   gccaaagtgg atgtttctgt ttggcaagga aggtaggatt tctgaaactc aggccttaac 120
   caataggttg gaagacaaga ccaattgaag agttaggaaa tgtgagtttt tgttacttct 180
   gttattccag tcttggtttc attgtc                                      206
<210> 419
   <211> 238
   <212> DNA
   <213> Homo sapiens
<220>
   <221> misc_feature
   <222> 159, 227
   <223> n = A,T,C or G
<400> 419
   agcagtgtac ataatattcc agtaggaaac tgcttccaag tttaagcatg agctccccaa 60
   actggagaaa acatattttg ctattctgag acaacaatca gaatacagac tttggattcc 120
   aggtcacagt ttgcttttta gacaaggtaa agcaaagana gccacattgt gccatcttca 180
   gctccagtgg ctttagcagt gactgtttga cataaaacat gtaaganttg cttgttgg   238
<210> 420
   <211> 504
   <212> DNA
   <213> Homo sapiens
<400> 420
   cggcgtgctt gctgctggag ggtgatggcc ctgcaaggct gtgggctccg acctcaccgg 60
   gagtcgacag cgagaggttc gccgaagagc gaggttctgg gcgagcgctg aacgccggcc 120
   ccaagcaccc cgggtcttta cacagtccgc gtccacagac tctgacgaag acgtggatct 180
   gctctcgctt tagctgctcg cggtcctcca gatcatgtcc gcgactcctg cgactccgcg 240
   cggaaaaaaa agtttgccag gcgtggactc aatgaccttt ccaagctgtg cgcctcgctg 300
   cctggaccgg gtctgagcgc ggctgcccag gttgaccttt ctgcgggagg gctttctcta 360
   cgtgctgttg tctcactggg tttttgtcgg agccccacgc cctccggcct ctgattcctg 420
   gaagaaaggg ttggtcccct cagcaccccc agcatcccgg aaaatgggga gcaaggctct 480
   gccagcgccc atcccgctcc accc                                        504
<210> 421
   <211> 814
   <212> DNA
   <213> Homo sapiens
<220>
   <221> misc_feature
   <222> 38, 93, 94, 95, 422, 440, 467, 474, 508, 519, 529, 535, 554, 557, 561, 565, 584, 594, 604, 619, 641, 655, 674, 679, 690, 695, 702, 704, 706, 712, 716, 724, 734, 737, 740, 743, 780, 781, 808, 813
   <223> n = A,T,C or G
<400> 421
   cggggacgga gctcggcgtg cttgctgctg gagggttntg gccctgcaag gctgtgggct 60
   ccgacctcac cgggagtcga cagcgagagg ttnnncgaag agcgaggttc tgggcgagcg 120
   ctgaacgccg gccccaagca ccccgggtct ttacacagtc cgcgtccaca gactctgacg 180
   aagacgtgga tctgctctcg ctttagctgc tcgcggtcct ccagatcatg tccgcgactc 240
   ctgcgactcc gcgcggaaaa aaaagtttgc caggcgtgga ctcaatgacc tttccaagct 300
   gtgcgcctcg ctgcctggac cgggtctgag cgcggctgcc caggttgacc ttttctgcgg 360
   aagggctttc tctacgtgct gttgctcatg ggtttttgtc ggagccccaa cgcccttccg 420
   gncttttgat tcctggaaan aaaaggggtt ggttcccctt caagcanccc caancattcc 480
   ccgggaaaaa atgggggagc caaagggntt ttggccaang gccccaatnc ccggnttcaa 540
   cccgttgggt tggnaanttt naccnaaatt aacttccttt cctncaaggc ccgnggaaaa 600
   aacnttttcc cgggccacng ggggggaacc aaccttgcaa nggggccttg taccnggtct 660
   tcaaacggcg ggtnccaana acccttgccn ccatngaaac cnantnggaa cncctngggg 720
   gttnttcccc aatnggngcn ccnaaaaaac aaccccggtt ccaaccattt aagggaaaan 780
   nggcgggggg gccccaaggg cccttttngg acnt 814
<210> 422
   <211> 375
   <212> DNA
   <213> Homo sapiens
<400> 422
   ctgacgaaga cgtggatctg ctctcgcttt agctgctcgc ggtcctccag atcatgtccg 60
   cgactcctgc gactccgcgc ggaaaaaaaa gtttgccagg cgtggactca atgacctttc 120
   caagctgtgc gcctcgctgc ctggaccggg tctgagcgcg gctgcccagg ttgacctttc 180
   tgcgggaggg ctttctctac gtgctgttgt ctcactgggt ttttgtcgga gccccacgcc 240
   ctccggcctc tgattcctgg aagaaagggt tggtcccctc agcaccccca gcatcccgga 300
   aaatggggag caaggctctg cagcgcccat cccgctccac cgtcgctgca gctcccaatt 360
   actcttctgc aggcg 375
<210> 423
   <211> 405
   <212> DNA
   <213> Homo sapiens
<400> 423
   ggggacggag ctcggcgtgc ttgctgctgg agggtgatgg ccctgcaagg ctgtgggctc 60
   cgacctcacc gggagtcgac agcgagaggt tcgccgaaga gcgaggttct gggcgagcgc 120
   tgaacgccgg ccccaagcac cccgggtctt tacacagtcc gcgtccacag actctgacga 180
   agacgtggat ctgctctcgc tttagctgct cgcggtcctc cagatcatgt ccgcgactcc 240
   tgcgactccg cgcggaaaaa aaagtttgcc aggcgtggac tcaatgacct ttccaagctg 300
   tgcgcctcgc tgcctggacc gggtctgagc gcggctgccc aggttgacct ttctgcggga 360
   gggctttctc tacgtgctgt tgtctcactg ggtttttgtc ggacc 405
<210> 424
   <211> 139
   <212> DNA
   <213> Homo sapiens
<400> 424
   ctcgtgttca gctgtcagaa taacagccaa taaaaactac aggagcaaaa cctctcagga 60
   aggtgcttta aaaaagatgc atgaggaaga acaccatcaa caaatgtcca tcttacaact 120
   gcaactgata caaatgaat 139
<210> 425
   <211> 273
   <212> DNA
   <213> Homo sapiens
<400> 425
   ttctggctgg gaagcgcgat tgtggcttta aaccaccatc atggtctagc aaagaggcaa 60
   agaccaagac caccaagaag cgccctcagc gtgcaacatc caatgtgttt gccatgtttg 120
   accagtcaca gattcaggag ttcaaagagg ccttcaacat gattgatcag aacagagatg 180
   gcttcatcga caaggaagat ttgcatgata tgcttgcttc tctagggaag aatcccactg 240
   atgcatacct tgatgccatg atgaatgagg ccc 273
<210> 426
   <211> 56
   <212> DNA
   <213> Homo sapiens
<400> 426
   gggaaccgcc attctgcctg ggaaccgcca ttctggccgg gaaccgccat tatgac 56
<210> 427
   <211> 365
   <212> DNA
   <213> Homo sapiens
<400> 427
   ggcgcattct tacctgtcgg ggtgcggcga gtgtctcacc tctctgcact tccaaggact 60
   cttgtcatct gccttaggcg ggaaatgctg ttgctggatt gcaaccccga ggtggatggt 120
   ctgaagcatt tgctggagac aggggcctcg gtcaacgcac ccccggatcc ctgcaagcag 180
   tcgcctgtcc acttagccgc aggaagcggc cttgcttgct ttcttctctg gcagctgcaa 240
   acgggcgctg acctcaacca gcaggatgtt ttaggagaag ctccactaca caaggcagca 300
   aaagttggaa gcctggagtg cctaagcctg cttgtagcca gtgatgccca aattgattta 360
   tgtag 365
<210> 428
   <211> 119
   <212> DNA
   <213> Homo sapiens
<400> 428
   gagcggtggc tgagaaatgt aaggattctg gaatacatat tccatgggac tttccttccc 60
   tctcctgctt cctcttttcc tgctccctaa cctttcgccg aatggggcag caccactga 119
<210> 429
   <211> 421
   <212> DNA
   <213> Homo sapiens
<220>
   <221> misc_feature
   <222> 130, 185, 246, 256, 336, 361, 385, 412
   <223> n = A, T, C or G
<400> 429
   tttttttttt tttttttgga aataagtcaa agcattgttt atttatgaca tatttacata 60
   tttacaaaac tgattttact caatacatca tcctgcgtaa tatcataaaa tgaacaccat 120
   atcctgggan taaaaatcca tatttcttaa taatttatgt atagcccaac ttttagaaca 180
   tagantatta tcaatttggc ttcccaaact acaaagtcct gtttataatt ttttctagcc 240
   aaggancaga gtaggntcaa caggcatatt aaagtaattt agttaaccct gaggtaatta 300
   ctaacttggc ataattttgg aatggggtat atatancaca ctttccatct ggcacttagg 360
   ntacttatta ctattcacac taccnttttg gtatttatcc acctcaattt tncaacttcc 420
   t                                                                 421
<210> 430
   <211> 481
   <212> DNA
   <213> Homo sapiens
<400> 430
   gggtagccgc ttttcgtcga ctcttaccgg ttggctgggc cagctgcgcc gcggctcaca 60
   gctgacgatg ggggacccca gcaagcagga catcttgacc atcttcaagc gcctccgctc 120
   ggtgcccact aacaaggtgt gttttgattg tggtgccaaa aaatcccagc tgggcaagca 180
   taacctatgg agtgttcctt tgcattgatt gctcagggtc ccaccggtca cttggtgttc 240
   acttgagttt tattcgatct acagagttgg attccagctg gtcatggttt cagttgcgat 300
   gcatgcaagt cggaggaaac gctagtgcat cttccttttt tcatcaacat gggtgttcca 360
   ccaatgacac caatgccaag tacaacagtc gtgctgctca gctctatagg gagaaaatca 420
   aatcgctcgc ctctcaagca acacggaagc atggcactga tctgtggctt gatagttgtg 480
   t                                                                 481
<210> 431
   <211> 136
   <212> DNA
   <213> Homo sapiens
<400> 431
   ggggtaagtt tagaaatacg gctgggcatg tccagccctg accacggcca gctctggagg 60
   gctgtccttt ggctgtaccc acttggaaga gaaagaaaaa gaaaaaaaaa aaaaaaaaaa 120
   aaaatttttt tttttt                                                 136
<210> 432
   <211> 578
   <212> DNA
   <213> Homo sapiens
<400> 432
   aaacaacaaa caccagaaaa attacctata ccaatgatag caaaaaacct tatgtgtgaa 60
   ctcgatgaag actgtgaaaa gaatagtaag agggactact taagttctag ttttctatgt 120
   tctgatgatg atagagcttc taaaaatatt tctatgaact ctgattcatc ttttcctgga 180
   atttctataa tggaaagtcc attagaaagt cagcccttag attcagatag aagcattaaa 240
   gaatcctctt ttgaagaatc aaatattgaa gatccactta ttgtaacacc agattgccaa 300
   gaaaagacct caccaaaagg tgtcgagaac cctgctgtac aagagagtaa ccaaaaaatg 360
   ttaggtcctc ctttggaggt gctgaaaacg ttagcctcta aaagaaatgc tgttgctttt 420
   cgaagtttta acagtcatat taatgcatcc aataactcag aaccatccag aatgaacatg 480
   acttctttag atgccaatgg atatttcgtg tgcctacagt ggttcatatc ccatggctat 540
   aacccctact caaaaaagaa gatcctgtat gccacatc                         578
<210> 433
   <211> 229
   <212> DNA
   <213> Homo sapiens
<220>
   <221> misc_feature
   <222> 35, 37
   <223> n = A,T,C or G
<400> 433
   gcctaggtgc ccaggctatg atgagtctgc ttttnangga ggtagggaat gacatcttcc 60
   ttggacccaa agcttaaaag taatgtatgc tttgctgacc actgtttgtt aggccttaaa 120
   caacattcac tgtggtggta tcaggcacac tgctatgtgc atcaattatt tttttgcttt 180
   ccaaacagaa tctctggggc acaagtttta cactcaagct aagtataac             229
<210> 434
   <211> 503
   <212> DNA
   <213> Homo sapiens
<400> 434
   tggtacgcct gcaggtaccg gtccggaatt cccgggtcga cccacgcgtc cggcgtcatg 60
   gagctgacct ggttcccatc tactcctttg gagagaatga agtgtacaag caggtgatct 120
   tcgaggaggg ctcctggggc cgatgggtcc agaagaagtt ccagaaatac attggtttcg 180
   ccccatgcat cttccatggt cgaggcctct tctcctccga cacctggggg ctggtgccct 240
   actccaagcc catcaccact gttgtgggag agcccatcac catccccaag ctggagcacc 300
   caacccagca agacatcgac ctgtaccaca ccatgtacat ggaggccctg gtgaagctct 360
   tcgacaagca caagaccaag ttcggcctcc cggagactga ggtcctggag gtgaactgag 420
   ccagccttcg gggccaattc cctggaggaa ccagctgcaa atcacttttt tgctctgtaa 480
   atttggaagt gtcatgggtg tct                                         503
<210> 435
   <211> 248
   <212> DNA
   <213> Homo sapiens
<400> 435
   gcgtcatgga gctgacctgg ttcccatcta ctcctttgga gagaatgaag tgtacaagca 60
   ggtgatcttc gaggagggct cctggggccg atgggtccag aagaagttcc agaaatacat 120
   tggtttcgcc ccatgcatct tccatggtcg aggcctcttc tcctccgaca cctgggggct 180
   ggtgcctact ccaagcccat caccactgtt gtgggagagc ccatcaccat ccccaagctg 240
   gagcacca                                                          248
<210> 436
   <211> 457
   <212> DNA
   <213> Homo sapiens
<400> 436
   atcttgtctc ttttcatcgt gatggtgtga tgctgacgag aatatcttat gctttcttca 60
   gcctgttgca atctgagcca atgattttct ttgcactgat cctttctact ctggagagaa 120
   gctcttttga cacagatcct gccccgttta atagactcca gctgctggca ctgccttctg 180
   agttctttca cttccgaatt cttatcgtcc tgcagcccca ccacagtcaa tgactaagtt 240
   cctctggact ttcacatgga tcgtaataga caacttcatc ctgtttttct taccagaccc 300
   taaaatgtgc ctccaagaca gtcgtgggaa cagtatggag ccagcagcag aagccactca 360
   cgaaccaatg gaggagaaca actcagaaac agacccaagt caatctaagg tttaactttt 420
   ataagtcttt caagagagtc caactgtgta gtaagca                          457
<210> 437
   <211> 589
   <212> DNA
   <213> Homo sapiens
<400> 437
   gcttccaggt ctccttccag catccacaca agtacctgct ccactacctg gtttccctcc 60
   agaactggct gaaccgccac agctggcagc ggacccctgt tgccgtcacc gcctgggccc 120
   tgctgcggga cagctaccat ggggcgctgt gcctccgctt ccaggcccag cacatcgccg 180
   tggcggtgct ctacctggcc ctgcaggtct acggagttga ggtgcccgcc gaggtcgagg 240
   ctgagaagcc gtggtggcag gtgtttaatg acgaccttac caagccaatc attgataata 300
   ttgtgtctga tctcattcag atttatacca tggacacaga gatcccctaa ggccctggcc 360
   caggcctgcc caaagagaag cccaggatgg tcggctgcct ggggacattg tcaccacgtc 420
   gccatgacgg ctggtcccca caggaccagc tgggaggact ggttgtgctg ctggagaagg 480
   gctggagaag gcaatggcat gctgccgctt tgccagtccc taaaagtcgc ggtgcaggtg 540
   atggtgggag ccgcgcctcc agcgggcagg ccgggagtgt actgtgtgc              589
<210> 438
   <211> 241
   <212> DNA
   <213> Homo sapiens
<400> 438
   cgcttccagg tctccttcca gcatccacac aagtacctgc tccactacct ggtttccctc 60
   cagaactggc tgaaccgcca cagctggcag cggacccctg ttgccgtcac cgcctgggcc 120
   ctgctgcggg acagctacca tggggcgctg tgcctccgct tccaggccca gcacatcgcc 180
   gtggcggtgc tctacctggc cctgcaggtc tacggagttg aggtgcccgc cgaggtcgag 240
   g                                                                 241
<210> 439
   <211> 221
   <212> DNA
   <213> Homo sapiens
<400> 439
   ttcagctctg caaacactgt cacatccttt cctggaaggg cactgaccat ccgtgcactg 60
   ccaataaccc agagagctgc tccgtttcac tttcacccca ggactttatc aacttgttca 120
   agttctgaat cccagcacat gacaacactt cagaagggtc cccctgctga ctggagagct 180
   gggaatatgg catttggaca cttcatttgt aaatagtgta c                     221
<210> 440
   <211> 228
   <212> DNA
   <213> Homo sapiens
<220>
   <221> misc_feature
   <222> 191
   <223> n = A,T,C or G
<400> 440
   gagctttctt aataaccgta cttctcaaaa tcagagtttt actgtttcaa taaatgttca 60
   ccctagattg taagtttttt gttgttgagc cctagatttt tttctactag tgtaaatctg 120
   tattccctcc aagtatggtg ataaggggac tgagtcttat ttacatttgt acaatcacta 180
   ctttacctgt ngtatttgca gtaagtcttt tgagccctat taaacctg              228
<210> 441
   <211> 531
   <212> DNA
   <213> Homo sapiens
<400> 441
   tttcttaata accgtacttc tcaaaatcag agttttactg tttcaataaa tgttcaccct 60
   agattgtaag ttttttgttg ttgagcccta gatttttttc tactagtgta aatctgtatt 120
   ccctccaagt atggtgataa ggggactgag tcttatttac atttgtacaa tcactacttt 180
   acctgttgta tttgcagtaa gtcttttgag ccctattaaa cctgtcaatt ttcttgtcct 240
   gtcagaaaac tgagattttg gctcaaaaat ggatgttatt aacaaagggg aacaatatag 300
   atgtcttagt acaaagaaaa tgaaatgtaa gaggagattg tctggagttc aggggataga 360
   gtgtcaagtc ttaaatggtt acatcttttt gctaagtgtt actcagaata tagttacaaa 420
   tatggtactt aaatatctag ctgaaatttg tttgtcccat gagcttctca catgagtcta 480
   ctgggcaatt ttatgtgagt tttggtcaaa attggtaatc tcttttatct t          531
<210> 442
   <211> 147
   <212> DNA
   <213> Homo sapiens
<220>
   <221> misc_feature
   <222> 112
   <223> n = A,T,C or G
<400> 442
   aacttgttac ccaataacaa tttaatgtta aatttggctt tcttctgtgt cccagcctct 60
   taaattaata gatgggcctt tccattatca ttatgaccgg acattgtaaa gnacttaagg 120
   taacacccag ttttctatta cttgccc 147
<210> 443
   <211> 518
   <212> DNA
   <213> Homo sapiens
<400> 443
   acctgaagaa tattagaaga aattgtgcac cctccacaaa acatacaaag tttaaaagtt 60
   tggatctttt tctcagcagg tatcagttgt aaataatgaa ttaggggcca aaatgcaaaa 120
   cgaaaaatga atcatctaca tgtagttagt aatttctagt ttgaactgta attgaatatt 180
   gtggcttcat atgtattatt ttatattgta cttttttcat tattgatggt ttggacttta 240
   ataagagaaa ttccatagtt tttaatatcc cagaagtgag acaatttgaa cagtgtattc 300
   tggaaaacaa cacactaact gaacagaagt gaatgcttat atatattatg atagccttaa 360
   acctttttcc tctaatgcct taactgtcaa ataattataa ccttttaaag cataggacta 420
   tagtcagcat gctagactga gaggtaaaca ctgatgcaat tagaacaggt actgatgctg 480
   tcagtgttta acactatgtt tagctgtgtt tatgctat 518
<210> 444
   <211> 76
   <212> DNA
   <213> Homo sapiens
<400> 444
   gctgctcatg agcagcatgg acgacctgat acgccactgt aacgggaagc tgggcagcta 60
   caaaatcaat ggccgg 76
<210> 445
   <211> 308
   <212> DNA
   <213> Homo sapiens
<400> 445
   gagcattatg agcattatgt cagaatagaa tagaattggg gttcgatctt aacaggccag 60
   aaatgcctgg gtttttttgg tttgtttttg tttttgtttt tttatcaaat cctgcctgac 120
   tgtctgcttg ttttgcctac catcgtgaca tctccatggc tgtaccacct tgtcgggtag 180
   cttatcagac tgatgttgac tgttgaatct catggcaaca ccagtcgatg ggctgtctga 240
   cattttggta tctttcatct gaccatccat atccaatgtt ctcatttaaa cattacccag 300
   catcattg 308
<210> 446
   <211> 530
   <212> DNA
   <213> Homo sapiens
<400> 446
   tgtgttaatg ttttctagca tgtactctgg tttcaacaga cacaaattta tatgttaacc 60
   cagttttctt gccgttctgt aagtgtttta ttcttagtgt gatttttttc cattgggatg 120
   tttttgattg aacttgttca ttttgttttg cttgggagga aaataaacaa ttttactttt 180
   ttcctttagg agcattatga gcattatgtc agaatagaat agaattgggg ttcgatctta 240
   acaggccaga aatgcctggg tttttttggt ttgtttttgt ttttgttttt ttatcaaatc 300
   ctgcctgact gtctgcttgt tttgcctacc atcgtgacat ctccatggct gtaccacctt 360
   gtcgggtagc ttatcagact gatgttgact gttgaatctc atggcaacac cagtcgatgg 420
   gctgtctgac attttggtat ctttcatctg accatccata tccaatgttc tcatttaaac 480
   attacccagc atcattgttt ataatcagaa actctggtcc ttctgtctgg           530
<210> 447
   <211> 104
   <212> DNA
   <213> Homo sapiens
<400> 447
   ggacgtgcct ggaaccacct cgtccacgtc cacgtccacc tgggggcctc gggaggctag 60
   gcccctcctc aaaggcccac cagcccggcg ctcatgctga gccc                  104
<210> 448
   <211> 417
   <212> DNA
   <213> Homo sapiens
<400> 448
   tatctttcat ctgaccatcc atatccaatg ttctcattta aacattaccc agcatcattg 60
   tttataatca gaaactctgg tccttctgtc tggtggcact taaagtcttt tgtgccataa 120
   tgcagcagta tggagggagg attttatgga gaaatgggga tagtcttcat gaccacaaat 180
   aaataaagga aaactaagct gcactgtggg ttttgaaaag gttattatac ttcttaacaa 240
   ttcttttttt cagggacttt tctagctgta tgactgttac ttgaccttct ttgaaaagca 300
   ttcccaaaat gctctatttt agatagttta acattaacca acataatttt ttttagatcg 360
   agtcagcata aatttctaag tcagcctcta gtcgtggttc atctctttca cctgcat    417
<210> 449
   <211> 630
   <212> DNA
   <213> Homo sapiens
<400> 449
   tttttttttt tttttttttt ttggaatcgc aagaattccc aggccctctt tttatttaca 60
   gtgataccaa accatccact tgcaaattct ttggtctccc atcagctgga attaagtagg 120
   tactgtgtat ctttgagatc atgtatttgt ctccactttg gtggatacaa gaaaggaagg 180
   cacgaacagc tgaaaaagaa gggtatcaca ccgctccagc tggaatccag caggaacctc 240
   tgagcatgcc acagctgaac acttaaaaga ggaaagaagg acagctgctc ttcatttatt 300
   ttgaaagcaa attcatttga aagtgcataa atggtcatca taagtcaaac gtatcaatta 360
   gaccttcaac ctaggaaaca aaattttttt ttctatttaa taatacacca cactgaaatt 420
   atttgccaat gaatcccaaa gatttggtac aaatagtaca attcgtattt gctttcctct 480
   ttcctttctt cagacaaaca ccaaataaaa tgcaggtgaa agagatgaac cacgactaga 540
   ggctgactta gaaatttatg ctgactcgat ctaaaaaaaa ttatgttggt taatgttaaa 600
   ctatctaaaa tagagcattt tgggaatgct 630
<210> 450
   <211> 596
   <212> DNA
   <213> Homo sapiens

<400> 450
   tttttttttt tttttttttt tttggggtaa aagttatatc ttattgccat gctacaaaat 60
   gtatgaagtt ggcactgata gggagaaata gagaacaaag ggtgggaagg gatagaggga 120
   aaattatgtt gttacatata caacaaggtt ttattttaat taacagtggt tacgttttgc 180
   caatattaaa aatgcaaacc aaaatttaaa atgctgatct gaaacagcat taagatacaa 240
   tgtatgcata gtacagtatc acttatgtct ttttattaga gaaatatgga atgtttataa 300
   aagaaattaa ccatgggggt aaaattcata tttcatatac aatttggcaa tggtagtccc 360
   actgttggac aattttttat aaaagaaaaa attaaaaatc taataagcta cctttataca 420
   aagttgctat atttatgcct ttacgtagga aaaaaacatt tataatgcaa attaggacat 480
   acaatagtct tacaatacta tacaatgtaa tgaaaataaa acataacaca aagtttgtcc 540
   tttataaaat gtatattttg cattactaat gcaaatgtgg cacactggtg actact 596
<210> 451
   <211> 559
   <212> DNA
   <213> Homo sapiens
<400> 451
   tggcgggttg ctttccaaaa tggcgcgggt gctgaaggct gcagccgcga atgccgtagg 60
   gcttttttcc agacttcaag ctcccattcc aacagtaaga gcttcttcca catcacagcc 120
   cttggatcaa gtgacaggtt ctgtgtggaa cctgggtcga ctcaaccatg tagccatagc 180
   agtgccagat ttggaaaagg ctgcagcatt ttataagaat attctggggg cccaggtaag 240
   tgaagcggtc cctcttcctg aacatggagt atctgttgtt tttgtcaacc tgggaaatac 300
   caagatggaa ctgcttcatc cattgggacg tgacagtcca attgcaggtt ttctgcagaa 360
   aaacaaggct ggaggaatgc atcacatctg catcgaggtg gataatatta atgcagctgt 420
   gatggatttg aaaaaaaaag aagatccgca gtctaagtga aggggtcaaa ataggagcac 480
   atggaaaacc agtgattttt ctccatccta aagactgtgg tggagtcctt gtggaactgg 540
   agcaagcttg acttatatt 559
<210> 452
   <211> 638
   <212> DNA
   <213> Homo sapiens
<400> 452
   tggcgggttg cgttccaaat ggcgcgggtg ctgaaggctg cagccgcgaa tgccgtaggg 60
   cttttttcca gacttcaagc tcccattcca acagtaagag cttcttccac atcacagccc 120
   ttggatcaag tgacaggttc tgtgtggaac ctgggtcgac tcaaccatgt agccatagca 180
   gtgccagatt tggaaaaggc tgcagcattt tataagaata ttctgggggc ccaggtaagt 240
   gaagcggtcc ctcttcctga acatggagta tctgttgttt ttgtcaacct gggaaatacc 300
   aagatggaac tgcttcatcc attgggacgt gacagtccaa ttgcaggttt tctgcagaaa 360
   aacaaggctg gaggaatgca tcacatctgc atcgaggtgg ataatattaa tgcagctgtg 420
   atggatttga aaaaaaaaga agatccgcag tctaagtgaa ggggtcaaaa taggagcaca 480
   tggaaaacca gtgatttttc tccatcctaa agactgtggt ggagtccttg tggaactgga 540
   gcaagcttga cttatatttg caagcaacta aattaattga cctgaaaaag cctatcaaat 600
   actatcaaaa tgtactatga cattgagtcc ttcactgc 638
<210> 453
   <211> 57
   <212> DNA
   <213> Homo sapiens
<400> 453
   gactacattt ggggatgatg cattccttta agattgaatg attctgccct tgggcag 57
<210> 454
   <211> 538
   <212> DNA
   <213> Homo sapiens
<400> 454
   gccgggctgc taattctgtt taattgttcc tgggctaaaa agaattagaa ggaagctgtc 60
   tgtttcccac tgcggttatg tttcagtaaa ttagacgtac tttctgatga atactaatta 120
   gccactgagc atttgcaccc actgtctttg ctggttgtgt gcagaacagc tgccaagttg 180
   cccaagaccc tcgctatccc atccccctct cttgctttcc acttttgggc ttcctttgcc 240
   tagattagaa gagatttcag ttccgagaaa gtaaaaggtg atccaaggaa gtaatcaccg 300
   agtgtctcat ggtttttcct tgttgacaaa attcaaaact cacacatgtg tagtctaatg 360
   atagcgctag gatttaaaga aagtgtttta gtgctgtgct tatttaggac tacatttggg 420
   gatgatgcat tcctttaaga ttgaatgatt ctgcccttgg gcagagctcc caattaggga 480
   ggattaggta agctttttgt ggcgatgggt aataccattc ttttcctcat tgtgcctg 538
<210> 455
   <211> 548
   <212> DNA
   <213> Homo sapiens
<400> 455
   tgaatcagta ggaatgtggg gaagggagtg aggggagacc ccctccttga ctcagcagtg 60
   gtgacggtcg gtgtgtcctg cagacctgaa gccaagatca agggggcttg agcaccagga 120
   gcccccgcag ttgctgaatg accagcggag ggcaggtgcc agcctgtggc aaaataggaa 180
   agaaaaggac aggatgggga cttcaccatt tttttcagcc ttaaattgtt ccttaaacct 240
   tcatgtcctt ttctctaatg tgtgttcttg tttggtaaaa taaaaaagtt tgtaaccctg 300
   agttctctaa agatatacat tcttttttac tggtttgtga agtcagaagg atgagagctg 360
   ctatttcttg gaaccgtgca ataaatatta gcatattcag tctcggttct gcctagagga 420
   cctatttgct tttctttatc tcgtaaccca taactcacag gacattaacc agggtgtcca 480
   agaacagtct gggaaagttt tgataattac ttcagcattg ctgtgtgatg ggagacattg 540
   ttttaaaa 548
<210> 456
   <211> 354
   <212> DNA
   <213> Homo sapiens
<400> 456
   tcagtgggag tgaatcagta ggaatgtggg gaagggagtg aggggagacc ccctccttga 60
   ctcagcagtg gtgacggtcg gtgtgtcctg cagacctgaa gccaagatca agggggcttg 120
   agcaccagga gcccccgcag ttgctgaatg accagcggag ggcaggtgcc agcctgtggc 180
   aaaataggaa agaaaaggac aggatgggga cttcaccatt tttttcagcc ttaaattgtt 240
   ccttaaacct tcatgtcctt ttctctaatg tgtgttcttg tttggtaaaa taaaaaagtt 300
   tgtaaccctg agttctctaa agatatacat tcttttttac tggtttgtga agtc 354
<210> 457
   <211> 570
   <212> DNA
   <213> Homo sapiens
<400> 457
   cttttatagg attcatttaa aggtgaataa aataatgaat gtgaaactca tattagagct 60
   taacatatag tagtaatgat ttataaaata tttgcctccc ttagaccaga gcagctacta 120
   aatttgattt taataataag ataaacaaat taataagatc acaaagttgt tatgtaataa 180
   cataaacagc tgtgttaaaa ttagtagtga cccatatcaa agaaacacaa ttacaaagag 240
   attaagaagg ataatattta aagtgtagct ttactcagtc ttttgtgtga aggtattctt 300
   agggataaaa caatgtattt ggaagctgct ggaagaatat ggtgcaaaga atatttttaa 360
   atgcttgtga atgttctgta accacaaaca tagatacata acagatcaaa gacatatttt 420
   agactgccat gtggacttaa atcatgggag gcggaagagt ggctccccaa agaggactat 480
   atcgtaatac cagaacttgt gaatatatta ctttaagtgg caaaagggac tttacagatg 540
   tgattaaaat taaggacctt gaaatggggg 570
<210> 458
   <211> 540
   <212> DNA
   <213> Homo sapiens
<400> 458
   aactagactt cttttatagg attcatttaa aggtgaataa aataatgaat gtgaaactca 60
   tattagagct taacatatag tagtaatgat ttataaaata tttgcctccc ttagaccaga 120
   gcagctacta aatttgattt taataataag ataaacaaat taataagatc acaaagttgt 180
   tatgtaataa cataaacagc tgtgttaaaa ttagtagtga cccatatcaa agaaacacaa 240
   ttacaaagag attaagaagg ataatattta aagtgtagct ttactcagtc ttttgtgtga 300
   aggtattctt agggataaaa caatgtattt ggaagctgct ggaagaatat ggtgcaaaga 360
   atatttttaa atgcttgtga atgttctgta accacaaaca tagatacata acagatcaaa 420
   gacatatttg agactggcat gtggacttaa atcatgggag gcggaagagt ggctccccaa 480
   agaggactat atcgtagtac cagaacttgt gaatatatta ctttaagtgg caaaagggac 540
<210> 459
   <211> 622
   <212> DNA
   <213> Homo sapiens
<400> 459
   acttaagatt ttttcaatgt aagaaaaatg caatgaaata atagctgcaa atacccacta 60
   ctaacaattg cttggccttc ttatatagac ctcccgaggt tctcatcttt tacatttcag 120
   gagtagaatc agttaaaaac taatctttat atgtaaggga tgagagagag aaagaggagg 180
   gtatgtgtat gcacacatgt gtgtgtgtgt ggtgggtagt aattttaatt caatgattta 240
   ctagagttcg atgtcgtttg ctgataaatg aagcaggagg aagagccagg tttggagggg 300
   acgagagaat gagttccatt tgtctcatat agaagttgaa gtaactgagt gatgatgggt 360
   agagatgtcc ctcaggggta gccacagtat tttatttact ttttattcac cacatgcagc 420
   aaggagcttt gttctccaaa atgctgtcaa ttatttttct aaattacagg tttgattgct 480
   tcactgtatt ttcatgtctc attactacct ttacgcttaa aaccagaaac tgtgccacag 540
   cgttaaagat tctgctaact tttaaaatac agaactctgg agatgccata attagattgc 600
   agatttatga gtcttctgga ta                                          622
<210> 460
   <211> 378
   <212> DNA
   <213> Homo sapiens
<400> 460
   acaatgggtt tgttctctgc cttataaatt gggggattct agaggagtct gcttttctcc 60
   caagaaggac ctcttctttt cttgcttttc atatgctctc cttgagatat cttgggtatt 120
   ctcatggctt taaatagcac ttatatccag aagactcata aatctgcaat ctaattatgg 180
   catctccaga gttctgtatt ttaaaagtta gcagaatctt taacgctgtg gcaaagtttc 240
   tggttttaag cgtaaaggta gtaatgagac atgaaaatac cgtgaagcca tcaaacctgt 300
   aatttaaaaa aataattgac agcattttgg agaacaaagc tccttgctgc atgtggggaa 360
   taaaaagtaa ataaaata                                              378
<210> 461
   <211> 396
   <212> DNA
   <213> Homo sapiens
<400> 461
   ccttctgctc tacgagaact atgggcagtc ggaaacggga ctaatttgtg ccacctactg 60
   gggaatgaag atcaagccgg gtttcatggg gaaggccact ccaccctacg acgtccaggt 120
   cattgatgac aagggcagca tcctgccacc taacacagaa ggaaacattg gcatcagaat 180
   caaacctgtc aggcctgtga gcctcttcat gtgctatgag ggtgacccag agaagacagc 240
   taaagtggaa tgtggggact tctacaacac tggggacaga ggaaagatgg atgaagaggg 300
   ctacatttgt ttcctgggga ggagtgatga catcattaat gcctctgggt atcgcatcgg 360
   gcctgcagag gttgaaagtg ctttggtgga gcaccc                           396
<210> 462
   <211> 529
   <212> DNA
   <213> Homo sapiens
<400> 462
   tttttttttt tttttttttt ttttttcggt agaaatgggg ttttaccatg ttgcccaggc 60
   tagtctcgaa ctcctgggct taagcaatcc acacacctcg cttccaaaaa agctggggtt 120
   acaggtgtga gccatcacac ccagcctaat atacaatctc aaatattttg ttttaaatca 180
   ttacttactg aactataaag taaaactaat ttttagacag cattttaata catattttac 240
   tttttaaagg ttataaagaa aacactaaca atatggaaaa tgcatattta aagaaaattg 300
   aaatcaaata taatcttatg gctcaaaatc attagtgtta atattttgat acctaccttc 360
   cccatctttt gcctacgaat actgggttaa gagtttttaa atagttttgt ccttgctttg 420
   taattttcgt atgttctcac aaaagagaag ctgaggaagc atttggctat tgggaaaatt 480
   aattaataga tgttaactta ccaagatata ctataataga ttagacagc          529
<210> 463
   <211> 485
   <212> DNA
   <213> Homo sapiens
<400> 463
   tttaaagtaa atgactcatg ttgaggaaag aggttattac ctaaatctgg actgcggcct 60
   aaggaaattc ccttaacctc tattctggtt tcctatttca aaatggttgt gtaggaggct 120
   aatggaagtt agttggttgc tatgatccaa aaactctatg ggtgaaaatt taaagtacag 180
   atttcttatt taatcgttaa acagctttag ttgtgagttc tatgtcctgg tataatggat 240
   cctgattatt aatgcattaa atatgcattc agtgaattca aatgttgcta attattcttt 300
   taccaatcaa agaaaactca aagcatggga ttaagagggg ttggccaaaa gtatttggac 360
   caggttgcat accaggacca tgaagaaatt gagaacagag cctacatctt ttatactatg 420
   gctcaaagca agggctgttg gaatgtgctg cttctccaaa gtaggactta tgaaaaaatg 480
   agggt                                                             485
<210> 464
   <211> 576
   <212> DNA
   <213> Homo sapiens
<400> 464
   tatcagcatc tgtagaggag aaagcagaat aagcactggg gtatttgata gacttgagaa 60
   taagagaacc ccaaagttgt caataggtat ttgctagaaa gttcagtggg tcagggtggg 120
   aatagcagct gaaattggca gggattttga ctattcaaat aatgggtgag tagaagggat 180
   ctgtggaata gccattatga cctcttgaaa ccaggcaact agggggtccc ttctagaatg 240
   atgctgcgta cctaagaaat tcagtaggga gtggagtcaa aatgatcaga aaagatagag 300
   atagttgtgg caaaagatga tctaagagtg tgtgtgtatg tgtgtgagtg agagagagaa 360
   atctcaagaa atagtggcta tggtgttgaa cactacatga aagcaaccta aaacagctgt 420
   gtgaagttag aaaaggtact ctggaccata ttgccctgta aaagctcagg aaaactaatt 480
   ttgcataaac ataagcaaca ggaaattatt gctgtcaaat ctcattcaga gttattgtac 540
   aaaaaaagag acaagaatcc ctatagacaa tgaaag                            576
<210> 465
   <211> 459
   <212> DNA
   <213> Homo sapiens
<400> 465
   ttatctaacg tttctaacag gggtgttaat gatattagca gcaagagcta tgagaaataa 60
   ctttagacat tatttcattg aaccttccca actgaaatta ttttatgatg ttataacatg 120
   gatagtaact caagtagcaa taagttacac agttgtgcca tttgtgcttc tttctataaa 180
   accatcactc acgttttaca gctcctggta ttattgcctg cacattcttg gtatcttagt 240
   attatcgttg ttgccagtga aaaaaactca aagaaggaag aatacacatg aaaacattca 300
   gctctcacaa tccaaaaagt ttgatgaagg agaaaattct ttgggacaga acagtttttc 360
   tacaacaaac aatgtttgca atcagaatca agaaatagcc tcgagacatt catcactaaa 420
   gcagtgatcg ggaaggctct gagggctgtt ttttttttt 459
<210> 466
   <211> 250
   <212> DNA
   <213> Homo sapiens
<400> 466
   tatacccagg atattatcta acgtgtctaa caggggtgtt aatgatatta gcagcaagag 60
   ctatgagaaa taactttaga cattatttca ttgaaccttc ccaactgaaa ttattttatg 120
   atgttataac atggatagta actcaagtag caataagtta cacagttgtg ccatttgtgc 180
   ttctttctat aaaaccatca ctcacgtttt acagctcctg gtattattgc ctgcacattc 240
   ttggtatctt 250
<210> 467
   <211> 509
   <212> DNA
   <213> Homo sapiens
<400> 467
   atactttatc tattttcggg caacttgctt ccctcatgaa ccatggacat ctcaatgtgc 60
   cattacacac aggagttata tgttaggtat tgttgtccca ttttacagaa gagaatccgc 120
   aaggttcaca gagtgaatca taggcataaa gtccttcagg tggtaaatgg caaggctggt 180
   gttccaacca gtcttctctg gctccaggga ctggctcctt cagactacat ttcaccagct 240
   gcctccagga acagaagacg ggaattcacc tttcatgcga catataccag aaacgtggac 300
   ctcagccacc ctgggtccta tttgatcccc agggccttca tttggccctc gaataaaaac 360
   cttatttttt tatctcctta cctttcccag aattcatagt aggacttggc tggtgaaagg 420
   ctggttgctg agaaggctac agtgtggcta ggctgcagtt ccctgttatt acattgcccc 480
   aggtattaat attgtatatt taggcagct 509
<210> 468
   <211> 554
   <212> DNA
   <213> Homo sapiens
<400> 468
   ggatttcaaa tctgagatga tactttatct attttcgggc aacttgcttc cctcatgaac 60
   catggacatc tcaatgtgcc attacacaca ggagttatat gttaggtatt gttgtcccat 120
   tttacagaag agaatccgca aggttcacag agtgaatcat aggcataaag tccttcaggt 180
   ggtaaatggc aaggctggtg ttccaaccag tcttctctgg ctccagggac tggctccttc 240
   agactacatt tcaccagctg cctccaggaa cagaagacgg gaattcacct ttcatgcgac 300
   atataccaga aacgtggacc tcagccaccc tgggtcctat ttgatcccca gggccttcat 360
   ttggccctcg aataaaaacc ttattttttt atctccttac ctttcccaga attcatagta 420
   ggacttggct ggtgaaaggc tggttgctga gaaggctaca gtgtggctag gctgcagttc 480
   cctgttatta cattgcccca ggtattaata ttgtatattt aggcagctgt tctcatccgt 540
   gcctggcagt gaaa                                                   554
<210> 469
   <211> 537
   <212> DNA
   <213> Homo sapiens
<400> 469
   attctgaccc cattgtgcac cttagtcatg gcaaactttc cagttgctcc ttgccaaaac 60
   tcaagaataa aagggcccaa gctagagagg ctgtcctcac aagcatcagc tgctgggggc 120
   ttccactcat tttcctctga aacaacagag aaagagacca tctctcattc gcagagcagc 180
   ccaaggcctt ctgaggagac tgtgagtctc ctctaagtca tttctctctg ctttgtagca 240
   gtggagctac caagggtgag atgagcaggt tgagaggcct ctgaagcctg ctgggcacaa 300
   tgctctgtga taagtttcag ctccactgga gcttatcatc caccagcaat cgacttcatg 360
   gctgctgctc agaggcccta ggtgctgcgc tgctcactgc cctcacgtct ctgggacttc 420
   cacacataaa gccatctctt tccattgcac tatggcactt gtagggagga tcccacactt 480
   agggcccaaa atgagaccat ttgagtcaaa tttctaattg tctttcaaat tttatta    537
<210> 470
   <211> 492
   <212> DNA
   <213> Homo sapiens
<400> 470
   attctgaccc cattgtgcac cttagtcatg gcaaactttc cagttgctcc ttgccaaaac 60
   tcaagaataa aagggcccaa gctagagagg ctgtcctcac aagcatcagc tgctgggggc 120
   ttccactcat tttcctctga aacaacagag aaagagacca tctctcattc gcagagcagc 180
   ccaaggcctt ctgaggagac tgtgagtctc ctctaagtca tttctctctg ctttgtagca 240
   gtggagctac caagggtgag atgagcaggt tgagaggcct ctgaagcctg ctgggcacaa 300
   tgctctgtga taagtttcag ctccactgga gcttatcatc caccagcaat cgacttcatg 360
   gctgctgctc agaggcccta ggtgctgcgc tgctcactgc cctcacgtct ctgggacttc 420
   cacacataaa gccatctctt tccattgcac tatggcactt gtagggagga tcccacactt 480
   agggcccaaa tg                                                     492
<210> 471
   <211> 509
   <212> DNA
   <213> Homo sapiens
<400> 471
   aagacattca aattagccac cactggagta gatgacctaa aagttcttac aactctcaat 60
   tatacccagt gatgtctcga ttagcactta ttataaaaat taaaatttat aattcaacat 120
   ttataccatc cagaaaaagt taaaatatat taatagccta tttctcttca ataaagcgta 180
   tatataactc tatttgttaa tgtttctatt ctccatgaca ttctgtttat agataagccc 240
   tatgctattt ctagtcaagt gctaatctct tgaatgaagc tgaattaggt agtcaactac 300
   tagatgtatc ctgaaaagca agtaatgtgt atatttcatt tattttatac ataagagcta 360
   cagactgttg tcacaatctt ttcaagggct attaaattca ttattttaac taacattttt 420
   gaacatctgt cttatgttgt taattgagga catttctgaa tgtataacaa cataagaata 480
   atagttgtta aacttcaaag agatgacag                                   509
<210> 472
   <211> 649
   <212> DNA
   <213> Homo sapiens
<400> 472
   caaattagcc accactggag tagatgacct aaaagttctt acaactctca attataccca 60
   gtgatgtctc gattagcact tattataaaa attaaaattt ataattcaac atttatacca 120
   tccagaaaaa gttaaaatat attaatagcc tatttctctt caataaagcg tatatataac 180
   tctatttgtt aatgtttcta ttctccatga cattctgttt atagataagc cctatgctat 240
   ttctagtcaa gtgctaatct cttgaatgaa gctgaattag gtagtcaact actagatgta 300
   tcctgaaaag caagtaatgt gtatatttca tttattttat acataagagc tacagactgt 360
   tgtcacaatc ttttcaaggg ctattaaatt cattatttta actaacattt ttgaacatct 420
   gtcttatgtt gttaattgag gacatttctg aatgtataac aacataagaa taatagtttt 480
   taaacttcaa agagatgaca ggttaatgag taaaggagaa atatgaaata tcacagaatt 540
   ccttgacact aaatgatgtt ttgcaaatac tgaacagaat gatgtttgta aactttccac 600
   tggttttcaa gagtcccaaa acattaggaa aatgtacatc acctaactt             649
<210> 473
   <211> 494
   <212> DNA
   <213> Homo sapiens
<400> 473
   atatcagaag taaaacaatt tttcttgttg actgctttgg taaaaaacag tttgatggat 60
   agttttacat ttcactggac tagataaaaa atggtgctaa tatttatgta gcttgatgct 120
   atagttgctt tggtatcaaa cttaatacct aacccatata agatccttat tatataattt 180
   tgtgatcagt aaaatgatat tttaaagagt gatcttaaaa atatgacctg gtcattgcac 240
   aacgtttgca tttgaaatga atttttgtac tatagggtgg atatggagtt attcagtgca 300
   agtgtgtgct taatatcaaa ccctatgcaa ggagctatgt ctagattttt ggtccgaatt 360
   tgcctccttc aagcctacta gtgtgagatg gaaaaaaatc gattgctctt ttaatattat 420
   ttccattttg aaattctcga cacttgaatg aaggcagtag aagcctcttt ttggatttct 480
   cttctaataa caaa                                                   494
<210> 474
   <211> 630
   <212> DNA
   <213> Homo sapiens
<400> 474
   aaaacatttt tcttgttgac tgctttggta aaaaacagtt tgatggatag ttttacattt 60
   cactggacta gataaaaaat ggtgctaata tttatgtagc ttgatgctat agttgctttg 120
   gtatcaaact taatacctaa cccatataag atccttatta tataattttg tgatcagtaa 180
   aatgatattt taaagagtga tcttaaaaat atgacctggt cattgcacaa cgtttgcatt 240
   tgaaatgaat ttttgtacta tagggtggat atggagttat tcagtgcaag tgtgtgctta 300
   atatcaaacc ctatgcaagg agctatgtct agatttttgg tccgaatttg cctccttcaa 360
   gcctactagt gtgagatgga aaaaaatcga ttgctctttt aatattattt ccattttgaa 420
   attctcgaca cttgaatgaa ggcagtagag gcctcttttt ggatttctct tctaataaca 480
   aaactttatt tagggaaggt ttccctgtgc tatcgtaagt ttgttttgag cactgcattc 540
   actttaaaat tctggaggaa caaaggctgg gcacataatc acaaagccca ggccacacaa 600
   taattccggg gttgtatttt ctaagaacta                                  630
<210> 475
   <211> 156
   <212> DNA
   <213> Homo sapiens
<400> 475
   gggggagata aggcaaagag gcacttttgg atttctccat ctgagcagct ctgtgattca 60
   ttatctgttc tagaaagcag cacacgcagt tccagcaaaa aaaaaaaaaa aaaaaaattt 120
   tttttttttt cccccctttt tttttttttt ttcccc                             156
<210> 476
   <211> 579
   <212> DNA
   <213> Homo sapiens
<400> 476
   attccgttgc tgtcggcggc cgggtcccga tgagcctcct gttgcctccg ctggcgctgc 60
   tgctgcttct cgcggcgctt gtggccccag ccacagccgc cactgcctac cggccggact 120
   ggaaccgtct gagcggccta acccgcgccc gggtagagac ctgcggggga tgacagctga 180
   accgcctaaa ggaggtgaag gctttcgtca cgcaggacat tccattctat cacaacctgg 240
   tgatgaaaca cctccctggg gccgaccctg agctcgtgct gctgggccgc cgctacgagg 300
   aactagagcg catcccactc agtgaaatga cccgcgaaga gatcaatgcg ctagtgcagg 360
   agctcggctt ctaccgcaag gcggcgcccg acgcgcaggt gccccccgag tacgtgtggg 420
   cgcccgcgaa gcccccagag gaaacttcgg accacgctga cctgtaggtc cgggggcgcg 480
   gcggagctgg gacctacctg cctgagtcct ggagacagaa tgaagcgctc agcatcccgg 540
   gaatacttct cttgctgaga gccgatgccc gtccccggg                        579
<210> 477
   <211> 472
   <212> DNA
   <213> Homo sapiens
<400> 477
   ggcttagcgg ataacaattt cacacaggag ctagcagaca ccacaagata ccaacagagc 60
   ttctgaaaca gatacccata gcattggaga gaaaaacagc tcacagtctg aggaagatga 120
   tattgaaaga aggaaagaag ttgaaagcat cttgaagaaa aactcagatt ggatatggga 180
   ttggtcaagt cggccggaaa atattccccc caaggagttc ctctaaacac ccgaagcgca 240
   cggccaccct cagcatgagg aacacgagcg tcatgaagaa agggggcata ttctctgcag 300
   aatttctgaa agatttcctt ccatctctgc tgctctctca tttgctggcc atcggattgg 360
   ggatctatat tggaaggcgt gtgacaacct ccaccagcac cttttgatga agaactggag 420
   tctgacttgg ttcgttagtg gattacttct gagcttgcaa catagctcac tg         472
<210> 478
   <211> 355
   <212> DNA
   <213> Homo sapiens
<400> 478
   tctacactta aagctttgga gcaattccca tcgaccagag ttggtccgac cagccttgga 60
   aaggtcactg aaaaatcttc aattggacta tggtgacctc tatcttatac attttccatt 120
   gtctgcaaag ccaggtgagg aagtgatccc aaaagatgac aatggaaaaa tactatttga 180
   cacagtggat ctctgtgcca catgggaggc catggagaag tgtaaagatg cacgattggc 240
   caagtccatc ggggtgtcca acttcaacca caggctgctg gagatgatcc tcaacaagcc 300
   agggctcaag tacaagcctg tctgcaacca ggtggaatgt catccttact tcaac      355
<210> 479
   <211> 510
   <212> DNA
   <213> Homo sapiens
<400> 479
   aagactactg aatctgctac caaaacagtg aatcagtgag tcgatgttct attttttgtt 60
   ttgtttcctc ccctatctgt attcccaaaa attactttgg ggctaattta acaagaactt 120
   taaattgtgt tttaattgta aaaatggcag ggggtggaat tattactcta tacattcaac 180
   agagactgaa tagatatgaa agctgatttt ttttaattac catgcttcac aatgttaagt 240
   tatatgggga gcaacagcaa acaggtgcta atttgttttg gatatagtat aagcagtgtc 300
   tgtgttttga aagaatagaa cacagtttgt agtgccactg ttgttttggg ggggcttttt 360
   tcttttcgga aatcttaaac cttaagatac taaggacgtt gttttggttg tactttggaa 420
   ttcttagtca caaaatatat tttgtttaca aaaatttctg taaaacaggt tataacagtg 480
   tttaaagtct cagtttcttg cttggggaac                                  510
<210> 480
   <211> 371
   <212> DNA
   <213> Homo sapiens
<400> 480
   ttccgttgct gtcggaattg aggaagagct gggggatgaa gctcgctttg ccggacataa 60
   cttccgtaat cccagtgtgc tgtgattcct ctgcttgcct ggagacgtgg aacctctgtc 120
   tcatcctcct ggaaccttgc tgtcctgatc tgtgatagtt caccccctga gatcccctga 180
   gccccagggt gcccagaact tccctgattg acctgctccg ctgctccttg gcttacctga 240
   cctcttgctg tctctgctcg ccctcctttc tgtgccctac tcattggggt tccgcacttt 300
   ccacttcttc ctttctcttt ctctcttccc tcaaaaacta gaaatgtgaa tgaggattat 360
   tataaaaggg g                                                      371
<210> 481
   <211> 543
   <212> DNA
   <213> Homo sapiens
<400> 481
   aattccgttg ctgtcggtgt ctggaggcca tcctccagaa ctctcctgac gccaaaatct 60
   tctgcctggt gcacaaaatg gatctggttc aggaggatca gcgtgacctg atttttaaag 120
   agcgagagga agacctgagg cgtctgtctc gcccgctgga gtgtgcttgt tttcgaacgt 180
   ccatctggga tgagacgctc tacaaagcct ggtccagcat cgtctaccag ctgattccca 240
   acgttcagca gctggagatg aacctcagga attttgccca aatcattgag gccgatgaag 300
   ttctgctgtt cgaaagagct acattcttgg ttatttccca ctaccagtgc aaagagcagc 360
   gcgacgtcca ccggtttgag aagatcagca acatcatcaa acagttcaag ctgagctgca 420
   gtaaattggc cgcttccttc cagagcatgg aagttaggaa ttccaacttc gctgctttca 480
   tcgacatctt cacctcaaat acgtacgtga tggtggtcat gtcagatccg tcgatccctt 540
   ctg                                                               543
<210> 482
   <211> 415
   <212> DNA
   <213> Homo sapiens
<400> 482
   ggcttactca ctatagggct tttttttttt tcgggtctat tctttaattt tactaaatta 60
   ggaacgcagc ttttacagaa caaataaccc caggggacgg ggccccccca ggatctaaca 120
   gcttttcagg gagctatgtt gcaagctcaa aagtaatcca ctaacgaacc aagtcaaact 180
   ccagttttta ataaaaaggg gctgggggag gttgtcaaac cccttccaat ataaatcccc 240
   aatccgatgg ccaccaaatg aaaaagcacc agggatggaa ggaaaacttt caaaaattct 300
   gcaaaaaata tgcccccttt tttaatgacc ctcgggttcc taatgctaag gggggccgcc 360
   cccttcgggg gttaaaaaag gaactccttg gggggaatat tttccggccg acttg      415
<210> 483
   <211> 240
   <212> DNA
   <213> Homo sapiens
<400> 483
   tttttttttt taaagtcatg gaggccatgg ggttggcttg aaaccacctt tggggggtcc 60
   aatcccttcc ttttttgcct aaattttatg tatacgggtt cttcaaatgc gtggtagggg 120
   ggggggcatc catatagtcc ctccaggttt atggagggtt cttctactat taggactttt 180
   cgcttcaaaa caaaggcttt tcaaatcatg aaaattttta attttcctgc tgttaaaaaa 240
<210> 484
   <211> 293
   <212> DNA
   <213> Homo sapiens
<400> 484
   tttttttttt aataaatctc ctaaggggat ggctactttt tctatctaaa taataatata 60
   tagacctatt cgatcagaga tacaggggac taacaatcac aatcctgtga tcgacatccg 120
   aacataagtc actatctatc agaataaaca atgatccaac gaataataga ggagtaaggg 180
   gacatgtcca aagcatcagg tatcgtcatg atcgaaaacc actgtcaagc aagacacaaa 240
   caaacaaaac agctttacac acaagtcagc agtccaagcg ttcatgtccc aag        293
<210> 485
   <211> 221
   <212> DNA
   <213> Homo sapiens
<400> 485
   tttttttttt tcaagggaca ctttaatggt taacttaagg gatcatcaat tttgcctcac 60
   tacctacaaa gggaatttca tcttgtcccc atgctgagta gggaaacagg gacaaagtta 120
   atcataatac cctacatcaa aaaaaaacta agctaacact gctaactttt tttttaacag 180
   gcaaaatata aatatatgcc ctctaaaatg cccaagggtt t                     221
<210> 486
   <211> 563
   <212> DNA
   <213> Homo sapiens
<400> 486
   ttccgttgct gtcgcctccg ctctgctctt cgtggaacac gaccgtggtg cccggccctt 60
   gggagccttg gggccagctg gcctgctgct ctccagtcaa gtagcgaagc tcctaccacc 120
   cagacaccca aacagccgtg gccccagagg tcctggccaa atatgggggc ctgcctaggt 180
   tggtggaaca gtgctcctta tgtaaactga gccctttgtt taaaaaacaa ttccaaatgt 240
   gaaactagaa tgagagggaa gagataacat ggcatgcagc acacacggct gctccagttc 300
   atggcctccc aggggtgctg ggatgcatcc caaagtggtt gtctgagaca gagttggaaa 360
   ccctcaccaa ctggcctctt caccttccac attatcccgc tgccaccggc tgccctgtct 420
   cactgcagat tcaggaccag cttgggctgc gtgcgttctg ccttgccagt cagccgagga 480
   tgtagttgtt gctgccgtcg tcccaccacc tcagggacca gagggctagg ttggcactgc 540
   ggccctcacc aggtcctggg ctc                                         563
<210> 487
   <211> 271
   <212> DNA
   <213> Homo sapiens
<400> 487
   ctcatatggt caggtcgctt caaaaaggaa gatgaaatcc cagagactgt ctcgttggag 60
   atgcttgatg ctgcaaagaa caagatgcga gtgaagatca gctatctaat gattgccctg 120
   acggtggtag gatgcatctt catggttatt gagggcaaga aggctgccca aagacacgag 180
   actttaacaa gcttgaactt agaaaagaaa gctcgtctga aagaggaagc agctatgaag 240
   gccaaaacag agtagcagag gtatccgtgt t                                271
<210> 488
   <211> 342
   <212> DNA
   <213> Homo sapiens
<400> 488
   ggcttgtaat acgactcact atagggcttt ttttttttcg aattaaaaaa attccgttag 60
   ccttttctcc atctcctcta attctggtag catctttgga cccctaacac ttggcatctg 120
   ctacttcaga caaacaaacc ctatgtaaat gacaaagaag gggcctccca accttctccc 180
   tgtgttacta tttcaaaagc actactcggg gcacaggggt acaaatttct tatggccact 240
   agcatctttt ttcaattttc aaaggaatca tcaaacatct gggtcaatta tacttaaatt 300
   acagaagccc ggaattttag gcaacaggcc cctcatttta cc                    342
<210> 489
   <211> 326
   <212> DNA
   <213> Homo sapiens
<400> 489
   tttttttttt aaaaagtcat ggaggccatg gggttggctt gaaaccagct ttggggggtt 60
   cgattccttc cttttttgtc taaattttat gtatacgggt tcttcaaatg tgtggtaggg 120
   tggggggcat ccatatagtc actccaggtt tatggagggt tcttctacta ttaggacttt 180
   tcgcttcgaa gcgaaggctt ctcaaatcat gaaaattatt aatattactg ctgttagaaa 240
   aatgaatgag cctaccgatg ataggatgtt tcatgtggtg tatgcatcgg ggtagtccga 300
   gtaacgtcgg ggcattccgg ataggc                                      326
<210> 490
   <211> 55
   <212> DNA
   <213> Homo sapiens
<400> 490
   tttttttttt tttttttttg agaaaccggg gggggttttt tttttaaaat tgggg 55
<210> 491
   <211> 558
   <212> DNA
   <213> Homo sapiens

<400> 491
   cgccgcgtcc ccttctcgct cctgcggggc cccagctggg accccttccg cgactggtac 60
   ccgcatagcc gcctcttcga ccaggccttc gggctgcccc ggctgccgga ggagtggtcg 120
   cagtggttag gcggcagcag ctggccaggc tacgtgcgcc ccctgccccc cgccgccatc 180
   gagagccccg cagtggccgc gcccgcctac agccgcgcgc tcagccggca actcagcagc 240
   ggggtcttcg gagatccggc acactgcgga ccgctggcgc gtgtccctgg atgtcaacca 300
   cttcgccccg gacgagctga cggtcaagac caaggatggc gtggtggaga tcaccggcaa 360
   gcacgaggag cggcaggacg agcatggcta catctcccgg tgcttcacgc ggaaatacac 420
   gctgcccccc ggtgtggacc ccacccaagt ttcctcctcc ctgtcccctg agggcacact 480
   gaccgtggag gcccccatgc ccaagctagc cacgcagtcc aacgagatca ccatcccagt 540
   caccttcgag tcgcgggc                                               558
<210> 492
   <211> 370
   <212> DNA
   <213> Homo sapiens
<400> 492
   ggctagcgga taacaatttc acacaggatg gattggtcag agtgaattga atattgtaag 60
   tcagccactg ggacccgagg atttctggga ccccgcagtt gggaggagga agtagtccag 120
   ccttccaggt ggcgtgagag gcaatgactc gttacctgcc gcccatcacc ttggaggcct 180
   tccctggcct tgagtagaaa agtcggggat cggggcaaga gaggctgagt acggatggga 240
   aactattgtg cacaagtctt tccagaggag tttcttaatg agatatttgt atttatttcc 300
   agaccaataa atttgtaact ttgcgaaaaa aaaaaagccc tatagtgagt cgtattacaa 360
   gccgaattcc 370
<210> 493
   <211> 560
   <212> DNA
   <213> Homo sapiens
<400> 493
   cagccagcat gaccgagcgc cgcgtcccct tctcgctcct gcggggcccc agctgggacc 60
   ccttccgcga ctggtacccg catagccgcc tcttcgacca ggccttcggg ctgccccggc 120
   tgccggagga gtggtcgcag tggttaggcg gcagcagctg gccaggctac gtgcgccccc 180
   tgccccccgc cgccatcgag agccccgcag tggccgcgcc cgcctacagc cgcgcgctca 240
   gccggcaact cagcagcggg gtctcggaga tccggcacac tgcggaccgc tggcgcgtgt 300
   ccctggatgt caaccacttc gccccggacg agctgacggt caagaccaag gatggcgtgg 360
   tggagatcac cggcaagcac gaggagcggc aggacgagca tggctacatc tcccggtgct 420
   tcacgcggaa atacacgctg ccccccggtg tggaccccac ccaagtttcc tcctccctgt 480
   cccctgaggg cacactgacc gtggaggccc ccatgcccaa gctagccacg cagtccaacg 540
   agatcaccat cccagtcacc 560
<210> 494
   <211> 443
   <212> DNA
   <213> Homo sapiens
<400> 494
   ggcttgtaat acgactcact atagggcttt tttttttgca agtgctgtgg gaagaaagtt 60
   agatttacgc cgatgaatat gatagtgaaa tggattttgg cgtaggtttg gtctagggtg 120
   tagcctgaga ataggggaaa tcagtgaatg aagcctccta tgatggcaaa tacagctcct 180
   attgatagga catagtggaa gtgagctaca acgtagtacg tgtcgtgtag tacgatgtct 240
   agtgatgagt ttgctaatac aatgccagtc aggccaccta cggtgaaaag aaagatgaat 300
   cctagggctc aaagcactgc agcagatcat ttcatattgc ctccgtggag tgtggcgagt 360
   cagctaaata ctttgacgcc ggtggggata gcgatgatta tggtagcatc atcctgtgtg 420
   aaattgttat ccgctaagcc gaa 443
<210> 495
   <211> 249
   <212> DNA
   <213> Homo sapiens
<400> 495
   tttttttttt cgaaggattt ggcaaagatt tgtttttttt tccatttcca gttttttaaa 60
   gtaaacacag atttgcttaa aataaagctg attttaaaag cccacaaaag ttgaacacaa 120
   aggagaggat taaattcccc aatgcagagt gataaaaagg aaaagatcct gagtaggtgc 180
   cttcagcaaa aaactgatca tccagggtga tcacctaata atcggagact taattcctta 240
   taatgcaaa 249
<210> 496
   <211> 434
   <212> DNA
   <213> Homo sapiens
<400> 496
   tttccgtatc tgcttcgggc ttccacctca tttttttcgc tttgcccatt ctgtttcagc 60
   cagtcgccaa gaatcatgaa agtcgccagt ggcagcaccg ccaccgccgc cgcgggcccc 120
   agctgcgcgc tgaaggccgg caagacagcg agcggtgcgg gcgaggtggt gcgctgtctg 180
   tctgagcaga gcgtggccat ctcgcgctgc gccgggggcg ccggggcgcg cctgcctgcc 240
   ctgctggacg agcagcaggt aaacgtgctg ctctacgaca tgaacggctg ttactcacgc 300
   ctcaaggagc tggtgcccac cctgccccag aaccgcaagg tgagcaaggt ggagattctc 360
   cagcacgtca tcgactacat cagggacctt cagttggagc tgaactcgga atccgaagtt 420
   ggaacccccg gggg                                                    434
<210> 497
   <211> 368
   <212> DNA
   <213> Homo sapiens
<400> 497
   tttttttttg cttatggagg gttcctctac tattaggact tttcgcttcg aagcgaaggc 60
   ttctcaaatc atgaaaatta ttaatattac tgctgttaga gaagtgaatg accctacaga 120
   tgataggatg tttcatgtgg tgtatgcatc ggggtagtcc gagtaacgtc ggggcattcc 180
   ggataggccg aaaaagtgtt gtgggaaaaa agttagattt accccgatga atatgatagt 240
   gaaatggatt ttggcgtagg tttggtctag ggtgtaccct gagaataggg gaaatcagtg 300
   aatgaagcct cctatgatgg caaatacagc tcctattgat aggacatagt ggaagtgagc 360
   tacaacgt                                                           368
<210> 498
   <211> 482
   <212> DNA
   <213> Homo sapiens
<400> 498
   ccagccttcc tgtcccgggc cagcgctctg acatgcagaa ggtgaccctg ggcctgcttg 60
   tgttcctggc aggctttcct gtcctggacg ccaatgacct agaagataaa aacagtcctt 120
   tctactatga ctggcacagc ctccaggttg gcgggctcat ctgcgctggg gttctgtgcg 180
   ccatgggcat catcatcgtc atgagtgcaa aatgcaaatg caagtttggc cagaagtccg 240
   gtcaccatcc aggggagact ccacctctca tcaccccagg ctcagcccaa agctgatgag 300
   gacagaccag ctgaaattgg gtggaggacc gttctctgtc cccaggtcct gtctctgcac 360
   agaaacttga actccaggat ggaattcttc ctcctctgct gggactcctt tgcatggcag 420
   ggcctcatct cacctctcgc aagagggtct ctttgttcaa ttttttttta tctaaaatga 480
   tt                                                                 482
<210> 499
   <211> 489
   <212> DNA
   <213> Homo sapiens
<400> 499
   tggcgagcag tttcccactt gccaaagatc ccttttaacc aacactagcc cttgttttta 60
   acacacgctc cagcccttca tcagcctggg cagtcttacc aaaatgttta aagtgatctc 120
   agaggggccc atggattaac gccctcatcc caaggtccgt cccatgacat aacactccac 180
   acccgcccca gccaacttca tgggtcactt tttctggaaa ataatgatct gtacagacag 240
   gacagaatga aactcctgcg gctctttggc ctgaaagttg ggaatggttg ggggagagaa 300
   gggcagcagc ttattggtgg tcttttcacc attggcagaa acagtgagag ctgtgtggtg 360
   cagaaatcca gaaatgaggt gtagggaatt ttgcctgcct tcctgcagac ctgagctggc 420
   tttggaatga ggttaaagtg tcagggacgt tgcctgagcc caaatgtgta gtgtggtctg 480
   ggcaggcag                                                         489
<210> 500
   <211> 25
   <212> DNA
   <213> Artificial Sequence
<220>
   <223> Oligonucleotide primer
<400> 500
   ggaatcaccg ctttgccatc ttcaa 25
<210> 501
   <211> 25
   <212> DNA
   <213> Artificial Sequence
<220>
   <223> Oligonucleotide primer
<400> 501
   aacttctacc gtttcgccac taagg 25
<210> 502
   <211> 23
   <212> DNA
   <213> Artificial Sequence
<220>
   <223> Oligonucleotide primer
<400> 502
   gaccgtgtac tgcgtgtcgt gcg 23
<210> 503
   <211> 23
   <212> DNA
   <213> Artificial Sequence
<220>
   <223> Oligonucleotide primer
<400> 503
   gcgtgctgtg cgtcatgtgc cag 23
<210> 504
   <211> 24
   <212> DNA
   <213> Artificial Sequence
<220>
   <223> Oligonucleotide primer
<400> 504
   gccgtcttca ggcaacaact ccca 24
<210> 505
   <211> 24
   <212> DNA
   <213> Artificial Sequence
<220>
   <223> Oligonucleotide primer
<400> 505
   tgctggacga ggctgtcatc ttgc 24
<210> 506
   <211> 25
   <212> DNA
   <213> Artificial Sequence
<220>
   <223> Oligonucleotide primer
<400> 506
   acagggagaa aactggttgt cctgg 25
<210> 507
   <211> 23
   <212> DNA
   <213> Artificial Sequence
<220>
   <223> Oligonucleotide primer
<400> 507
   aaggcagaac ccatccactc caa 23
<210> 508
   <211> 25
   <212> DNA
   <213> Artificial Sequence
<220>
   <223> Oligonucleotide primer
<400> 508
   gctgctggat tcgtttggca taact 25
<210> 509
   <211> 25
   <212> DNA
   <213> Artificial Sequence
<220>
   <223> Oligonucleotide primer
<400> 509
   tcaatacggt ttgcttaggt cgtcg 25
<210> 510
   <211> 24
   <212> DNA
   <213> Artificial Sequence
<220>
   <223> Oligonucleotide primer
<400> 510
   tctcctctga gttcaaccgc tgct 24
<210> 511
   <211> 24
   <212> DNA
   <213> Artificial Sequence
<220>
   <223> Oligonucleotide primer
<400> 511
   tcgtcgccaa cttgagtctc ctct 24
<210> 512
   <211> 406
   <212> PRT
   <213> Homo sapiens
<400> 512
   Met Ala Glu Asn Gly Lys Asn Cys Asp Gln Arg Arg Val Ala Met Asn
    1               5                  10                  15
   Lys Glu His His Asn Gly Asn Phe Thr Asp Pro Ser Ser Val Asn Glu
               20                  25                  30
   Lys Lys Arg Arg Glu Arg Glu Glu Arg Gln Asn Ile Val Leu Trp Arg
           35                  40                  45
   Gln Pro Leu Ile Thr Leu Gln Tyr Phe Ser Leu Glu Ile Leu Val Ile
       50                  55                  60
   Leu Lys Glu Trp Thr Ser Lys Leu Trp His Arg Gln Ser Ile Val Val
   65                  70                  75                  80
   Ser Phe Leu Leu Leu Leu Ala Val Leu Ile Ala Thr Tyr Tyr Val Glu
                   85                  90                  95
   Gly Val His Gln Gln Tyr Val Gln Arg Ile Glu Lys Gln Phe Leu Leu
               100                 105                 110
   Tyr Ala Tyr Trp Ile Gly Leu Gly Ile Leu Ser Ser Val Gly Leu Gly
           115                 120                 125
   Thr Gly Leu His Thr Phe Leu Leu Tyr Leu Gly Pro His Ile Ala Ser
       130                 135                 140
   Val Thr Leu Ala Ala Tyr Glu Cys Asn Ser Val Asn Phe Pro Glu Pro
   145                 150                 155                 160
   Pro Tyr Pro Asp Gln Ile Ile Cys Pro Asp Glu Glu Gly Thr Glu Gly
                   165                 170                 175
   Thr Ile Ser Leu Trp Ser Ile Ile Ser Lys Val Arg Ile Glu Ala Cys
               180                 185                 190
   Met Trp Gly Ile Gly Thr Ala Ile Gly Glu Leu Pro Pro Tyr Phe Met
           195                 200                 205
   Ala Arg Ala Ala Arg Leu Ser Gly Ala Glu Pro Asp Asp Glu Glu Tyr
       210                 215                 220
   Gln Glu Phe Glu Glu Met Leu Glu His Ala Glu Ser Ala Gln Asp Phe
   225                 230                 235                 240
   Ala Ser Arg Ala Lys Leu Ala Val Gln Lys Leu Val Gln Lys Val Gly
                   245                 250                 255
   Phe Phe Gly Ile Leu Ala Cys Ala Ser Ile Pro Asn Pro Leu Phe Asp
               260                 265                 270
   Leu Ala Gly Ile Thr Cys Gly His Phe Leu Val Pro Phe Trp Thr Phe
           275                 280                 285
   Phe Gly Ala Thr Leu Ile Gly Lys Ala Ile Ile Lys Met His Ile Gln
       290                 295                 300
   Lys Ile Phe Val Ile Ile Thr Phe Ser Lys His Ile Val Glu Gln Met
   305                 310                 315                 320
   Val Ala Phe Ile Gly Ala Val Pro Gly Ile Gly Pro Ser Leu Gln Lys
                   325                 330                 335
   Pro Phe Gln Glu Tyr Leu Glu Ala Gln Arg Gln Lys Leu His His Lys
               340                 345                 350
   Ser Glu Met Gly Thr Pro Gln Gly Glu Asn Trp Leu Ser Trp Met Phe
           355                 360                 365
   Glu Lys Leu Val Val Val Met Val Cys Tyr Phe Ile Leu Ser Ile Ile
       370                 375                 380
   Asn Ser Met Ala Gln Ser Tyr Ala Lys Arg Ile Gln Gln Arg Leu Asn
   385                 390                 395                 400
   Ser Glu Glu Lys Thr Lys
                   405
<210> 513
   <211> 1221
   <212> DNA
   <213> Homo sapiens
<400> 513
   atggcagaga atggaaaaaa ttgtgaccag agacgtgtag caatgaacaa ggaacatcat 60
   aatggaaatt tcacagaccc ctcttcagtg aatgaaaaga agaggaggga gcgggaagaa 120
   aggcagaata ttgtcctgtg gagacagccg ctcattacct tgcagtattt ttctctggaa 180
   atccttgtaa tcttgaagga atggacctca aaattatggc atcgtcaaag cattgtggtg 240
   tcttttttac tgctgcttgc tgtgcttata gctacgtatt atgttgaagg agtgcatcaa 300
   cagtatgtgc aacgtataga gaaacagttt cttttgtatg cctactggat aggcttagga 360
   attttgtctt ctgttgggct tggaacaggg ctgcacacct ttctgcttta tctgggtcca 420
   catatagcct cagttacatt agctgcttat gaatgcaatt cagttaattt tcccgaacca 480
   ccctatcctg atcagattat ttgtccagat gaagagggca ctgaaggaac catttctttg 540
   tggagtatca tctcaaaagt taggattgaa gcctgcatgt ggggtatcgg tacagcaatc 600
   ggagagctgc ctccatattt catggccaga gcagctcgcc tctcaggtgc tgaaccagat 660
   gatgaagagt atcaggaatt tgaagagatg ctggaacatg cagagtctgc acaagacttt 720
   gcctcccggg ccaaactggc agttcaaaaa ctagtacaga aagttggatt ttttggaatt 780
   ttggcctgtg cttcaattcc aaatccttta tttgatctgg ctggaataac gtgtggacac 840
   tttctggtac ctttttggac cttctttggt gcaaccctaa ttggaaaagc aataataaaa 900
   atgcatatcc agaaaatttt tgttataata acattcagca agcacatagt ggagcaaatg 960
   gtggctttca ttggtgctgt ccccggcata ggtccatctc tgcagaagcc atttcaggag 1020
   tacctggagg ctcaacggca gaagcttcac cacaaaagcg aaatgggcac accacaggga 1080
   gaaaactggt tgtcctggat gtttgaaaag ttggtcgttg tcatggtgtg ttacttcatc 1140
   ctatctatca ttaactccat ggcacaaagt tatgccaaac gaatccagca gcggttgaac 1200
   tcagaggaga aaactaaata a                                           1221
<210> 514
   <211> 338
   <212> DNA
   <213> Homo sapiens
<400> 514
   gtgctgtccc cggcataggt ccatctctgc agaagccatt tcaggagtac ctggaggctc 60
   aacggcagaa gcttcaccac aaaagcgaaa tgggcacacc acagggagaa aactgcttgt 120
   cctggatgtt tgaaaagtcg gtcgatgtca tggtgtgtta cttcatccta tctatcatta 180
   actccatggc acaaagttat gccaaacgaa tccagcagcg gttgaactca gaggagaaaa 240
   ctaaataagt agagaaagtt ttaaactgca gaaattggag tggatgggtt ctgccttata 300
   ttgggaggac tccaagccgg gaaggaaaat tccctttt                         338
<210> 515
   <211> 186
   <212> DNA
   <213> Homo sapiens
<400> 515
   tgtgttaatg ttttctagca tgtactctgg tttcaacaga cacaaattta tatgttaacc 60
   cagttttctt gccgttctgt aagtgtttta ttcttagtgt gatttttttc cattgggatg 120
   tttttgattg aacttgttca ttttgttttg cttgggagga aaataaacaa ttttactttt 180
   ttcctt                                                            186
<210> 516
   <211> 118
   <212> DNA
   <213> Homo sapiens
<400> 516
   acagggagaa aactggttgt cctggatgtt tgaaaagttg gtcgttgtca tggtgtgtta 60
   cttcatccta tctatcatta actccatggc acaaagttat gccaaacgaa tccagcag   118

## Description

The present invention relates to DKFZp566I133, which is differentially expressed in breast cancer cells.

### BACKGROUND OF THE INVENTION

Cancer, like many diseases, is not the result of a single, well-defined cause, but rather can be viewed as several diseases, each caused by different aberrations in informational pathways, that ultimately result in apparently similar pathologic phenotypes. Identification of polynucleotides that correspond to genes that are differentially expressed in cancerous, pre-cancerous, or low metastatic potential cells relative to normal cells of the same tissue type, provides the basis for diagnostic tools, facilitates drug discovery by providing for targets for candidate agents, and further serves to identify therapeutic targets for cancer therapies that are more tailored for the type of cancer to be treated.

Identification of differentially expressed gene products also furthers the understanding of the progression and nature of complex diseases such as cancer, and is key to identifying the genetic factors that are responsible for the phenotypes associated with development of, for example, the metastatic phenotype. Identification of gene products that are differentially expressed at various stages, and in various types of cancers, can both provide for early diagnostic tests, and further serve as therapeutic targets. Additionally, the product of a differentially expressed gene can be the basis for screening assays to identify chemotherapeutic agents that modulate its activity (e.g. its expression, biological activity, and the like).

Early disease diagnosis is of central importance to halting disease progression, and reducing morbidity. Analysis of a patient's tumor to identify the gene products that are differentially expressed, and administration of therapeutic agent(s) designed to modulate the activity of those differentially expressed gene products, provides the basis for more specific, rational cancer therapy that may result in diminished adverse side effects relative to conventional therapies. Furthermore, confirmation that a tumor poses less risk to the patient (*e.g.*, that the tumor is benign) can avoid unnecessary therapies. In short, identification of genes and the encoded gene products that are differentially expressed in cancerous cells can provide the basis of therapeutics, diagnostics, prognostics, therametrics, and the like.

Breast cancer is a leading cause of death among women. One of the priorities in breast cancer research is the discovery of new biochemical markers that can be used for diagnosis, prognosis and monitoring of breast cancer. The prognostic usefulness of these markers depends on the ability of the marker to distinguish between patients with breast cancer who require aggressive therapeutic treatment and patients who should be monitored.

While the pathogenesis of breast cancer is unclear, transformation of non-tumorigenic breast epithelium to a malignant phenotype may be the result of genetic factors, especially in women under 30 (Miki, et al., Science, 266: 66-71, 1994). However, it is likely that other, non-genetic factors are also significant in the etiology of the disease. Regardless of its origin, breast cancer morbidity increases significantly if a lesion is not detected early in its progression. Thus, considerable effort has focused on the elucidation of early cellular events surrounding transformation in breast tissue. Such effort has led to the identification of several potential breast cancer markers.

Thus, the identification of new markers associated with breast cancer, and the identification of genes involved in transforming cells into the cancerous phenotype, remains a significant goal in the management of this disease. In exemplary aspects, the invention described herein provides breast cancer diagnostics, prognostics, therametrics, and therapeutics based upon polynucleotides and/or their encoded gene products.

### SUMMARY OF THE INVENTION

The invention provides method for detecting a cancerous breast cell, said method comprising:
detecting a level of DKFZp566I133 or fragment thereof in a test sample obtained from a cell of a subject, and
comparing the level of DKFZp566I133 to a control level of DKFZp566I133,
wherein the presence of a cancerous breast cell is indicated by detection of said level and comparison to a control level of DKFZp566I133.

According to a preferred embodiment the level of DKFZp566I133 is detected by measuring a level of a DKFZp566I133 gene product, which can be a polypeptide or a nucleic acid. According to a further preferred embodiment the detecting step of the above method uses a polymerase chain reaction or hybridization. The sample to be used in the above method is preferably a sample of breast tissue. According to a further preferred embodiment the level of the DKFZp566I133 or fragment thereof is indicative of the cancerous state of the cell of the test sample.

In particular, the invention relates to the use of an agent selected from the group consisting of an antibody, an antisense polynucleotide, and an RNAi molecule for inhibition of DKFZp566I133 activity for the preparation of a pharmaceutical composition for inhibiting a cancerous phenotype of a breast cell. According to a preferred embodiment of the invention the cancerous phenotype may be aberrant cellular proliferation relative to a normal cell or loss of contact inhibition of cell growth. According to a further preferred embodiment of the invention the inhibition of DKFZp566I133 activity is associated with a reduction in a level of DKFZp566I133 protein or with a reduction in a level of DKFZp566I133 RNA. According to a still further preferred embodiment of the invention the inhibition the inhibition of DKFZp566I133 activity is associated with a reduction in a level of activity of DKFZp566I133 protein.

In another aspect, the pharmaceutical composition is for treating a subject with breast cancer.

In another aspect, the invention provides a method for assessing the tumor burden of a subject, the method comprising detecting a level of DKFZp566I133 in a test sample from a subject suspected of or having a tumor. Detection of the level of DKFZp566I133 in the test sample is indicative of the tumor burden in the subject.

In another aspect, the invention provides a method for identifying agents that modulate (i.e. increase or decrease) the biological activity of a gene product differentially expressed in a cancerous breast cell as compared to a normal breast cell, said method comprising:
contacting a candidate agent selected from the group consisting of an antibody, an antisense polynucleotide, and an RNAi molecule with a cancerous cell expressing a DKFZp566I133 gene product; and
detecting modulation of expression of the DKFZp566I133 gene product relative to a level of expression of the DKFZp566I133 gene product in the absence of the candidate agent.

According to a preferred embodiment of the invention, the assessment of expression is achieved by detecting either a polynucleotide or a polypeptide gene product.

In specific embodiments, the detecting is by identifying an increase or decrease in expression of the differentially expressed gene product. In other specific embodiments, the gene product is mRNA or cDNA prepared from the mRNA gene product In further embodiments, the gene product is a polypeptide.

### BRIEF DESCRIPTION OF THE FIGURES

Fig. 1 is three panels of autoradiographs showing expression of GAK polypeptide in different cell lines.
Fig. 2 is a graph of a hydropathy plot and a table showing the hydrophobic regions of DKFZp566I133.
Fig. 3 is six panels of photographs of MDA-231 cells exposed to C180-7, C180-8 and positive control antisense (AS) and control (RC) oligonucleotides.
Fig. 4 is an alignment of spot ID 22793 and spot ID 26883.
Fig. 5 is a figure of three sequence alignments showing the mapping of each of three sequences onto VMP1 (DKFZ).

### DETAILED DESCRIPTION OF THE INVENTION

Described herein are polynucleotides, as well as polypeptides encoded thereby, that are differentially expressed in breast cancer cells. Methods are disclosed in which these polynucleotides and polypeptides are used for detecting and reducing the growth of breast cancer cells. Also disclosed are methods in which the polynucleotides and polypeptides of the invention are used in a variety of diagnostic and therapeutic applications for breast cancer.

Before the present invention is described, it is to be understood that this invention is not limited to particular embodiments described, as such may, of course, vary. It is also to be understood that the terminology used herein is for the purpose of describing particular embodiments only, and is not intended to be limiting.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

It must be noted that as used herein and in the appended claims, the singular forms "a", "and", and "the" include plural referents unless the context clearly dictates otherwise. Thus, for example, reference to "a polynucleotide" includes a plurality of such polynucleotides and reference to "the breast cancer cell" includes reference to one or more cells and equivalents thereof known to those skilled in the art, and so forth.

The publications and applications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Definitions

The terms "polynucleotide" and "nucleic acid", used interchangeably herein, refer to polymeric forms of nucleotides of any length, either ribonucleotides or deoxynucleotides. Thus, these terms include, but are not limited to, single-, double-, or multi-stranded DNA or RNA, genonic DNA, cDNA, DNA-RNA hybrids, or a polymer comprising purine and pyrimidine bases or other natural, chemically or biochemically modified, non-natural, or derivatized nucleotide bases. These terms further include, but are not limited to, mRNA or cDNA that comprise intronic sequences (see, *e.g.*, Niwa et al. (1999) Cell 99(7):691-702). The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidites and thus can be an oligodeoxynucleoside phosphoramidate or a mixed phosphoramidate-phosphodiester oligomer. Peyrottes et al. (1996) Nucl. Acids Res. 24:1841-1848; Chaturvedi et al. (1996) Nucl. Acids Res. 24:2318-2323. A polynucleotide may comprise modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support. The term "polynucleotide" also encompasses peptidic nucleic acids (Pooga et al Curr Cancer Drug Targets. (2001) 1:231-9).

A "gene product" is a biopolymeric product that is expressed or produced by a gene. A gene product may be, for example, an unspliced RNA, an mRNA, a splice variant mRNA, a polypeptide, a post-translationally modified polypeptide, a splice variant polypeptide etc. Also encompassed by this term is biopolymeric products that are made using an RNA gene product as a template (i.e. cDNA of the RNA). A gene product may be made enzymatically, recombinantly, chemically, or within a cell to which the gene is native. In many embodiments, if the gene product is proteinaceous, it exhibits a biological activity. In many embodiments, if the gene product is a nucleic acid, it can be translated into a proteinaceous gene product that exhibits a biological activity.

A composition (e.g. a polynucleotide, polypeptide, antibody, or host cell) that is "isolated" or "in substantially isolated form" refers to a composition that is in an environment different from that in which the composition naturally occurs. For example, a polynucleotide that is in substantially isolated form is outside of the host cell in which the polynucleotide naturally occurs, and could be a purified fragment of DNA, could be part of a heterologous vector, or could be contained within a host cell that is not a host cell from which the polynucleotide naturally occurs. The term "isolated" does not refer to a genomic or cDNA library, whole cell total protein or mRNA preparation, genomic DNA preparation, or an isolated human chromosome. A composition which is in substantially isolated form is usually substantially purified.

As used herein, the term "substantially purified" refers to a compound (*e.g.*, a polynucleotide, a polypeptide or an antibody, etc.,) that is removed from its natural environment and is usually at least 60% free, preferably 75% free, and most preferably 90% free from other components with which it is naturally associated. Thus, for example, a composition containing A is "substantially free of" B when at least 85% by weight of the total A+B in the composition is A. Preferably, A comprises at least about 90% by weight of the total of A+B in the composition, more preferably at least about 95% or even 99% by weight. In the case of polynucleotides, "A" and "B" may be two different genes positioned on different chromosomes or adjacently on the same chromosome, or two isolated cDNA species, for example.

The terms "polypeptide" and "protein", interchangeably used herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

"Heterologous" refers to materials that are derived from different sources (*e.g.*, from different genes, different species, etc.).

As used herein, the terms "a gene that is differentially expressed in a breast cancer cell," and "a polynucleotide that is differentially expressed in a breast cancer cell" are used interchangeably herein, and generally refer to a polynucleotide that represents or corresponds to a gene that is differentially expressed in a cancerous breast cell when compared with a cell of the same cell type that is not cancerous, e.g., mRNA is found at levels at least about 25%, at least about 50% to about 75%, at least about 90%, at least about 1.5-fold, at least about 2-fold, at least about 5-fold, at least about 10-fold, or at least about 50-fold or more, different (*e.g.*, higher or lower). The comparison can be made in tissue, for example, if one is using in situ hybridization or another assay method that allows some degree of discrimination among cell types in the tissue. The comparison may also or alternatively be made between cells removed from their tissue source.

"Differentially expressed polynucleotide" as used herein refers to a nucleic acid molecule (RNA or DNA) comprising a sequence that represents a differentially expressed gene, *e.g.*, the differentially expressed polynucleotide comprises a sequence (*e.g.*, an open reading frame encoding a gene product; a non-coding sequence) that uniquely identifies a differentially expressed gene so that detection of the differentially expressed polynucleotide in a sample is correlated with the presence of a differentially expressed gene in a sample. "Differentially expressed polynucleotides" is also meant to encompass fragments of the disclosed polynucleotides, *e.g.*, fragments retaining biological activity, as well as nucleic acids homologous, substantially similar, or substantially identical (*e.g.*, having about 90% sequence identity) to the disclosed polynucleotides.

By "cyclin G associated kinase", or "GAK" is meant any polypeptide composition that exhibits cyclin G associated kinase activity. Examples of cyclin G associated kinase include the polypeptide defined by NCBI accession number XM_003450, NM_005255, NP_005246 and NM_031030. Assays for determining whether a polypeptide has cyclin G associated kinase activity are described in Ausubel et al., eds., 1998, Current Protocols in Molecular Biology, John Wiley & Sons, NY. Variants of the human cyclin G associated kinase that retain biological activity may be produced by, *inter alia,* substituting amino acids that are in equivalent positions between two cyclin G associated kinases, such as the cyclin G associated kinases from rat and humans.

With regard to cyclin G associated kinases, further references of interest include: Kanaoka et al, FEBS Lett. 1997 Jan 27;402(1):73-80; Kimura et al, Genomics. 1997 Sep 1;44(2):179-87; Greener et al, J Biol Chem. 2000 Jan 14;275(2):1365-70; and Korolchuk et al, Traffic. 2002 Jun;3(6):428-39.

"DKFZP566I133" and "DKFZ" are used interchangeably herein to refer to a polypeptide composition that exhibits DKFZP566I133 activity. Assays for determining whether a polypeptide has DKFZP566I133 activity (i.e. for determining whether DKFZP566I133 may have intracytoplasmatic vacuole promoting activity) are described in Dusetti et al, (Biochem Biophys Res Commun. 2002 Jan 18;290(2):641-9). Variants of the DKFZP566I133 that retain biological activity may be produced by, *inter alia,* substituting amino acids that are in equivalent positions between two DKFZP566I133, such as the DKFZp566I133 from rat and humans. DKFZ is also known as VMP1, or vacuole membrane protein 1.

Alternatively, "DKFZP566I133", or "DKFZ" refers to an amino acid sequence defined by NCBI accession number NP_112200, AAH09758, NM_138839, and NM_030938, polynucleotides encoding the amino acid sequences set forth in these accession numbers (SEQ ID N0:512 and SEQ ID NO:513, respectively).

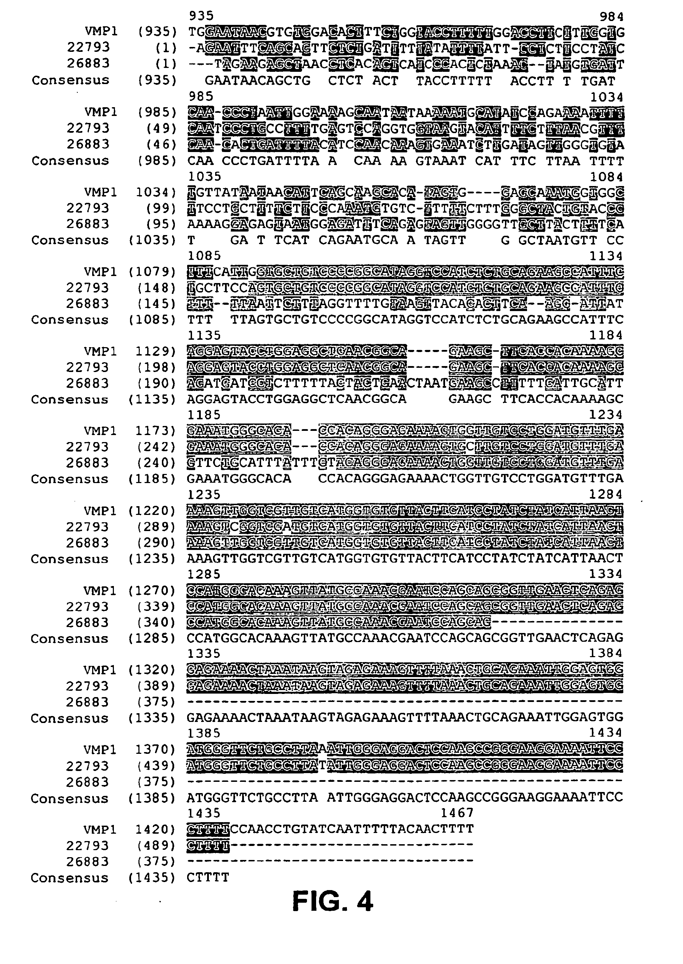

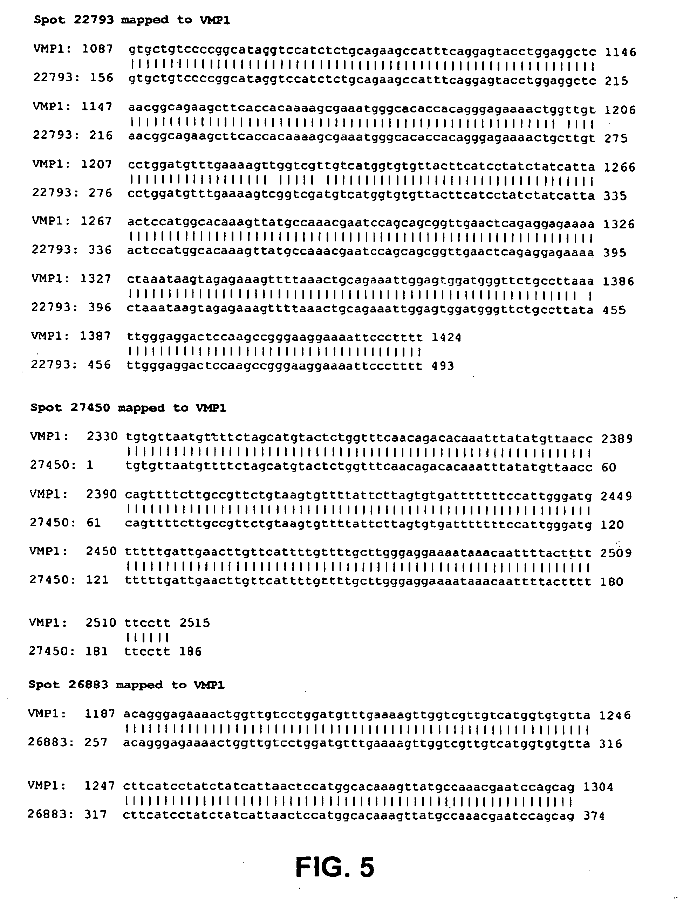

In addition, "DKFZP566I133", or "DKFZ" refers to the polynucleotide sequences represented by Spot ID NOS 22793, 26883 and 27450 (SEQ ID NOS: 274-275 and SEQ ID NOS: 276-277 and SEQ ID NOS:459-460, respectively). Figure 4 shows an alignment between Spot ID NOS: 22793, 26883 and VMP1 (NM_030938) (i.e. DKFZ), identifying a VMP1 or DKFZ gene product as corresponding to these spot IDs. Figure 5 depicts fragments of Spot ID NOS 22793, 26883, 27450 which align with VMP1 (SEQ ID NOS 514, 515, and 516 respectively). These fragments, or their encoded products, may also be used as a DKFZ identifying sequence.

"Corresponds to" or "represents" when used in the context of, for example, a polynucleotide or sequence that "corresponds to" or "represents" a gene means that at least a portion of a sequence of the polynucleotide is present in the gene or in the nucleic acid gene product (*e.g.*, mRNA or cDNA). A subject nucleic acid may also be "identified" by a polynucleotide if the polynucleotide corresponds to or represents the gene. Several genes identified by the polynucleotides of the sequence listing may be found in table 1. Genes identified by a polynucleotide may have all or a portion of the identifying sequence wholly present within an exon of a genomic sequence of the gene, or different portions of the sequence of the polynucleotide may be present in different exons (*e.g.*, such that the contiguous polynucleotide sequence is present in an mRNA, either pre- or post-splicing, that is an expression product of the gene). In some embodiments, the polynucleotide may represent or correspond to a gene that is modified in a cancerous cell relative to a normal cell. The gene in the cancerous cell may contain a deletion, insertion, substitution, or translocation relative to the polynucleotide and may have altered regulatory sequences, or may encode a splice variant gene product, for example. The gene in the cancerous cell may be modified by insertion of an endogenous retrovirus, a transposable element, or other naturally occurring or non-naturally occurring nucleic acid. In most cases, a polynucleotide corresponds to or represents a gene if the sequence of the polynucleotide is most identical to the sequence of a gene or its product (e.g. mRNA or cDNA) as compared to other genes or their products. In most embodiments, the most identical gene is determined using a sequence comparison of a polynucleotide to a database of polynucleotides (e.g. GenBank) using the BLAST program at default settings For example, if the most similar gene in the human genome to an exemplary polynucleotide is the protein kinase C gene, the exemplary polynucleotide corresponds to protein kinase C. In most cases, the sequence of a fragment of an exemplary polynucleotide is at least 95%, 96%, 97%, 98%, 99% or up to 100% identical to a sequence of at least 15, 20, 25, 30, 35, 40, 45, or 50 contiguous nucleotides of a corresponding gene or its product (mRNA or cDNA), when nucleotides that are "N" represent G, A, T or C.

An "identifying sequence" is a minimal fragment of a sequence of contiguous nucleotides that uniquely identifies or defines a polynucleotide sequence or its complement. In many embodiments, a fragment of a polynucleotide uniquely identifies or defines a polynucleotide sequence or its complement. In some embodiments, the entire contiguous sequence of a gene, cDNA, EST, or other provided sequence is an identifying sequence.

"Diagnosis" as used herein generally includes determination of a subject's susceptibility to a disease or disorder, determination as to whether a subject is presently affected by a disease or disorder, prognosis of a subject affected by a disease or disorder (*e.g.*, identification of pre-metastatic or metastatic cancerous states, stages of cancer, or responsiveness of cancer to therapy), and use of therametrics (*e.g.*, monitoring a subject's condition to provide information as to the effect or efficacy of therapy).

As used herein, the term "a polypeptide associated with breast cancer" refers to a polypeptide encoded by a polynucleotide that is differentially expressed in a breast cancer cell. Several examples of polypeptides associated with breast cancer are shown in Table 1.

The term "biological sample" encompasses a variety of sample types obtained from an organism and can be used in a diagnostic or monitoring assay. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

The terms "individual," "subject," "host," and "patient," used interchangeably herein and refer to any mammalian subject for whom diagnosis, treatment, or therapy is desired, particularly humans. Other subjects may include cattle, dogs, cats, guinea pigs, rabbits, rats, mice, horses, and the like.

A "host cell", as used herein, refers to a microorganism or a eukaryotic cell or cell line cultured as a unicellular entity which can be, or has been, used as a recipient for a recombinant vector or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation.

The terms "cancer", "neoplasm", "tumor", and "carcinoma", are used interchangeably herein to refer to cells which exhibit relatively autonomous growth, so that they exhibit an aberrant growth phenotype characterized by a significant loss of control of cell proliferation. In general, cells of interest for detection or treatment in the present application include precancerous (*e.g.*, benign), malignant, pre-metastatic, metastatic, and non-metastatic cells. Detection of cancerous cells is of particular interest.

The term "normal" as used in the context of "normal cell," is meant to refer to a cell of an untransformed phenotype or exhibiting a morphology of a non-transformed cell of the tissue type being examined.

"Cancerous phenotype" generally refers to any of a variety of biological phenomena that are characteristic of a cancerous cell, which phenomena can vary with the type of cancer. The cancerous phenotype is generally identified by abnormalities in, for example, cell growth or proliferation (*e.g.*, uncontrolled growth or proliferation), regulation of the cell cycle, cell mobility, cell-cell interaction, or metastasis, etc.

"Therapeutic target" generally refers to a gene or gene product that, upon modulation of its activity (*e.g.*, by modulation of expression, biological activity, and the like), can provide for modulation of the cancerous phenotype.

As used throughout, "modulation" is meant to refer to an increase or a decrease in the indicated phenomenon (*e.g.*, modulation of a biological activity refers to an increase in a biological activity or a decrease in a biological activity).

### POLYNUCLEOTIDE COMPOSITIONS

Described herein are isolated polynucleotides that represent genes that are differentially expressed in breast cancer cells. The polynucleotides, as well as polypeptides encoded thereby, find use in a variety of therapeutic and diagnostic methods. Also disclosed are polynucleotides having a sequence set forth in any one of the polynucleotide sequences provided herein; polynucleotides obtained from the biological materials described herein or other biological sources (particularly human sources) by hybridization under stringent conditions (particularly conditions of high stringency); genes corresponding to the provided polynucleotides; cDNAs corresponding to the provided polynucleotides; variants of the provided polynucleotides and their corresponding genes, particularly those variants that retain a biological activity of the encoded gene product (*e.g.*, a biological activity ascribed to a gene product corresponding to the provided polynucleotides as a result of the assignment of the gene product to a protein family(ies) and/or identification of a functional domain present in the gene product). Other nucleic acid compositions will be readily apparent to one of ordinary skill in the art. "Polynucleotide" and "nucleic acid" as used herein with reference to nucleic acids of the composition is not intended to be limiting as to the length or structure of the nucleic acid unless specifically indicated.

Also disclosed are polynucleotides that represent genes that are expressed in human tissue, specifically human breast tissue, particularly polynucleotides that are differentially expressed in cancerous breast cells. Nucleic acid compositions described herein of particular interest are at least about 15 bp in length, at least about 30 bp in length, at least about 50 bp in length, at least about 100 bp, at least about 200 bp in length, at least about 300 bp in length, at least about 500 bp in length, at least about 800 bp in length, at least about 1 kb in length, at least about 2.0 kb in length, at least about 3.0 kb in length, at least about 5 kb in length, at least about 10 kb in length, at least about 50kb in length and are usually less than about 200 kb in length. These polynucleotides (or polynucleotide fragments) have uses that include, but are not limited to, diagnostic probes and primers as starting materials for probes and primers, as discussed herein.

The subject polynucleotides usually comprise a sequence set forth in any one of the polynucleotide sequences provided herein, for example, in the sequence listing, in a table (e.g. identified by an NCBI accession number), a cDNA deposited at the A.T.C.C., or a fragment or variant thereof. A "fragment" or "portion" of a polynucleotide is a contiguous sequence of residues at least about 10 nt to about 12 nt, 15 nt, 16 nt, 18 nt or 20 nt in length, usually at least about 22 nt, 24 nt, 25 nt, 30 nt, 40 nt, 50 nt, 60nt, 70 nt, 80 nt, 90 nt, 100 nt to at least about 150 nt, 200 nt, 250 nt, 300 nt, 350 nt, 400 nt, 500 nt, 800 nt or up to about 1000 nt, 1500 or 2000 nt in length. In some embodiments, a fragment of a polynucleotide is the coding sequence of a polynucleotide. A fragment of a polynucleotide may start at position 1 (i.e. the first nucleotide) of a nucleotide sequence provided herein, or may start at about position 10, 20, 30, 50, 75, 100, 150, 200, 250, 300, 350, 400, 450, 500, 600, 700, 800, 900, 1000, 1500 or 2000, or an ATG translational initiation codon of a nucleotide sequence provided herein. In this context "about" includes the particularly recited value or a value larger or smaller by several (5, 4, 3, 2, or 1) nucleotides. The described polynucleotides and fragments thereof find use as hybridization probes, PCR primers, BLAST probes, or as an identifying sequence, for example.

The subject nucleic acids may be variants or degenerate variants of a sequence provided herein. In general, a variants of a polynucleotide provided herein have a fragment of sequence identity that is greater than at least about 65%, greater than at least about 70%, greater than at least about 75%, greater than at least about 80%, greater than at least about 85%, or greater than at least about 90%, 95%, 96%, 97%, 98%, 99% or more (i.e. 100%) as compared to an identically sized fragment of a provided sequence, as determined by the Smith-Waterman homology search algorithm as implemented in MPSRCH program (Oxford Molecular). For the purposes of this invention, a preferred method of calculating percent identity is the Smith-Waterman algorithm. Global DNA sequence identity should be greater than 65% as determined by the Smith-Waterman homology search algorithm as implemented in MPSRCH program (Oxford Molecular) using an gap search with the following search parameters: gap open penalty, 12; and gap extension penalty, 1.

The subject nucleic acid compositions include full-length cDNAs or mRNAs that encompass an identifying sequence of contiguous nucleotides from any one of the polynucleotide sequences provided herein.

As discussed above, the polynucleotides useful in the methods described herein also include polynucleotide variants having sequence similarity or sequence identity. Nucleic acids having sequence similarity are detected by hybridization under low stringency conditions, for example, at 50°C and 10XSSC (0.9 M saline/0.09 M sodium citrate) and remain bound when subjected to washing at 55°C in 1XSSC. Sequence identity can be determined by hybridization under high stringency conditions, for example, at 50°C or higher and 0.1XSSC (9 mM saline/0.9 mM sodium citrate). Hybridization methods and conditions are well known in the art, see, *e.g.*, USPN 5,707,829. Nucleic acids that are substantially identical to the provided polynucleotide sequences, *e.g.* allelic variants, genetically altered versions of the gene, *etc.,* bind to the provided polynucleotide sequences under stringent hybridization conditions. By using probes, particularly labeled probes of DNA sequences, one can isolate homologous or related genes. The source of homologous genes can be any species, *e.g.* primate species, particularly human; rodents, such as rats and mice; canines, felines, bovines, ovines, equines, yeast, nematodes, etc.

In one embodiment, hybridization is performed using a fragment of at least 15 contiguous nucleotides (nt) of at least one of the polynucleotide sequences provided herein. That is, when at least 15 contiguous nt of one of the disclosed polynucleotide sequences is used as a probe, the probe will preferentially hybridize with a nucleic acid comprising the complementary sequence, allowing the identification and retrieval of the nucleic acids that uniquely hybridize to the selected probe. Probes from more than one polynucleotide sequence provided herein can hybridize with the same nucleic acid if the cDNA from which they were derived corresponds to one mRNA.

Polynucleotides described herein include those having a sequence of naturally occurring variants of the nucleotide sequences (*e.g.*, degenerate variants (*e.g.*, sequences that encode the same polypeptides but, due to the degenerate nature of the genetic code, different in nucleotide sequence), allelic variants, *etc*.). Variants of the polynucleotides are identified by hybridization of putative variants with nucleotide sequences disclosed herein, preferably by hybridization under stringent conditions. For example, by using appropriate wash conditions, variants of the polynucleotides described herein can be identified where the allelic variant exhibits at most about 25-30% base pair (bp) mismatches relative to the selected polynucleotide probe. In general, allelic variants contain 15-25% bp mismatches, and can contain as little as even 5-15%, or 2-5%, or 1-2% bp mismatches, as well as a single bp mismatch.

Also disclosed are homologs corresponding to any one of the polynucleotide sequences provided herein, where the source of homologous genes can be any mammalian species, *e.g.*, primate species, particularly human; rodents, such as rats; canines, felines, bovines, ovines, equines, yeast, nematodes, etc. Between mammalian species, *e.g.*, human and mouse, homologs generally have substantial sequence similarity, *e.g.*, at least 75% sequence identity, usually at least 80%%, at least 85, at least 90%, at least 95%, at least 96%, at least 97%, at least 98%, at least 99% or even 100% identity between nucleotide sequences. Sequence similarity is calculated based on a reference sequence, which may be a subset of a larger sequence, such as a conserved motif, coding region, flanking region, *etc.* A reference sequence will usually be at least about a fragment of a polynucleotide sequence and may extend to the complete sequence that is being compared. Algorithms for sequence analysis are known in the art, such as gapped BLAST, described in Altschul, et al. Nucleic Acids Res. (1997) 25:3389-3402, or TeraBLAST available from TimeLogic Corp. (Crystal Bay, Nevada).

Moreover, representative examples of polynucleotide fragments (useful, for example, as probes), include, for example, fragments comprising, or alternatively consisting of, a sequence from about nucleotide number 1-50, 51-100, 101-150, 151-200, 201-250, 251-300, 301-350, 351-400, 401-450, 451-500, 501-550, 551-600, 651-700,701-750, 751-800, 800-850, 851-900, 901-950,951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900, 1901-1950, 1951 -2000,2001 -2050,2051 -2100, 2101 -2150,2151 -2200,2201 -2250,2251 - 2300, 2301-2350, 2351-2400, 2401-2450, 2451-2500, 2501-2550, 2551-2600, 2601-2650, 2651-2700, 2701-2750, 2751-2800, 2801-2850, 2851-2900, 2901-2950, 2951-3000, 3001-3050, 3051-3100, 3101-3150, 3151-3200, 3201-3250, 3251-3300, 3301-3350, 3351-3400, 3401-3450, 3451-3500, 3501-3550, 3551-3600, 3601-3650, 3651-3700, 3701-3750, 3751-3800, 3801-3850, 3851-3900, 3901-3950, 3951-4000, 4001-4050, 4051-4100, 4101-4150, 4151-4200, 4201-4250, 4251-4300, 4301-4350, 4351-4400, 4401-4450, 4451-4500, 4501-4550, 4551-4600, 4601-4650, 4651-4700, 4701-4750, 4751-4800, 4801-4850, 4851-4900, 4901-4950, 4951-5000, 5001-5050, 5051-5100, 5101-5150, 5151-5200, 5201-5250, 5251-5300, 5301-5350, 5351-5400, 5401- 5450, 5451-5500, 5501-5550, 5551-5600, 5601-5650, 5651-5700, 5701-5750, 5751-5800, 5801-5850, 5851-5900, 5901-5950, 5951-6000, 6001-6050, 6051-6100, 6101-6150, and 6151 of a subject nucleic acid, or the complementary strand thereto. In this context "about" includes the particularly recited range or a range larger or smaller by several (5, 4, 3, 2, or 1) nucleotides, at either terminus or at both termini. In some embodiments, these fragments encode a polypeptide which has a functional activity (e.g., biological activity) whereas in other embodiments, these fragments are probes, or starting materials for probes. Polynucleotides which hybridize to one or more of these nucleic acid molecules under stringent hybridization conditions or alternatively, under lower stringency conditions, are also disclosed, as are polypeptides encoded by these polynucleotides or fragments.

The subject nucleic acids can be cDNAs or genomic DNAs, as well as fragments thereof, particularly fragments that encode a biologically active gene product and/or are useful in the methods disclosed herein (e.g., in diagnosis, as a unique identifier of a differentially expressed gene of interest, etc.). The term "cDNA" as used herein is intended to include all nucleic acids that share the arrangement of sequence elements found in native mature mRNA species, where sequence elements are exons and 3' and 5' non-coding regions. Normally mRNA species have contiguous exons, with the intervening introns, when present, being removed by nuclear RNA splicing, to create a continuous open reading frame encoding a polypeptide, mRNA species can also exist with both exons and introns, where the introns may be removed by alternative splicing. Furthermore it should be noted that different species of mRNAs encoded by the same genomic sequence can exist at varying levels in a cell, and detection of these various levels of mRNA species can be indicative of differential expression of the encoded gene product in the cell.

A genomic sequence of interest comprises the nucleic acid present between the initiation codon and the stop codon, as defined in the listed sequences, including all of the introns that are normally present in a native chromosome. It can further include the 3' and 5' untranslated regions found in the mature mRNA. It can further include specific transcriptional and translational regulatory sequences, such as promoters, enhancers, etc., including about 1 kb, but possibly more, of flanking genomic DNA at either the 5' and 3' end of the transcribed region. The genomic DNA can be isolated as a fragment of 100 kbp or smaller; and substantially free of flanking chromosomal sequence. The genomic DNA flanking the coding region, either 3' and 5', or internal regulatory sequences as sometimes found in introns, contains sequences required for proper tissue, stage-specific, or disease-state specific expression.

The nucleic acid compositions described herein can encode all or a part of the naturally-occurring polypeptides. Double or single stranded fragments can be obtained from the DNA sequence by chemically synthesizing oligonucleotides in accordance with conventional methods, by restriction enzyme digestion, by PCR amplification, etc.

Probes specific to the polynucleotides described herein can be generated using the polynucleotide sequences disclosed herein. The probes are usually a fragment of a polynucleotide sequences provided herein. The probes can be synthesized chemically or can be generated from longer polynucleotides using restriction enzymes. The probes can be labeled, for example, with a radioactive, biotinylated, or fluorescent tag. Preferably, probes are designed based upon an identifying sequence of any one of the polynucleotide sequences provided herein. More preferably, probes are designed based on a contiguous sequence of one of the subject polynucleotides that remain unmasked following application of a masking program for masking low complexity (*e.g.*, XBLAST, RepeatMasker, etc.) to the sequence., *i.e.,* one would select an unmasked region, as indicated by the polynucleotides outside the poly-n stretches of the masked sequence produced by the masking program.

The polynucleotides are isolated and obtained in substantial purity, generally as other than an intact chromosome. Usually, the polynucleotides, either as DNA or RNA, will be obtained substantially free of other naturally-occurring nucleic acid sequences that they are usually associated with , generally being at least about 50%, usually at least about 90% pure and are typically "recombinant", *e.g.*, flanked by one or more nucleotides with which it is not normally associated on a naturally occurring chromosome.

The polynucleotides described herein can be provided as a linear molecule or within a circular molecule, and can be provided within autonomously replicating molecules (vectors) or within molecules without replication sequences. Expression of the polynucleotides can be regulated by their own or by other regulatory sequences known in the art. The polynucleotides can be introduced into suitable host cells using a variety of techniques available in the art, such as transferrin polycation-mediated DNA transfer, transfection with naked or encapsulated nucleic acids, liposome-mediated DNA transfer, intracellular transportation of DNA-coated latex beads, protoplast fusion, viral infection, electroporation, gene gun, calcium phosphate-mediated transfection, and the like.

The nucleic acid compositions described herein can be used to, for example, produce polypeptides, as probes for the detection of mRNA in biological samples (*e.g.*, extracts of human cells) or cDNA produced from such samples, to generate additional copies of the polynucleotides, to generate ribozymes or antisense oligonucleotides, and as single stranded DNA probes or as triple-strand forming oligonucleotides. The probes described herein can be used to, for example, determine the presence or absence of any one of the polynucleotide provided herein or variants thereof in a sample. These and other uses are described in more detail below.

### POLYPEPTIDES AND VARIANTS THEREOF

Also disclosed are polypeptides encoded by polynucleotides that represent genes that are differentially expressed in breast cancer cells. Such polypeptides are referred to herein as "polypeptides associated with breast cancer." The polypeptides can be used to generate antibodies specific for a polypeptide associated with breast cancer, which antibodies are in turn useful in diagnostic methods, prognostics methods, therametric methods, and the like as discussed in more detail herein. Polypeptides are also useful as targets for therapeutic intervention, as discussed in more detail herein.

The polypeptides described herein include those encoded by the disclosed polynucleotides and the genes to which these polynucleotides correspond, as well as nucleic acids that, by virtue of the degeneracy of the genetic code, are not identical in sequence to the disclosed polynucleotides. Further polypeptides described herein include polypeptides that are encoded by polynucleotides that hybridize to polynucleotide of the sequence listing. Thus, also disclosed is a polypeptide encoded by a polynucleotide having the sequence of any one of the polynucleotide sequences provided herein, or a variant thereof.

In general, the term "polypeptide" as used herein refers to both the full length polypeptide encoded by the recited polynucleotide, the polypeptide encoded by the gene represented by the recited polynucleotide, as well as portions or fragments thereof. "Polypeptides" also includes variants of the naturally occurring proteins, where such variants are homologous or substantially similar to the naturally occurring protein, and can be of an origin of the same or different species as the naturally occurring protein (*e.g.*, human, murine, or some other species that naturally expresses the recited polypeptide, usually a mammalian species). In general, variant polypeptides have a sequence that has at least about 80%, usually at least about 90%, and more usually at least about 98% sequence identity with a differentially expressed polypeptide described herein, as measured by BLAST 2.0 using the parameters described above. The variant polypeptides can be naturally or non-naturally glycosylated, *i.e.*, the polypeptide has a glycosylation pattern that differs from the glycosylation pattern found in the corresponding naturally occurring protein.

Also disclosure are homologs of the disclosed polypeptides (or fragments thereof) where the homologs are isolated from other species, *i.e*. other animal or plant species, where such homologs, usually mammalian species, *e.g.* rodents, such as mice, rats; domestic animals, *e.g.*, horse, cow, dog, cat; and humans. By "homolog" is meant a polypeptide having at least about 35%, usually at least about 40% and more usually at least about 60% amino acid sequence identity to a particular differentially expressed protein as identified above, where sequence identity is determined using the BLAST 2.0 algorithm, with the parameters described *supra.*

In general, the polypeptides are provided in a non-naturally occurring environment, *e.g.* are separated from their naturally occurring environment. In certain embodiments, the subject protein is present in a composition that is enriched for the protein as compared to a cell or extract of a cell that naturally produces the protein. As such, isolated polypeptide is provided, where by "isolated" or "in substantially isolated form" is meant that the protein is present in a composition that is substantially free of other polypeptides, where by substantially free is meant that less than 90%, usually less than 60% and more usually less than 50% of the composition is made up of other polypeptides of a cell that the protein is naturally found.

Also disclosed are variants; variants of polypeptides include mutants, fragments, and fusions. Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/hydrophilicity, and/or steric bulk of the amino acid substituted.

Variants can be designed so as to retain or have enhanced biological activity of a particular region of the protein (*e.g.*, a functional domain and/or, where the polypeptide is a member of a protein family, a region associated with a consensus sequence). For example, muteins can be made which are optimized for increased antigenicity, i.e. amino acid variants of a polypeptide may be made that increase the antigenicity of the polypeptide. Selection of amino acid alterations for production of variants can be based upon the accessibility (interior vs. exterior) of the amino acid (*see, e.g.,* Go et al, Int. J Peptide Protein Res. (1980) 15:211), the thermostability of the variant polypeptide (*see, e.g.,* Querol et al., Prot. Eng. (1996) 9:265), desired glycosylation sites (see, e.g., Olsen and Thomsen, J. Gen. Microbiol. (1991) 137:579), desired disulfide bridges (*see, e.g.,* Clarke et al., Biochemistry (1993) 32:4322; and Wakarchuk et al., Protein Eng. (1994) 7:1379), desired metal binding sites (*see, e.g.,* Toma et al., Biochemistry (1991) 30:97, and Haezerbrouck et al., Protein Eng. (1993) 6:643), and desired substitutions with in proline loops (*see, e.g.,* Masul et al., Appl. Env. Microbiol. (1994) 60:3579). Cysteine-depleted muteins can be produced as disclosed in USPN 4,959,314.Variants also include fragments of the polypeptides disclosed herein, particularly biologically active fragments and/or fragments corresponding to functional domains. Fragments of interest will typically be at least about 10 aa to at least about 15 aa in length, usually at least about 50 aa in length, and can be as long as 300 aa in length or longer, but will usually not exceed about 1000 aa in length, where the fragment will have a stretch of amino acids that is identical to a polypeptide encoded by a polynucleotide having a sequence of any one of the polynucleotide sequences provided herein, or a homolog thereof. The protein variants described herein are encoded by polynucleotides described herein.

The genetic code can be used to select the appropriate codons to construct the corresponding variants.

A fragment of a subject polypeptide is, for example, a polypeptide having an amino acid sequence which is a portion of a subject polypeptide e.g. a polypeptide encoded by a subject polynucleotide that is identified by any one of the sequence of SEQ ID NOS 1 - 499 or its complement. The polypeptide fragments are preferably at least about 9 aa, at least about 15 aa, and more preferably at least about 20 aa, still more preferably at least about 30 aa, and even more preferably, at least about 40 aa, at least about 50 aa, at least about 75 aa, at least about 100 aa, at least about 125 aa or at least about 150 aa in length. A fragment "at least 20 aa in length," for example, is intended to include 20 or more contiguous amino acids from, for example, the polypeptide encoded by a cDNA, in a cDNA clone contained in a deposited library, or a nucleotide sequence shown in SEQ ID NOS:1-499 or the complementary stand thereof. In this context "about" includes the particularly recited value or a value larger or smaller by several (5, 4, 3, 2, or 1) amino acids. These polypeptide fragments have uses that include, but are not limited to, production of antibodies as discussed herein. Of course, larger fragments (e.g., at least 150, 175, 200, 250, 500, 600, 1000, or 2000 amino acids in length) are also encompassed by the invention.

Moreover, representative examples of polypeptides fragments (useful in, for example, as antigens for antibody production), include, for example, fragments comprising, or alternatively consisting of, a sequence from about amino acid number 1-10, 5-10, 10-20, 21-31, 31-40, 41-61, 61-81, 91-120, 121-140, 141-162, 162-200, 201-240, 241-280, 281- 320, 321-360, 360-400, 400-450, 451-500, 500-600, 600-700, 700-800, 800-900 and the like. In this context "about" includes the particularly recited range or a range larger or smaller by several (5, 4, 3, 2, or 1) amino acids, at either terminus or at both termini. In some embodiments, these fragments has a functional activity (e.g., biological activity) whereas in other embodiments, these fragments may be used to make an antibody.

Further polypeptide variants may are described in PCT publications WO/00-55173, WO/01-07611 and WO/02-16429

### VECTORS, HOST CELLS AND PROTEIN PRODUCTION

Also disclosed are vectors containing the polynucleotide described herein, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides of the invention may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria.

Representative examples of appropriate hosts include,but are not limited to, bacterial cells, such as E. coli, Streptomyces and Salmonella typhimurium cells; fungal cells, such as yeast cells (e.g., Saccharomyces cerevisiae or Pichia pastoris (ATCC Accession No. 201178)); insect cells such as Drosophila S2 and Spodoptera Sf9 cells; animal cells such as CHO, COS, 293, and Bowes melanoma cells; and plant cells. 5 Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNHSA, pNH16a, pNH18A, pNH46A, available from Stratagene Cloning Systems, Inc.; and ptrc99a, pKK223-3, pKK233-3, pDR540, pRITS available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Preferred expression vectors for use in yeast systems include, but are not limited to pYES2, pYD1, pTEFI/Zeo, pYES2/GS, pPICZ, pGAPZ, pGAPZalph, pPIC9, pPIC3.5, pHIL-D2, pHIL-S1, pPIC3.5K, pPIC9K, and PAO815 (all available from Invitrogen, Carload, CA). Other suitable vectors will be readily apparent to the skilled artisan.

Nucleic acids of interest may be cloned into a suitable vector by route methods. Suitable vectors include plasmids, cosmids, recombinant viral vectors e.g. retroviral vectors, YACs, BACs and the like, phage vectors.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides described herein may in fact be expressed by a host cell lacking a recombinant vector.

A polypeptide can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast higher plant, insect, and mammalian cells. Depending upon the host employed in a recombinant production procedure, the polypeptides may be glycosylated or may be non-glycosylated. In addition, polypeptides may also include an initial modified methionine residue, in some cases as a result of host mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

Suitable methods and compositions for polypeptide expression may be found in PCT publications WO/00-55173, WO/01-07611 and WO/02-16429.

In addition, polypeptides can be chemically synthesized using techniques known in the art (e.g., see Creighton, 1983, Proteins: Structures and Molecular Principles, W.H. Freeman & Co., N.Y., and Hunkapiller et al., Nature, 310:105-111 (1984)). For example, a polypeptide corresponding to a fragment of a polypeptide can be synthesized by use of a peptide synthesizer. Furthermore, if desired, nonclassical amino acids or chemical amino acid analogs can be introduced as a substitution or addition into the polypeptide sequence. Non-classical amino acids include, but are not limited to, to the D-isomers of the common amino acids, 2,4-diaminobutyric acid, a-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, 5 hydroxyproline, sarcosine, citrulline, homocitrulline, cysteic acid, t-butylglycine, t-butylalanine, phenylglycine, cyclohexylalanine, b-alanine, fluoro-amino acids, designer amino acids such as b-methyl amino acids, Ca-methyl amino acids, Na-methyl amino acids, and amino acid analogs in general. Furthermore, the amino acid can be D (dextrorotary) or L (levorotary).

Non-naturally occurring variants may be produced using art-known mutagenesis techniques, which include, but are not limited to oligonucleotide mediated mutagenesis, alanine scanning, PCR mutagenesis, site directed mutagenesis (see, e.g., Carter et al., Nucl. Acids Res. 73:4331 (1986); and Zoller et al., Nucl. Acids Res. 70:6487 (1982)), cassette mutagenesis (see, e.g., Wells et al., Gene 34:315 (1985)), restriction selection mutagenesis (see, e.g., Wells et al., Philos. Trans. R. Soc. London SerA 377:415 (1986)).

Scanning amino acid analysis can also be employed to identify one or more amino acids along a contiguous sequence. Among the preferred scanning amino acids are relatively small neutral amino acids. Such amino acids include alanine, glycine, serine and cysteine. Alanine is typically a preferred scanning amino acid among this group because it eliminates the side-chain beyond the beta-carbon and is less likely to alter the main-chain conformation of the variant. Alanine is also typically preferred because it is the most common amino acid. If alanine substituting does not yield adequate amounts of variant, an isoteric amino acid can be used.

Any cyseine reside not involved in maintaining the proper conformation of a polypeptide may also be substituted, generally with serine, to improve the oxidative stability of the molecule and prevent aberrant crosslinking. Conversely, cysteine bonds may be added to the polypeptide to improve its stability.

Also disclosed are polypeptides which are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited, to specific chemical cleavage by cyanogen bromide, trypsin, chymotrypsin, papain, V8 protease, NaBH4; acetylation, formylation, oxidation, reduction; metabolic synthesis in the presence of tunicamycin; etc.

Additional post-translational modifications include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The polypeptides may also be modified with a detectable label, such as an enzymatic, fluorescent, isotopic or affinity label to allow for detection and isolation of the protein.

Also described herein are chemically modified derivatives of the disclosed polypeptides which may provide additional advantages such as increased solubility, stability and circulating time of the polypeptide, or decreased immunogenicity (see U.S. Patent No. 4,179,337). The chemical moieties for derivitization may be selected from water soluble polymers such as polyethylene glycol, ethylene glycol/propylene glycol copolymers, carboxymethylcellulose, dextran, polyvinyl alcohol and the like. The 5 polypeptides may be modified at random positions within the molecule, or at predetermined positions within the molecule and may include one, two, three or more attached chemical moieties.

Suitable methods and compositions for production of modified polypeptides may be found in PCT publications WO/00-55173, WO/01-07611 and WO/02-16429.

### ANTIBODIES AN OTHER POLYPEPTIDE OR POLYNUCLEOTIDE BINDING MOLECULES

Also disclosed are antibodies, which may be isolated antibodies, that are specific for a polypeptide encoded by a polynucleotide described herein and/or a polypeptide of a gene that corresponds to a polynucleotide described herein. Antibodies can be provided in a composition comprising the antibody and a buffer and/or a pharmaceutically acceptable excipient. Antibodies specific for a polypeptide associated with breast cancer are useful in a variety of diagnostic and therapeutic methods, as discussed in detail herein.

Gene products, including polypeptides, mRNA (particularly mRNAs having distinct secondary and/or tertiary structures), cDNA, or complete gene, can be prepared and used for raising antibodies for experimental, diagnostic, and therapeutic purposes. Antibodies may be used to identify a gene corresponding to a polynucleotide. The polynucleotide or related cDNA is expressed as described above, and antibodies are prepared. These antibodies are specific to an epitope on the polypeptide encoded by the polynucleotide, and can precipitate or bind to the corresponding native protein in a cell or tissue preparation or in a cell-free extract of an in vitro expression system.

### Antibodies

Further polypeptides described herein relate to antibodies and T-cell antigen receptors (TCR) which immunospecifically bind a subject polypeptide, subject polypeptide fragment, or variant thereof, and/or an epitope thereof (as determined by immunoassays well known in the art for assaying specific antibody-antigen binding). Antibodies include, but are not limited to, polyclonal, monoclonal, multispecific, human, humanized or chimeric antibodies, single chain antibodies, Fab fragments, F(ab') fragments, fragments produced by a Fab expression library anti-idiotypic (anti-Id) antibodies (including, e.g., anti-Id antibodies to antibodies described herein), and epitope-binding fragments of any of the above. The term "antibody," as used herein, refers to immunoglobulin molecules and immunologically active portions of immunoglobulin molecules, i.e., molecules that contain an antigen binding site that immunospecifically binds an antigen. The immunoglobulin molecules can be of any type (e.g., IgG, IgE, IgM, IgD, IgA and IgY), class (e.g., IgG1, IgG2, IgG3, IgG4, IgA1 and IgA2) or subclass of immunoglobulin molecule.

Most preferably the antibodies are human antigen-binding antibody fragments and include, but are not limited to, Fab. Fab' and F(ab')2, Fd, single-chain Fvs (scFv), single-chain antibodies, disulfide-linked Fvs (sdFv) and fragments comprising either a V_{L} or V_{H} domain. Antigen-binding antibody fragments, including single-chain antibodies, may comprise the variable region(s) alone or in combination with the entirety or a portion of the following: hinge region, C_{H}1, C_{H}2, and C_{H}3 domains. Also disclosed are antigen-binding fragments also comprising any combination of variable region(s) with a hinge region, C_{H}1, C_{H}2, and C_{H}3 domains. The antibodies described herein may be from any animal origin including birds and mammals. Preferably, the antibodies are human, murine (e.g., mouse and rat), donkey, ship rabbit, goat, guinea pig, camel, horse, or chicken. As used herein, "human" antibodies include antibodies having the amino acid sequence of a human immunoglobulin and include antibodies isolated from, human immunoglobulin libraries or from animals transgenic for one or more human immunoglobulin and that do not express endogenous immunoglobulins, as described infra and, for example in, U.S. Patent No. 5,939,598 by Kucherlapati et al.

The antibodies may be monospecific, bispecific, trispecific or of greater multispecificity. Multispecific antibodies may be specific for different epitopes of a polypeptide or may be specific for both a polypeptide as well as for a heterologous epitope, such as a heterologous polypeptide or solid support material. See, e.g., PCT publications WO 93/17715; WO 92/08802; WO 91/00360; WO 92/05793; Tutt, et al., J. Immunol. 147:60-69 (1991); U.S. Patent Nos. 4,474,893; 4,714,681; 4,925,648; 5,573,920; 5,601,819; Kostelny et al., J. Immunol. 148:1547-1553 (1992).

Antibodies may be described or specified in terms of the epitope(s) or portion(s) of a polypeptide which they recognize or specifically bind. The epitope(s) or polypeptide portion(s) may be specified as described herein, e.g., by N-terminal and C-terminal positions, or by size in contiguous amino acid residues. Antibodies which specifically bind any epitope or polypeptide described herein may also be excluded. Therefore, described herein are antibodies that specifically bind polypeptides described herein, and allow for the exclusion of the same.

Antibodies may also be described or specified in terms of their cross-reactivity. Antibodies that do not bind any other analog, ortholog, or homolog of a polypeptide described herein are included. Antibodies that bind polypeptides with at least 95%, at least 90%, at least 85%, at least 80%, at least 75%, at least 70%, at least 65%, at least 60%, at least 55%, and at least 50% identity (as calculated using methods known in the art and described herein) to a polypeptide described herein are also disclosed.

In specific embodiments, antibodies described herein cross-react with murine, rat and/or rabbit homologs of human proteins and the corresponding epitopes thereof. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide described herein are also disclosed. In a specific embodiment, the above-described cross-reactivity is with respect to any single specific antigenic or immunogenic polypeptide, or combination(s) of 2, 3, 4, 5, or more of the specific antigenic and/or immunogenic polypeptides disclosed herein. Further disclosed are antibodies which bind polypeptides encoded by polynucleotides which hybridize to a polynucleotide described herein under stringent hybridization conditions (as described herein). Antibodies may also be described or specified in terms of their binding affinity to a polypeptide described herein. Preferred binding affinities include those with a dissociation constant or Kd less than 5 X 10⁻² M, 10⁻² M, 5 X 10⁻³ M, 10⁻³ M, 5 X 10⁻⁴ M, 10⁻⁴ M, 5 X 10⁻⁵ M, 10⁻⁵ M; 5 X 10⁻⁶ M, 10 ⁻⁶M, 5 X 10⁻⁷ M, 10⁻⁷ M, 5 X ID⁻⁸ M, 10⁻⁸ M, 5 X 10⁻⁹ M, 10⁻⁹ M, 5 X 10⁻¹⁰ M, 10⁻¹⁰ M, 5 X 10⁻¹¹ M, 10⁻¹¹ M, 5 X 10⁻¹² M, 10⁻¹² M, 5 X 10⁻³³ M, 10⁻¹³ M, 5 X 10⁻¹⁴ M, 10⁻¹⁴ M, 5 X 10⁻¹⁵ M or 10⁻¹⁵ M.

Also disclosed are antibodies that competitively inhibit binding of an antibody to an epitope described herein as determined by any method known in the art for determining competitive binding, for example, the immunoassays described herein. In preferred embodiments, the antibody competitively inhibits binding to the epitope by at least 95%, at least 90%, at least 85 %, at least 80%, at least 75%, at least 70%, at least 60%, or at least 50%.

Methods for making screening, humanizing, and modifying different types of antibody are well known in the art and may be found in PCT publications WO/00-55173, WO/01-07611 and WO/02-16429.

Antibodies may act as agonists or antagonists of the polypeptides described herein. For example, described herein are antibodies which disrupt the receptor/ligand interactions with the polypeptides described herein either partially or fully. Preferably, antibodies bind an antigenic epitope disclosed herein, or a portion thereof. Described herein are both receptor-specific antibodies and ligand-specific antibodies. Also disclosed are receptor-specific antibodies which do not prevent ligand binding but prevent receptor activation. Receptor activation (i.e., signaling) may be determined by techniques described herein or otherwise known in the art. For example, receptor activation can be determined by detecting the phosphorylation (e.g., tyrosine or serine/threonine) of the receptor or its substrate by immunoprecipitation followed by western blot analysis (for example, as described supra). In specific embodiments, antibodies are provided that inhibit ligand activity or receptor activity by at least 95%, at least 90%, at least 85%, at least 80%, at least 75%, at least 70%, at least 60%, or at least 50% of the activity in absence of the antibody.

Also disclosed are receptor-specific antibodies which both prevent ligand binding and receptor activation as well as antibodies that recognize the receptor-ligand complex, and, preferably, do not specifically recognize the unbound receptor or the unbound ligand. Likewise, described herein are neutralizing antibodies which bind the ligand and prevent binding of the ligand to the receptor, as well as antibodies which bind the ligand, thereby preventing receptor activation, but do not prevent the ligand from binding the receptor. Further disclosed are antibodies which activate the receptor. These antibodies may act as receptor agonists, i.e., potentiate or activate either all or a subset of the biological activities of the ligand-mediated receptor activation, for example, by inducing dimerization of the receptor. The antibodies may be specified as agonists, antagonists or inverse agonists for biological activities comprising the specific biological activities of the peptides disclosed herein. The above antibody agonists can be made using methods known in the art. See, e.g., PCT publication WO 96/40281; U.S. Patent No. 5,811,097; Deng et al., Blood 92(6): 1981-1988 (1998); Chen et al., Cancer Res. 58(16):3668-4998 (1998); Harrop et al., J. Immunol. 161(4): 1786-1794 (1998); Zhu et al., Cancer Res. 58(15):3209-3214 (1998): Yoon et al., J. Immunol. 160(7):3 170-3179 (1998); Prat et al., J. Cell. Sci. 11 1(Pt2):237-247 (1998); Pitard et al., J. Immunol. Methods 205(2): 177-190 (1997); Liautard et al., Cytokine 9(4):233-241 (1997); Carlson et al., J. Biol. Chem 272(I1): 11295-1 1301 (1997); Taryman et al., Neuron 14(4):755-762 (1995); Muller et al., Structure 6(9): 1153-1167 (1998); Bartunek et al., Cytokine 8(1):14-20 (1996).

Antibodies described herein may be used, for example, but not limited to, to purify, detect, and target the polypeptides described herein, including both in vitro and in vivo diagnostic and therapeutic methods. For example, the antibodies have use in immunoassays for qualitatively and quantitatively measuring levels of the polypeptides described herein in biological samples. See, e.g., Harlow et al., Antibodies: A Laboratory Manual, (Cold Spring Harbor Laboratory Press, 2nd ed.1988).

Further methods and compositions involving antibodies may be found in PCT publications WO/00-55173, WO/01-07611 and WO/02-16429.

### Polynucleolides Encoding Antibodies

Also disclosed are polynucleotides comprising a nucleotide sequence encoding an antibody described herein and fragments thereof. Also disclosed are polynucleotides that hybridize under stringent or alternatively, under lower stringency hybridization conditions, e.g., as defined supra, to polynucleotides that encode an antibody, preferably, that specifically binds to a polypeptide described herein, preferably, an antibody that binds to a subject polypeptide.

The polynucleotides may be obtained, and the nucleotide sequence of the polynucleotides determined, by any method known in the art. For example, if the nucleotide sequence of the antibody is known, a polynucleotide encoding the antibody may be assembled from chemically synthesized oligonucleotides (e.g., as described in Kutmeier et al., BioTechniques 17:242 (1994)). which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (e.g., an antibody cDNA library, or a cDNA library generated from, or nucleic acid, preferably poly A+ RNA, isolated from, any tissue or cells expressing the antibody, such as hybridoma cells selected to express an antibody described herein) by PCR amplification using synthetic primers hybridizable to the 3' and 5' ends of the sequence or by cloning using an oligonucleotide probe specific for the particular gene sequence to identify, e.g., a cDNA clone from a cDNA library that encodes the antibody. Amplified nucleic acids generated by' PCR may then be cloned into replicable cloning vectors using any method well known in the art.

Once the nucleotide sequence and corresponding amino acid sequence of the antibody is determined, the nucleotide sequence of the antibody may be manipulated using methods well known in the art for the manipulation of nucleotide sequences, e.g., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY), to generate antibodies having a different amino acid sequence, for example to create amino acid substitutions, deletions, and/or insertions.

### Methods of Producing Antibodies

The antibodies described herein can be produced by any method known in the art for the synthesis of antibodies, in particular, by chemical synthesis or preferably, by recombinant expression techniques. Recombinant expression of an antibody or fragment, derivative or analog thereof, (e.g., a heavy or light chain of an antibody or a single chain antibody), requires construction of an expression vector containing a polynucleotide that encodes the antibody. Once a polynucleotide encoding an antibody molecule or a heavy or light chain of an antibody, or portion thereof (preferably containing the heavy or light chain variable domain), has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. Described herein are replicable vectors comprising a nucleotide sequence encoding an antibody molecule described herein, or a heavy or light chain thereof, or a heavy or light chain variable domain, operably linked to a promoter. Such vectors may include the nucleotide sequence encoding the constant region of the antibody molecule (see, e.g., PCT Publication WO 86/05807; PCT Publication WO 89/01036; and U.S. Patent No. 5,122,464) and the variable domain of the antibody may be cloned into such a vector for expression of the entire heavy or light chain.

The expression vector is transferred to a host cell by conventional techniques and the transfected cells are then cultured by conventional techniques to produce an antibody described herein. Also disclosed are host cells containing a polynucleotide encoding an antibody described herein, or a heavy or light chain thereof, or a single chain antibody described herein, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules described herein. Such host-expression systems represent vehicles by which the coding sequences of interest may be produced and subsequently purified, but also represent cells which may, when transformed or transfected with the appropriate nucleotide coding sequences, express an antibody molecule of the invention in situ. These include but are not limited to microorganisms such as bacteria (e.g., E. coli, B. subtilis) transformed with recombinant bacteriophage DNA, plasmid DNA or cosmid DNA expression vectors containing antibody coding sequences; yeast (e.g., Saccharomyces, Pichia) transformed with recombinant yeast expression vectors containing antibody coding sequences; insect cell systems infected with recombinant virus expression vectors (e.g., baculovirus) containing antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harboring recombinant expression constructs containing promoters derived from the genome of mammalian cells (e.g., metallothionein promoter) or from mammalian viruses (e.g., the adenovirus late promoter; the vaccinia virus 7.5K promoter). Preferably, bacterial cells such as Escherichia coli, and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., Gene 45:101 (1986); Cockett etal., Bio/Technology 8:2 (1990)).

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited, to the E. coli expression vector pUR278 (Ruther et al., EMBO J. 2:1791 (1983)), in which the antibody coding sequence may be ligated individually into the vector in frame with the lac Z coding region so that a fusion protein is produced; pIN vectors (Inouye & Inouye, Nucleic Acids Res. 13:3101-3109 (1985); Van Heeke & Schuster, J. Biol. Chem. 24:5503-5509 (1989)); and the like. pGEX vectors may also be used to express foreign polypeptides as fusion proteins with glutathione S-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera fnigiperda* ceils. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, e.g., the late promoter and tripartite leader sequence. This chimeric gene may then be inserted in the adenovirus genome by in vitro or in vivo recombination. Insertion in a non- essential region of the viral genome (e.g., region E1 or E3) will result in a recombinant virus that is viable and capable of expressing the antibody molecule in infected hosts, (e.g., see Logan & Shenk, Proc. Natl. Acad. Sci. USA 81:355-359 (1984)). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., Methods in Enzymol. 153:51-544 (1987)).

Antibodies production is well known in the art. Exemplary methods and compositions for making antibodies may be found in PCT publications WO/00-55173, WO/01-07611 and WO/02-16429.

### Immunophenotyping

The antibodies described herein may be utilized for immunophenotyping of cell lines and biological samples. The translation product of the gene described herein may be useful as a cell specific marker, or more specifically as a cellular marker that is differentially expressed at various stages of differentiation and/or maturation of particular cell types. Monoclonal antibodies directed against a specific epitope, or combination of epitopes, will allow for the screening of cellular populations expressing the marker. Various techniques can be utilized using monoclonal antibodies to screen for cellular populations expressing the marker(s), and include magnetic separation using antibody-coated magnetic beads, "panning" with antibody attached to a solid matrix (i.e., plate), and flow cytometry (See, e.g., U.S. Patent 5,985,660; and Morrison et al. Cell, 96:737-49 (1999)).

These techniques allow for the screening of particular populations of cells, such as might be found with hematological malignancies (i.e. minimal residual disease (MRD) in acute leukemic patients) and "non-self cells in transplantations to prevent Graft-versus-Host Disease (GVHD). Alternatively, these techniques allow for the screening of hematopoietic stem and progenitor cells capable of undergoing proliferation and/or differentiation, as might be found in human umbilical cord blood.

### Assays For Antibody Binding

The antibodies described herein may be assayed for immunospecific binding by any method known in the art The immunoassays which can be used include but are not limited to competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e.g., Ausubel et al, eds, 1994, Current Protocols in Molecular Biology, Vol. 1, John Wiley & Sons, Inc., New York,).

Exemplary immunoassays are described briefly below (but are not intended by way of limitation).

Immunoprecipitation protocols generally comprise lysing a population of cells in a lysis buffer such as R1PA buffer (1% NP-40 or Triton X- 100, 1 % sodium deoxycholate, 0.1% SDS, 0.15 M NaCl, 0.01 M sodium phosphate at pH 7.2, 1% Trasylol) supplemented with protein phosphatase and/or protease inhibitors (e.g., EDTA, PMSF, aprotinin, sodium vanadate), adding the antibody of interest to the cell lysate, incubating for a period of time (e.g., 1-4 hours) at 4° C, adding protein A and/or protein G sepharose beads to the cell lysate, incubating for about an hour or more at 4° C, washing the beads in lysis buffer and resuspending the beads in SDS/sample buffer. The ability of the antibody of interest to immunoprecipitate a particular antigen can be assessed by, e.g., western blot analysis. One of skill in the art would be knowledgeable as to the parameters that can be modified to increase the binding of the antibody to an antigen and decrease the background (e.g., pre-clearing the cell lysate with sepharose beads). For further discussion regarding immunoprecipitation protocols see, e.g., Ausubel et al, eds, 1994, Current Protocols in Molecular Biology, Vol. 1, John Wiley & Sons, Inc., New York at 10.16.1.

Western blot analysis generally comprises preparing protein samples, electrophoresis of the protein samples in a polyacrylamide gel (e.g., 8% 20% SDS-PAGE depending on the molecular weight of the antigen), transferring the protein sample from the polyacrylamide gel to a membrane such as nitrocellulose, PVDF or nylon, blocking the membrane in blocking solution (e.g., PBS with 3% BSA or non-fat milk), washing the membrane in washing buffer (e.g., PBS-Tween 20), blocking the membrane with primary antibody (the antibody of interest) diluted in blocking buffer, washing the membrane in washing buffer, blocking the membrane with a secondary antibody (which recognizes the primary antibody, e.g., an anti-human antibody) conjugated to an enzymatic substrate (e.g., horseradish peroxidase or alkaline phosphatase) or radioactive molecule (e.g., 32P or 1251) diluted in blocking buffer, washing the membrane in wash buffer, and detecting the presence of the antigen. One of skill in the art would be knowledgeable as to the parameters that can be modified to increase the signal detected and to reduce the background noise. For further discussion regarding western blot protocols see, e.g., Ausubel et al, eds, 1994, Current Protocols in Molecular Biology, Vol. 1, John Wiley & Sons, Inc., New York at 10.8.1.

ELISAs comprise preparing antigen, coating the well of a 96 well microtiter plate with the antigen, adding the antibody of interest conjugated to a detectable compound such as an enzymatic substrate (e.g., horseradish peroxidase or alkaline phosphatase) to the well and incubating for a period of time, and detecting the presence of the antigen. In ELISAs the antibody of interest does not have to be conjugated to a detectable compound; instead, a second antibody (which recognizes the antibody of interest) conjugated to a detectable compound may be added to the well. Further, instead of coating the well with the antigen, the antibody may be coated to the well. In this case, a second antibody conjugated to a detectable compound may be added following the addition of the antigen of interest to the coated well. One of skill in the art would be knowledgeable as to the parameters that can be modified to increase the signal detected as well as other variations of ELISAs known in the art. For further discussion regarding ELISAs see, e.g., Ausubel et al, eds, 1994, Current Protocols in Molecular Biology, Vol. 1, John Wiley & Sons, Inc., New York at 11.2.1.

The binding affinity of an antibody to an antigen and the off-rate of an antibody-antigen interaction can be determined by competitive binding assays. One example of a competitive binding assay is a radioimmunoassay comprising the incubation of labeled antigen (e.g., 3H or 1251) with the antibody of interest in the presence of increasing amounts of unlabeled antigen, and the detection of the antibody bound to the labeled antigen. The affinity of the antibody of interest for a particular antigen and the binding off-rates can be determined from the data by scatchard plot analysis. Competition with a second antibody can also be determined using radioimmunoassays. In this case, the antigen is incubated with antibody of interest conjugated to a labeled compound (e.g., 3H or 1251) in the presence of increasing amounts of an unlabeled second antibody.

### Therapeutic Uses

Also disclosed are antibody-based therapies which involve administering antibodies described herein to an animal, preferably a mammal, and most preferably a human, patient for treating one or more of the disclosed diseases, disorders, or conditions. Therapeutic compounds described herein include, but are not limited to, antibodies described herein (including fragments, analogs and derivatives thereof as described herein) and nucleic acids encoding antibodies described herein (including fragments, analogs and derivatives thereof and anti-idiotypic antibodies as described herein). The antibodies described herein can be used to treat, inhibit or prevent diseases, disorders or conditions associated with aberrant expression and/or activity of a polypeptide described herein, including, but not limited to, any one or more of the diseases, disorders, or conditions described herein. The treatment and/or prevention of diseases, disorders, or conditions associated with aberrant expression and/or activity of a polypeptide described herein includes, but is not limited to, alleviating symptoms associated with those diseases, disorders or conditions. Antibodies described herein may be provided in pharmaceutically acceptable compositions as known in the art or as described herein.

A summary of the ways in which the antibodies described herein may be used therapeutically includes binding polynucleotides or polypeptides described herein locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies described herein for diagnostic, monitoring or therapeutic purposes without undue experimentation.

The antibodies described herein may be advantageously utilized in combination with other monoclonal or chimeric antibodies, or with lymphokines or hematopoietic growth factors (such as, e.g., IL-2, IL-3 and IL-7), for example, which serve to increase the number or activity of effector cells which interact with the antibodies.

The antibodies described herein may be administered alone or in combination with other types of treatments (e.g., radiation therapy, chemotherapy, hormonal therapy, immunotherapy and anti-tumor agents). Generally, administration of products of a species origin or species reactivity (in the case of antibodies) that is the same species as that of the patient is preferred. Thus, in a preferred embodiment, human antibodies, fragments derivatives, analogs, or nucleic acids, are administered to a human patient for therapy or prophylaxis.

It is preferred to use high affinity and/or potent in vivo inhibiting and/or neutralizing antibodies against polypeptides or polynucleotides described herein, fragments or regions thereof, for both immunoassays directed to and therapy of disorders related to polynucleotides or polypeptides, including fragments thereof, described herein. Such antibodies, fragments, or regions, will preferably have an affinity for polynucleotides or polypeptides described herein, including fragments thereof. Preferred binding affinities include those with a dissociation constant or Kd less than 5 X10⁻² M, 10⁻² M, 5 X 10⁻³ M, 10⁻³ M, 5 X 10⁻⁴ M, 10⁻⁴ M, 5 X 10⁻⁵ M,10⁻⁵ M, 5 X 10⁻⁶ M, 10⁻⁶ M, 5 X 10⁻⁷ M, 10⁻⁷ M, 5 X ID⁻⁸ M, 10⁻⁸ M, 5 X 10⁻⁹ M, 10⁻⁹ M, 5 X 10⁻¹⁰ M, 10⁻¹⁰ M, 5 X 10⁻¹¹ M, 10⁻¹¹ M, 5 X 10⁻¹² M, 10⁻¹² M, 5 X 10⁻¹³ M, 10⁻¹³ M, 5 X 10⁻¹⁴ M, 10⁻¹⁴ M, 5 X10⁻¹⁵ M, or 10⁻¹⁵ M.

### KITS

Also described are kits for practicing the subject methods, as described above. The subject kits include at least one or more of: a subject nucleic acid, isolated polypeptide or an antibody thereto. Other optional components of the kit include: restriction enzymes, control primers and plasmids; buffers, cells, carriers adjuvents etc. The nucleic acids of the kit may also have restrictions sites, multiple cloning sites, primer sites, etc to facilitate their ligation other plasmids. The various components of the kit may be present in separate containers or certain compatible components may be precombined into a single container, as desired. In many embodiments, kits with unit doses of the active agent, e.g. in oral or injectable doses, are provided. In certain embodiments, controls, such as samples from a cancerous or non-cancerous cell are disclosed. Further embodiments of the kit include an antibody for a subject polypeptide and a chemotherapeutic agent to be used in combination with the polypeptide as a treatment.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

### COMPUTER-RELATED EMBODIMENTS

In general, a library of polynucleotides is a collection of sequence information, which information is provided in either biochemical form (*e.g*., as a collection of polynucleotide molecules), or in electronic form (*e.g*., as a collection of polynucleotide sequences stored in a computer-readable form, as in a computer system and/or as part of a computer program). The sequence information of the polynucleotides can be used in a variety of ways, *e.g*., as a resource for gene discovery, as a representation of sequences expressed in a selected cell type (*e.g*., cell type markers), and/or as markers of a given disease or disease state. For example, in the instant case, the sequences of polynucleotides and polypeptides corresponding to genes differentially expressed in cancer, particular in breast cancer, as well as the nucleic acid and amino acid sequences of the genes themselves, can be provided in electronic form in a computer database.

In general, a disease marker is a representation of a gene product that is present in all cells affected by disease either at an increased or decreased level relative to a normal cell (*e.g*., a cell of the same or similar type that is not substantially affected by disease). For example, a polynucleotide sequence in a library can be a polynucleotide that represents an mRNA, polypeptide, or other gene product encoded by the polynucleotide, that is either overexpressed or underexpressed in a cancerous breast cell affected by cancer relative to a normal (*i.e*., substantially disease-free) breast cell.

The nucleotide sequence information of the library can be embodied in any suitable form, *e.g*., electronic or biochemical forms. For example, a library of sequence information embodied in electronic form comprises an accessible computer data file (or, in biochemical form, a collection of nucleic acid molecules) that contains the representative nucleotide sequences of genes that are differentially expressed (*e.g.,* overexpressed or underexpressed) as between, for example, i) a cancerous cell and a normal cell; ii) a cancerous cell and a dysplastic cell; iii) a cancerous cell and a cell affected by a disease or condition other than cancer; iv) a metastatic cancerous cell and a normal cell and/or non-metastatic cancerous cell; v) a malignant cancerous cell and a non-malignant cancerous cell (or a normal cell) and/or vi) a dysplastic cell relative to a normal cell. Other combinations and comparisons of cells affected by various diseases or stages of disease will be readily apparent to the ordinarily skilled artisan. Biochemical embodiments of the library include a collection of nucleic acids that have the sequences of the genes in the library, where the nucleic acids can correspond to the entire gene in the library or to a fragment thereof, as described in greater detail below.

The polynucleotide libraries described herein generally comprise sequence information of a plurality of polynucleotide sequences, where at least one of the polynucleotides has a sequence of any of sequence described herein. By plurality is meant at least 2, usually at least 3 and can include up to all of the sequences described herein. The length and number of polynucleotides in the library will vary with the nature of the library, *e.g*., if the library is an oligonucleotide array, a cDNA array, a computer database of the sequence information, etc.

Where the library is an electronic library, the nucleic acid sequence information can be present in a variety of media. "Media" refers to a manufacture, other than an isolated nucleic acid molecule, that contains the sequence information of the present invention. Such a manufacture provides the genome sequence or a subset thereof in a form that can be examined by means not directly applicable to the sequence as it exists in a nucleic acid. For example, the nucleotide sequence described herein, *e.g.* the nucleic acid sequences of any of the polynucleotides of the sequences described herein, can be recorded on computer readable media, *e.g*. any medium that can be read and accessed directly by a computer. Such media include, but are not limited to: magnetic storage media, such as a floppy disc, a hard disc storage medium, and a magnetic tape; optical storage media such as CD-ROM; electrical storage media such as RAM and ROM; and hybrids of these categories such as magnetic/optical storage media.

One of skill in the art can readily appreciate how any of the presently known computer readable mediums can be used to create a manufacture comprising a recording of the present sequence information. "Recorded" refers to a process for storing information on computer readable medium, using any such methods as known in the art. Any convenient data storage structure can be chosen, based on the means used to access the stored information. A variety of data processor programs and formats can be used for storage, *e.g*. word processing text file, database format, *etc.* In addition to the sequence information, electronic versions of libraries comprising one or more sequence described herein can be provided in conjunction or connection with other computer-readable information and/or other types of computer-readable files (*e.g*., searchable files, executable files, *etc,* including, but not limited to, for example, search program software, *etc*.).

By providing the nucleotide sequence in computer readable form, the information can be accessed for a variety of purposes. Computer software to access sequence information (e.g. the NCBI sequence database) is publicly available. For example, the gapped BLAST (Altschul et al., Nucleic Acids Res. (1997) 25:3389-3402) and BLAZE (Brutlag et al., Comp. Chem. (1993) 17:203) search algorithms on a Sybase system, or the TeraBLAST (TimeLogic, Crystal Bay, Nevada) program optionally running on a specialized computer platform available from TimeLogic, can be used to identify open reading frames (ORFs) within the genome that contain homology to ORFs from other organisms.

As used herein, "a computer-based system" refers to the hardware means, software means, and data storage means used to analyze the nucleotide sequence information of the present invention. The minimum hardware of the computer-based systems described herein comprises a central processing unit (CPU), input means, output means, and data storage means. A skilled artisan can readily appreciate that any one of the currently available computer-based system are suitable for use in the present invention. The data storage means can comprise any manufacture comprising a recording of the present sequence information as described above, or a memory access means that can access such a manufacture.

"Search means" refers to one or more programs implemented on the computer-based system, to compare a target sequence or target structural motif, or expression levels of a polynucleotide in a sample, with the stored sequence information. Search means can be used to identify fragments or regions of the genome that match a particular target sequence or target motif. A variety of known algorithms are publicly known and commercially available, *e.g*. MacPattern (EMBL), TeraBLAST (TimeLogic), BLASTN and BLASTX (NCBI). A "target sequence" can be any polynucleotide or amino acid sequence of six or more contiguous nucleotides or two or more amino acids, preferably from about 10 to 100 amino acids or from about 30 to 300 nt. A variety of means for comparing nucleic acids or polypeptides may be used to compare accomplish a sequence comparison (*e.g*., to analyze target sequences, target motifs, or relative expression levels) with the data storage means. A skilled artisan can readily recognize that any one of the publicly available homology search programs can be used to search the computer based systems described herein to compare of target sequences and motifs. Computer programs to analyze expression levels in a sample and in controls are also known in the art.

A "target structural motif," or "target motif," refers to any rationally selected sequence or combination of sequences in which the sequence(s) are chosen based on a three-dimensional configuration that is formed upon the folding of the target motif, or on consensus sequences of regulatory or active sites. There are a variety of target motifs known in the art. Protein target motifs include, but are not limited to, enzyme active sites and signal sequences, kinase domains, receptor binding domains, SH2 domains, SH3 domains, phosphorylation sites, protein interaction domains, transmembrane domains, etc. Nucleic acid target motifs include, but are not limited to, hairpin structures, promoter sequences and other expression elements such as binding sites for transcription factors.

A variety of structural formats for the input and output means can be used to input and output the information in the computer-based systems of the present invention. One format for an output means ranks the relative expression levels of different polynucleotides. Such presentation provides a skilled artisan with a ranking of relative expression levels to determine a gene expression profile. A gene expression profile can be generated from, for example, a cDNA library prepared from mRNA isolated from a test cell suspected of being cancerous or pre-cancerous, comparing the sequences or partial sequences of the clones against the sequences in an electronic database, where the sequences of the electronic database represent genes differentially expressed in a cancerous cell, *e.g*., a cancerous breast cell. The number of clones having a sequence that has substantial similarity to a sequence that represents a gene differentially expressed in a cancerous cell is then determined, and the number of clones corresponding to each of such genes is determined. An increased number of clones that correspond to differentially expressed gene is present in the cDNA library of the test cell (relative to, for example, the number of clones expected in a cDNA of a normal cell) indicates that the test cell is cancerous.

As discussed above, the "library" as used herein also encompasses biochemical libraries of the polynucleotides of the sequences described herein, *e.g*., collections of nucleic acids representing the provided polynucleotides. The biochemical libraries can take a variety of forms, *e.g*., a solution of cDNAs, a pattern of probe nucleic acids stably associated with a surface of a solid support (*i.e.,* an array) and the like. Of particular interest are nucleic acid arrays in which one or more of the genes described herein is represented by a sequence on the array. By array is meant an article of manufacture that has at least a substrate with at least two distinct nucleic acid targets on one of its surfaces, where the number of distinct nucleic acids can be considerably higher, typically being at least 10 nt, usually at least 20 nt and often at least 25 nt. A variety of different array formats have been developed and are known to those of skill in the art. The arrays described herein find use in a variety of applications, including gene expression analysis, drug screening, mutation analysis and the like, as disclosed in the above-listed exemplary patent documents.

In addition to the above nucleic acid libraries, analogous libraries of polypeptides are also provided, where the polypeptides of the library will represent at least a portion of the polypeptides encoded by a gene corresponding to a sequence described herein.

### DIAGNOSTIC AND OTHER METHODS INVOLVING DETECTION OF DIFFERENTIALLY EXPRESSED GENES

Also disclosed are methods of using the polynucleotides described herein in, for example, diagnosis of cancer and classification of cancer cells according to expression profiles. In specific non-limiting embodiments, the methods are useful for detecting breast cancer cells, facilitating diagnosis of cancer and the severity of a cancer (*e.g*., tumor grade, tumor burden, and the like) in a subject, facilitating a determination of the prognosis of a subject, and assessing the responsiveness of the subject to therapy (*e.g*., by providing a measure of therapeutic effect through, for example, assessing tumor burden during or following a chemotherapeutic regimen). Detection can be based on detection of a polynucleotide that is differentially expressed in a breast cancer cell, and/or detection of a polypeptide encoded by a polynucleotide that is differentially expressed in a breast cancer cell ("a polypeptide associated with breast cancer"). The detection methods described herein can be conducted *in vitro* or *in vivo,* on isolated cells, or in whole tissues or a bodily fluid, *e.g*., blood, plasma, serum, urine, and the like).

In general, methods described herein involving detection of a gene product (*e.g*., mRNA, cDNA generated from such mRNA, and polypeptides) involve contacting a sample with a probe specific for the gene product of interest. "Probe" as used herein in such methods is meant to refer to a molecule that specifically binds a gene product of interest (*e.g*., the probe binds to the target gene product with a specificity sufficient to distinguish binding to target over non-specific binding to non-target (background) molecules). "Probes" include, but are not necessarily limited to, nucleic acid probes (*e.g*., DNA, RNA, modified nucleic acid, and the like), antibodies (*e.g*., antibodies, antibody fragments that retain binding to a target epitope, single chain antibodies, and the like), or other polypeptide, peptide, or molecule (*e.g*., receptor ligand) that specifically binds a target gene product of interest.

The probe and sample suspected of having the gene product of interest are contacted under conditions suitable for binding of the probe to the gene product. For example, contacting is generally for a time sufficient to allow binding of the probe to the gene product (*e.g*., from several minutes to a few hours), and at a temperature and conditions of osmolarity and the like that provide for binding of the probe to the gene product at a level that is sufficiently distinguishable from background binding of the probe (*e.g*., under conditions that minimize non-specific binding). Suitable conditions for probe-target gene product binding can be readily determined using controls and other techniques available and known to one of ordinary skill in the art.

In this embodiment, the probe can be an antibody or other polypeptide, peptide, or molecule (*e.g*., receptor ligand) that specifically binds a target polypeptide of interest.

The detection methods can be provided as part of a kit. Thus, also disclosed are kits for detecting the presence and/or a level of a polynucleotide that is differentially expressed in a breast cancer cell (*e.g*., by detection of an mRNA encoded by the differentially expressed gene of interest), and/or a polypeptide encoded thereby, in a biological sample. Procedures using these kits can be performed by clinical laboratories, experimental laboratories, medical practitioners, or private individuals. The kits for detecting a polypeptide encoded by a polynucleotide that is differentially expressed in a breast cancer cell comprise a moiety that specifically binds the polypeptide, which may be a specific antibody. The kits for detecting a polynucleotide that is differentially expressed in a breast cancer cell comprise a moiety that specifically hybridizes to such a polynucleotide. The kit may optionally provide additional components that are useful in the procedure, including, but not limited to, buffers, developing reagents, labels, reacting surfaces, means for detection, control samples, standards, instructions, and interpretive information.

### Detecting a polypeptide encoded by a polynucleotide that is differentially expressed in a breast cancer cell

In some embodiments, methods are provided for a detecting breast cancer cell by detecting in a cell, particularly a breast cell, a polypeptide encoded by a gene differentially expressed in a breast cancer cell. Any of a variety of known methods can be used for detection, including, but not limited to, immunoassay, using an antibody specific for the encoded polypeptide, e.g., by enzyme-linked immunosorbent assay (ELISA), radioimmunoassay (RIA), and the like; and functional assays for the encoded polypeptide, e.g., binding activity or enzymatic activity.

For example, an immunofluorescence assay can be easily performed on cells without first isolating the encoded polypeptide. The cells are first fixed onto a solid support, such as a microscope slide or microtiter well. This fixing step can permeabilize the cell membrane. The permeablization of the cell membrane permits the polypeptide-specific probe (*e.g,* antibody) to bind. Alternatively, where the polypeptide is secreted or membrane-bound, or is otherwise accessible at the cell-surface (*e.g*., receptors, and other molecule stably-associated with the outer cell membrane or otherwise stably associated with the cell membrane, such permeabilization may not be necessary.

Next, the fixed cells are exposed to an antibody specific for the encoded polypeptide. To increase the sensitivity of the assay, the fixed cells may be further exposed to a second antibody, which is labeled and binds to the first antibody, which is specific for the encoded polypeptide. Typically, the secondary antibody is detectably labeled, e.g., with a fluorescent marker. The cells which express the encoded polypeptide will be fluorescently labeled and easily visualized under the microscope. See, for example, Hashido et al. (1992) Biochem. Biophys. Res. Comm. 187:1241-1248.

As will be readily apparent to the ordinarily skilled artisan upon reading the present specification, the detection methods and other methods described herein can be varied. Such variations are within the intended scope of the invention. For example, in the above detection scheme, the probe for use in detection can be immobilized on a solid support, and the test sample contacted with the immobilized probe. Binding of the test sample to the probe can then be detected in a variety of ways, *e.g.,* by detecting a detectable label bound to the test sample.

Also disclosed are methods for detecting the presence of and/or measuring a level of a polypeptide in a biological sample, which polypeptide is encoded by a polynucleotide that represents a gene differentially expressed in cancer, particularly in a polynucleotide that represents a gene differentially cancer cell, using a probe specific for the encoded polypeptide. In this embodiment, the probe can be a an antibody or other polypeptide, peptide, or molecule (*e.g*., receptor ligand) that specifically binds a target polypeptide of interest.

The methods generally comprise: a) contacting the sample with an antibody specific for a differentially expressed polypeptide in a test cell; and b) detecting binding between the antibody and molecules of the sample. The level of antibody binding (either qualitative or quantitative) indicates the cancerous state of the cell. For example, where the differentially expressed gene is increased in cancerous cells, detection of an increased level of antibody binding to the test sample relative to antibody binding level associated with a normal cell indicates that the test cell is cancerous.

Suitable controls include a sample known not to contain the encoded polypeptide; and a sample contacted with an antibody not specific for the encoded polypeptide, e.g., an anti-idiotype antibody. A variety of methods to detect specific antibody-antigen interactions are known in the art and can be used in the method, including, but not limited to, standard immunohistological methods, immunoprecipitation, an enzyme immunoassay, and a radioimmunoassay.

In general, the specific antibody will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g., ¹⁵²Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like.

The antibody may be attached (coupled) to an insoluble support, such as a polystyrene plate or a bead. Indirect labels include second antibodies specific for antibodies specific for the encoded polypeptide ("first specific antibody"), wherein the second antibody is labeled as described above; and members of specific binding pairs, e.g., biotin-avidin, and the like. The biological sample may be brought into contact with and immobilized on a solid support or carrier, such as nitrocellulose, that is capable of immobilizing cells, cell particles, or soluble proteins. The support may then be washed with suitable buffers, followed by contacting with a detectably-labeled first specific antibody. Detection methods are known in the art and will be chosen as appropriate to the signal emitted by the detectable label. Detection is generally accomplished in comparison to suitable controls, and to appropriate standards.

In some embodiments, the methods are adapted for use *in vivo,* e.g., to locate or identify sites where breast cancer cells are present. In these embodiments, a detectably-labeled moiety, e.g., an antibody, which is specific for a breast cancer-associated polypeptide is administered to an individual (e.g., by injection), and labeled cells are located using standard imaging techniques, including, but not limited to, magnetic resonance imaging, computed tomography scanning, and the like. In this manner, breast cancer cells are differentially labeled.

### Detecting a polynucleotide that represents a gene differentially expressed in a breast cancer cell

In some embodiments, methods are provided for detecting a breast cancer cell by detecting expression in the cell of a transcript or that is differentially expressed in a breast cancer cell. Any of a variety of known methods can be used for detection, including, but not limited to, detection of a transcript by hybridization with a polynucleotide that hybridizes to a polynucleotide that is differentially expressed in a breast cancer cell; detection of a transcript by a polymerase chain reaction using specific oligonucleotide primers; *in situ* hybridization of a cell using as a probe a polynucleotide that hybridizes to a gene that is differentially expressed in a breast cancer cell and the like.

In many embodiments, the levels of a subject gene product are measured. By measured is meant qualitatively or quantitatively estimating the level of the gene product in a first biological sample either directly (e.g. by determining or estimating absolute levels of gene product) or relatively by comparing the levels to a second control biological sample. In many embodiments the second control biological sample is obtained from an individual not having not having breast cancer. As will be appreciated in the art, once a standard control level of gene expression is known, it can be used repeatedly as a standard for comparison. Other control samples include samples of cancerous breast tissue.

The methods can be used to detect and/or measure mRNA levels of a gene that is differentially expressed in a breast cancer cell. In some embodiments, the methods comprise: a) contacting a sample with a polynucleotide that corresponds to a differentially expressed gene described herein under conditions that allow hybridization; and b) detecting hybridization, if any. Detection of differential hybridization, when compared to a suitable control, is an indication of the presence in the sample of a polynucleotide that is differentially expressed in a breast cancer cell. Appropriate controls include, for example, a sample that is known not to contain a polynucleotide that is differentially expressed in a breast cancer cell. Conditions that allow hybridization are known in the art, and have been described in more detail above.

Detection can also be accomplished by any known method, including, but not limited to, *in situ* hybridization, PCR (polymerase chain reaction), RT-PCR (reverse transcription-PCR), and "Northern" or RNA blotting, arrays, microarrays, etc, or combinations of such techniques, using a suitably labeled polynucleotide. A variety of labels and labeling methods for polynucleotides are known in the art and can be used in the assay methods of the invention. Specific hybridization can be determined by comparison to appropriate controls.

Polynucleotide generally comprising at least 12 contiguous nt of a polynucleotide provided herein, as shown in the Sequence Listing or of the sequences of the genes corresponding to the polynucleotides of the Sequence Listing, are used for a variety of purposes, such as probes for detection of and/or measurement of, transcription levels of a polynucleotide that is differentially expressed in a breast cancer cell. Additional disclosure about preferred regions of the disclosed polynucleotide sequences is found in the Examples. A probe that hybridizes specifically to a polynucleotide disclosed herein should provide a detection signal at least 2-, 5-, 10-, or 20-fold higher than the background hybridization provided with other unrelated sequences. It should be noted that "probe" as used in this context of detection of nucleic acid is meant to refer to a polynucleotide sequence used to detect a differentially expressed gene product in a test sample. As will be readily appreciated by the ordinarily skilled artisan, the probe can be detectably labeled and contacted with, for example, an array comprising immobilized polynucleotides obtained from a test sample (*e.g.,* mRNA). Alternatively, the probe can be immobilized on an array and the test sample detectably labeled. These and other variations of the methods of the invention are well within the skill in the art and are within the scope of the invention.

Labeled nucleic acid probes may be used to detect expression of a gene corresponding to the provided polynucleotide. In Northern blots, mRNA is separated electrophoretically and contacted with a probe. A probe is detected as hybridizing to an mRNA species of a particular size. The amount of hybridization can be quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of detectable labels can be used such as chromophores, fluorophores, and enzymes. Other examples of nucleotide hybridization assays are described in WO92/02526 and USPN 5,124,246.

PCR is another means for detecting small amounts of target nucleic acids, methods for which may be found in Sambrook, et al. Molecular Cloning: A Laboratory Manual, CSH Press 1989, pp.14.2-14.33.

A detectable label may be included in the amplification reaction. Suitable detectable labels include fluorochromes,(*e.g*. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein, 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA)), radioactive labels, (*e.g*. ³²P, ³⁵S, ³H, *etc*.), and the like. The label may be a two stage system, where the polynucleotides is conjugated to biotin, haptens, *etc.* having a high affinity binding partner, *e.g*. avidin, specific antibodies, *etc.,* where the binding partner is conjugated to a detectable label. The label may be conjugated to one or both of the primers. Alternatively, the pool of nucleotides used in the amplification is labeled, so as to incorporate the label into the amplification product.

### Arrays

Polynucleotide arrays provide a high throughput technique that can assay a large number of polynucleotides or polypeptides in a sample. This technology can be used as a tool to test for differential expression.

A variety of methods of producing arrays, as well as variations of these methods, are known in the art and contemplated for use in the invention. For example, arrays can be created by spotting polynucleotide probes onto a substrate (*e.g*., glass, nitrocellulose, *etc.*) in a two-dimensional matrix or array having bound probes. The probes can be bound to the substrate by either covalent bonds or by non-specific interactions, such as hydrophobic interactions.

Samples of polynucleotides can be detectably labeled (*e.g*., using radioactive or fluorescent labels) and then hybridized to the probes. Double stranded polynucleotides, comprising the labeled sample polynucleotides bound to probe polynucleotides, can be detected once the unbound portion of the sample is washed away. Alternatively, the polynucleotides of the test sample can be immobilized on the array, and the probes detectably labeled. Techniques for constructing arrays and methods of using these arrays are described in, for example, Schena et al. (1996) Proc Natl Acad Sci U S A. 93(20):10614-9; Schena et al. (1995) Science 270(5235):467-70; Shalon et al. (1996) Genome Res. 6(7):639-45, USPN 5,807,522, EP 799 897; WO 97/29212; WO 97/27317; EP 785 280; WO 97/02357; USPN 5,593,839; USPN 5,578,832; EP 728 520; USPN 5,599,695; EP 721 016; USPN 5,556,752; WO 95/22058; and USPN 5,631,734. In most embodiments, the "probe" is detectably labeled. In other embodiments, the probe is immobilized on the array and not detectably labeled.

Arrays can be used, for example, to examine differential expression of genes and can be used to determine gene function. For example, arrays can be used to detect differential expression of a gene corresponding to a polynucleotide described herein, where expression is compared between a test cell and control cell (*e.g*., cancer cells and normal cells). For example, high expression of a particular message in a cancer cell, which is not observed in a corresponding normal cell, can indicate a cancer specific gene product. Exemplary uses of arrays are further described in, for example, Pappalarado et al., Sem. Radiation Oncol. (1998) 8:217; and Ramsay, Nature Biotechnol. (1998) 16:40. Furthermore, many variations on methods of detection using arrays are well within the skill in the art and within the scope of the present invention. For example, rather than immobilizing the probe to a solid support, the test sample can be immobilized on a solid support which is then contacted with the probe.

### DIAGNOSIS, PROGNOSIS, ASSESSMENT OF THERAPY (THERAMETRICS), AND MANAGEMENT OF CANCER

The polynucleotides described herein, as well as their gene products and corresponding genes and gene products, are of particular interest as genetic or biochemical markers (e.g., in blood or tissues) that will detect the earliest changes along the carcinogenesis pathway and/or to monitor the efficacy of various therapies and preventive interventions.

For example, the level of expression of certain polynucleotides can be indicative of a poorer prognosis, and therefore warrant more aggressive chemo- or radio-therapy for a patient or vice versa. The correlation of novel surrogate tumor specific features with response to treatment and outcome in patients can define prognostic indicators that allow the design of tailored therapy based on the molecular profile of the tumor. These therapies include antibody targeting, antagonists (*e.g*., small molecules), and gene therapy.

Determining expression of certain polynucleotides and comparison of a patient's profile with known expression in normal tissue and variants of the disease allows a determination of the best possible treatment for a patient, both in terms of specificity of treatment and in terms of comfort level of the patient. Surrogate tumor markers, such as polynucleotide expression, can also be used to better classify, and thus diagnose and treat, different forms and disease states of cancer. Two classifications widely used in oncology that can benefit from identification of the expression levels of the genes corresponding to the polynucleotides described herein are staging of the cancerous disorder, and grading the nature of the cancerous tissue.

The polynucleotides that correspond to differentially expressed genes, as well as their encoded gene products, can be useful to monitor patients having or susceptible to cancer to detect potentially malignant events at a molecular level before they are detectable at a gross morphological level. In addition, the polynucleotides described herein, as well as the genes corresponding to such polynucleotides, can be useful as therametrics, *e.g*., to assess the effectiveness of therapy by using the polynucleotides or their encoded gene products, to assess, for example, tumor burden in the patient before, during, and after therapy.

Furthermore, a polynucleotide identified as corresponding to a gene that is differentially expressed in, and thus is important for, one type of cancer can also have implications for development or risk of development of other types of cancer, e.g., where a polynucleotide represents a gene differentially expressed across various cancer types. Thus, for example, expression of a polynucleotide corresponding to a gene that has clinical implications for breast cancer can also have clinical implications for metastatic breast cancer, colon cancer, or ovarian cancer.

Staging. Staging is a process used by physicians to describe how advanced the cancerous state is in a patient. Staging assists the physician in determining a prognosis, planning treatment and evaluating the results of such treatment. Staging systems vary with the types of cancer, but generally involve the following "TNM" system: the type of tumor, indicated by T; whether the cancer has metastasized to nearby lymph nodes, indicated by N; and whether the cancer has metastasized to more distant parts of the body, indicated by M. Generally, if a cancer is only detectable in the area of the primary lesion without having spread to any lymph nodes it is called Stage I. If it has spread only to the closest lymph nodes, it is called Stage II. In Stage III, the cancer has generally spread to the lymph nodes in near proximity to the site of the primary lesion. Cancers that have spread to a distant part of the body, such as the liver, bone, brain or other site, are Stage IV, the most advanced stage.

The polynucleotides and corresponding genes and gene products described herein can facilitate fine-tuning of the staging process by identifying markers for the aggressiveness of a cancer, *e.g*. the metastatic potential, as well as the presence in different areas of the body. Thus, a Stage II cancer with a polynucleotide signifying a high metastatic potential cancer can be used to change a borderline Stage II tumor to a Stage III tumor, justifying more aggressive therapy. Conversely, the presence of a polynucleotide signifying a lower metastatic potential allows more conservative staging of a tumor.

One type of breast cancer is ductal carcinoma in situ (DCIS): DCIS is when the breast cancer cells are completely contained within the breast ducts (the channels in the breast that carry milk to the nipple), and have not spread into the surrounding breast tissue. This may also be referred to as non-invasive or intraductal cancer, as the cancer cells have not yet spread into the surrounding breast tissue and so usually have not spread into any other part of the body.

Lobular carcinoma in situ breast cancer (LCIS) means that cell changes are found in the lining of the lobules of the breast. It can be present in both breasts. It is also referred to as non-invasive cancer as it has not spread into the surrounding breast tissue.

Invasive breast cancer can be staged as follows: Stage 1 tumours: these measure less than two centimetres. The lymph glands in the armpit are not affected and there are no signs that the cancer has spread elsewhere in the body; Stage 2 tumours: these measure between two and five centimetres, or the lymph glands in the armpit are affected, or both. However, there are no signs that the cancer has spread further; Stage 3 tumours: these are larger than five centimetres and may be attached to surrounding structures such as the muscle or skin. The lymph glands are usually affected, but there are no signs that the cancer has spread beyond the breast or the lymph glands in the armpit; Stage 4 tumours: these are of any size, but the lymph glands are usually affected and the cancer has spread to other parts of the body. This is secondary breast cancer.

Grading of cancers. Grade is a term used to describe how closely a tumor resembles normal tissue of its same type. The microscopic appearance of a tumor is used to identify tumor grade based on parameters such as cell morphology, cellular organization, and other markers of differentiation. As a general rule, the grade of a tumor corresponds to its rate of growth or aggressiveness, with undifferentiated or high-grade tumors generally being more aggressive than well-differentiated or low-grade tumors.

The polynucleotides of the Sequence Listing, and their corresponding genes and gene products, can be especially valuable in determining the grade of the tumor, as they not only can aid in determining the differentiation status of the cells of a tumor, they can also identify factors other than differentiation that are valuable in determining the aggressiveness of a tumor, such as metastatic potential.

Low grade means that the cancer cells look very like the normal cells of the breast. They are usually slowly growing and are less likely to spread. In high grade tumors the cells look very abnormal. They are likely to grow more quickly and are more likely to spread.

Assessment of proliferation of cells in tumor. The differential expression level of the polynucleotides described herein can facilitate assessment of the rate of proliferation of tumor cells, and thus provide an indicator of the aggressiveness of the rate of tumor growth. For example, assessment of the relative expression levels of genes involved in cell cycle can provide an indication of cellular proliferation, and thus serve as a marker of proliferation.

### Detection of breast cancer.

The polynucleotides corresponding to genes that exhibit the appropriate expression pattern can be used to detect breast cancer in a subject. Breast cancer is one of the most common neoplasms in women, and prevention and early detection are key factors in controlling and curing breast cancer. The expression of appropriate polynucleotides can be used in the diagnosis, prognosis and management of breast cancer. Detection of breast cancer can be determined using expression levels of any of these sequences alone or in combination with the levels of expression of other known cancer genes. Determination of the aggressive nature and/or the metastatic potential of a breast cancer can be determined by comparing levels of one or more gene products of the genes corresponding to the polynucleotides described herein, and comparing total levels of another sequence known to vary in cancerous tissue, *e.g*., expression of p53, DCC, ras, FAP (see, e.g., Fearon ER, et al., Cell (1990) 61(5):759; Hamilton SR et al., Cancer (1993) 72:957; Bodmer W, et al., Nat Genet. (1994) 4(3):217; Fearon ER, Ann N YAcad Sci. (1995) 768:101). For example, development of breast cancer can be detected by examining the level of expression of a gene corresponding to a polynucleotides described herein to the levels of oncogenes (*e.g*. ras) or tumor suppressor genes (*e.g.* FAP or p53). Thus expression of specific marker polynucleotides can be used to discriminate between normal and cancerous breast tissue, to discriminate between breast cancers with different cells of origin, to discriminate between breast cancers with different potential metastatic rates, etc. For a review of other markers of cancer, see, *e.g*., Hanahan et al. (2000) Cell 100:57-70.

### Treatment of breast cancer

Also disclosed are methods for reducing growth of breast cancer cells. The methods provide for decreasing the expression of a gene that is differentially expressed in a breast cancer cell or decreasing the level of and/or decreasing an activity of a breast cancer-associated polypeptide. In general, the methods comprise contacting a breast cancer cell with a substance that modulates (1) expression of a gene that is differentially expressed in breast cancer; or (2) a level of and/or an activity of a breast cancer-associated polypeptide.

"Reducing growth of breast cancer cells" includes, but is not limited to, reducing proliferation of breast cancer cells, and reducing the incidence of a non-cancerous breast cell becoming a cancerous breast cell. Whether a reduction in breast cancer cell growth has been achieved can be readily determined using any known assay, including, but not limited to, [³H]-thymidine incorporation; counting cell number over a period of time; detecting and/or measuring a marker associated with breast cancer (e.g., PSA).

Also disclosed are methods for treating breast cancer, generally comprising administering to an individual in need thereof a substance that reduces breast cancer cell growth, in an amount sufficient to reduce breast cancer cell growth and treat the breast cancer. Whether a substance, or a specific amount of the substance, is effective in treating breast cancer can be assessed using any of a variety of known diagnostic assays for breast cancer, including, but not limited to, proctoscopy, rectal examination, biopsy, contrast radiographic studies, CAT scan, and detection of a tumor marker associated with breast cancer in the blood of the individual (*e.g*., PSA (breast-specific antigen)). The substance can be administered systemically or locally. Thus, in some embodiments, the substance is administered locally, and breast cancer growth is decreased at the site of administration. Local administration may be useful in treating, e.g., a solid tumor.

A substance that reduces breast cancer cell growth can be targeted to a breast cancer cell. Thus, in some embodiments, a method of delivering a drug to a breast cancer cell is disclosed, comprising administering a drug-antibody complex to a subject, wherein the antibody is specific for a breast cancer-associated polypeptide, and the drug is one that reduces breast cancer cell growth, a variety of which are known in the art. Targeting can be accomplished by coupling (e.g., linking, directly or via a linker molecule, either covalently or non-covalently, so as to form a drug-antibody complex) a drug to an antibody specific for a breast cancer-associated polypeptide. Methods of coupling a drug to an antibody are well known in the art and need not be elaborated upon herein.

### Tumor classification and patient stratification

Also disclosed are methods of classifying tumors, and thus grouping or "stratifying" patients, according to the expression profile of selected differentially expressed genes in a tumor. Differentially expressed genes can be analyzed for correlation with other differentially expressed genes in a single tumor type or across tumor types. Genes that demonstrate consistent correlation in expression profile in a given cancer cell type (*e.g*., in a breast cancer cell or type of breast cancer) can be grouped together, e.g., when one gene is overexpressed in a tumor, a second gene is also usually overexpressed. Tumors can then be classified according to the expression profile of one or more genes selected from one or more groups.

The tumor of each patient in a pool of potential patients can be classified as described above. Patients having similarly classified tumors can then be selected for participation in an investigative or clinical trial of a cancer therapeutic where a homogeneous population is desired. The tumor classification of a patient can also be used in assessing the efficacy of a cancer therapeutic in a heterogeneous patient population. In addition, therapy for a patient having a tumor of a given expression profile can then be selected accordingly.

In another embodiment, differentially expressed gene products (*e.g*., polypeptides or polynucleotides encoding such polypeptides) may be effectively used in treatment through vaccination. The growth of cancer cells is naturally limited in part due to immune surveillance. Stimulation of the immune system using a particular tumor-specific antigen enhances the effect towards the tumor expressing the antigen. An active vaccine comprising a polypeptide encoded by the cDNA described herein would be appropriately administered to subjects having an alteration, e.g., overabundance, of the corresponding RNA, or those predisposed for developing cancer cells with an alteration of the same RNA. Polypeptide antigens are typically combined with an adjuvant as part of a vaccine composition. The vaccine is preferably administered first as a priming dose, and then again as a boosting dose, usually at least four weeks later. Further boosting doses may be given to enhance the effect. The dose and its timing are usually determined by the person responsible for the treatment.

Also disclosed is the selection of a therapeutic regimen based upon the expression profile of differentially expressed genes in the patient's tumor. For example, a tumor can be analyzed for its expression profile of the genes corresponding to SEQ ID NOS:1-499 as described herein, *e.g*., the tumor is analyzed to determine which genes are expressed at elevated levels or at decreased levels relative to normal cells of the same tissue type. The expression patterns of the tumor are then compared to the expression patterns of tumors that respond to a selected therapy. Where the expression profiles of the test tumor cell and the expression profile of a tumor cell of known drug responsivity at least substantially match (*e.g*., selected sets of genes at elevated levels in the tumor of known drug responsivity and are also at elevated levels in the test tumor cell), then the therapeutic agent selected for therapy is the drug to which tumors with that expression pattern respond.

### Pattern matching in diagnosis using arrays

In another embodiment, the diagnostic and/or prognostic methods described herein involve detection of expression of a selected set of genes in a test sample to produce a test expression pattern (TEP). The TEP is compared to a reference expression pattern (REP), which is generated by detection of expression of the selected set of genes in a reference sample (*e.g*., a positive or negative control sample). The selected set of genes includes at least one of the genes described herein, which genes correspond to the polynucleotide sequences described herein. Of particular interest is a selected set of genes that includes gene differentially expressed in the disease for which the test sample is to be screened.

### IDENTIFICATION OF THERAPEUTIC TARGETS AND ANTI-CANCER THERAPEUTIC AGENTS

Also disclosed are methods for identification of agents having the ability to modulate activity of a differentially expressed gene product, as well as methods for identifying a differentially expressed gene product as a therapeutic target for treatment of cancer, especially breast cancer.

Identification of compounds that modulate activity of a differentially expressed gene product can be accomplished using any of a variety of drug screening techniques. Such agents are candidates for development of cancer therapies. Of particular interest are screening assays for agents that have tolerable toxicity for normal, non-cancerous human cells. The screening assays described herein are generally based upon the ability of the agent to modulate an activity of a differentially expressed gene product and/or to inhibit or suppress phenomenon associated with cancer (*e.g*., cell proliferation, colony formation, cell cycle arrest, metastasis, and the like).

### Screening of candidate agents

Screening assays can be based upon any of a variety of techniques readily available and known to one of ordinary skill in the art. In general, the screening assays involve contacting a cancerous cell (preferably a cancerous cell such as a cancerous breast cell) with a candidate agent, and assessing the effect upon biological activity of a differentially expressed gene product. The effect upon a biological activity can be detected by, for example, detection of expression of a gene product of a differentially expressed gene (*e.g*., a decrease in mRNA or polypeptide levels, would in turn cause a decrease in biological activity of the gene product). Alternatively or in addition, the effect of the candidate agent can be assessed by examining the effect of the candidate agent in a functional assay. For example, where the differentially expressed gene product is an enzyme, then the effect upon biological activity can be assessed by detecting a level of enzymatic activity associated with the differentially expressed gene product. The functional assay will be selected according to the differentially expressed gene product. In general, where the differentially expressed gene is increased in expression in a cancerous cell, agents of interest are those that decrease activity of the differentially expressed gene product.

Assays described infra can be readily adapted in the screening assay embodiments of the invention. Exemplary assays useful in screening candidate agents include, but are not limited to, hybridization-based assays (*e.g*., use of nucleic acid probes or primers to assess expression levels), antibody-based assays (*e.g*., to assess levels of polypeptide gene products), binding assays (*e.g*., to detect interaction of a candidate agent with a differentially expressed polypeptide, which assays may be competitive assays where a natural or synthetic ligand for the polypeptide is available), and the like. Additional exemplary assays include, but are not necessarily limited to, cell proliferation assays, antisense knockout assays, assays to detect inhibition of cell cycle, assays of induction of cell death/apoptosis, and the like. Generally such assays are conducted *in vitro,* but many assays can be adapted for *in vivo* analyses, *e.g*., in an animal model of the cancer.

### Identification of therapeutic targets

Also disclosed is identification of differentially expressed genes and gene products as therapeutic targets. In some respects, this is the converse of the assays described above for identification of agents having activity in modulating (*e.g*., decreasing or increasing) activity of a differentially expressed gene product.

In this embodiment, therapeutic targets are identified by examining the effect(s) of an agent that can be demonstrated or has been demonstrated to modulate a cancerous phenotype (*e.g*., inhibit or suppress or prevent development of a cancerous phenotype). Such agents are generally referred to herein as an "anti-cancer agent", which agents encompass chemotherapeutic agents. For example, the agent can be an antisense oligonucleotide that is specific for a selected gene transcript. For example, the antisense oligonucleotide may have a sequence corresponding to a sequence of a differentially expressed gene described herein, *e.g*., a sequence of one of SEQ ID NOS: 1-499.

Assays for identification of therapeutic targets can be conducted in a variety of ways using methods that are well known to one of ordinary skill in the art. For example, a test cancerous cell that expresses or overexpresses a differentially expressed gene is contacted with an anti-cancer agent, the effect upon a cancerous phenotype and a biological activity of the candidate gene product assessed. The biological activity of the candidate gene product can be assayed be examining, for example, modulation of expression of a gene encoding the candidate gene product (*e.g*., as detected by, for example, an increase or decrease in transcript levels or polypeptide levels), or modulation of an enzymatic or other activity of the gene product. The cancerous phenotype can be, for example, cellular proliferation, loss of contact inhibition of growth (*e.g*., colony formation), tumor growth (*in vitro* or *in vivo),* and the like. Alternatively or in addition, the effect of modulation of a biological activity of the candidate target gene upon cell death/apoptosis or cell cycle regulation can be assessed.

Inhibition or suppression of a cancerous phenotype, or an increase in cell death or apoptosis as a result of modulation of biological activity of a candidate gene product indicates that the candidate gene product is a suitable target for cancer therapy. Assays described infra can be readily adapted for assays for identification of therapeutic targets. Generally such assays are conducted *in vitro,* but many assays can be adapted for *in vivo* analyses, *e.g*., in an appropriate, art-accepted animal model of the cancer.

### Candidate agents

The term "agent" as used herein describes any molecule, *e.g*. protein or pharmaceutical, with the capability of modulating a biological activity of a gene product of a differentially expressed gene. Generally a plurality of assay mixtures are run in parallel with different agent concentrations to obtain a differential response to the various concentrations. Typically, one of these concentrations serves as a negative control, *i.e.* at zero concentration or below the level of detection.

Candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts (including extracts from human tissue to identify endogenous factors affecting differentially expressed gene products) are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

Exemplary candidate agents of particular interest include, but are not limited to, antisense and RNAi polynucleotides, and antibodies, soluble receptors, and the like. Antibodies and soluble receptors are of particular interest as candidate agents where the target differentially expressed gene product is secreted or accessible at the cell-surface (*e.g*., receptors and other molecule stably-associated with the outer cell membrane).

For method that involve RNAi (RNA interference), a double stranded RNA (dsRNA) molecule is usually used. The dsRNA is prepared to be substantially identical to at least a segment of a subject polynucleotide (e.g. a cDNA or gene). In general, the dsRNA is selected to have at least 70%, 75%, 80%, 85% or 90% sequence identity with the subject polynucleotide over at least a segment of the candidate gene. In other instances, the sequence identity is even higher, such as 95%, 97% or 99%, and in still other instances, there is 100% sequence identity with the subject polynucleotide over at least a segment of the subject polynucleotide. The size of the segment over which there is sequence identity can vary depending upon the size of the subject polynucleotide. In general, however, there is substantial sequence identity over at least 15, 20, 25, 30, 35, 40 or 50 nucleotides. In other instances, there is substantial sequence identity over at least 100, 200, 300, 400, 500 or 1000 nucleotides; in still other instances, there is substantial sequence identity over the entire length of the subject polynucleotide, i.e., the coding and non-coding region of the candidate gene.

Because only substantial sequence similarity between the subject polynucleotide and the dsRNA is necessary, sequence variations between these two species arising from genetic mutations, evolutionary divergence and polymorphisms can be tolerated. Moreover, as described further infra, the dsRNA can include various modified or nucleotide analogs.

Usually the dsRNA consists of two separate complementary RNA strands. However, in some instances, the dsRNA may be formed by a single strand of RNA that is self-complementary, such that the strand loops back upon itself to form a hairpin loop. Regardless of form, RNA duplex formation can occur inside or outside of a cell.

The size of the dsRNA that is utilized varies according to the size of the subject polynucleotide whose expression is to be suppressed and is sufficiently long to be effective in reducing expression of the subject polynucleotide in a cell. Generally, the dsRNA is at least 10-15 nucleotides long. In certain applications, the dsRNA is less than 20, 21, 22, 23, 24 or 25 nucleotides in length. In other instances, the dsRNA is at least 50, 100, 150 or 200 nucleotides in length. The dsRNA can be longer still in certain other applications, such as at least 300, 400, 500 or 600 nucleotides. Typically, the dsRNA is not longer than 3000 nucleotides. The optimal size for any particular subject polynucleotide can be determined by one of ordinary skill in the art without undue experimentation by varying the size of the dsRNA in a systematic fashion and determining whether the size selected is effective in interfering with expression of the subject polynucleotide.

dsRNA can be prepared according to any of a number of methods that are known in the art, including in vitro and in vivo methods, as well as by synthetic chemistry approaches.

*In vitro methods.* Certain methods generally involve inserting the segment corresponding to the candidate gene that is to be transcribed between a promoter or pair of promoters that are oriented to drive transcription of the inserted segment and then utilizing an appropriate RNA polymerase to carry out transcription. One such arrangement involves positioning a DNA fragment corresponding to the candidate gene or segment thereof into a vector such that it is flanked by two opposable polymerase-specific promoters that can be same or different. Transcription from such promoters produces two complementary RNA strands that can subsequently anneal to form the desired dsRNA. Exemplary plasmids for use in such systems include the plasmid (PCR 4.0 TOPO) (available from Invitrogen). Another example is the vector pGEM-T (Promega, Madison, WI) in which the oppositely oriented promoters are T7 and SP6; the T3 promoter can also be utilized.

In a second arrangement, DNA fragments corresponding to the segment of the subject polynucleotide that is to be transcribed is inserted both in the sense and antisense orientation downstream of a single promoter. In this system, the sense and antisense fragments are cotranscribed to generate a single RNA strand that is self-complementary and thus can form dsRNA.

Various other *in vitro* methods have been described. Examples of such methods include, but are not limited to, the methods described by Sadher et al. (Biochem. Int. 14:1015, 1987); by Bhattacharyya (Nature 343:484, 1990); and by Livache, et al. (U.S. Patent No. 5,795,715).

Single-stranded RNA can also be produced using a combination of enzymatic and organic synthesis or by total organic synthesis. The use of synthetic chemical methods enable one to introduce desired modified nucleotides or nucleotide analogs into the dsRNA.

*In vivo methods.* dsRNA can also be prepared in vivo according to a number of established methods (see, e.g., Sambrook, et al. (1989) Molecular Cloning: A Laboratory Manual, 2nd ed.; Transcription and Translation (B.D. Hames, and S.J. Higgins, Eds., 1984); DNA Cloning, volumes I and II (D.N. Glover, Ed., 1985); and Oligonucleotide Synthesis (M.J. Gait, Ed., 1984).

Once the single-stranded RNA has been formed, the complementary strands are allowed to anneal to form duplex RNA. Transcripts are typically treated with DNAase and further purified according to established protocols to remove proteins. Usually such purification methods are not conducted with phenol:chloroform. The resulting purified transcripts are subsequently dissolved in RNAase free water or a buffer of suitable composition.

dsRNA is generated by annealing the sense and anti-sense RNA in vitro. Generally, the strands are initially denatured to keep the strands separate and to avoid self-annealing. During the annealing process, typically certain ratios of the sense and antisense strands are combined to facilitate the annealing process. In some instances, a molar ratio of sense to antisense strands of 3:7 is used; in other instances, a ratio of 4:6 is utilized; and in still other instances, the ratio is 1:1.

The buffer composition utilized during the annealing process can in some instances affect the efficacy of the annealing process and subsequent transfection procedure. While some have indicated that the buffered solution used to carry out the annealing process should include a potassium salt such as potassium chloride (e.g. at a concentration of about 80 mM). In some embodiments, the buffer is substantially postassium free. Once single-stranded RNA has annealed to form duplex RNA, typically any single-strand overhangs are removed using an enzyme that specifically cleaves such overhangs (e.g., RNAase A or RNAase T).

Once the dsRNA has been formed, it is introduced into a reference cell, which can include an individual cell or a population of cells (e.g., a tissue, an embryo and an entire organism). The cell can be from essentially any source, including animal, plant, viral, bacterial, fungal and other sources. If a tissue, the tissue can include dividing or nondividing and differentiated or undifferentiated cells. Further, the tissue can include germ line cells and somatic cells. Examples of differentiated cells that can be utilized include, but are not limited to, neurons, glial cells, blood cells, megakaryocytes, lymphocytes, macrophages, neutrophils, eosinophils, basophils, mast cells, leukocytes, granulocytes, keratinocytes, adipocytes, osteoblasts, osteoclasts, hepatocytes, cells of the endocrine or exocrine glands, fibroblasts, myocytes, cardiomyocytes, and endothelial cells. The cell can be an individual cell of an embryo, and can be a blastocyte or an oocyte.

Certain methods are conducted using model systems for particular cellular states (e.g., a disease). For instance, certain methods provided herein are conducted with a cancer cell lines that serves as a model system for investigating genes that are correlated with various cancers.

A number of options can be utilized to deliver the dsRNA into a cell or population of cells such as in a cell culture, tissue or embryo. For instance, RNA can be directly introduced intracellularly. Various physical methods are generally utilized in such instances, such as administration by microinjection (see, e.g., Zemicka-Goetz, et al. (1997) Development 124:1133-1137; and Wianny, et al. (1998) Chromosoma 107: 430-439).

Other options for cellular delivery include permeabilizing the cell membrane and electroporation in the presence of the dsRNA, liposome-mediated transfection, or transfection using chemicals such as calcium phosphate. A number of established gene therapy techniques can also be utilized to introduce the dsRNA into a cell. By introducing a viral construct within a viral particle, for instance, one can achieve efficient introduction of an expression construct into the cell and transcription of the RNA encoded by the construct.

If the dsRNA is to be introduced into an organism or tissue, gene gun technology is an option that can be employed. This generally involves immobilizing the dsRNA on a gold particle which is subsequently fired into the desired tissue. Research has also shown that mammalian cells have transport mechanisms for taking in dsRNA (see, e.g., Asher, et al. (1969) Nature 223:715-717). Consequently, another delivery option is to administer the dsRNA extracellularly into a body cavity, interstitial space or into the blood system of the mammal for subsequent uptake by such transport processes. The blood and lymph systems and the cerebrospinal fluid are potential sites for injecting dsRNA. Oral, topical, parenteral, rectal and intraperitoneal administration are also possible modes of administration.

The composition introduced can also include various other agents in addition to the dsRNA. Examples of such agents include, but are not limited to, those that stabilize the dsRNA, enhance cellular uptake and/or increase the extent of interference. Typically, the dsRNA is introduced in a buffer that is compatible with the composition of the cell into which the RNA is introduced to prevent the cell from being shocked. The minimum size of the dsRNA that effectively achieves gene silencing can also influence the choice of delivery system and solution composition.

Sufficient dsRNA is introduced into the tissue to cause a detectable change in expression of a taget gene (assuming the candidate gene is in fact being expressed in the cell into which the dsRNA is introduced) using available detection methodologies. Thus, in some instances, sufficient dsRNA is introduced to achieve at least a 5-10% reduction in candidate gene expression as compared to a cell in which the dsRNA is not introduced. In other instances, inhibition is at least 20, 30, 40 or 50%. In still other instances, the inhibition is at least 60, 70, 80, 90 or 95%. Expression in some instances is essentially completely inhibited to undetectable levels.

The amount of dsRNA introduced depends upon various factors such as the mode of administration utilized, the size of the dsRNA, the number of cells into which dsRNA is administered, and the age and size of an animal if dsRNA is introduced into an animal. An appropriate amount can be determined by those of ordinary skill in the art by initially administering dsRNA at several different concentrations for example, for example. In certain instances when dsRNA is introduced into a cell culture, the amount of dsRNA introduced into the cells varies from about 0.5 to 3 µg per 10⁶ cells.

A number of options are available to detect interference of candidate gene expression (i.e., to detect candidate gene silencing). In general, inhibition in expression is detected by detecting a decrease in the level of the protein encoded by the candidate gene, determining the level of mRNA transcribed from the gene and/or detecting a change in phenotype associated with candidate gene expression.

### USE OF POLYPEPTIDES TO SCREEN FOR PEPTIDE ANALOGS AND ANTAGONISTS

Polypeptides encoded by differentially expressed genes identified herein can be used to screen peptide libraries to identify binding partners, such as receptors, from among the encoded polypeptides. Peptide libraries can be synthesized according to methods known in the art (*see, e.g.,* USPN 5,010,175 and WO 91/17823).

Agonists or antagonists of the polypeptides described herein can be screened using any available method known in the art, such as signal transduction, antibody binding, receptor binding, mitogenic assays, chemotaxis assays, etc. The assay conditions ideally should resemble the conditions under which the native activity is exhibited *in vivo,* that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide can require concentrations equal to or greater than the native concentration, while inhibitors capable of binding irreversibly to the polypeptide can be added in concentrations on the order of the native concentration.

Such screening and experimentation can lead to identification of a polypeptide binding partner, such as a receptor, encoded by a gene or a cDNA corresponding to a polynucleotide described herein, and at least one peptide agonist or antagonist of the binding partner. Such agonists and antagonists can be used to modulate, enhance, or inhibit receptor function in cells to which the receptor is native, or in cells that possess the receptor as a result of genetic engineering. Further, if the receptor shares biologically important characteristics with a known receptor, information about agonist/antagonist binding can facilitate development of improved agonists/antagonists of the known receptor.

### VACCINE AND USES

The differentially expressed nucleic acids and polypeptides produced by the nucleic acids described herein can also be used to modulate primary immune response to prevent or treat cancer. Every immune response is a complex and intricately regulated sequence of events involving several cell types. It is triggered when an antigen enters the body and encounters a specialized class of cells called antigen-presenting cells (APCs). These APCs capture a minute amount of the antigen and display it in a form that can be recognized by antigen-specific helper T lymphocytes. The helper (Th) cells become activated and, in turn, promote the activation of other classes of lymphocytes, such as B cells or cytotoxic T cells. The activated lymphocytes then proliferate and carry out their specific effector functions, which in many cases successfully activate or eliminate the antigen. Thus, activating the immune response to a particular antigen associated with a cancer cell can protect the patient from developing cancer or result in lymphocytes eliminating cancer cells expressing the antigen.

Gene products, including polypeptides, mRNA (particularly mRNAs having distinct secondary and/or tertiary structures), cDNA, or complete gene, can be prepared and used in vaccines for the treatment or prevention of hyperproliferative disorders and cancers. The nucleic acids and polypeptides can be utilized to enhance the immune response, prevent tumor progression, prevent hyperproliferative cell growth, and the like. Methods for selecting nucleic acids and polypeptides that are capable of enhancing the immune response are known in the art. Preferably, the gene products for use in a vaccine are gene products which are present on the surface of a cell and are recognizable by lymphocytes and antibodies.

The gene products may be formulated with pharmaceutically acceptable carriers into pharmaceutical compositions by methods known in the art. The composition is useful as a vaccine to prevent or treat cancer. The composition may further comprise at least one co-immunostimulatory molecule, including but not limited to one or more major histocompatibility complex (MHC) molecules, such as a class I or class II molecule, preferably a class I molecule. The composition may further comprise other stimulator molecules including B7.1, B7.2, ICAM-1, ICAM-2, LFA-1, LFA-3, CD72 and the like, immunostimulatory polynucleotides (which comprise an 5'-CG-3' wherein the cytosine is unmethylated), and cytokines which include but are not limited to IL-1 through IL-15, TNF-a, IFN-γ, RANTES, G-CSF, M-CSF, IFN-α, CTAP III, ENA-78, GRO, I-309, PF-4, IP-10, LD-78, MGSA, MIP-1α, MIP-1β, or combination thereof, and the like for immunopotentiation. In one embodiment, the imrnunopotentiators of particular interest are those that facilitate a Th1 immune response.

The gene products may also be prepared with a carrier that will protect the gene products against rapid elimination from the body, such as a controlled release formulation, including implants and microencapsulated delivery systems. Biodegradable polymers can be used, such as ethylene vinyl acetate, polyanhydrides, polyglycolic acid, collagen, polyorthoesters, polylactic acid, and the like. Methods for preparation of such formulations are known in the art.

In the methods of preventing or treating cancer, the gene products may be administered via one of several routes including but not limited to transdermal, transmucosal, intravenous, intramuscular, subcutaneous, intradermal, intraperitoneal, intrathecal, intrapleural, intrauterine, rectal, vaginal, topical, intratumor, and the like. For transmucosal or transdermal administration, penetrants appropriate to the barrier to be permeated are used in the formulation. Such penetrants are generally known in the art, and include, for example, administration bile salts and fusidic acid derivatives. In addition, detergents may be used to facilitate permeation. Transmucosal administration may be by nasal sprays or suppositories. For oral administration, the gene products are formulated into conventional oral administration form such as capsules, tablets, elixirs and the like.

The gene product is administered to a patient in an amount effective to prevent or treat cancer. In general, it is desirable to provide the patient with a dosage of gene product of at least about 1 pg per Kg body weight, preferably at least about 1 ng per Kg body weight, more preferably at least about 1 µg or greater per Kg body weight of the recipient. A range of from about 1 ng per Kg body weight to about 100 mg per Kg body weight is preferred although a lower or higher dose may be administered. The dose is effective to prime, stimulate and/or cause the clonal expansion of antigen-specific T lymphocytes, preferably cytotoxic T lymphocytes, which in turn are capable of preventing or treating cancer in the recipient. The dose is administered at least once and may be provided as a bolus or a continuous administration. Multiple administrations of the dose over a period of several weeks to months may be preferable. Subsequent doses may be administered as indicated.

In another method of treatment, autologous cytotoxic lymphocytes or tumor infiltrating lymphocytes may be obtained from a patient with cancer. The lymphocytes are grown in culture, and antigen-specific lymphocytes are expanded by culturing in the presence of the specific gene products alone or in combination with at least one co-immunostimulatory molecule with cytokines. The antigen-specific lymphocytes are then infused back into the patient in an amount effective to reduce or eliminate the tumors in the patient. Cancer vaccines and their uses are further described in USPN 5,961,978; USPN 5,993,829; USPN 6,132,980; and WO 00/38706.

### PHARMACEUTICAL COMPOSITIONS AND USES

Pharmaceutical compositions can comprise polypeptides, receptors that specifically bind a polypeptide produced by a differentially expressed gene (*e.g.*, antibodies, or polynucleotides (including antisense nucleotides and ribozymes) described herein in a therapeutically effective amount. The compositions can be used to treat primary tumors as well as metastases of primary tumors. In addition, the pharmaceutical compositions can be used in conjunction with conventional methods of cancer treatment, *e.g*., to sensitize tumors to radiation or conventional chemotherapy.

Where the pharmaceutical composition comprises a receptor (such as an antibody) that specifically binds to a gene product encoded by a differentially expressed gene, the receptor can be coupled to a drug for delivery to a treatment site or coupled to a detectable label to facilitate imaging of a site comprising breast cancer cells. Methods for coupling antibodies to drugs and detectable labels are well known in the art, as are methods for imaging using detectable labels.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature.

The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation is determined by routine experimentation and is within the judgment of the clinician. For purposes of the present invention, an effective dose will generally be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, lipid aggregates and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles.

Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington: The Science and Practice of Pharmacy (1995) Alfonso Gennaro, Lippincott, Williams, & Wilkins.

### DELIVERY METHODS

Once formulated, the compositions contemplated by the invention can be (1) administered directly to the subject (*e.g*., as polynucleotide, polypeptides, small molecule agonists or antagonists, and the like); or (2) delivered ex vivo, to cells derived from the subject (*e.g.,* as in *ex vivo* gene therapy). Direct delivery of the compositions will generally be accomplished by parenteral injection, e.g., subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the ex vivo delivery and reimplantation of transformed cells into a subject are known in the art and described in *e.g*., International Publication No. WO 93/14778. Examples of cells useful in ex vivo applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once differential expression of a gene corresponding to a polynucleotide described herein has been found to correlate with a proliferative disorder, such as neoplasia, dysplasia, and hyperplasia, the disorder can be amenable to treatment by administration of a therapeutic agent based on the provided polynucleotide, corresponding polypeptide or other corresponding molecule (e.g., antisense, ribozyme, etc.). In other embodiments, the disorder can be amenable to treatment by administration of a small molecule drug that, for example, serves as an inhibitor (antagonist) of the function of the encoded gene product of a gene having increased expression in cancerous cells relative to normal cells or as an agonists for gene products that are decreased in expression in cancerous cells (*e.g*., to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic composition agents includes local or systemic administration, including injection, oral administration, particle gun or catheterized administration, and topical administration. In general, the therapeutic polynucleotide composition contains an expression construct comprising a promoter operably linked to a polynucleotide of at least 12, 22, 25, 30, or 35 contiguous nt of the polynucleotide disclosed herein. Various methods can be used to administer the therapeutic composition directly to a specific site in the body. For example, a small metastatic lesion is located and the therapeutic composition injected several times in several different locations within the body of the tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. The antisense composition is directly administered to the surface of the tumor, for example, by topical application of the composition. X-ray imaging is used to assist in certain of the above delivery methods.

Targeted delivery of therapeutic compositions containing an antisense polynucleotide, subgenomic polynucleotides, or antibodies to specific tissues can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. (USA) (1990) 87:3655; Wu et al., J. Biol. Chem. (1991) 266:338. Therapeutic compositions containing a polynucleotide are administered in a range of about 100 ng to about 200 mg of DNA for local administration in a gene therapy protocol. Concentration ranges of about 500 ng to about 50 mg, about 1 µg to about 2 mg, about 5 µg to about 500 µg, and about 20 µg to about 100 µg of DNA can also be used during a gene therapy protocol. Factors such as method of action (e.g., for enhancing or inhibiting levels of the encoded gene product) and efficacy of transformation and expression are considerations that will affect the dosage required for ultimate efficacy of the antisense subgenomic polynucleotides.

Where greater expression is desired over a larger area of tissue, larger amounts of antisense subgenomic polynucleotides or the same amounts readministered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect. For polynucleotide related genes encoding polypeptides or proteins with anti-inflammatory activity, suitable use, doses, and administration are described in USPN 5,654,173.

The therapeutic polynucleotides and polypeptides described herein can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., WO 90/07936; WO 94/03622; WO 93/25698; WO 93/25234; USPN 5,219,740; WO 93/11230; WO 93/10218; USPN 4,777,127; GB Patent No. 2,200,651; EP 0 345 242; and WO 91/02805), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532), and adeno-associated virus (AAV) vectors (see, e.g., WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., USPN 5,814,482; WO 95/07994; WO 96/17072; WO 95/30763; and WO 97/42338) and nucleic charge neutralization or fusion with cell membranes. Naked DNA can also be employed. Exemplary naked DNA introduction methods are described in WO 90/11092 and USPN 5,580,859. Liposomes that can act as gene delivery vehicles are described in USPN 5,422,120; WO 95/13796; WO 94/23697; WO 91/14445; and EP 0524968. Additional approaches are described in Philip, Mol. Cell Biol. (1994) 14:2411, and in Woffendin, Proc. Natl. Acad. Sci. (1994) 91:1581.

Further non-viral delivery suitable for use includes mechanical delivery systems such as the approach described in Woffendin et al., Proc. Natl. Acad. Sci. USA (1994) 91(24):11581. Moreover, the coding sequence and the product of expression of such can be delivered through deposition of photopolymerized hydrogel materials or use of ionizing radiation (see, e.g., USPN 5,206,152 and WO 92/11033). Other conventional methods for gene delivery that can be used for delivery of the coding sequence include, for example, use of hand-held gene transfer particle gun (see, e.g., USPN 5,149,655); use of ionizing radiation for activating transferred gene (see, e.g., USPN 5,206,152 and WO 92/11033).

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### EXAMPLE 1:

### SOURCE OF BIOLOGICAL MATERIALS

The cells used for detecting differential expression of breast cancer related genes were those previously described for the HMT-3522 tumor reversion model, disclosed in U.S. Patent Nos. 5,846,536 and 6,123,941. The model utilizes both non-tumorigenic (HMT-3522 S1) and tumorigenic (HMT-3522 T4-2) cells derived by serial passaging from a single reduction mammoplasty. In two dimensional (2D) monolayers on plastic, both S 1 and T4-2 cells display similar morphology. But in three dimensional (3D) matrigel cultures, S1 form phenotypically normal mammary tissue structures while T4-2 cells fail to organize into these structures and instead disseminate into the matrix. This assay was designated as a tumor reversion model, in that the T4-2 cells can be induced to form S1-like structures in 3D by treatment with beta-1 integrin or EGFR blocking antibodies, or by treating with a chemical inhibitor of the EGFR signaling pathway (tyrophostin AG 1478). These treated T4-2 cells, called T4R cells, are non-tumorigenic.

### EXAMPLE 2:

### CELL GROWTH AND RNA ISOLATION

Growth of Cells 2D and 3D for Microarray Experiments: HMT3522 S1 and T4-2 cells were grown 2D and 3D and T4-2 cells reverted with anti-EGFR, anti-beta 1 integrin, or tyrophostin AG 1478 as previously described (Weaver et al J Cell Biol. 137:231-45, 1997; and Wang et al PNAS 95:14821-14826, 1998). Anti-EGFR (mAb 225) was purchased from Oncogene and introduced into the matrigel at the time of gelation at a concentration of 4 ug/ml purified mouse IgG1. Anti-beta 1 integrin (mAb AIIB2) was a gift from C. Damsky at the University of California at San Francisco and was also introduced into the matrigel at the time of gelation at a concentration of 100 ug/ml ascites protein (which corresponds to 4-10 ug/ml purified rat IgG1). Tyrophostin AG 1478 was purchased from Calbiochem and used at a concentration of 100 nM.

Isolation of RNA for Microarray Experiments: RNA was prepared from: S 1 passage 60 2D cultures; T4-2 passage 41 2D cultures; S1 passage 59 3D cultures; and T4-2 and T4-2 revertant (with anti-EGFR, anti-beta 1 integrin, and tyrophostin) passage 35 3D cultures.

All RNA for microarray experiments was isolated using the commercially available RNeasy Mini Kit from Qiagen. Isolation of total RNA from cells grown 2D was performed as instructed in the kit handbook. Briefly, media was aspirated from the cells and kit Buffer RLT was added directly to the flask. The cell lysate was collected with a rubber cell scraper, and the lysate passed 5 times through a 20-G needle fitted to a syringe. One volume of 70% ethanol was added to the homogenized lysate and mixed well by pipetting. Up to 700 ul of sample was applied to an RNeasy mini spin column sitting in a 2-ml collection tube and centrifuged for 15 seconds at >8000 x g. 700 ul Buffer RW1 was added to the column and centrifuged for 15 seconds at >8000 x g to wash. The column was transferred to a new collection tube. 500 ul Buffer RPE was added to the column and centrifuged for 15 seconds at >8000 x g to wash. Another 500 ul Buffer RPE was added to the column for additional washing, and the column centrifuged for 2 minutes at maximum speed to dry. The column was transferred to a new collection tube and RNA eluted from the column with 30 ul RNase-free water by centrifuging for 1 minute at >8000 x g.

Isolation of total RNA from cells grown 3D was performed as described above, except cells were isolated from matrigel prior to RNA isolation. The cells were isolated as colonies from matrigel using ice-cold PBS/EDTA (0.01 M sodium phosphate pH 7.2 containing 138 mM sodium chloride and 5 mM EDTA). See Weaver et al, J Cell Biol 137:231-245, 1997; and Wang et al. PNAS 95:14821-14826, 1998.

### EXAMPLE 3:

### DETECTION AND IDENTIFICATION OF GENES EXHIBITING DIFFERENTIAL EXPRESSION

The relative expression levels of a selected sequence (which in turn is representative of a single transcript) were examined in the tumorigenic versus non-tumorigenic cell lines described above, following culturing of the cells (S1, T4-2 and T4R) in either two-dimensional (2D) monolayers or three-dimensional (3D) matrigel cultures as described above. Differential expression for a selected sequence was assessed by hybridizing mRNA from S1 and T4-2 2D cultures, and S 1, T4-2 and T4R 3D cultures to microarray chips as described below, as follows: Exp1 = T4-2 2D/S1 2D; Exp2 = T4-2 3D/S 3D; Exp3 = S1 3D/S1 2D; Exp4 = T4-2 3D/T4-2 2D; Exp5 = T4-2 3D/T4R (anti-EGFR) 3D; Exp6 = T4-2 3D/T4R (anti-betal integrin) 3D; and Exp7 = T4-2 3D/T4R (tyrophostin AG 1478) 3D.

Each array used had an identical spatial layout and control spot set. Each microarray was divided into two areas, each area having an array with, on each half, twelve groupings of 32 x 12 spots for a total of about 9,216 spots on each array. The two areas are spotted identically which provide for at least two duplicates of each clone per array. Spotting was accomplished using PCR amplified products from 0.5kb to 2.0 kb and spotted using a Molecular Dynamics Gen III spotter according to the manufacturer's recommendations. The first row of each of the 24 regions on the array had about 32 control spots, including 4 negative control spots and 8 test polynucleotides.

The test polynucleotides were spiked into each sample before the labeling reaction with a range of concentrations from 2-600 pg/slide and ratios of 1:1. For each array design, two slides were hybridized with the test samples reverse-labeled in the labeling reaction. This provided for about 4 duplicate measurements for each clone, two of one color and two of the other, for each sample.

Identification Of Differentially Expressed Genes: "Differentially expressed" in the context of the present example meant that there was a difference in expression of a particular gene between tumorigenic vs. non-tumorigenic cells, or cells grown in three-dimensional culture vs. cells grown in two-dimensional culture. To identify differentially expressed genes, total RNA was first reverse transcribed into cDNA using a primer containing a T7 RNA polymerase promoter, followed by second strand DNA synthesis. cDNA was then transcribed *in vitro* to produce antisense RNA using the T7 promoter-mediated expression (see, *e.g.,* Luo et al. (1999) Nature Med 5:117-122), and the antisense RNA was then converted into cDNA. The second set of cDNAs were again transcribed i*n vitro,* using the T7 promoter, to provide antisense RNA. Optionally, the RNA was again converted into cDNA, allowing for up to a third round of T7-mediated amplification to produce more antisense RNA. Thus the procedure provided for two or three rounds of *in vitro* transcription to produce the final RNA used for fluorescent labeling.

Fluorescent probes were generated by first adding control RNA to the antisense RNA mix, and producing fluorescently labeled cDNA from the RNA starting material. Fluorescently labeled cDNAs prepared from tumorigenic RNA sample were compared to fluorescently labeled cDNAs prepared from non-tumorigenic cell RNA sample. For example, the cDNA probes from the non-tumorigenic cells were labeled with Cy3 fluorescent dye (green) and the cDNA probes prepared from the tumorigenic cells were labeled with Cy5 fluorescent dye (red).

The differential expression assay was performed by mixing equal amounts of probes from tumorigenic cells and non-tumorigenic cells, and/or cells grown in 3D vs. those grown in 2D. The arrays were prehybridized by incubation for about 2 hrs at 60°C in 5X SSC/0.2% SDS/1 mM EDTA, and then washed three times in water and twice in isopropanol. Following prehybridization of the array, the probe mixture was then hybridized to the array under conditions of high stringency (overnight at 42°C in 50% formamide, 5X SSC, and 0.2% SDS). After hybridization, the array was washed at 55°C three times as follows: 1) first wash in 1X SSC/0.2% SDS; 2) second wash in 0.1 X SSC/0.2% SDS; and 3) third wash in 0.1X SSC.

The arrays were then scanned for green and red fluorescence using a Molecular Dynamics Generation III dual color laser-scanner/detector. The images were processed using BioDiscovery Autogene software, and the data from each scan set normalized to provide for a ratio of expression relative to non-tumorigenic or tumorigenic cells grown two-dimensionally or three-dimensionally. Data from the microarray experiments was analyzed according to the algorithms described in U.S. application serial no. 60/252,358, filed November 20, 2000, by E.J. Moler, M.A. Boyle, and F.M. Randazzo, and entitled "Precision and accuracy in cDNA microarray data".

The experiment was repeated, this time labeling the two probes with the opposite color in order to perform the assay in both "color directions." Each experiment was sometimes repeated with two more slides (one in each color direction). The level fluorescence for each sequence on the array expressed as a ratio of the geometric mean of 8 replicate spots/genes from the four arrays or 4 replicate spots/gene from 2 arrays or some other permutation. The data were normalized using the spiked positive controls present in each duplicated area, and the precision of this normalization was included in the final determination of the significance of each differential. The fluorescence intensity of each spot was also compared to the negative controls in each duplicated area to determine which spots have detected significant expression levels in each sample.

A statistical analysis of the fluorescent intensities was applied to each set of duplicate spots to assess the precision and significance of each differential measurement, resulting in a p-value testing the null hypothesis that there is no differential in the expression level between the tumorigenic and non-tumorigenic cells or cells grown two-dimensionally versus three-dimensionally. During initial analysis of the microarrays, the hypothesis was accepted if p>10⁻³, and the differential ratio was set to 1.000 for those spots. All other spots have a significant difference in expression between the two samples compared. For example, if the tumorigenic sample has detectable expression and the non-tumorigenic does not, the ratio is truncated at 1000 since the value for expression in the non-tumorigenic sample would be zero, and the ratio would not be a mathematically useful value (e.g., infinity). If the non-tumorigenic sample has detectable expression and the tumorigenic does not, the ratio is truncated to 0.001, since the value for expression in the tumor sample would be zero and the ratio would not be a mathematically useful value. These latter two situations are referred to herein as "on/off." Database tables were populated using a 95% confidence level (p>0.05).

In general, a polynucleotide is said to represent a significantly differentially expressed gene between two samples when there is detectable levels of expression in at least one sample and the ratio value is greater than at least about 1.2 fold, at least about 1.5 fold, or at least about 2 fold, where the ratio value is calculated using the method described above.

A differential expression ratio of 1 indicates that the expression level of the gene in tumorigenic cells was not statistically different from expression of that gene in the specific non-tumorigenic cells compared. A differential expression ratio significantly greater than 1 in tumorigenic breast cells relative to non-tumorigenic breast cells indicates that the gene is increased in expression in tumorigenic cells relative to non-tumorigenic cells, suggesting that the gene plays a role in the development of the tumorigenic phenotype, and may be involved in promoting metastasis of the cell. Detection of gene products from such genes can provide an indicator that the cell is cancerous, and may provide a therapeutic and/or diagnostic target. Likewise, a differential expression ratio significantly less than 1 in tumorigenic breast cells relative to non-tumorigenic breast cells indicates that, for example, the gene is involved in suppression of the tumorigenic phenotype. Increasing activity of the gene product encoded by such a gene, or replacing such activity, can provide the basis for chemotherapy. Such gene can also serve as markers of cancerous cells, *e.g*., the absence or decreased presence of the gene product in a breast cell relative to a non-tumorigenic breast cell indicates that the cell is cancerous.

Using the above methodology, three hundred and sixty-seven (367) genes or products thereof were identified from 20,000 chip clones analyzed as being overexpressed 2-fold or more in one or more of these experiments, with a p-value of 0.001 or less. These identified genes or products thereof are listed in Table 1, according to the Spot ID of the spotted polynucleotide, the Sample ID, the corresponding GenBank Accession Number (No.), the GenBank description (if available) for the corresponding Genbank Accession Number, and the GenBank score (p-value; the probability that the association between the SEQ ID NO. and the gene or product thereof occurred by chance).

The differential hybridization results from the seven differential expression microarray experiments listed above are provided in Table 2, where sequences have a measurement corresponding to its ratio of expression in the 7 experiments, e.g. spot ID 10594 is 2.2-fold overexpressed in 3D T4-2 cells as compared to 3D S1 cells. SEQ ID NOS:1-499, representing the sequences corresponding to the spot Ids listed in Tables 1 and 2 are provided in the sequence listing. Table 11 is a lookup table showing the relationship between the spot Ids (i.e. the nucleic acids spotted on the microarray) and the sequences provided in the sequence listing.

The following Examples 4, 5, and 6 are not embodiments of the invention but represent examples which are useful for understanding the invention.

### EXAMPLE 4

### CYCLING ASSOCIATED KINASE (GAK)

A gene or product thereof called cyclin G associated kinase, or GAK, was identified as being overexpressed in 3D T4-2 cultures relative to both 3D S1 cultures (ratio: 7.9296) and 2D T4-2 cultures (ratio: 34.6682) (Sample ID RG:1056692:10012:C11, Spot ID 19990). GAK corresponds to Genbank Accession number XM_003450.

### EXAMPLES 5

### ANTISENSE REGULATION OF GAK EXPRESSION

Additional functional information on GAK was generated using antisense knockout technology. A number of different oligonucleotides complementary to GAK mRNA were designed (AS) with corresponding controls (RC): GGAATCACCGCTTTGCCATCTTCAA (SEQ ID NO:500; CHIR159-1AS, gak:P1868AS), AACTTCTACCGTTTCGCCACTAAGG (SEQ ID NO:501; CHIR159-1RC, gak:P1868RC); GACCGTGTACTGCGTGTCGTGCG (SEQ ID NO:502; CHIR159-7AS, gak:P0839AS) and GCGTGCTGTGCGTCATGTGCCAG (SEQ ID NO:502; CHIR159-7RC, gak:P0839RC), and tested for their ability to suppress expression of GAK in human malignant colorectal carcinoma SW620 cells, human breast cancer MDA231 cells, and human breast cancer T4-2 cells. For each transfection mixture, a carrier molecule, preferably a lipitoid or cholesteroid, was prepared to a working concentration of 0.5 mM in water, sonicated to yield a uniform solution, and filtered through a 0.45 pm PVDF membrane. The antisense or control oligonucleotide was then prepared to a working concentration of 100 µM in sterile Millipore water. The oligonucleotide was further diluted in OptiMEM^{™} (GibcoBRL), in a microfuge tube, to 2 µM, or approximately 20 µg oligo/ml of OptiMEM^{™}. In a separate microfuge tube, lipitoid or cholesteroid, typically in the amount of about 1.5-2 nmol lipitoid/µg antisense oligonucleotide, was diluted into the same volume of OptiMEM^{™} used to dilute the oligonucleotide. The diluted antisense oligonucleotide was immediately added to the diluted lipitoid and mixed by pipetting up and down. Oligonucleotide was added to the cells to a final concentration of 300 nM.

The level of target mRNA (GAK) in the transfected cells was quantitated in the cancer cell lines using the methods using primers CHIR159_2896 (GCCGTCTTCAGGCAACAACTCCCA; SEQ ID NO: 504; forward) and CHIR159_3089 (TGCTGGACGAGGCTGTCATCTTGC; SEQ ID NO:505; reverse). RNA was extracted as above according to manufacturer's directions.

Quantitative PCR (qPCR) was performed by first isolating the RNA from the above mentioned tissue/cells using a Qiagen RNeasy mini prep kit. A total of 0.5 micrograms of RNA was used to generate a first strand cDNA using Stratagene MuLV Reverse Transcriptase, using recommended concentrations of buffer, enzyme, and Rnasin. Concentrations and volumes of dNTP, and oligo dT, or random hexamers were lower than recommended to reduce the level of background primer dimerization in the qPCR.

The cDNA is then used for qPCR to determine the levels of expression of GAK using the GeneAmp 7000 by ABI as recommended by the manufacturer. Primers for actin were also used in order to normalized the values, and eliminate possible variations in cDNA template concentrations, pipetting error, etc.

For each 20 µl reaction, extracted RNA (generally 0.2-1 µg total) was placed into a sterile 0.5 or 1.5 ml microcentrifuge tube, and water was added to a total volume of 12.5 µl. To each tube was added 7.5 µl of a buffer/enzyme mixture, prepared by mixing (in the order listed) 2.5 µl H₂O, 2.0 µl 10X reaction buffer, 10 µl oligo dT (20 pmol), 1.0 µl dNTP mix (10 mM each), 0.5 µl RNAsin® (20u) (Ambion, Inc., Hialeah, FL), and 0.5 µl MMLV reverse transcriptase (50u) (Ambion, Inc.). The contents were mixed by pipetting up and down, and the reaction mixture was incubated at 42°C for 1 hour. The contents of each tube were centrifuged prior to amplification.

An amplification mixture was prepared by mixing in the following order: 1X PCR buffer II, 3 mM MgCl₂, 140 µM each dNTP, 0.175 pmol each oligo, 1:50,000 dil of SYBR® Green, 0.25 mg/ml BSA, 1 unit *Taq* polymerase, and H₂O to 20 µl. (PCR buffer II is available in 10X concentration from Perkin-Elmer, Norwalk, CT). In 1X concentration it contains 10 mM Tris pH 8.3 and 50 mM KCI. SYBR® Green (Molecular Probes, Eugene, OR) is a dye which fluoresces when bound to double stranded DNA. As double stranded PCR product is produced during amplification, the fluorescence from SYBR® Green increases. To each 20 µl aliquot of amplification mixture, 2 µl of template RT was added, and amplification was carried out according to standard protocols.

Table 3 shows that the antisense oligonucleotides described above reduced expression of GAK mRNA as compared to controls in all three cell lines. GAK mRNA reduction ranged from about 50% to about 90%, as compared to cells transfected with reverse (i.e. sense) control oligonucleotides.

**Table 3: antisense regulation of GAK mRNA**

| Oligo | Cell Line | Gene Message | Actin Message | Ratio | Percent KO |
|---|---|---|---|---|---|
| CHIR159-1AS | SW620 | 0.0923 | 0.669 | 0.138 | 90.7 |
| CHIR159-1RC | SW620 | 1.01 | 0.680 | 1.49 | |
| CHIR159-7AS | SW620 | 0.0555 | 0.678 | 0.082 | 85.4 |
| CHIR159-7RC | SW620 | 0.335 | 0.598 | 0.560 | |
| CHIR159-1AS | MDA231 | 0.358 | 0.687 | 0.521 | 59.3 |
| CHIR159-1RC | MDA231 | 1.00 | 0.784 | 1.28 | |
| CHIR159-7AS | MDA231 | 0.262 | 0.674 | 0.389 | 69.4 |
| CHIR159-7RC | MDA231 | 0.840 | 0.659 | 1.27 | |
| CHIR159-1AS | T4-2 | 0.307 | 0.707 | 0.434 | 72.9 |
| CHIR159-1RC | T4-2 | 1.23 | 0.770 | 1.60 | |
| CHIR159-7AS | T4-2 | 0.214 | 0.649 | 0.330 | 49.8 |
| CHIR159-7RC | T4-2 | 0.506 | 0.770 | 0.657 | |

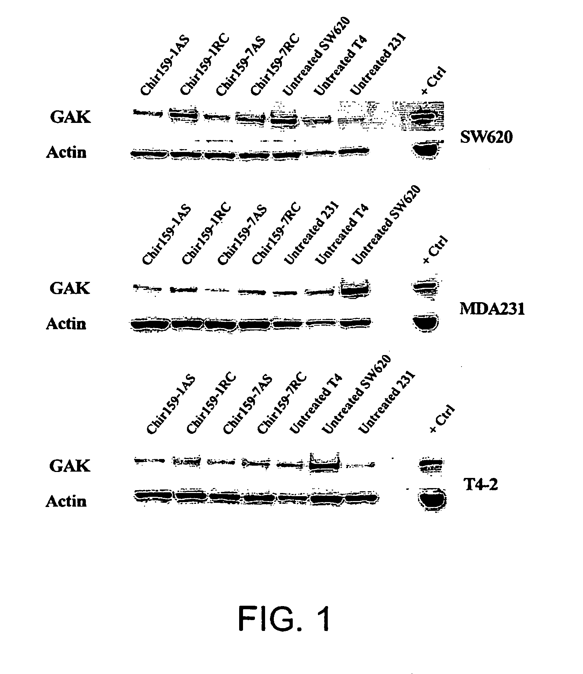

Reduction of GAK protein by antisense polynucleotides in SW620, MDA231 and T4-2 was confirmed using an antibody that specifically recognizes GAK. Figure 1 shows a western (i.e. protein) blot of protein extracts of the above cell lines decorated with anti-GAK antibodies. GAK protein expression is reduced in cell lines receiving GAK antisense oligonucleotides.

### EXAMPLE 6

### ROLE OF GAK IN ANCHORAGE INDEPENDENT CELL GROWTH

The effect of GAK gene expression upon anchorage-independent cell growth of SW620 and MBA-231 cells was measured by colony formation in soft agar. Soft agar assays were performed by first coating a non-tissue culture treated plate with PolyHEMA to prevent cells from attaching to the plate. Non-transfected cells were harvested using 0.05% trypsin and washing twice in media. The cells are counted using a hemacytometer and resuspended to 10⁴ per ml in media. 50 µl aliquots are placed in poly-HEMA coated 96-well plates and transfected. For each transfection mixture, a carrier molecule, preferably a lipitoid or cholesteroid, was prepared to a working concentration of 0.5 mM in water, sonicated to yield a uniform solution, and filtered through a 0.45 µm PVDF membrane. The antisense or control oligonucleotide was then prepared to a working concentration of 100 µM in sterile Millipore water. The oligonucleotide was further diluted in OptiMEM^{™} (Gibco/BRL), in a microfuge tube, to 2 µM, or approximately 20 µg oligo/ml of OptiMEM^{™}. In a separate microfuge tube, lipitoid or cholesteroid, typically in the amount of about 1.5-2 nmol lipitoid/µg antisense oligonucleotide, was diluted into the same volume of OptiMEM^{™} used to dilute the oligonucleotide. The diluted antisense oligonucleotide was immediately added to the diluted lipitoid and mixed by pipetting up and down. Oligonucleotide was added to the cells to a final concentration of 300 nM. Following transfection (∼30 minutes), 3% GTG agarose is added to the cells for a final concentration of 0.35% agarose. After the cell layer agar solidifies, 100 µl of media is dribbled on top of each well. Colonies form in 7 days. For a read-out of growth, 20 µl of Alamar Blue is added to each well and the plate is shaken for 15 minutes. Fluorecence readings (530nm excitation 590 nm emission) are taken after incubation for 6-24 hours.

The data presented in Table 4 shows that the application of GAK antisense oligonucleotides to SW620 and MDA 231 cells results in inhibition of colony formation and shows that GAK plays a role in production anchorage-independent cell growth. Table 4 shows the average fluorescence reading for several experiments. The standard deviation (St. Dev) of the fluorescence reading and coefficient of variation (%CV) is also shown.

**Table 4: GAK and anchorage-independent cell growth.**

| Oligo | Cell Line | Average | St. Dev | %CV |
|---|---|---|---|---|
| Blank | SW620 | 12868.17 | 208.78 | 1.78 |
| Untreated | SW620 | 31075.17 | 1944.36 | 7.66 |
| Pos Control | SW620 | 5717.17 | 1108.71 | 23.75 |
| Neg Control | SW620 | 7576.17 | 465.95 | 7.63 |
| Chir159-1AS | SW620 | 9701.5 | 2281.36 | 28.8 |
| Chir159-1RC | SW620 | 17765.5 | 1958.45 | 13.5 |
| Blank | MDA231 | 12726.83 | 232.45 | 2 |
| Untreated | MDA231 | 87272.17 | 0 | 0 |
| Pos Control | MDA231 | 10645.17 | 1591.08 | 18.31 |
| Neg Control | MDA231 | 24159.5 | 2850.58 | 14.45 |
| Chir159-1AS | MDA231 | 8613.5 | 4852.76 | 69 |
| Chir159-1RC | MDA231 | 17859.17 | 1535.55 | 10.53 |

### EXAMPLE 7

### DKFZP566I133 (DKFZ)

Several previously uncharacterized genes were identified as being induced in these experiments. One such gene was represented by two spots, Spot ID Nos 22793 and 26883 (gene assignment DKFZp566I133). This gene was expressed at a ratio of about 2.2 in two 2-dimensional (2D) T4-2 vs. 2D S1 experiments, and also at a ratio of about 2 when 3-dimensional (3D) T4-2 cells were compared to the various tumor reversion cultures. However, the ratio of expression increased to an average of 3.2 when 3-dimensional (3D) T4-2 cultures were compared to 2D S1 cultures. In contrast, there was essentially no difference in expression levels when 3D S1 cultures were compared to 2D S1 cultures, suggesting that expression of this gene is specifically elevated in the tumorigenic cell line T4-2, and even further elevated when the tumorigenic cell line is grown in three dimensional cultures (see Table 5).

**Table 5.**

| Spot | 2D T4-2/ | 3D T42/ | 3D S1/ | 3D T4-2 | 3D T4-2/EGFRAb | 3D T4-2/B1 integrin | 3D T4-2/ |
|---|---|---|---|---|---|---|---|
| ID | 2D S1 | 3D S1 | 2D S1 | /2D T4-2 | | Ab | Tyr |
| 22893 | 1.90387 | 2.64711 | 0.522161 | 1 | 2.17956 | 1.75287 | 2.055538 |
| 26883 | 2.43428 | 3.74613 | 0.524466 | 1 | 2.467573 | 2.029468 | 2.002817 |

These array data were confirmed by qPCR using the methods described above and the gene specific PCR primers CHIR180_1207 ACAGGGAGAAAACTGGTTGTCCTGG (SEQ ID NO:506; Forward) and CHIR180_1403 AAGGCAGAACCCATCCACTCCAA (SEQ ID NO:507; Reverse). Independent cultures were used for these experiments, and data was normalized to B-catenin. These data are shown in Table 6.

**Table 6.**

| 2D S1 | 2D T4-2 | 3D S1 | 3D T4-2 | 3D EGFR Ab | 3D B1 Integrin Ab | 3D Tyr |
|---|---|---|---|---|---|---|
| 0.165 | 0.421 | 0.14 | 0.475 | 0.231 | 0.175 | 0.174 |

DKFZ corresponds to Genbank Accession numbers NP_112200, AAH09758, and NM_030938. Orthologs of DKFZ are identified in species other than Homo sapiens include NM_138839 from *Rattus norvegicus.*

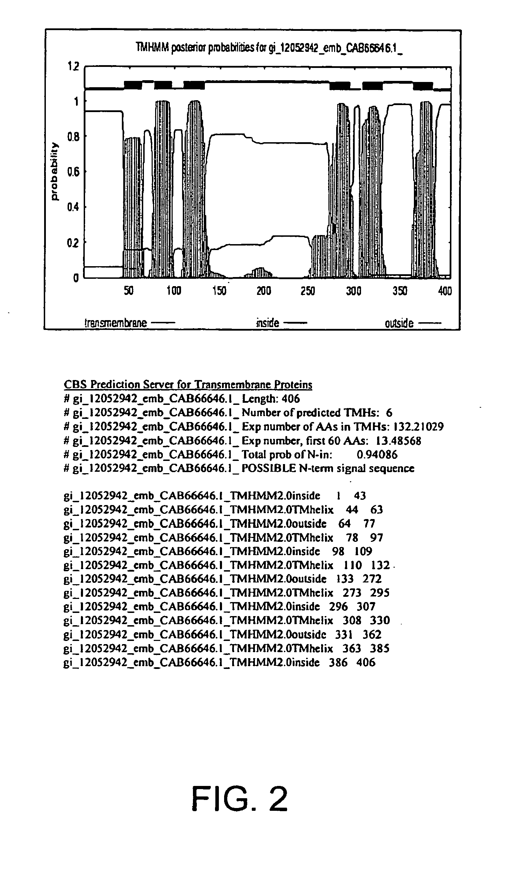

Analysis of the sequence of DKFZ using a transmembrane helix prediction algorithm (Sonhammer, et al, A hidden Markov model for predicting transmembrane helices in protein sequences, In Proc. of Sixth Int. Conf. on Intelligent Systems for Molecular Biology, p. 175-82, Ed. J. Glasgow, T. Littlejohn, F. Major, R. Lathrop, D. Sankoff, and C. Sensen, Menlo Park, CA: AAAI Press, 1998) indicates that the DKFZ protein has six transmembrane regions (Fig.2), and, as such, is likely to be a transmembrane protein.

### EXAMPLE 8

### ANTISENSE REGULATION OF DKFZ EXPRESSION

Additional functional information on DKFZ was generated using antisense knockout technology. A number of different oligonucleotides complementary to DKFZ mRNA were designed (AS) with corresponding controls (RC): GCTGCTGGATTCGTTTGGCATAACT (SEQ IDNO: 508; CHIR180-7AS, DKFZp566I1:P1301AS), TCAATACGGTTTGCTTAGGTCGTCG (SEQ ID NO:509; CHIR180-7RC, DKFZp566I1:P1301RC), TCTCCTCTGAGTTCAACCGCTGCT (SEQ ID NOS:510; CHIR180-BAS, DKFZp566I1:P1320AS) and TCGTCGCCAACTTGAGTCTCCTCT (SEQ ID NO:511; CHIR180-BRC, DKFZp566I1:P1320AS), and tested for their ability to suppress expression of DKFZ in human malignant colorectal carcinoma SW620 cells, human breast cancer MDA231 cells, and human breast cancer T4-2 cells, as described above.

Table 7 shows that the antisense (AS) oligonucleotides described above reduced expression of DKFZ mRNA as compared to controls in all three cell lines. DKFZ mRNA reduction ranged from about 95% to about 99%, as compared to cells transfected with reverse (i.e. sense) control (RC) oligonucleotides.

**Table 7: antisense regulation of DKFZ mRNA**

| Oligo | Cell Line | Gene Message | Actin Message | Ratio | Percent KO |
|---|---|---|---|---|---|
| CHIR180-7AS | SW620 | 0.0157 | 0.772 | 0.020 | 99.3 |
| CHIR180-7RC | SW620 | 1.99 | 0.736 | 2.70 | |
| CHIR180-8AS | SW620 | 0.0387 | 0.681 | 0.057 | 97.9 |
| CHIR180-8RC | SW620 | 1.89 | 0.703 | 2.69 | |
| CHIR180-7AS | MDA231 | 0.0471 | 3.58 | 0.013 | 98.5 |
| CHIR180-7RC | MDA231 | 1.99 | 2.33 | 0.854 | |
| CHIR180-8AS | MDA231 | 0.00935 | 1.74 | 0.00537 | 99.5 |
| CHIR180-8RC | MDA231 | 1.14 | 1.01 | 1.13 | |
| CHIR180-7AS | T4-2 | 0.119 | 0.667 | 0.178 | 95.4 |
| CHIR180-7RC | T4-2 | 2.8 | 0.728 | 3.85 | |
| CHIR180-8AS | T4-2 | 0.0852 | 0.751 | 0.113 | 95.6 |
| CHIR180-8RC | T4-2 | 1.6 | 0.620 | 2.58 | |

### EXAMPLE 9

### EFFECT OF DKFZ EXPRESSION ON CELL PROLIFERATION

The effect of gene expression on the inhibition of cell proliferation was assessed in metastatic breast cancer cell line MDA-231 and breast cancer cell line T4-2.

Cells were plated to approximately 60-80% confluency in 96-well dishes. Antisense or reverse control oligonucleotide was diluted to 2 µM in OptiMEM^{™} and added to OptiMEM^{™} into which a delivery vehicle, preferably a lipitoid or cholesteroid, had been diluted. The oligo/delivery vehicle mixture was then further diluted into medium with serum on the cells. The final concentration of oligonucleotide for all experiments was 300 nM, and the final ratio of oligo to delivery vehicle for all experiments was 1.5 nmol lipitoid/µg oligonucleotide.

Antisense oligonucleotides were prepared. Cells were transfected for 4 hours or overnight at 37°C and the transfection mixture was replaced with fresh medium. Plates are incubated for 4 days, with a plate harvested for each day0-day4. To determine differences in cell number, a CyQuant Cell Proliferation Assay kit (Molecular Probes) was used per manufacturer's instructions. Fluorecence readings (480nm excitation 520 nm emission) are taken after incubation for 5 minutes.

The results of these assays are shown in Tables 7A and 8. The data show that DKFZ antisense polynucleotides significantly reduce cell proliferation as compared to controls, and, as such, DKFZ plays a role in production or maintenance of the cancerous phenotype in cancerous breast cells.

**Table 7A:**

| Cell proliferation | | | | | | |
|---|---|---|---|---|---|---|
| Oligo | Cell Line | Ave Day0 | Ave Day1 | Ave Day2 | Av3 Day3 | Ave Day4 |
| Untreated | MDA231 | 4233 | 4858 | 9544 | 10981 | 16776 |
| Untreated | MDA231 | 3849 | 4036 | 8686 | 9855 | 14865 |
| Pos Control | MDA231 | 3630 | 2236 | 3564 | 4536 | 7477 |
| Neg Control | MDA231 | 4913 | 5127 | 8331 | 8887 | 13620 |
| CHIR180-7AS | MDA231 | 3848 | 3476 | 6942 | 8715 | 11925 |
| CHIR180-7RC | MDA231 | 4895 | 4700 | 8484 | 10318 | 14226 |
| Untreated | T4-2 | 4062 | 3389 | 5438 | 10579 | 15617 |
| Untreated | T4-2 | 4209 | 3802 | 6346 | 11802 | 16275 |
| Pos Control | T4-2 | 3985 | 2712 | 4081 | 6404 | 9685 |
| Neg Control | T4-2 | 4051 | 3901 | 4356 | 9425 | 12964 |
| CHIR180-7AS | T4-2 | 3792 | 3201 | 3849 | 7376 | 10911 |
| CHIR180-7RC | T4-2 | 3967 | 3840 | 4321 | 8382 | 12293 |

**Table 8**

| | Standard Deviations | | | | | P-Value of T-Test | | | | |
|---|---|---|---|---|---|---|---|---|---|---|
| Oligo | Day 0 | Day 1 | Day 2 | Day 3 | Day 4 | Day0 | Day1 | Day2 | Day3 | Day4 |
| Untreated | 337 | 269 | 299 | 697 | 1333 | 0.1306 | 0.1063 | 0.1804 | 0.0926 | 0.1225 |
| Untreated | 99 | 631 | 867 | 547 | 1047 | 0.1306 | 0.1063 | 0.1804 | 0.0926 | 0.1225 |
| Pos Control | 94 | 118 | 89 | 441 | 974 | 0.0000 | 0.0001 | 0.0003 | 0.0001 | 0.0010 |
| Neg Control | 2 | 252 | 697 | 195 | 780 | 0.0000 | 0.0001 | 0.0003 | 0.0001 | 0.0010 |
| CHIR180-7 AS | 292 | 16 | 435 | 398 | 418 | 0.0072 | 0.0276 | 0.0059 | 0.0140 | 0.0028 |
| CHIRI80-7 RC | 208 | 6 | 244 | 533 | 440 | 0.0072 | 0.0276 | 0.0059 | 0.0140 | 0.0028 |
| Untreated | 64 | 283 | 789 | 1593 | 1226 | 0.2550 | 0.0921 | 0.1257 | 0.2794 | 0.4352 |
| Untreated | 22 | 158 | 205 | 577 | 478 | 0.2550 | 0.0921 | 0.1257 | 0.2794 | 0.4352 |
| Pos Control | 122 | 213 | 6 | 475 | 957 | 0.4320 | 0.0065 | 0.2624 | 0.0051 | 0.0293 |
| Neg Control | 47 | 335 | 464 | 809 | 1417 | 0.4320 | 0.0065 | 0.2624 | 0.0051 | 0.0293 |
| CHIR180-7 AS | 170 | 679 | 263 | 127 | 1330 | 0.2638 | 0.0976 | 0.3516 | 0.0040 | 0.0039 |
| CHIR180-7 RC | 22 | 453 | 646 | 579 | 884 | 0.2638 | 0.0976 | 0.3516 | 0.0040 | 0.0039 |

### EXAMPLE 10

### ROLE OF DKFZ IN ANCHORAGE INDEPENDENT CELL GROWTH

The effect of DKFZ gene expression upon anchorage-independent cell growth of MDA435 and MCF7 human breast cancer cells was measured by colony formation in soft agar. Soft agar assays were conducted by the method described for GAK, above.

The data presented in Table 9 shows that the application of DKFZ antisense oligonucleotides to MDA435 and MCF7 cells results in inhibition of colony formation and shows that DKFZ plays a role in anchorage-independent cell growth of cancer cells. Table 9 shows the average fluorescence reading for several experiments. The standard deviation (St. Dev) of the fluorescence reading and coefficient of variation (%CV) and probability (P-value) is also shown.

**Table 9**

| Oligo | Cell Line | Average | St. Dev | %CV | P-Value |
|---|---|---|---|---|---|
| Untreated | MDA435 | 31190 | 5838 | 19 | 0.1342 |
| Untreated | MDA435 | 38623 | 3620 | 9 | 0.1342 |
| Pos Control | MDA435 | 4776 | 818 | 17 | 0.0156 |
| Neg Control | MDA435 | 16315 | 481 | 3 | 0.0156 |
| Chir180-7AS | MDA435 | 21161 | 3439 | 16 | 0.0274 |
| Chir180-7RC | MDA435 | 28868 | 1902 | 7 | 0.0274 |
| Untreated | MCF7 | 18954 | 1478 | 8 | 0.1476 |
| Untreated | MCF7 | 14383 | 4163 | 29 | 0.1476 |
| Pos Control | MCF7 | 1036 | 194 | 19 | 0.0036 |
| Neg Control | MCF7 | 9478 | 2382 | 25 | 0.0036 |
| Chir180-7AS | MCF7 | 4752 | 2002 | 42 | 0.0139 |
| Chir180-7RC | MCF7 | 9570 | 18 | 0 | 0.0139 |

The effect of DKFZ gene expression upon invasiveness of MDA231 human breast cancer cells was measured by a matrigel assay. A 3-dimensional reconstituted basement membrane culture of cells was generated as described previously (Peterson et al., (1992) Proc. Natl. Acad. Sci. USA 89:9064-9068) using a commercially prepared reconstituted basement membrane (Matrigel; Collaborative Research, Waltham, MA) and examined using methods well known in the art.

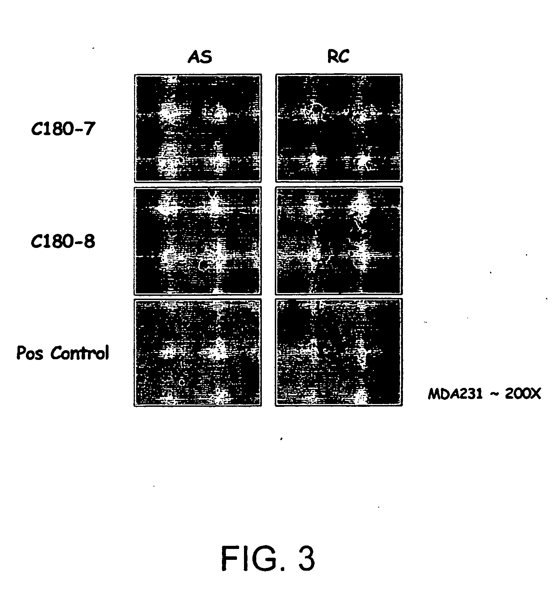

Table 10 (quantitated using Alamar Blue similar to the soft agar assay) and Figure 3 provides exemplary results of the Matrigel invasion/motility assay to test the invasiveness of MDA231 cells with reduced expression of DKFZ. In general, these data show that a reduction in the expression of DKFZ significantly decreases the invasiveness of MDA231 cells.

**Table 10:**

| Oligo | Cell Line | Average | St. Dev | %CV | P-Value |
|---|---|---|---|---|---|
| Untreated | MDA231 | 28316 | 13663 | 48 | 6.9080 |
| Untreated | MDA231 | 26840 | 15669 | 58 | 0.9080 |
| Pos Control | MDA231 | 2756 | 487 | 18 | 0.0002 |
| Neg Control | MDA231 | 14301 | 1386 | 10 | 0.0002 |
| Chir180-7AS | MDA231 | 10508 | 1963 | 19 | 0.0287 |
| **Chir180-7RC** | MDA231 | 14310 | 153 | 1 | 0.0287 |

### EXAMPLE 11

### EXPRESSION OF DKFZ IN CANCER TISSUES

The following peptides were used for polyclonal antibody production: peptide 809: gvhqqyvqriek (SEQ ID NO:380), corresponding to amino acids 97-108 of the DKFZ protein and peptide 810: sgaepddeeyqef (SEQ ID NO:381), corresponding to amino acids 215-227 of the DKFZ protein.

Antibodies specific for DKFZ are used in FACS and immunolocalization analysis to show that DKFZ is associated with membrane, and up-regulated in cancer tissues of biopsies from cancer patients.

Further, antibodies specific for DKFZ are used to modulate DKFZ activity in cancerous breast, and is further used, alone or conjugated to a toxic moiety, as a treatment for breast cancer.

**Table 1**

| SPOTID | SAMPLE ID | GENBANK NO | GENBANK DESCRIPTION | GENBANK SCORE |
|---|---|---|---|---|
| 10594 | I:1871362:05B01:A04 | M62994 | Homo sapiens thyroid autoantigen (truncated actin-binding protein) mRNA, complete cds | 8.6E-36 |
| 21851 | M00055153A:A12 | | | |
| 20990 | I:1986550:13B02:G12 | XM 005667 | Homo sapiens lipocalin 2 (oncogene 24p3) (LCN2), mRNA | 0 |
| 18641 | I:3473302:09A01:A09 | AB046098 | Macaca fascicularis brain cDNA, clone:QccE-15843 | 5.8E-57 |
| 17229 | I:1506962:09A01:G01 | AL365454 | Homo sapiens mRNA full length insert cDNA clone EUROIMAGE 926491 | 2.6E-110 |
| 25930 | 035JN020.F01 | AJ010446 | Homo sapiens mRNA for immunoglobulin kappa light chain,anti-RhD, therad 24 | 0 |
| 20701 | RG:730349:10010:G12 | U28387 | Human hexokinase II pseudogene, complete cds | 0 |
| 20346 | RG:1839794:10015:E11 | U28387 | Human hexokinase II pseudogene, complete cds | 0 |
| 21247 | M00054680C:A06 | U28387 | Human hexokinase II pseudogene, complete cds | 9,9E-80 |
| 23062 | M00056353C:E10 | XM 011013 | Homo sapiens filamin B, beta (actin-binding protein-278) (FLNB), mRNA | 0 |
| 25666 | 035Jn031.B01 | AF191633 | Homo sapiens filamin (FLNB) gene, exon 48 and complete cds | 0 |
| 19001 | I:2171401:09A02:E09 | AF123887 | Homo sapiens ERO1L (ERO1L) mRNA, partial cds | 3.3E-104 |
| 10897 | I:1852047:02A01:A10 | U22384 | Human lysyl oxidase gene, partial cds | 0 |
| 1960 | M00023297B:A10 | M33376 | Human pseudo-chlordecone reductase mRNA, complete cds | 0 |
| 26381 | 035JN029.H02 | AB037838 | Homo sapiens mRNA for KIAA1417 protein, partial cds | 0 |
| 26719 | 035JN030.A02 | X68277 | H.sapiens CL 100 mRNA for protein tyrosine phosphatase | 0 |
| 27152 | 037XN007.A09 | XM 048479 | Homo sapiens hypothetical protein FLJ14642 (FLJ14642), mRNA | 7.3E-58 |
| 10926 | I:2047770:08B02:G04 | AK000969 | Homo sapiens cDNA FLJ10107 fis, clone HEMBA1002583 | 3.8E-94 |
| 28980 | 035JN003.C12 | XM 027456 | Homo sapiens hypothetical gene supported by AK000584 (LOC89942), mRNA | 0 |
| 1236 | M00022024A:F02 | | | |
| 29350 | 035JN008,D06 | XM 043864 | Homo sapiens phosphoinositide-3-kinase, regulatory subunit, polypeptide 1 (p85 alpha) (PIK3R1), mRNA | 0 |
| 26242 | 035JN015.B02 | AL137717 | Homo sapiens mRNA; cDNA DKFZp434J1630 (from clone DKF2p434J1630) | 2.6E-70 |
| 4098 | M00001439D:C09 | BC002446 | Homo sapiens, MRJ gene for a member of the DNAJ protein family, clone MGC:1152 IMAGE:3346070, mRNA, complete cds | 0 |
| 17432 | I:1965049:16B02:D07 | XM 051165 | Homo sapiens DKFZP586A0522 protein (DKFZP586A0522), mRNA | 0 |
| 1785 | SL198 | XM 051165 | Homo sapiens DKFZP586A0522 protein (DKFZP586A0522), mRNA | 0 |
| 28856 | 035JN032.E11 | X62996 | H.sapiens mitochondrial genome (consensus sequence) | 0 |
| 18791 | RG:229957:10007:D03 | D42042 | Human mRNA for KIAA0085 gene, partial cds | 0 |
| 22950 | M00056922C:C09 | | | |
| 1882 | M000221968:D09 | Z29083 | H.sapiens 5T4 gene for 5T4 Oncofetal antigen | 0 |
| 23886 | M00055408A:F10 | | | |
| 24995 | M00055215C:E11 | XM 012880 | Homo sapiens hypothetical protein MGC1936 (MGC1936), mRNA | 0 |
| 24477 | M00055510B:F08 | AF240697 | Homo sapiens retinol dehydrogenase homolog isoform-2 (RDH) mRNA, complete cds | 0 |
| 21681 | M00056771C:A12 | X02152 | Human mRNA for lactate dehydrogenase-A (LDH-A, EC 1.1.1.27) | 0 |
| 9557 | I:1335140:05A02:C08 | X02152 | Human mRNA for lactate dehydrogenase-A (LDH-A, EC 1.1.1.27) | 0 |
| 22033 | M00056574B:A07 | | | |
| 873 | M00007979C:C05 | X00663 | Human mRNA fragment for epidermal growth factor (EGF) receptor | 0 |
| 17144 | RG:25254:10004:D07 | M97675 | Human transmembrane receptor (ror1) mRNA, complete cds | 0 |
| 26970 | 035JN015.F09 | AF097514 | Homo sapiens stearoyl-CoA desaturase (SCD) mRNA, complete cds | 0 |
| 21402 | M00054507C: D07 | | | |
| 27074 | 035Jn031.B03 | AF061741 | Homo sapiens retinal short-chain dehydrogenase/reductase retSDR1 mRNA, complete cds | 0 |
| 10963 | I:1258790:05A02:B10 | AF072752 | Homo sapiens ten integrin EGF-like repeat domains protein precursor (ITGBL1) mRNA, complete cds | 0 |
| 29525 | 035JN026.D12 | | | |
| 25514 | 035JN011.F01 | U62961 | Human succinyl CoA:3-oxoacid CoA transferase precursor (OXCT) mRNA, complete cds | 0 |
| 26612 | 035JN016.C08 | NM 000240 | Homo sapiens monoamine oxidase A (MAOA), nuclear gene encoding mitochondrial protein, mRNA | 0 |
| 24600 | M00055490C:G11 | U57059 | Homo sapiens Apo-2 ligand mRNA, complete cds | 0 |
| 9741 | I:3126828:12A02:G02 | U37518 | Human TNF-related apoptosis inducing ligand TRAIL mRNA, complete cds 0 | |
| 23689 | M00054752A:E11 | XM 001468 | Homo sapiens S100 calcium-binding protein A10 (annexin II ligand, calpactin I, light polypeptide (p11)) (S100A10), mRNA | 0 |
| 22352 | M00042842B:E02 | XM 001468 | Homo sapiens S100 calcium-binding protein A10 (annexin II ligand, calpactin I, light polypeptide (p11)) (S100A10), mRNA | 0 |
| 23806 | RG:2007319:20003:G10 | | | |
| 12285 | I:1404669:04A01:G12 | BC002517 | Homo sapiens, Pirin, clone MGC:2083 IMAGE:3140037, mRNA, complete cds | 0 |
| 27638 | 035JN011.D10 | AK002155 | Homo sapiens cDNA FLJ11293 fis, clone PLACE1009670, highly similar to Homo sapiens genethonin 1 mRNA | 0 |
| 9663 | 1:2488567:11A02:H08 | XM 006027 | Homo sapiens brain-derived neurotrophic factor (BDNF), mRNA | 0 |
| 26850 | 035JN003.803 | XM 031551 | Homo sapiens similar to carbohydrate (N-acetylglucosamine-6-O) sulfotransferase 2 (H. sapiens) (LOC90414), mRNA | 0 |
| 10204 | I:1491445:02B01:F09 | AF131765 | Homo sapiens clone 24833 nonsyndromic hearing impairment protein mRNA sequence, complete cds | 0 |
| 1318 | 2192-6 | | | |
| 25922 | 035JN020.B01 | AB020673 | Homo sapiens mRNA for KIAA0866 protein, complete cds | 0 |
| 26347 | 035JN025.G02 | | | |
| 20361 | I:395116:17A02: E05 | | | |
| 28672 | 035JN012.A05 | AF126181 | Homo sapiens breast cancer-associated gene 1 protein (BCG1) mRNA, complete cds | 0 |
| 25520 | 035JN011.A07 | D86956 | Human mRNA for KIAA0201 gene, complete cds | 0 |
| 1723 | M00005694A:A09 | BC001980 | Homo sapiens, clone IMAGE:3462291, mRNA | 0 |
| 28863 | 037XN002.A05 | | | |
| 25526 | 035JN011.D07 | AF086281 | Homo sapiens full length insert cDNA clone ZD45G11 | 0 |
| 27936 | 035JN008.A04 | X59445 | H.sapiens mRNA for colon carcinoma Manganese Superoxide Dismutase | 0 |
| 26851 | 035JN001.C03 | XM 033944 | Homo sapiens superoxide dismutase 2, mitochondrial (SOD2), mRNA | 0 |
| 25107 | M00054825A:E04 | AF075061 | Homo sapiens full length insert cDNA YP07G10 | 0 |
| 24912 | M00054505D:D06 | AF075061 | Homo sapiens full length insert cDNA YP07G10 | 0 |
| 25169 | M00055510D:D04 | M11167 | Human 28S ribosomal RNA gene | 1.2E-76 |
| 25600 | 035JN023.A01 | BC003107 | Homo sapiens, inhibitor of DNA binding 3, dominant negative helix-loop-helix protein, clone MGC:1988 IMAGE:3543936, mRNA, complete | 0 |
| 28706 | 035JN016.805 | X55181 | Human ETS2 gene, 3'end | 0 |
| 26377 | 035JN029.F02 | Y14436 | Homo sapiens mRNA for phosphatidic acid phosphatase type 2 | 0 |
| 19460 | I:438655:14B02:B04 | AF007133 | Homo sapiens clone 23764 mRNA sequence | 4.5E-113 |
| 25243 | RG:1667183:10014:F12 | BC000013 | Homo sapiens, insulin-like growth factor binding protein 3, clone MGC:2305 IMAGE:3506666, mRNA, complete cds | 0 |
| 20018 | I:1213574:17B01:A11 | AB037925 | Homo sapiens MAIL mRNA, complete cds | 3.7E-106 |
| 918 | M00026895D:H03 | BC006433 | Homo sapiens, Ras-related GTP-binding protein, clone MGC:13077 IMAGE:3835186, mRNA, complete cds | 0 |
| 25027 | RG:1983823:20002:806 | | | |
| 29089 | 035JN017.B06 | XM 037534 | Homo sapiens phosphodiesterase 7A (PDE7A), mRNA | 0 |
| 9141 | I:1347384:02A02:C07 | U78579 | Human type I phosphatidylinositol-4-phosphate 5-kinase beta (STM7) mRNA, partial cds | 0 |
| 12005 | I:1259230:05A01:C06 | D87075 | Human mRNA for KIAA0238 gene, partial cds | 0 |
| 12148 | I:3360476:03B01:B12 | XM 040922 | Homo sapiens interleukin 13 receptor, alpha 2 (IL13RA2), mRNA | 0 |
| 17394 | RG:1943755:10016:A07 | AF346607 | Homo sapiens interleukin-1 receptor associated kinase 1b (IRAK) mRNA, complete cds, alternatively spliced | 0 |
| 27017 | 035JN021.F03 | XM 051742 | Homo sapiens spermine synthase (SMS), mRNA | 0 |
| 25809 | 035JN002.807 | XM 009699 | Homo sapiens nuclear receptor interacting protein 1 (NRIP1), mRNA | 0 |
| 8719 | I:2600080:10A01:H01 | XM 009665 | Homo sapiens Kreisler (mouse) maf-related leucine zipper homolog (KRML), mRNA | 0 |
| 21030 | RG:1714832:10015:C06 | XM 029957 | Homo sapiens Rab acceptor 1 (prenylated) (RABAC1), mRNA | 0 |
| 11436 | I:1470085:03B01:F05 | XM 038976 | Homo sapiens N-ethylmaleimide-sensitive factor attachment protein, alpha (NAPA), mRNA | 0 |
| 10374 | I:1513989:03B02:C03 | XM 009010 | Homo sapiens complement component 3 (C3), mRNA | 1.4E.96 |
| 19037 | I:417827:15A01:G10 | X79538 | H.sapiens nuk_34 mRNA for translation initiation factor | 1.9E-28 |
| 398 | M00027016A:C05 | XM 031470 | Homo sapiens aldolase C, fructose-bisphosphate (ALDOC), mRNA | 4E-62 |
| 18773 | I:1211682:14A02:C09 | XM 008477 | Homo sapiens aldolase C, fructose-bisphosphate (ALDOC), mRNA | 0 |
| 3583 | M00023407B:C10 | | | |
| 3418 | M00001470A:C03 | XM 043951 | Homo sapiens CDP-diacylglycerol-inositol 3-phosphatidyltransferase (phosphatidylinositol synthase) (CDIPT), mRNA | 0 |
| 18985 | I:1402615:09A02:E03 | AF191148 | Homo sapiens type I transmembrane protein Fn14 mRNA, complete cds | 7.9E-64 |
| 25861 | 035JN010.D01 | XM 047975 | Homo sapiens hydroxyacyl glutathione hydrolase (HAGH), mRNA | 0 |
| 3317 | M00003974D:E04 | AF136185 | Homo sapiens collagen type XVII (COL17A1) gene, 3' UTR, long form | 0 |
| 8743 | I:1858905:04A01:D01 | U36775 | Human ribonuclease 4 gene, partial cds | 2.1E-57 |
| 26240 | 035JN015.A02 | XM 007493 | Homo sapiens ribonuclease, RNase A family, 4 (RNASE4), mRNA | 0 |
| 28562 | 037XND07.B11 | X00947 | Human alpha 1-antichymotrypsin gene fragment | 0 |
| 16877 | I:2362945:15A01:C07 | XM 029378 | Homo sapiens checkpoint suppressor 1 (CHES1), mRNA | 1.9E-91 |
| 25955 | 035JN022.C01 | AF035620 | Homo sapiens BRCA1-associated protein 2 (BRAP2) mRNA, complete cds | 0 |
| 26308 | 035JN023.C02 | XM 041470 | Homo sapiens zinc finger protein 145 (Kruppel-like, expressed in promyelocytic leukemia) (ZNF145), mRNA | 0 |
| 4140 | 2239-4 | X03083 | Human lactate dehydrogenase-A gene exon 7 and 3' flanking region | 0 |
| 3436 | 2239-1 | X03083 | Human lactate dehydrogenase-A gene exon 7 and 3' flanking region | 0 |
| 25612 | 035JN023.G01 | M94856 | Human fatty acid binding protein homologue (PA-FABP) mRNA, complete cds | 0 |
| 12257 | I:1448135:04A01:A06 | X15535 | H.sapiens lysosomal acid phosphatase gene (EC 3.1.3.2) Exon 11 | 0 |
| 9111 | I:1958902:04A02:D07 | D87258 | Homo sapiens mRNA for serin protease with IGF-binding motif, complete cds | 0 |
| 17620 | I:875567:15B01:B08 | XM 045326 | Homo sapiens MAX-interacting protein 1 (MXI1), mRNA | 0 |
| 26025 | 035JN030,F01 | XM 032511 | Homo sapiens procollagen-proline, 2-oxoglutarate 4-dioxygenase (proline 4-hydroxylase), alpha polypeptide I (P4HA1), mRNA | 0 |
| 19271 | RG:686684:10010:D04 | AF005216 | Homo sapiens receptor-associated tyrosine kinase (JAK2) mRNA, complete cds | 0 |
| 4151 | 2035-1 | D87953 | Human mRNA for RTP, complete cds | 0 |
| 26569 | 035JN010.F02 | AB004788 | Homo sapiens mRNA for BNIP3L, complete cds | 0 |
| 10344 | I:2859338:11 B02:003 | XM 005052 | Homo sapiens angiopoietin 1 (ANGPT1), mRNA | 1.3E-97 |
| 832 | M00021649B:D05 | XM 004628 | Homo sapiens hypoxia-inducible protein 2 (HIG2), mRNA | 0 |
| 12071 | I:1798283:06A01:D06 | S72481 | pantophysin [human, keratinocyte line HaCaT, mRNA, 2106 nt] | 0 |
| 12271 | I:1445767:04A01:H06 | X12701 | H.sapiens mRNA for endothelial plasminogen activator inhibitor PAI | 1.8E-130 |
| 11433 | I:1526282:03A01:E05 | XM 033627 | Homo sapiens glycoprotein (transmembrane) nmb (GPNMB), mRNA | 3.7E-117 |
| 20917 | RG:222350:10007:C12 | X00663 | Human mRNA fragment for epidermal growth factor (EGF) receptor | 1.7E-122 |
| 25810 | 035JN004.B07 | X00588 | Human mRNA for precursor of epidermal growth factor receptor | 0 |
| 12039 | I:3506985:07A01:D06 | M24795 | Human CD36 antigen mRNA, complete cds | 0 |
| 25499 | 035JN005.G07 | XM 028224 | Homo sapiens N-acetylglucosamine-phosphate mutase (AGM1), mRNA | 0 |
| 25557 | 035JN013.007 | BC010135 | Homo sapiens, cyclin C, clone IMAGE:4106819, mRNA | 0 |
| 9917 | I:1283532:05A01:G09 | XM 004148 | Homo sapiens 5T4 oncofetal trophoblast glycoprotein (5T4), mRNA | 2.4E-70 |
| 19505 | RG:204653:10007:A10 | XM 003789 | Homo sapiens colony stimulating factor 1 receptor, formerly McDonough feline sarcoma viral (v-fms) oncogene homolog (CSF1 R), mRNA | 0 |
| 17491 | RG:277866:10008:807 | XM 003789 | Homo sapiens colony stimulating factor 1 receptor, formerly McDonough feline sarcoma viral (v-fms) oncogene homolog (CSF1R), mRNA | 0 |
| 10683 | I:1686726:06A01:F10 | XM 003789 | Homo sapiens colony stimulating factor 1 receptor, formerly McDonough feline sarcoma viral (v-fms) oncogene homolog (CSF1R), mRNA | 0 |
| 1936 | M00008020C:H09 | X68277 | H.sapiens CL 100 mRNA for protein tyrosine phosphatase | 0 |
| 828 | M00021638B:F03 | X68277 | H.sapiens CL 100 mRNA for protein tyrosine phosphatase | 0 |
| 9558 | I:1824443:05B02:C08 | XM 003708 | Homo sapiens gamma-aminobutyric acid (GABA) A receptor, pi (GABRP), mRNA | 0 |
| 20164 | I:1997963:14B02:B05 | XM 003631 | Homo sapiens solute carrier family 25 (mitochondrial carrier, adenine nucleotide translocator), member 4 (SLC25A4), mRNA | 0 |
| 969 | NIH50_40026 | BC008664 | Homo sapiens, clone MGC:9281 IMAGE:3871960, mRNA, complete cds | 0 |
| 9910 | I:1805840:05B01:C09 | XM 003399 | Homo sapiens mannosidase, beta A, lysosomal (MANBA), mRNA | 0 |
| 2427 | M00005767D:B03 | XM 047441 | Homo sapiens RAP1, GTP-GDP dissociation stimulator 1 (RAP1GDS1), mRNA | 0 |
| 19990 | RG:1056692:10012:C111 | XM 003450 | Homo sapiens cyclin G associated kinase (GAK), mRNA | 0 |
| 20605 | I:690313:16A01:G12 | XM 011152 | Homo sapiens insulin-like growth factor binding protein 7 (IGFBP7), mRNA | 0 |
| 10650 | I:2456393:07BOI:E10 | AK001580 | Homo sapiens cDNA FLJ10718 fis, clone NT2RP3001096, weakly similar to Rattus norvegicus leprecan mRNA | 0 |
| 25963 | 035JN022.G01 | X53002 | Human mRNA for integrin beta-5 subunit | 0 |
| 25562 | 035JN015.F07 | X53002 | Human mRNA for integrin beta-5 subunit | 0 |
| 9377 | I:2782593:12A01:A02 | X60656 | H.sapiens mRNA for elongation factor 1-beta | 1.4E-46 |
| 17618 | I:707667:15B01:A08 | XM 002273 | Homo sapiens inhibitor of DNA binding 2, dominant negative helix-loop-helix protein (ID2), mRNA | 3.5E-117 |
| 12136 | I:3208994:03B01:D06 | U16267 | Human AMP deaminase isoform L, alternatively spliced (AMPD2) mRNA, exons 1A, 2 and 3, partial cds | 0 |
| 17373 | I:1538189:14A02:G07 | XM 046818 | Homo sapiens similar to receptor tyrosine kinase-like orphan receptor 1 (H. sapiens) (LOC92711), mRNA | 8.3E-123 |
| 18577 | RG:503209:10010:A09 | XM 049305 | Homo sapiens Lysosomal-associated multispanning membrane protein-5 (LAPTM5), mRNA | 0 |
| 3143 | M00001605D:C02 | BC003107 | Homo sapiens, inhibitor of DNA binding 3, dominant negative helix-loop-helix protein, clone MGC:1988 IMAGE:3543936, mRNA, complete | 1.7E-88 |
| 17737 | RG:155066:10006:E02 | AL050147 | Homo sapiens mRNA; cDNA DKFZp586E0820 (from clone DKFZp586E0820); partial cds | 0 |
| 20029 | I:1923613:17A01:G11 | AF113123 | Homo sapiens carbonyl reductase mRNA, complete cds | 0 |
| 18537 | NIH50_40304 | BC001380 | Homo sapiens, succinate dehydrogenase complex, subunit A, flavoprotein (Fp), clone MGC:1484 IMAGE:3051442, mRNA, complete cds | 0 |
| 10090 | NIH50_40304 | | | |
| 12102 | I:2832414:11B01:C06 | XM 048045 | Homo sapiens katanin p80 (WD40-containing) subunit B 1 (KATNB1), mRNA | 0 |
| 8487 | I:1375115:05A01:D01 | BC001174 | Homo sapiens, exostoses (multiple) 1, clone MGC:2129 IMAGE:3502232, mRNA, complete cds | 0 |
| 9252 | I:1673876:06B01:B02 | BC000917 | Homo sapiens, clone MGC:5184 IMAGE:3048750, mRNA, complete cds | 0 |
| 25605 | 035JN021.D01 | BC000671 | Homo sapiens, claudin 4, clone MGC:1778 IMAGE:3349211, mRNA, complete cds | 0 |
| 29652 | M00001610C:D05 | BC000588 | Homo sapiens, HIRA-interacting protein 3, clone MGC:1814 IMAGE:3345739, mRNA, complete cds | 0 |
| 10858 | I:2458933:04B01:E04 | X97544 | H.sapiens mRNA for TIM17 preprotein translocase | 8.7E-62 |
| 1261 | M00023419C:806 | U89606 | Human pyridoxal kinase mRNA, complete cds | 0 |
| 4156 | 2243-4 | X93334 | Homo sapiens mitochondrial DNA, complete genome | 0 |
| 3452 | 2243-1 | X93334 | Homo sapiens mitochondrial DNA, complete genome | 0 |
| 2748 | 2242-6 | X93334 | Homo sapiens mitochondrial DNA. complete genome | 0 |
| 2046 | 2248-3 | X93334 | Homo sapiens mitochondrial DNA, complete genome | 0 |
| 2044 | 2242-4 | X93334 | Homo sapiens mitochondrial DNA, complete genome | 0 |
| 1342 | 2248-2 | X93334 | Homo sapiens mitochondrial DNA, complete genome | 0 |
| 1326 | 2244-3 | X93334 | Homo sapiens mitochondrial DNA, complete genome | 0 |
| 9981 | I:1720149:06A01:G09 | AF069604 | Homo sapiens myosin light chain kinase isoform 4 (MLCK) mRNA, partial cds | 0 |
| 27917 | 035JN002.H04 | XM 015978 | Homo sapiens hypothetical protein FLJ22969 (FLJ22969), mRNA | 1.8E-92 |
| 8488 | I:1808529:05B01:D01 | AJ293647 | Homo sapiens partial IL4RA gene for interleukin-4 receptor alfa chain, exon 11, ECSSQV allele | 1.1E-125 |
| 22793 | M00057283C:D06 | AF161410 | Homo sapiens HSPC292 mRNA, partial cds | 0 |
| 26883 | 035JN005.C03 | AF161410 | Homo sapiens HSPC292 mRNA, partial cds | 0 |
| 11540 | I:1909488:10B01:B11 | XM 027739 | Homo sapiens duodenal cytochrome b (FLJ23462), mRNA | 0 |
| 17707 | I:489882:14A01:F02 | X99474 | H.sapiens mRNA for chloride channel, CIC-6c | 0 |
| 20649 | NIH50_41452 | Z14136 | H.sapiens gene for spermidine/spermine N1-acetyltransferase | 0 |
| 24004 | M00056163C:H09 | AF107495 | Homo sapiens FWP001 and putative FWP002 mRNA, complete cds | 0 |
| 11836 | I:1806769:01B02:F11 | X93036 | H.sapiens mRNA for MAT8 protein | 0 |
| 24932 | M00054963C:C09 | M26152 | Homo sapiens serum amyloid A (SAA) mRNA, complete cds | 0 |
| 19143 | RG:149960:10006:D04 | AK003448 | Mus musculus 18 days embryo cDNA, RIKEN full-length enriched library, clone:1110004P15, full insert sequence | 8.9E-21 |
| 26257 | 035JN013.B08 | J04056 | Human carbonyl reductase mRNA, complete cds | 0 |
| 21239 | M00054679B:B03 | J02619 | Human Z type alpha-1-antitrypsin gene, complete cds (exons 2-5) | 0 |
| 16959 | I:1426031:14B01:B07 | AY035783 | Homo sapiens laminin 5 beta 3 subunit (LAMB3) mRNA, complete cds | 3.8E-121 |
| 2568 | M00022158D:C11 | XM 036609 | Homo sapiens laminin, beta 3 (nicein (125kD), kalinin (140kD), BM600 (125kD)) (LAMB3), mRNA | 0 |
| 25936 | 035JN020.A07 | XM 036608 | Homo sapiens laminin, beta 3 (nicein (125kD). kalinin (140kD), BM600 (125kD)) (LAM83), mRNA | 0 |
| 23041 | M00054797C:G10 | XM 046649 | Homo sapiens nuclear factor of kappa light polypeptide gene enhancer in B-cells inhibitor, alpha (NFKBIA), mRNA | 0 |
| 9206 | I:1822716:05B01:C08 | BC008059 | Homo sapiens, clone IMAGE:2967491, mRNA | 0 |
| 25105 | M00054824C:H04 | BC009110 | Homo sapiens, clone MGC:17355 IMAGE:3453825, mRNA, complete cds | 0 |
| 24779 | M00057061D:G07 | | | |
| 22451 | M00043372B:B06 | X00947 | Human alpha 1-antichymotrypsin gene fragment | 0 |
| 22291 | M00054785D:G05 | X00947 | Human alpha 1-antichymotrypsin gene fragment | 0 |
| 21143 | M00055146A:D11 | | | |
| 24751 | M00054676B:D07 | X03083 | Human lactate dehydrogenase-A gene exon 7 and 3' flanking region | 0 |
| 24294 | M00056163D:E01 | X03083 | Human lactate dehydrogenase-A gene exon 7 and 3' flanking region | 9.4E-110 |
| 24006 | M00056163D:E01 | X03083 | Human lactate dehydrogenase-A gene exon 7 and 3' flanking region | 0 |
| 25678 | 035Jn031.H01 | AK001670 | Homo sapiens cDNA FLJ10808 fis, clone NT2RP4000879, weakly similar to UBIQUITIN-ACTIVATING ENZYME E1 | 4.9E-53 |
| 22027 | M00056534C:E08 | XM 003512 | Homo sapiens amphiregulin (schwannoma-derived growth factor) (AREG), mRNA | 0 |
| 29495 | 035JN022.E12 | D83761 | Homo sapiens mRNA for mother against dpp (Mad) related protein, complete cds | 0 |
| 24577 | M00056654B:G02 | XM 038306 | Homo sapiens dual specificity phosphatase 6 (DUSP6), mRNA | 0 |
| 23527 | M00055865C:D04 | | | |
| 17090 | I:341491:13B01:A01 | BC004490 | Homo sapiens, v-fos FBJ murine osteosarcoma viral oncogene homolog, clone MGC:11074 IMAGE:3688670, mRNA, complete cds | 3.8E-98 |
| 25137 | M00057167A:C07 | | | |
| 23772 | M00056360A:E07 | BC004490 | Homo sapiens, v-fos FBJ murine osteosarcoma viral oncogene homolog, clone MGC:11074 IMAGE:3688670, mRNA, complete cds | 0 |
| 1659 | M00001350B:D10 | BC004490 | Homo sapiens, v-fos FBJ murine osteosarcoma viral oncogene homolog, clone MGC:11074 IMAGE:3688670, mRNA, complete cds | 0 |
| 8497 | I:2170638:05A01:A07 | BC006169 | Homo sapiens, Similar to SH3-domain binding protein 5 (BTK-associated), clone MGC:13234 IMAGE:4025362, mRNA, complete cds | 5.2E-125 |
| 25272 | M00054621A:D09 | AF161435 | Homo sapiens HSPC317 mRNA, partial cds | 0 |
| 21216 | M00056194B:G06 | XM 002844 | Homo sapiens procollagen-lysine, 2-oxoglutarate 5-dioxygenase (lysine hydroxylase) 2 (PLOD2), mRNA | 0 |
| 11939 | I:2938757:02A02:B05 | D43767 | Human mRNA for chemokine, complete cds | 0 |
| 9191 | I:1421929:05A01:D02 | X63629 | H.sapiens mRNA for p cadherin | 2.4E-90 |
| 3429 | 2024-3 | AF002697 | Homo sapiens E1B 19K/Bcl-2-binding protein Nip3 mRNA. nuclear gene encoding mitochondrial protein, complete cds | 0 |
| 2725 | 2024-1 | AF002697 | Homo sapiens E1 B 19K/BcA-2-binding protein Nip3 mRNA, nuclear gene encoding mitochondrial protein, complete cds | 0 |
| 19923 | I:1001356:13A01:811 | BC006318 | Homo sapiens, erythrocyte membrane protein band 4.9 (dematin), clone MGC:12740 IMAGE:4125804, mRNA, complete cds | 1.7E-103 |
| 20457 | I:1923289:19A01:E06 | XM 035603 | Homo sapiens gap junction protein, beta 5 (connexin 31.1) (GJB5), mRNA | 0 |
| 24773 | M00057055D:B11 | | | |
| 24119 | M00042886D:H10 | BC006260 | Homo sapiens, Similar to N-myc downstream regulated, clone MGC:11293 IMAGE:3946764, mRNA, complete cds | 4.4E-114 |
| 3908 | M00027080A:E06 | M60756 | Human histone H2B.1 mRNA, 3' end | 0 |
| 8560 | I:2346704:06B01:H01 | AJ000334 | Homo sapiens mRNA for cytosolic asparaginyl-tRNA synthetase | 0 |
| 24588 | M00055411A:C10 | L19779 | Homo sapiens histone H2A.2 mRNA, complete cds | 0 |
| 4047 | M00007997C:B08 | XM 009091 | Homo sapiens glycogen synthase 1 (muscle) (GYS1), mRNA | 0 |
| 28344 | 035JN011.E11 | XM 050471 | Homo sapiens glycogen synthase 1 (muscle) (GYS1), mRNA | 0 |
| 27561 | 035JN001.F04 | XM 001472 | Homo sapiens v-jun avian sarcoma virus 17 oncogene homolog (JUN), mRNA | 0 |
| 3272 | M00022165C:E12 | NM 001024 | Homo sapiens ribosomal protein S21 (RPS21 ), mRNA | 0 |
| 26735 | 035JN030.A08 | XM 010408 | Homo sapiens RAB9-like protein (RAB9L), mRNA | 0 |
| 24900 | M00054500D:C08 | BC004427 | Homo sapiens, proteasome (prosome, macropain) subunit, alpha type, 7, clone MGC:3755 IMAGE:2819923, mRNA, complete cds | 0 |
| 9472 | I:2510171:04B01:H08 | X04503 | Human SLPI mRNA fragment for secretory leucocyte protease inhibitor | 0 |
| 9979 | I:1623318:06A01:F09 | L31409 | Homo sapiens creatine transporter mRNA, complete cds | 2.2E-45 |
| 21996 | M00042467B:804 | L00160 | Human phosphoglycerate kinase (pgk) mRNA, exons 2 to last | 0 |
| 22312 | M00055035D:F05 | | | |
| 11327 | I:3139773:05A01:H11 | L00160 | Human phosphoglycerate kinase (pgk) mRNA, exons 2 to last | 2.6E-21 |
| 18240 | RG:1927470:10015:H08 | V00572 | Human mRNA encoding phosphoglycerate kinase | 0 |
| 21922 | M00054848A:D12 | AF139065 | Homo sapiens desmoplakin I mRNA, partial cds | 0 |
| 22290 | M00057002D:H01 | | | |
| 10390 | I:1405391:03802:C09 | AF056979 | Homo sapiens clone YAN1 interferon-gamma receptor mRNA, complete cds | 0 |
| 2212 | M00008098B:F06 | U19247 | Homo sapiens interferon-gamma receptor alpha chain gene, exon 7 and complete cds | 0 |
| 20213 | RG:221172:10007:C11 | S74774 | p59fyn(T)=OKT3-induced calcium influx regulator [human, Jurkat J6 T cell line, mRNA Partial, 1605 nt] | 2.9E-103 |
| 24955 | M00055929D:D04 | | | |
| 19574 | I:635178:13B02:C10 | XM 033944 | Homo sapiens superoxide dismutase 2, mitochondrial (SOD2), mRNA | 0 |
| 19969 | RG:501476:10010:A05 | U14394 | Human tissue inhibitor of metalloproteinases-3 mRNA, complete cds | 0 |
| 8570 | I:1696224:06B01:E07 | X70684 | C.aethiops mRNA for heat shock protein 70 | 5.6E-25 |
| 18519 | I:1997703:13A01:D09 | X52947 | Human mRNA for cardiac gap junction protein | 0 |
| 9616 | I:3200341:06B02:H02 | Y00106 | Human gene for beta-adrenergic receptor (beta-2 subtype) | 0 |
| 22334 | M00055067D:H12 | | | |
| 17459 | I:2056395:13A02:B07 | M77349 | Human transforming growth factor-beta induced gene product (BIGH3) mRNA, complete cds | 2.5E-121 |
| 25193 | M00056763B:A12 | X68277 | H.sapiens CL 100 mRNA for protein tyrosine phosphatase | 0 |
| 25191 | M00056763B:A12 | X68277 | H.sapiens CL 100 mRNA for protein tyrosine phosphatase | 0 |
| 9448 | I:2455617:04B01:D02 | XM 051799 | Homo sapiens guanosine monophosphate reductase (GMPR), mRNA | 0 |
| 25224 | RG:950682:10003:D06 | BC002536 | Homo sapiens, phosphofructokinase, platelet, clone MGC:2192 IMAGE:3140233, mRNA, complete cds | 0 |
| 20218 | RG:2158297:10016:E11 | BC002536 | Homo sapiens, phosphofructokinase, platelet, clone MGC:2192 IMAGE:3140233, mRNA, complete cds | 0 |
| 3089 | NIH50_26184 | D25328 | Human mRNA for platelet-type phosphofructokinase, complete cds | 2E-108 |
| 23985 | NIH5D_26184 | | | |
| 19953 | NIH50_26184 | D25328 | Human mRNA for platelet-type phosphofructokinase, complete cds | 2E-108 |
| 11506 | NIH50_26184 | | | |
| 22362 | M00056349A:F08 | M10546 | Human mitochondrial DNA, fragment M1, encoding transfer RNAs, cytochrome oxidase I, and 2 URFs | 1.2E-86 |
| 25516 | 035JN011.G01 | XM 011470 | Homo sapiens myristoylated alanine-rich protein kinase C substrate (MARCKS, 80K-L) (MACS), mRNA | 0 |
| 25757 | 037XN005.H07 | AF017116 | Homo sapiens type-2 phosphatidic acid phosphohydrolase (PAP2) mRNA, complete cds | 0 |
| 24814 | M00042773B:E09 | M17733 | Human thymosin beta-4 mRNA, complete cds | 0 |
| 21994 | M00042465B:E04 | M17733 | Human thymosin beta-4 mRNA, complete cds | 0 |
| 27,117 | 037XN001.H03 | BC001631 | Homo sapiens, prothymosin beta 4, clone MGC:2219 IMAGE:3536637, mRNA, complete cds | 0 |
| 24681 | NIH50_41452 | | | |
| 22745 | M00056592A:B08 | NM 003739 | Homo sapiens aldo-keto reductase family 1, member C3 (3-alpha hydroxysteroid dehydrogenase, type II) (AKR1C3), mRNA | 0 |
| 24233 | M00055873C:B06 | | | |
| 2001 | M00001381A:F03 | XM 035387 | Homo sapiens ribosomal protein, large, P1 (RPLP1), mRNA | 0 |
| 21179 | NIH50_43550 | | | |
| 17147 | NIH50_43550 | AK026515 | Homo sapiens cDNA: FLJ22862 fis, clone KAT01966, highly similar to HSLDHAR Human mRNA for lactate dehydrogenase-A | 0 |
| 8700 | NIH50_43550 | | | |
| 21214 | M00056193B:D06 | BC006260 | Homo sapiens, Similar to N-myc downstream regulated, clone MGC:11293 IMAGE:3946764, mRNA, complete cds | 0 |
| 26422 | 037XN003.D08 | BC006260 | Homo sapiens, Similar to N-myc downstream regulated, clone MGC:11293 IMAGE:3946764, mRNA, complete cds | 0 |
| 22837 | M00055891C:B09 | | | |
| 21965 | M00057029A:G09 | | | |
| 25541 | 035JN013.D01 | AK026310 | Homo sapiens cDNA: FLJ22657 fis, clone HS107791, highly similar to HUMCYB5 Human cytochrome b5 mRNA | 0 |
| 18302 | I:1738248:09B02:G08 | XM 016114 | Homo sapiens hypothetical protein FLJ22501 (FLJ22501), mRNA | 0 |
| 24049 | M00054706B:G04 | AF107495 | Homo sapiens FWP001 and putative FWP002 mRNA, complete cds | 0 |
| 26326 | 035JN023.D08 | AK025906 | Homo sapiens cDNA: FLJ22253 fis, clone HRC02763 | 0 |
| 2254 | M00004085C:C02 | AK025703 | Homo sapiens cDNA: FLJ22050 fis, clone HEP09454 | 0 |
| 10296 | I:2868216:07B02:D09 | AK025703 | Homo sapiens cDNA: FLJ22050 fis, clone HEP09454 | 0 |
| 20044 | I:2547084:09B01:F05 | XM 016847 | Homo sapiens hypothetical protein FLJ22002 (FLJ22002), mRNA | 0 |
| 28806 | 035JN028.D05 | AK025504 | Homo sapiens cDNA: FLJ21851 fis, clone HEP01962 | 0 |
| 17566 | I:446969:17B02:G07 | AK023217 | Homo sapiens cDNA FLJ13155 fis, clone NT2RP3003433 | 2E-115 |
| 19005 | I:2674167:09A02:G09 | AK022968 | Homo sapiens cDNA FLJ12906 fis, clone NT2RP2004373 | 0 |
| 3567 | M00023369D:C05 | | | |
| 21983 | M00057081B:H03 | | | |
| 458 | M00022134B:E08 | XM 037412 | Homo sapiens hypothetical gene supported by BC008993 (LOC91283), mRNA | 0 |
| 22331 | M00057138A:E11 | | | |
| 21411 | M00055833D:B03 | | | |
| 22972 | M00056956D:B01 | | | |
| 24533 | RG:1643392:10014:C11 | | | |
| 24853 | M00056617D:F07 | AK020869 | Mus musculus adult retina cDNA, RIKEN full-length enriched library, clone:A930017 A02, full insert sequence | 6.5E-59 |
| 23753 | M00054915A:G02 | | | |
| 21502 | M00056193B:D06 | | | |
| 18180 | RG:39422:10005:B02 | | | |
| 23918 | M00056278C:E03 | | | |
| 24144 | RG:1982961:20001:H05 | | | |
| 19996 | RG:1283072:10012:F11 | BC009107 | Homo sapiens, clone MGC:17352 IMAGE:3449913, mRNA, complete cds | 0 |
| 11528 | I:1899534:10B01:D05 | | | |
| 20506 | I:1969044:18B01:E12 | AB048286 | Homo sapiens GS1999full mRNA, complete cds | 0 |
| 23833 | RG:1656861:10014:E10 | | | |
| 20042 | I:1873176:09B01:E05 | BC001909 | Homo sapiens, clone IMAGE:3537447, mRNA, partial cds | 0 |
| 24977 | M00055820D:F01 | | | |
| 11646 | I:1723142:08B02:G11 | AK014612 | Mus musculus 0 day neonate skin cDNA, RIKEN full-length enriched library, clone:4633401105, full insert sequence | 4.6E-45 |
| 24872 | RG:773612:10011:D06 | | | |
| 10577 | I:2174196:08A01:A10 | | | |
| 21710 | RG:1091554:10003:G01 | | | |
| 18556 | RG:31082:10004:F09 | | | |
| 29433 | 035JN014.F12 | AK001805 | Homo sapiens cDNA FLJ10943 fis, clone OVARC1001360 | 0 |
| 29273 | 037XN005.F12 | | | |
| 28763 | 035JN018.G11 | AJ310543 | Homo sapiens mRNA for EGLN1 protein | 1.9E-40 |
| 27887 | RG:2364147:8119908:A10 | | | |
| 27450 | 035JN032.F09 | | | |
| 27255 | 035JN006.E09 | XM 027456 | Homo sapiens hypothetical gene supported by AK000584 (LOC89942), mRNA | 1.2E-57 |
| 27226 | 035JN004.F09 | | | |
| 26550 | 035JN008.D08 | | | |
| 26508 | 035JN004.G02 | | | |
| 26483 | RG:2377371:8119908:C08 | | | |
| 26334 | 035JN023.H08 | AF364547 | Homo sapiens methylmalonyl-CoA epimerase mRNA, complete cds; nuclear gene for mitochondrial product | 0 |
| 26027 | 035JN030.G01 | | | |
| 25977 | 035JN022.F07 | | | |
| 25965 | 035JN022.H01 | | | |
| 25844 | 035JN008.C07 | | | |
| 25834 | 035JN008.F01 | AB048289 | Bos taurus lae mRNA for lipoate-activating enzyme, complete cds | 3.1E-35 |
| 25816 | 035JN004.E07 | | | |
| 25746 | 037XN007.B07 | | | |
| 25742 | 037XN007.H01 | | | |
| 25741 | 037XN005.H01 | | | |
| 25712 | 037XN003.A07 | | | |
| 25642 | 035Jn027.F01 | | | |
| 25621 | 035JN021.D07 | AK027321 | Homo sapiens cDNA FLJ14415 fis, clone HEMBA1004889, weakly similar to Human C3f mRNA | 0 |
| 25614 | 035JN023.H01 | | | |
| 25603 | 035JN021.C01 | | | |
| 25556 | 035JN015.C07 | | | |
| 25555 | 035JN013.C07 | | | |
| 25540 | 035JN015.C01 | | | |
| 23576 | RG:1984769:20002:D10 | | | |
| 22566 | RG:1996656:20003:C03 | | | |
| 9036 | DD182 | | | |
| 4164 | M00007932B:E06 | | | |
| 4146 | 2179-5 | | | |
| 4091 | M00026845A:E01 | | | |
| 4072 | M00023398A:G12 | | | |
| 4022 | M00022127D:B06 | | | |
| 3965 | M00005406A:f04 | | | |
| 3954 | M00005400B:E1 | | | |
| 3872 | M00007974D:B04 | | | |
| 3869 | M00003868C:A03 | | | |
| 3838 | M00007052A:C09 | XM 048272 | Homo sapiens similar to Ras-related GTP-binding protein (H. sapiens) (LOC92951), mRNA | 0 |
| 3806 | 2168-2 | | | |
| 3798 | 2138-4 | | | |
| 3792 | 2171-5 | | | |
| 3788 | 2156-4 | | | |
| 3767 | M00001355D:H12 | | | |
| 3458 | M00007160D:E10 | | | |
| 3251 | M00005471A:a04 | | | |
| 3194 | DF821 | | | |
| 3102 | 2167-1 | | | |
| 3094 | 2138-3 | | | |
| 2671 | M00023431A:002 | | | |
| 2634 | M00008025D:A04 | | | |
| 2567 | M00008061B:A12 | | | |
| 2317 | M00001502D:E09 | | | |
| 1958 | M00023296B:B09 | | | |
| 1680 | 2169-5 | | | |
| 1625 | M00001542C:G08 | | | |
| 1445 | M00023335C:C09 | | | |
| 1320 | 2207-5 | | | |
| 974 | 2161-1 | | | |
| 726 | DO15 | | | |
| 718 | ER418 | | | |
| 703 | M00004189D:A11 | | | |
| 652 | M00007070A:C08 | | | |
| 630 | 2203-2 | | | |
| 593 | M00001373A:A06 | X93036 | H.sapiens mRNA for MAT8 protein | 0 |
| 532 | M00022005A:H05 | | | |
| 272 | 2168-5 | | | |
| 256 | M00001406C:H12 | | | |
| 57 | M00023371B:H02 | | | |

**Table 2**

| SEQ ID NO | SPOT ID | 2D T4-2/2D S1 | 3D T4-2/3D S1 | 3D S1/2D S1 | 3D T4-2/2D T4-2 | 3D T4-2/EGFR Ab | 3D T4-2/B1 Integrin Ab | 3D T4-2/Tyr |
|---|---|---|---|---|---|---|---|---|
| 1 | 10594 | 0.6 | 2.2 | 0.6 | 1.9 | 3.0 | 1.0 | 2.9 |
| 2 | 21851 | 1.0 | 1.0 | 1.0 | 3.5 | 1.3 | 1.0 | 1.0 |
| 3 | 20990 | 1.6 | 4.6 | 1.0 | 1.5 | 1.0 | 1.0 | 1.0 |
| 4 | 18641 | 1.0 | 0.6 | 2.6 | 1.7 | 1.0 | 1.6 | 1.0 |
| 5 | 17229 | 0.3 | 0.8 | 1.0 | 2.1 | 1.0 | 1.0 | 1.0 |
| 6 | 25930 | 1.0 | 1.0 | 1.0 | 1.0 | 1.0 | 1.0 | 1.0 |
| 7 | 20701 | 1.6 | 2.9 | 1.3 | 2.7 | 4.5 | 1.9 | 5.8 |
| 8 | 20346 | 1.7 | 2.7 | 1.4 | 2.6 | 4.3 | 2.0 | 5.2 |
| 9 | 21247 | 1.0 | 4.4 | 1.5 | 3.0 | 3.4 | 2.6 | 4.7 |
| 10 | 23062 | 0.6 | 2.5 | 0.6 | 1.8 | 3.3 | 1.4 | 2.7 |
| 11 | 25666 | 1.0 | 2.9 | 0.6 | 2.0 | 3.6 | 1.0 | 2.3 |
| 12 | 19001 | 8.5 | 14.2 | 1.0 | 1.0 | 4.8 | 1.7 | 8.0 |
| 13 | 10897 | 1.0 | 3.1 | 4.5 | 1000.0 | 13.3 | 4.6 | 18.4 |
| 14 | 1960 | 0.3 | 1.5 | 3.0 | 13.7 | 3.9 | 2.4 | 4.9 |
| 15 | 26381 | 1.0 | 1.0 | 1.0 | 0.9 | 1.0 | 1.0 | 1.0 |
| 16 | 26719 | 0.4 | 1.0 | 0.6 | 2.8 | 1.2 | 1.7 | 1.0 |
| 17 | 27152 | 4.2 | 3.0 | 2.2 | 1.5 | 1.3 | 1.0 | 1.3 |
| 18 | 10926 | 0.7 | 1.9 | 0.9 | 2.1 | 3.7 | 1.5 | 3.3 |
| 19 | 28980 | 0.6 | 1.4 | 1.0 | 2.4 | 1.0 | 1.0 | 1.0 |
| 20 | 1236 | 1.0 | 2.8 | 0.8 | 2.1 | 2.2 | 1.8 | 3.2 |
| 21 | 29350 | 0.5 | 0.6 | 1.2 | 2.1 | 1.4 | 1.0 | 1.0 |
| 22 | 26242 | 1.0 | 1.0 | 0.6 | 2.2 | 1.0 | 1.0 | 2.0 |
| 23 | 4098 | 1.4 | 3.9 | 0.6 | 2.1 | 2.7 | 1.3 | 3.1 |
| 24 | 17432 | 0.4 | 0.3 | 2.4 | 2.1 | 0.3 | 0.9 | 0.3 |
| 25 | 1785 | 0.5 | 0.4 | 2.4 | 2.0 | 0.3 | 1.0 | 0.3 |
| 26 | 28856 | 8.5 | 0.9 | 2.5 | 0.3 | 0.6 | 1.0 | 0.5 |
| 27 | 18791 | 1.0 | 0.2 | 0.3 | 4.1 | 1.0 | 1.0 | 1.3 |
| 28 | 22950 | 3.9 | 4.1 | 1.2 | 1.0 | 2.1 | 1.0 | 2.4 |
| 29 | 1882 | 2.4 | 4.1 | 0.9 | 1.8 | 3.2 | 1.5 | 4.7 |
| 30 | 23886 | 1.0 | 1.0 | 1.2 | 2.1 | 1.0 | 1.0 | 1.0 |
| 31 | 24995 | 2.0 | 1.6 | 2.1 | 1.0 | 1.0 | 1.0 | 1.0 |
| 32 | 24477 | 1.0 | 1.9 | 1.0 | 4.2 | 2.7 | 1.3 | 1.8 |
| 33 | 21681 | 1.7 | 7.1 | 0.6 | 2.0 | 2.8 | 1.0 | 3.6 |
| 34 | 9557 | 1.6 | 7.5 | 0.8 | 1.0 | 3.0 | 1.0 | 2.5 |
| 35 | 22033 | 2.8 | 3.7 | 1.0 | 0.9 | 2.2 | 1.0 | 2.7 |
| 36 | 873 | 1.0 | 4.0 | 1.0 | 2.7 | 1.7 | 1.0 | 1.0 |
| 37 | 17144 | 1.0 | 0.5 | 3.6 | 1.4 | 1.0 | 1.0 | 1.0 |
| 38 | 26970 | 6.0 | 15.3 | 0.2 | 0.6 | 2.9 | 1.0 | 5.4 |
| 39 | 21402 | 0.2 | 1.0 | 2.8 | 6.9 | 2.4 | 1.0 | 3.6 |
| 40 | 27074 | 1.7 | 2.5 | 2.3 | 3.2 | 1.6 | 1.0 | 2.0 |
| 41 | 10963 | 0.5 | 0.3 | 2.1 | 0.5 | 1.0 | 1.0 | 0.7 |
| 42 | 29525 | 0.6 | 1.0 | 0.7 | 2.4 | 1.7 | 1.3 | 1.0 |
| 43 | 25514 | 1000.0 | 1.0 | 1.0 | 1.0 | 0.5 | 1.0 | 1.0 |
| 44 | 26612 | 0.4 | 0.5 | 1.6 | 2.8 | 0.8 | 1.0 | 0.8 |
| 45 | 24600 | 1.6 | 2.7 | 1.0 | 2.0 | 1.0 | 1.2 | 1.4 |
| 46 | 9741 | 2.3 | 5.0 | 1.0 | 2.2 | 1.7 | 1.0 | 1.0 |
| 47 | 23689 | 1.0 | 2.6 | 0.8 | 1.8 | 2.3 | 1.0 | 2.7 |
| 48 | 22352 | 1.0 | 2.9 | 0.7 | 1.6 | 2.4 | 1.0 | 2.4 |
| 49 | 23806 | 1.0 | 0.4 | 1.3 | 2.3 | 1.0 | 1.4 | 1.4 |
| 50 | 12285 | 1.0 | 1.0 | 1.0 | 1.0 | 0.8 | 1.0 | 0.5 |
| 51 | 27638 | 0.6 | 1.0 | 0.8 | 2.2 | 2.1 | 1.0 | 1.0 |
| 52 | 9663 | 1.0 | 1.0 | 1.0 | 1000.0 | 1.0 | 1.0 | 1.0 |
| 53 | 26850 | 1.0 | 0.2 | 9.1 | 2.1 | 1.3 | 1.6 | 2.2 |
| 54 | 10204 | 2.9 | 2.3 | 0.8 | 0.6 | 3.1 | 1.4 | 2.4 |
| 55 | 1318 | 2.0 | 0.9 | 2.3 | 0.5 | 0.6 | 1.1 | 0.7 |
| 56 | 25922 | 1.0 | 0.8 | 1.0 | 1.0 | 1.0 | 1.0 | 1.0 |
| 57 | 26347 | 1.0 | 1.0 | 1.0 | 1.0 | 1.0 | 1.0 | 1.0 |
| 58 | 20361 | 1.0 | 1.0 | 1.0 | 2.0 | 1.0 | 1.0 | 1.0 |
| 59 | 28672 | 0.6 | 2.1 | 0.6 | 2.1 | 1.4 | 1.0 | 1.7 |
| 60 | 25520 | 0.5 | 0.3 | 2.3 | 1.3 | 1.0 | 0.7 | 0.5 |
| 61 | 1723 | 1.0 | 0.5 | 5.1 | 3.5 | 1.0 | 3.1 | 1.0 |
| 62 | 28863 | 0.8 | 1.3 | 1.0 | 2.3 | 1.7 | 1.7 | 1.7 |
| 63 | 25526 | 5.9 | 1.7 | 1.0 | 0.6 | 0.6 | 0.7 | 0.4 |
| 64 | 27936 | 1.0 | 1.0 | 3.2 | 3.1 | 1.9 | 3.1 | 1.5 |
| 65 | 26851 | 1.0 | 0.7 | 3.2 | 2.7 | 1.6 | 2.4 | 1.3 |
| 66 | 25107 | 1.0 | 5.8 | 1.0 | 2.6 | 2.6 | 1.6 | 2.6 |
| 67 | 24912 | 1.0 | 2.9 | 1.0 | 2.4 | 1.6 | 1.3 | 1.8 |
| 68 | 25169 | 1.0 | 0.7 | 2.5 | 1.5 | 1.0 | 1.0 | 1.0 |
| 69 | 25600 | 1.6 | 1.4 | 2.9 | 2.1 | 0.7 | 0.9 | 0.5 |
| 70 | 28706 | 0.2 | 0.5 | 0.6 | 2.1 | 1.3 | 1.2 | 1.0 |
| 71 | 26377 | 0.6 | 0.3 | 2.2 | 1.0 | 1.2 | 1.3 | 1.0 |
| 72 | 19460 | 2.4 | 1.5 | 2.5 | 1.3 | 1.0 | 1.0 | 0.8 |
| 73 | 25243 | 1.0 | 0.7 | 2.2 | 1.0 | 1.0 | 1.0 | 1.0 |
| 74 | 20018 | 1.0 | 1.0 | 1.0 | 2.6 | 1.0 | 1.0 | 1.0 |
| 75 | 918 | 1.0 | 1.7 | 1.3 | 2.1 | 2.0 | 1.6 | 2.4 |
| 76 | 25027 | 1.0 | 1.0 | 1.0 | 1.0 | 1.0 | 1.0 | 1.0 |
| 77 | 29089 | 0.6 | 0.5 | 0.8 | 2.1 | 1.0 | 1.0 | 1.0 |
| 78 | 9141 | 1.0 | 1.0 | 1.0 | 1.0 | 1.0 | 1.0 | 1.0 |
| 79 | 12005 | 1.0 | 1.0 | 2.2 | 1.0 | 1.0 | 1.0 | 1.0 |
| 80 | 12148 | 1.0 | 1.0 | 1.0 | 1.0 | 1.0 | 1.0 | 1.0 |
| 81 | 17394 | 0.4 | 0.6 | 2.1 | 2.0 | 1.0 | 1.0 | 1.0 |
| 82 | 27017 | 2.8 | 3.3 | 0.8 | 1.0 | 2.4 | 1.8 | 2.8 |
| 83 | 25809 | 1.0 | 1.0 | 1000.0 | 1.0 | 1.0 | 1.0 | 1.0 |
| 84 | 8719 | 0.1 | 1.0 | 2.3 | 2.1 | 0.4 | 0.5 | 0.3 |
| 85 | 21030 | 0.4 | 1.0 | 1.3 | 2.1 | 1.4 | 1.6 | 1.4 |
| 86 | 11436 | 0.7 | 0.4 | 2.0 | 1.0 | 0.6 | 0.8 | 0.6 |
| 87 | 10374 | 1.5 | 1.5 | 3.5 | 2.7 | 0.4 | 1.0 | 0.3 |
| 88 | 19037 | 3.0 | 3.3 | 0.9 | 1.5 | 2.7 | 1.4 | 3.7 |
| 89 | 398 | 1.6 | 6.9 | 1.1 | 3.3 | 2.4 | 1.0 | 4.5 |
| 90 | 18773 | 1.9 | 5.1 | 1.0 | 3.9 | 3.8 | 2.0 | 6.1 |
| 91 | 3583 | 0.5 | 0.7 | 1.0 | 2.0 | 2.5 | 1.0 | 1.5 |
| 92 | 3418 | 1.8 | 3.2 | 1.2 | 2.4 | 1.6 | 1.0 | 1.2 |
| 93 | 18985 | 9.2 | 3.1 | 1.0 | 0.6 | 2.3 | 1.1 | 2.5 |
| 94 | 25861 | 3.4 | 1.5 | 2.0 | 0.8 | 0.8 | 0.9 | 0.6 |
| 95 | 3317 | 0.9 | 2.3 | 1.0 | 3.4 | 1.9 | 1.0 | 1.0 |
| 96 | 8743 | 0.2 | 0.7 | 1.0 | 4.3 | 1.8 | 1.0 | 1.7 |
| 97 | 26240 | 0.2 | 1.0 | 1.0 | 5.3 | 1.9 | 1.9 | 1.1 |
| 98 | 28562 | 0.3 | 0.2 | 2.0 | 1.0 | 0.5 | 0.5 | 0.6 |
| 99 | 16877 | 1.0 | 2.6 | 1.1 | 2.6 | 1.7 | 1.5 | 1.3 |
| 100 | 25955 | 1.0 | 1.0 | 1.0 | 1.0 | 1.0 | 1.0 | 1.0 |
| 101 | 26308 | 0.2 | 0.4 | 1.0 | 2.2 | 0.7 | 0.8 | 0.6 |
| 102 | 4140 | 1.9 | 6.7 | 0.7 | 2.1 | 3.0 | 1.0 | 3.5 |
| 103 | 3436 | 1.8 | 6.3 | 0.6 | 2.2 | 3.1 | 1.3 | 3.3 |
| 104 | 25612 | 1.0 | 12.5 | 1.0 | 1.0 | 2.1 | 1.0 | 2.9 |
| 105 | 12257 | 1.0 | 1.0 | 2.0 | 1.0 | 0.8 | 0.9 | 0.8 |
| 106 | 9111 | 0.5 | 0.5 | 2.2 | 1.3 | 1.5 | 1.0 | 0.7 |
| 107 | 17620 | 0.3 | 0.8 | 1.0 | 3.2 | 2.7 | 2.1 | 1.0 |
| 108 | 26025 | 1.0 | 2.9 | 1.1 | 2.2 | 2.3 | 1.0 | 2.6 |
| 109 | 19271 | 0.5 | 1.3 | 0.7 | 2.2 | 1.6 | 1.2 | 1.5 |
| 110 | 4151 | 0.4 | 4.2 | 1.2 | 11.1 | 4.2 | 1.0 | 2.9 |
| 111 | 26569 | 0.7 | 2.2 | 0.8 | 2.9 | 2.3 | 1.7 | 2.6 |
| 112 | 10344 | 1.0 | 1.0 | 1.0 | 1.0 | 1.0 | 1.0 | 1.0 |
| 113 | 832 | 1.0 | 3.3 | 1.0 | 2.4 | 3.7 | 2.2 | 4.0 |
| 114 | 12071 | 1.8 | 1.5 | 2.2 | 1.0 | 1.3 | 0.8 | 1.4 |
| 115 | 12271 | 0.6 | 4.9 | 1.9 | 14.9 | 20.8 | 4.0 | 24.1 |
| 116 | 11433 | 0.5 | 0.4 | 5.7 | 3.0 | 1.7 | 1.8 | 1.0 |
| 117 | 20917 | 1.0 | 2.8 | 0.9 | 2.6 | 1.7 | 1.4 | 1.7 |
| 118 | 25810 | 1.1 | 3.8 | 1.0 | 2.9 | 1.5 | 1.3 | 1.5 |
| 119 | 12039 | 1.0 | 1.0 | 3.6 | 1.0 | 1.0 | 1.0 | 1.0 |
| 120 | 25499 | 1.0 | 1.0 | 1.0 | 1.0 | 1.0 | 1.0 | 1.0 |
| 121 | 25557 | 1.0 | 1.8 | 1.0 | 0.8 | 1.0 | 1.0 | 1.0 |
| 122 | 9917 | 2.5 | 2.7 | 0.7 | 1.6 | 3.8 | 1.2 | 3.6 |
| 123 | 19505 | 0.4 | 1.7 | 0.7 | 3.8 | 1.7 | 1.6 | 1.4 |
| 124 | 17491 | 0.6 | 1.7 | 0.7 | 2.5 | 1.6 | 1.3 | 1.4 |
| 125 | 10683 | 0.4 | 1.9 | 0.6 | 3.6 | 1.7 | 1.4 | 1.1 |
| 126 | 1936 | 0.2 | 0.6 | 0.6 | 3.1 | 1.0 | 1.8 | 1.0 |
| 127 | 828 | 0.1 | 1.0 | 0.5 | 3.0 | 1.0 | 1.7 | 1.2 |
| 128 | 9558 | 1.0 | 1.0 | 1.0 | 1.0 | 1.0 | 1.0 | 1.0 |
| 129 | 20164 | 2.0 | 1.1 | 2.5 | 1.7 | 1.0 | 1.0 | 0.8 |
| 130 | 969 | 1.0 | 1.0 | 2.7 | 1.0 | 1.0 | 1.5 | 0.7 |
| 131 | 9910 | 0.4 | 1.0 | 0.8 | 3.2 | 1.9 | 1.3 | 1.4 |
| 132 | 2427 | 1.3 | 0.7 | 3.0 | 2.8 | 0.8 | 1.9 | 1.0 |
| 133 | 19990 | 1.0 | 7.9 | 2.8 | 34.7 | 1.0 | 1.0 | 1.0 |
| 134 | 20605 | 3.0 | 1.2 | 2.1 | 1.0 | 1.3 | 1.2 | 0.8 |
| 135 | 10650 | 0.5 | 1.7 | 0.5 | 2.9 | 2.8 | 0.6 | 3.4 |
| 136 | 25963 | 2.6 | 3.5 | 0.7 | 1.0 | 3.3 | 1.0 | 2.3 |
| 137 | 25562 | 3.2 | 5.9 | 0.7 | 1.0 | 4.2 | 1.0 | 4.8 |
| 138 | 9377 | 0.6 | 1.0 | 1.0 | 2.1 | 1.9 | 2.0 | 1.6 |
| 139 | 17618 | 1.0 | 0.7 | 2.3 | 3.2 | 0.8 | 0.7 | 0.8 |
| 140 | 12136 | 1.0 | 1.0 | 3.8 | 1.0 | 1.0 | 1.0 | 1.0 |
| 141 | 17373 | 1.0 | 0.4 | 6.1 | 2.4 | 1.0 | 1.0 | 1.0 |
| 142 | 18577 | 1.0 | 0.3 | 0.3 | 4.6 | 1.0 | 1.0 | 1.0 |
| 143 | 3143 | 1.7 | 1.3 | 2.6 | 2.3 | 0.7 | 1.0 | 0.5 |
| 144 | 17737 | 6.1 | 0.7 | 3.4 | 0.3 | 0.5 | 1.3 | 0.4 |
| 145 | 20029 | 1.0 | 0.6 | 2.3 | 1.0 | 1.0 | 1.0 | 0.5 |
| 146 | 18537 | 1.0 | 1.3 | 2.1 | 2.6 | 1.3 | 1.0 | 1.2 |
| 147 | 10090 | 1.0 | 1.7 | 2.1 | 2.8 | 1.5 | 1.0 | 1.2 |
| 148 | 12102 | 1.0 | 1.0 | 3.9 | 1.0 | 1.0 | 1.0 | 1.0 |
| 149 | 8487 | 4.7 | 2.4 | 1.0 | 1.0 | 2.3 | 1.1 | 2.2 |
| 150 | 9252 | 1.3 | 3.8 | 0.3 | 1.0 | 2.1 | 1.6 | 2.5 |
| 151 | 25605 | 1.0 | 1.0 | 1.0 | 1.0 | 0.5 | 0.5 | 1.0 |
| 152 | 29652 | 1.0 | 2.9 | 1.5 | 2.9 | 2.0 | 1.5 | 2.1 |
| 153 | 10858 | 1.0 | 0.8 | 2.0 | 1.0 | 1.0 | 1.0 | 0.7 |
| 154 | 1261 | 0.2 | 0.6 | 1.0 | 2.9 | 0.8 | 0.8 | 0.9 |
| 155 | 4156 | 12.4 | 0.8 | 3.1 | 0.2 | 0.6 | 1.0 | 0.3 |
| 156 | 3452 | 10.6 | 0.8 | 2.8 | 0.3 | 0.6 | 1.0 | 0.4 |
| 157 | 2748 | 10.8 | 0.8 | 3.1 | 0.2 | 0.5 | 1.0 | 0.4 |
| 158 | 2046 | 9.2 | 1.0 | 2.4 | 0.3 | 0.5 | 1.2 | 0.4 |
| 159 | 2044 | 11.7 | 0.8 | 2.8 | 0.2 | 0.6 | 1.4 | 0.4 |
| 160 | 1342 | 10.5 | 0.9 | 2.8 | 0.2 | 0.5 | 1.2 | 0.4 |
| 161 | 1326 | 12.2 | 1.0 | 2.7 | 0.2 | 0.5 | 1.0 | 0.4 |
| 162 | 9981 | 0.2 | 1.5 | 0.3 | 2.5 | 1.2 | 1.6 | 0.5 |
| 163 | 27917 | 1.9 | 2.5 | 0.5 | 1.0 | 2.1 | 1.4 | 2.3 |
| 164 | 8488 | 4.3 | 2.4 | 1.0 | 0.5 | 2.9 | 0.9 | 3.6 |
| 165 | 22793 | 1.9 | 2.6 | 0.5 | 1.0 | 2.2 | 1.8 | 2.1 |
| 166 | 26883 | 2.4 | 3.7 | 0.5 | 1.0 | 2.5 | 2.0 | 2.0 |
| 167 | 11540 | 0.7 | 1.0 | 1.3 | 2.8 | 0.8 | 1.0 | 0.5 |
| 168 | 17707 | 1.0 | 0.6 | 2.6 | 1.0 | 1.0 | 1.0 | 1.0 |
| 169 | 20649 | 2.3 | 2.6 | 0.5 | 0.4 | 3.0 | 1.0 | 3.1 |
| 170 | 24004 | 1.0 | 2.5 | 1.8 | 3.6 | 2.3 | 1.0 | 2.8 |
| 171 | 11836 | 1.2 | 5.0 | 0.9 | 3.7 | 1.3 | 1.0 | 0.8 |
| 172 | 24932 | 1.8 | 0.8 | 6.5 | 2.1 | 0.8 | 1.0 | 0.5 |
| 173 | 19143 | 0.6 | 1.6 | 0.7 | 2.0 | 1.7 | 1.2 | 1.4 |
| 174 | 26257 | 1.9 | 1.3 | 2.2 | 1.7 | 0.7 | 1.0 | 0.6 |
| 175 | 21239 | 9.4 | 9.2 | 0.5 | 0.4 | 2.4 | 1.0 | 2.7 |
| 176 | 16959 | 0.6 | 2.1 | 0.8 | 2.1 | 3.0 | 1.4 | 2.5 |
| 177 | 2568 | 0.7 | 1.9 | 0.7 | 2.2 | 3.0 | 1.3 | 2.4 |
| 178 | 25936 | 1.0 | 2.4 | 0.7 | 2.0 | 3.1 | 1.5 | 2.4 |
| 179 | 23041 | 0.7 | 1.0 | 2.1 | 2.6 | 1.0 | 1.4 | 1.0 |
| 180 | 9206 | 5.7 | 1.8 | 4.6 | 1.0 | 1.0 | 0.7 | 0.9 |
| 181 | 25105 | 1.6 | 1.3 | 2.1 | 1.0 | 1.0 | 0.7 | 0.8 |
| 182 | 24779 | 1.0 | 1.0 | 1.0 | 2.9 | 2.3 | 1.4 | 1.2 |
| 183 | 22451 | 1.0 | 0.2 | 2.1 | 1.0 | 1.4 | 0.6 | 1.0 |
| 184 | 22291 | 0.2 | 0.2 | 2.1 | 1.0 | 0.6 | 0.6 | 0.5 |
| 185 | 21143 | 1.0 | 7.2 | 0.7 | 2.0 | 2.6 | 1.1 | 2.4 |
| 186 | 24751 | 1.7 | 5.0 | 0.7 | 2.1 | 2.4 | 1.3 | 4.0 |
| 187 | 24294 | 1.7 | 3.9 | 0.8 | 2.4 | 2.6 | 1.1 | 3.9 |
| 188 | 24006 | 1.7 | 6.3 | 0.8 | 2.5 | 2.4 | 1.0 | 4.0 |
| 189 | 25678 | 1.0 | 1.0 | 1.0 | 1.0 | 1.0 | 1.0 | 1.0 |
| 190 | 22027 | 8.7 | 7.0 | 0.4 | 0.2 | 5.1 | 2.0 | 5.2 |
| 191 | 29495 | 1.0 | 1.0 | 1.0 | 1.0 | 1.0 | 1.0 | 1.0 |
| 192 | 24577 | 6.8 | 3.2 | 0.8 | 0.4 | 3.8 | 1.3 | 2.1 |
| 193 | 23527 | 0.3 | 2.1 | 1.6 | 6.4 | 2.7 | 2.1 | 3.4 |
| 194 | 17090 | 1.0 | 4.9 | 0.7 | 2.3 | 3.1 | 2.3 | 3.6 |
| 195 | 25137 | 1.0 | 1.0 | 0.4 | 3.8 | 1.0 | 2.5 | 4.1 |
| 196 | 23772 | 0.6 | 6.8 | 0.5 | 3.7 | 12.6 | 3.6 | 9.2 |
| 197 | 1659 | 1.0 | 7.5 | 0.3 | 3.2 | 17.8 | 4.1 | 20.3 |
| 198 | 8497 | 1.3 | 0.4 | 2.2 | 0.5 | 1.0 | 1.0 | 1.0 |
| 199 | 25272 | 8.0 | 6.0 | 1.0 | 0.6 | 2.2 | 1.0 | 2.9 |
| 200 | 21216 | 1.0 | 1.0 | 0.6 | 2.0 | 2.5 | 2.0 | 2.2 |
| 201 | 11939 | 1.0 | 1.0 | 1.0 | 1.0 | 1.0 | 1.0 | 1.0 |
| 202 | 9191 | 1.8 | 2.2 | 1.3 | 1.1 | 2.2 | 1.0 | 2.0 |
| 203 | 3429 | 0.7 | 3.4 | 0.8 | 3.5 | 3.0 | 1.5 | 3.7 |
| 204 | 2725 | 0.8 | 3.4 | 1.0 | 3.4 | 2.6 | 1.6 | 4.1 |
| 205 | 19923 | 1.0 | 1.1 | 2.9 | 1.0 | 1.7 | 1.4 | 1.2 |
| 206 | 20457 | 1.0 | 2.0 | 1.0 | 2.3 | 2.9 | 1.0 | 2.3 |
| 207 | 24773 | 0.2 | 1.0 | 0.8 | 2.0 | 1.6 | 1.0 | 1.0 |
| 208 | 24119 | 0.2 | 4.6 | 1.1 | 15.9 | 2.7 | 1.0 | 3.4 |
| 209 | 3908 | 0.3 | 0.5 | 1.1 | 2.3 | 1.7 | 1.0 | 1.0 |
| 210 | 8560 | 1.9 | 0.7 | 2.2 | 0.5 | 1.0 | 1.0 | 0.7 |
| 211 | 24588 | 0.3 | 0.5 | 1.0 | 2.0 | 1.0 | 1.0 | 1.4 |
| 212 | 4047 | 0.5 | 1.2 | 1.0 | 2.1 | 1.9 | 1.0 | 1.8 |
| 213 | 28344 | 0.8 | 1.0 | 1.0 | 2.0 | 1.7 | 1.5 | 2.7 |
| 214 | 27561 | 1.0 | 1.0 | 1.0 | 2.4 | 1.2 | 1.2 | 1.3 |
| 215 | 3272 | 0.6 | 0.8 | 1.0 | 2.1 | 1.3 | 1.6 | 1.0 |
| 216 | 26735 | 1.0 | 1.0 | 1.0 | 1.0 | 1.0 | 1.0 | 1.0 |
| 217 | 24900 | 0.3 | 0.8 | 2.9 | 5.7 | 2.2 | 1.1 | 3.0 |
| 218 | 9472 | 2.2 | 5.0 | 1.0 | 2.0 | 1.5 | 0.8 | 1.7 |
| 219 | 9979 | 1.3 | 3.3 | 1.5 | 3.9 | 3.4 | 1.4 | 2.5 |
| 220 | 21996 | 1.0 | 4.7 | 1.0 | 3.4 | 2.5 | 1.0 | 2.4 |
| 221 | 22312 | 1.2 | 4.4 | 1.2 | 3.3 | 2.2 | 1.1 | 2.2 |
| 222 | 11327 | 1.4 | 6.2 | 1.4 | 2.7 | 2.7 | 1.0 | 2.2 |
| 223 | 18240 | 2.0 | 4.5 | 1.0 | 2.2 | 2.1 | 1.0 | 2.7 |
| 224 | 21922 | 0.7 | 1.4 | 0.8 | 2.1 | 1.8 | 1.0 | 1.3 |
| 225 | 22290 | 0.7 | 1.6 | 0.9 | 2.1 | 1.5 | 1.2 | 1.3 |
| 226 | 10390 | 1.3 | 1.0 | 2.6 | 1.6 | 0.8 | 1.0 | 0.6 |
| 227 | 2212 | 1.9 | 1.0 | 2.8 | 1.0 | 0.6 | 1.6 | 0.8 |
| 228 | 20213 | 0.4 | 1.0 | 1.0 | 1.0 | 1.0 | 1.0 | 1.0 |
| 229 | 24955 | 0.9 | 2.9 | 1.0 | 3.3 | 0.8 | 0.8 | 1.0 |
| 230 | 19574 | 1.0 | 0.6 | 3.7 | 3.1 | 1.5 | 2.6 | 1.4 |
| 231 | 19969 | 1.0 | 1.0 | 1.0 | 3.1 | 1.0 | 1.0 | 1.0 |
| 232 | 8570 | 0.4 | 1.2 | 1.0 | 2.6 | 1.2 | 0.8 | 0.6 |
| 233 | 18519 | 3.5 | 2.9 | 2.6 | 1.8 | 1.8 | 1.0 | 2.0 |
| 234 | 9616 | 0.6 | 2.0 | 1.0 | 2.3 | 1.2 | 1.2 | 1.0 |
| 235 | 22334 | 0.2 | 0.7 | 2.9 | 8.5 | 1.7 | 1.1 | 3.4 |
| 236 | 17459 | 0.1 | 0.7 | 2.7 | 18.8 | 4.0 | 1.3 | 4.6 |
| 237 | 25193 | 1.0 | 0.8 | 1.0 | 2.3 | 1.0 | 1.3 | 1.0 |
| 238 | 25191 | 0.2 | 0.8 | 0.7 | 2.5 | 1.3 | 1.5 | 1.2 |
| 239 | 9448 | 0.6 | 1.0 | 1.0 | 2.3 | 0.8 | 0.8 | 0.5 |
| 240 | 25224 | 5.6 | 14.4 | 1.0 | 2.3 | 6.0 | 1.5 | 9.6 |
| 241 | 20218 | 6.1 | 12.3 | 0.7 | 1.7 | 5.6 | 1.6 | 9.0 |
| 242 | 3089 | 7.0 | 15.7 | 0.7 | 2.3 | 7.3 | 1.8 | 8.0 |
| 243 | 23985 | 5.8 | 17.2 | 0.9 | 2.1 | 6.8 | 1.8 | 8.1 |
| 244 | 19953 | 6.2 | 13.5 | 0.8 | 1.8 | 6.4 | 1.7 | 10.4 |
| 245 | 11506 | 4.1 | 13.3 | 1.0 | 1.4 | 4.4 | 1.6 | 7.2 |
| 246 | 22362 | 1.0 | 0.7 | 4.1 | 2.1 | 1.2 | 1.8 | 1.0 |
| 247 | 25516 | 0.7 | 10.1 | 0.4 | 4.0 | 14.7 | 4.7 | 8.1 |
| 248 | 25757 | 0.6 | 0.4 | 2.4 | 1.0 | 1.0 | 1.3 | 0.9 |
| 249 | 24814 | 0.5 | 2.8 | 0.3 | 1.0 | 3.5 | 1.4 | 4.4 |
| 250 | 21994 | 0.5 | 3.2 | 0.3 | 1.0 | 3.6 | 1.0 | 4.3 |
| 251 | 27117 | 1.0 | 2.8 | 0.3 | 1.0 | 3.9 | 1.0 | 4.9 |
| 252 | 24681 | 1.8 | 2.6 | 0.6 | 0.5 | 3.2 | 1.5 | 3.0 |
| 253 | 22745 | 0.3 | 2.4 | 1.4 | 8.1 | 2.8 | 2.3 | 3.5 |
| 254 | 24233 | 1.9 | 3.9 | 1.3 | 2.3 | 1.3 | 0.8 | 2.2 |
| 255 | 2001 | 1.0 | 1.0 | 1.5 | 2.1 | 1.0 | 1.0 | 1.0 |
| 256 | 21179 | 2.0 | 7.9 | 0.7 | 1.9 | 2.1 | 1.0 | 4.3 |
| 257 | 17147 | 1.3 | 4.3 | 0.7 | 1.7 | 2.4 | 1.2 | 3.9 |
| 258 | 8700 | 1.5 | 7.3 | 0.7 | 1.6 | 3.1 | 1.0 | 2.7 |
| 259 | 21214 | 0.3 | 5.4 | 1.2 | 15.5 | 3.1 | 1.0 | 3.6 |
| 260 | 26422 | 0.4 | 3.7 | 1.0 | 12.7 | 3.9 | 1.0 | 3.3 |
| 261 | 22837 | 0.7 | 1.0 | 2.1 | 2.4 | 1.2 | 1.5 | 0.9 |
| 262 | 21965 | 1.0 | 1.0 | 1.0 | 2.2 | 2.4 | 1.0 | 1.0 |
| 263 | 25541 | 4.5 | 2.7 | 2.7 | 0.8 | 1.0 | 1.3 | 0.8 |
| 264 | 18302 | 1.1 | 0.9 | 2.1 | 1.0 | 1.0 | 1.0 | 1.0 |
| 265 | 24049 | 1.0 | 2.6 | 1.5 | 2.5 | 2.3 | 1.4 | 2.4 |
| 266 | 26326 | 9.2 | 1.5 | 3.2 | 0.7 | 0.7 | 0.9 | 1.0 |
| 267 | 2254 | 1.6 | 3.3 | 1.0 | 2.8 | 2.0 | 1.1 | 3.1 |
| 268 | 10296 | 0.9 | 1.7 | 2.9 | 5.0 | 2.1 | 1.0 | 1.3 |
| 269 | 20044 | 1.0 | 0.8 | 2.0 | 1.0 | 1.0 | 1.0 | 1.0 |
| 270 | 28806 | 2.8 | 1.1 | 2.1 | 1.0 | 0.9 | 1.2 | 0.8 |
| 271 | 17566 | 7.5 | 4.2 | 0.7 | 0.5 | 2.5 | 1.0 | 2.5 |
| 272 | 19005 | 1.0 | 0.8 | 1.0 | 2.1 | 1.0 | 1.0 | 1.0 |
| 273 | 3567 | 1.0 | 1.0 | 1.0 | 1.0 | 1.0 | 1.0 | 1.0 |
| 274 | 21983 | 0.1 | 1.0 | 3.1 | 5.6 | 3.4 | 1.0 | 4.7 |
| 275 | 458 | 1.0 | 2.1 | 0.6 | 1.0 | 1.6 | 2.1 | 2.3 |
| 276 | 22331 | 0.6 | 2.1 | 0.4 | 1.0 | 2.2 | 1.0 | 2.8 |
| 277 | 21411 | 0.7 | 1.5 | 1.0 | 2.5 | 1.0 | 1.0 | 1.0 |
| 278 | 22972 | 1.0 | 2.2 | 0.5 | 1.0 | 2.2 | 1.4 | 2.4 |
| 279 | 24533 | 1.0 | 2.5 | 1.0 | 2.0 | 2.0 | 2.7 | 3.2 |
| 280 | 24853 | 1.0 | 2.6 | 2.1 | 2.1 | 2.4 | 1.3 | 2.1 |
| 281 | 23753 | 0.7 | 1.5 | 1.3 | 2.1 | 2.0 | 1.7 | 2.3 |
| 282 | 21502 | 0.3 | 4.8 | 1.0 | 10.8 | 2.6 | 1.0 | 2.9 |
| 283 | 18180 | 0.3 | 0.8 | 0.8 | 2.4 | 0.9 | 1.4 | 0.7 |
| 284 | 23918 | 0.7 | 2.3 | 0.4 | 1.0 | 2.4 | 1.2 | 3.5 |
| 285 | 24144 | 1.0 | 1.0 | 1.0 | 1.0 | 1.0 | 1.6 | 1.0 |
| 286 | 19996 | 1.5 | 2.5 | 0.7 | 1.2 | 2.1 | 0.9 | 2.5 |
| 287 | 11528 | 1.0 | 1.0 | 1.0 | 2.1 | 1.0 | 1.0 | 1.0 |
| 288 | 20506 | 2.2 | 0.9 | 3.2 | 0.8 | 1.3 | 1.6 | 1.0 |
| 289 | 23833 | 1.0 | 0.5 | 2.1 | 1.0 | 1.0 | 1.0 | 0.7 |
| 290 | 20042 | 3.8 | 1.6 | 2.3 | 0.8 | 1.0 | 1.0 | 1.0 |
| 291 | 24977 | 1.0 | 1.0 | 2.1 | 1.0 | 2.3 | 1.4 | 1.4 |
| 292 | 11646. | 1.0 | 1.0 | 0.8 | 1000.0 | 1.0 | 1.0 | 1.7 |
| 293 | 24872 | 1.0 | 1.4 | 0.8 | 2.5 | 1.4 | 1.2 | 1.3 |
| 294 | 10577 | 1.0 | 1.0 | 1.0 | 1.0 | 1.0 | 1.0 | 1.0 |
| 295 | 21710 | 1.0 | 0.2 | 2.2 | 0.7 | 1.6 | 1.0 | 1.2 |
| 296 | 18556 | 0.0 | 1.0 | 1.0 | 1.0 | 1.0 | 1.0 | 1.0 |
| 297 | 29433 | 1.0 | 0.5 | 1.0 | 2.1 | 1.0 | 1.0 | 1.0 |
| 298 | 29273 | 1.0 | 2.2 | 1.0 | 2.2 | 1.0 | 1.3 | 1.0 |
| 299 | 28763 | 1.6 | 2.7 | 1.0 | 2.2 | 1.8 | 1.3 | 2.5 |
| 300 | 27887 | 0.1 | 0.2 | 1.1 | 2.7 | 0.8 | 1.0 | 0.6 |
| 301 | 27450 | 2.6 | 11.3 | 0.2 | 1.0 | 4.4 | 3.3 | 7.3 |
| 302 | 27255 | 0.6 | 1.6 | 0.8 | 2.3 | 1.7 | 1.4 | 1.5 |
| 303 | 27226 | 1.0 | 1.3 | 1.0 | 2.6 | 1.8 | 1.0 | 1.0 |
| 304 | 26550 | 4.2 | 17.9 | 0.2 | 1.0 | 6.9 | 2.9 | 9.2 |
| 305 | 26508 | 1.0 | 1.4 | 1.0 | 1.0 | 1.0 | 1.0 | 1.0 |
| 306 | 26483 | 1.2 | 2.2 | 0.6 | 1.0 | 2.1 | 1.4 | 2.7 |
| 307 | 26334 | 1.0 | 0.5 | 3.0 | 1.0 | 0.6 | 0.8 | 0.5 |
| 308 | 26027 | 1.0 | 1.0 | 1.0 | 1.5 | 1.0 | 1.0 | 1.0 |
| 309 | 25977 | 1.0 | 1.0 | 1.0 | 1.0 | 1.0 | 1.0 | 1.0 |
| 310 | 25965 | 1.0 | 1.0 | 1.0 | 1.0 | 1.0 | 1.0 | 1.0 |
| 311 | 25844 | 1.0 | 1.0 | 1.0 | 1.0 | 1.0 | 1.0 | 1.0 |
| 312 | 25834 | 1000.0 | 1.0 | 1.0 | 1.0 | 0.4 | 1.0 | 1.0 |
| 313 | 25816 | 1.0 | 1.0 | 1.0 | 1.0 | 1.0 | 1.0 | 1.0 |
| 314 | 25746 | 1.0 | 1.0 | 1.0 | 1.0 | 1.0 | 1.0 | 1.0 |
| 315 | 25742 | 1.0 | 1.0 | 1.0 | 1.0 | 0.5 | 1.0 | 1.0 |
| 316 | 25741 | 1.0 | 1.0 | 1.0 | 1.0 | 1.0 | 1.0 | 1.0 |
| 317 | 25712 | 1.0 | 1.0 | 1.0 | 1.0 | 1.0 | 1.0 | 1.0 |
| 318 | 25642 | 1.0 | 1.0 | 1.0 | 1.0 | 1.0 | 1.0 | 1.0 |
| 319 | 25621 | 0.6 | 0.8 | 2.1 | 2.0 | 1.3 | 1.2 | 1.0 |
| 320 | 25614 | 1.0 | 1.0 | 1.0 | 1.0 | 1.0 | 1.0 | 1.0 |
| 321 | 25603 | 1.0 | 1.0 | 1.0 | 1.0 | 1.0 | 1.0 | 1.0 |
| 322 | 25556 | 1.8 | 0.7 | 1.0 | 0.8 | 1.0 | 1.0 | 1.0 |
| 323 | 25555 | 1.0 | 2.9 | 1.0 | 1.0 | 1.5 | 1.3 | 1.0 |
| 324 | 25540 | 1.0 | 1.0 | 1.0 | 1.2 | 1.0 | 1.0 | 1.0 |
| 325 | 23576 | 0.0 | 1.0 | 1.0 | 1.0 | 1.0 | 1.5 | 1.4 |
| 326 | 22566 | 1.0 | 1.0 | 1.0 | 1.0 | 1.0 | 1.3 | 1.0 |
| 327 | 9036 | 1.9 | 3.8 | 0.6 | 1.8 | 2.6 | 1.3 | 3.1 |
| 328 | 4164 | 1.0 | 1.0 | 2.2 | 1.0 | 1.5 | 1.0 | 0.9 |
| 329 | 4146 | 0.8 | 3.7 | 0.9 | 4.4 | 3.3 | 1.0 | 4.3 |
| 330 | 4091 | 1.0 | 1.0 | 1.0 | 2.5 | 1.7 | 1.0 | 0.9 |
| 331 | 4072 | 1.0 | 1.0 | 2.1 | 1.0 | 5.9 | 2.0 | 5.1 |
| 332 | 4022 | 3.5 | 4.5 | 0.8 | 1.0 | 3.0 | 1.0 | 3.2 |
| 333 | 3965 | 1.9 | 5.6 | 0.4 | 1.0 | 5.5 | 2.3 | 4.1 |
| 334 | 3954 | 1.0 | 2.7 | 1.3 | 3.6 | 2.8 | 1.9 | 2.6 |
| 335 | 3872 | 1.0 | 3.2 | 1.3 | 2.8 | 4.0 | 1.8 | 3.9 |
| 336 | 3869 | 1.0 | 1.0 | 5.8 | 3.8 | 1.0 | 0.7 | 0.6 |
| 337 | 3838 | 1.0 | 1.6 | 1.2 | 2.0 | 2.6 | 1.7 | 1.9 |
| 338 | 3806 | 0.6 | 2.6 | 0.9 | 3.7 | 3.0 | 1.0 | 3.4 |
| 339 | 3798 | 10.2 | 0.9 | 2.9 | 0.3 | 0.7 | 1.0 | 0.4 |
| 340 | 3792 | 1.0 | 1.0 | 1.0 | 2.7 | 2.9 | 1.0 | 2.5 |
| 341 | 3788 | 1.7 | 5.4 | 1.2 | 3.4 | 2.5 | 1.3 | 2.7 |
| 342 | 3767 | 1.1 | 2.2 | 0.7 | 1.7 | 2.5 | 1.0 | 2.5 |
| 343 | 3458 | 1.2 | 3.3 | 0.7 | 2.0 | 2.6 | 1.0 | 2.2 |
| 344 | 3251 | 0.4 | 0.5 | 1.4 | 2.7 | 1.4 | 1.0 | 1.0 |
| 345 | 3194 | 1.0 | 2.3 | 1.3 | 3.1 | 2.2 | 1.3 | 3.2 |
| 346 | 3102 | 0.5 | 3.2 | 1.0 | 4.8 | 2.9 | 1.0 | 2.3 |
| 347 | 3094 | 11.5 | 0.8 | 2.7 | 0.3 | 0.6 | 1.0 | 0.4 |
| 348 | 2671 | 0.8 | 1.6 | 1.0 | 2.2 | 2.8 | 1.9 | 1.0 |
| 349 | 2634 | 0.9 | 2.8 | 0.4 | 1.0 | 3.8 | 1.7 | 4.0 |
| 350 | 2567 | 4.6 | 3.3 | 0.8 | 0.6 | 2.6 | 1.0 | 3.3 |
| 351 | 2317 | 1.0 | 1.0 | 2.4 | 1.0 | 1.0 | 1.0 | 1.1 |
| 352 | 1958 | 0.3 | 0.6 | 1.0 | 2.6 | 0.9 | 0.8 | 0.9 |
| 353 | 1680 | 0.3 | 4.7 | 1.0 | 17.7 | 2.7 | 1.0 | 4.5 |
| 354 | 1625 | 2.2 | 7.8 | 0.5 | 1.8 | 3.1 | 1.7 | 3.4 |
| 355 | 1445 | 0.2 | 0.6 | 1.0 | 2.7 | 0.8 | 0.9 | 0.9 |
| 356 | 1320 | 4.9 | 1.0 | 2.4 | 0.4 | 0.6 | 1.2 | 0.5 |
| 357 | 974 | 0.6 | 3.1 | 1.1 | 3.2 | 2.4 | 1.4 | 3.7 |
| 358 | 726 | 1.0 | 1.0 | 1.0 | 1.0 | 1.0 | 1.0 | 1.0 |
| 359 | 718 | 0.4 | 2.6 | 0.5 | 2.7 | 1.6 | 1.0 | 1.0 |
| 360 | 703 | 1.0 | 4.1 | 1.0 | 2.4 | 1.6 | 1.0 | 1.7 |
| 361 | 652 | 2.8 | 4.4 | 1.6 | 2.3 | 1.0 | 1.6 | 1.0 |
| 362 | 630 | 6.9 | 1.0 | 2.2 | 0.3 | 0.6 | 1.0 | 0.5 |
| 363 | 593 | 1.0 | 4.3 | 1.0 | 2.3 | 1.0 | 1.0 | 1.0 |
| 364 | 532 | 1.3 | 4.7 | 1.0 | 2.4 | 2.6 | 2.2 | 4.0 |
| 365 | 272 | 0.7 | 2.7 | 0.9 | 3.1 | 2.4 | 1.3 | 4.3 |
| 366 | 256 | 0.6 | 3.2 | 0.5 | 1.9 | 2.8 | 1.0 | 3.4 |
| 367 | 57 | 0.5 | 1.4 | 1.0 | 2.3 | 0.9 | 1.0 | 0.7 |

**Table 11**

| **SEQ ID NO** | **SPOT ID** |
|---|---|
| 1 | 10594 |
| 2 | 21851 |
| 3 | 20990 |
| 4 | 18641 |
| 5 | 19037 |
| 6 | 398 |
| 7 | 18773 |
| 8 | 3583 |
| 9 | 3418 |
| 10 | 145306 |
| 11 | 3418 |
| 12 | 3418 |
| 13 | 18985 |
| 14 | 17229 |
| 15 | 25930 |
| 16 | 25930 |
| 17 | 20701 |
| 18 | 20346 |
| 19 | 20346 |
| 20 | 21247 |
| 21 | 21247 |
| 22 | 23062 |
| 23 | 25666 |
| 24 | 25666 |
| 25 | 19001 |
| 26 | 10897 |
| 27 | 10897 |
| 28 | 10897 |
| 29 | 1960 |
| 30 | 146262 |
| 31 | 26381 |
| 32 | 26381 |
| 33 | 26719 |
| 34 | 26719 |
| 35 | 27152 |
| 36 | 10926 |
| 37 | 28980 |
| 38 | 1236 |
| 39 | 29350 |
| 40 | 29350 |
| 41 | 26242 |
| 42 | 4098 |
| 43 | 145253 |
| 44 | 4098 |
| 45 | 17432 |
| 46 | 17432 |
| 47 | 1785 |
| 48 | 1785 |
| 49 | 1785 |
| 50 | 28856 |
| 51 | 28856 |
| 52 | 18791 |
| 53 | 18791 |
| 54 | 22950 |
| 55 | 22950 |
| 56 | 1882 |
| 57 | 23886 |
| 58 | 24995 |
| 59 | 24995 |
| 60 | 24477 |
| 61 | 21681 |
| 62 | 21681 |
| 63 | 9557 |
| 64 | 9557 |
| 65 | 22033 |
| 66 | 873 |
| 67 | 17144 |
| 68 | 26970 |
| 69 | 26970 |
| 70 | 21402 |
| 71 | 27074 |
| 72 | 27074 |
| 73 | 10963 |
| 74 | 10963 |
| 75 | 29525 |
| 76 | 29525 |
| 77 | 25514 |
| 78 | 25514 |
| 79 | 26612 |
| 80 | 26612 |
| 81 | 24600 |
| 82 | 9741 |
| 83 | 9741 |
| 84 | 9741 |
| 85 | 23689 |
| 86 | 23689 |
| 87 | 22352 |
| 88 | 23806 |
| 89 | 12285 |
| 90 | 27638 |
| 91 | 27638 |
| 92 | 9663 |
| 93 | 9663 |
| 94 | 26850 |
| 95 | 10204 |
| 96 | 10204 |
| 97 | 10204 |
| 98 | 25922 |
| 99 | 25922 |
| 100 | 26347 |
| 101 | 26347 |
| 102 | 20361 |
| 103 | 20361 |
| 104 | 28672 |
| 105 | 28672 |
| 106 | 25520 |
| 107 | 25520 |
| 108 | 1723 |
| 109 | 1723 |
| 110 | 28863 |
| 111 | 25526 |
| 112 | 25526 |
| 113 | 27936 |
| 114 | 27936 |
| 115 | 26851 |
| 116 | 25107 |
| 117 | 25107 |
| 118 | 25107 |
| 119 | 24912 |
| 120 | 24912 |
| 121 | 25169 |
| 122 | 25600 |
| 123 | 25600 |
| 124 | 28706 |
| 125 | 28706 |
| 126 | 26377 |
| 127 | 26377 |
| 128 | 19460 |
| 129 | 25243 |
| 130 | 20018 |
| 131 | 20018 |
| 132 | 918 |
| 133 | 25027 |
| 134 | 29089 |
| 135 | 29089 |
| 136 | 9141 |
| 137 | 9141 |
| 138 | 9141 |
| 139 | 12005 |
| 140 | 12148 |
| 141 | 12148 |
| 142 | 17394 |
| 143 | 27017 |
| 144 | 27017 |
| 145 | 25809 |
| 146 | 8719 |
| 147 | 8719 |
| 148 | 21030 |
| 149 | 21030 |
| 150 | 11436 |
| 151 | 11436 |
| 152 | 10374 |
| 153 | 10374 |
| 154 | 25861 |
| 155 | 25861 |
| 156 | 3317 |
| 157 | 3317 |
| 158 | 8743 |
| 159 | 26240 |
| 160 | 26240 |
| 161 | 28562 |
| 162 | 16877 |
| 163 | 25955 |
| 164 | 26308 |
| 165 | 26308 |
| 166 | 4140 |
| 167 | 3436 |
| 168 | 25612 |
| 169 | 25612 |
| 170 | 12257 |
| 171 | 12257 |
| 172 | 9111 |
| 173 | 9111 |
| 174 | 17620 |
| 175 | 26025 |
| 176 | 26025 |
| 177 | 19271 |
| 178 | 4151 |
| 179 | 4151 |
| 180 | 26569 |
| 181 | 26569 |
| 182 | 10344 |
| 183 | 10344 |
| 184 | 10344 |
| 185 | 832 |
| 186 | 832 |
| 187 | 12071 |
| 188 | 12071 |
| 189 | 12271 |
| 190 | 11433 |
| 191 | 20917 |
| 192 | 25810 |
| 193 | 12039 |
| 194 | 12039 |
| 195 | 25499 |
| 196 | 25499 |
| 197 | 25557 |
| 198 | 25557 |
| 199 | 9917 |
| 200 | 19505 |
| 201 | 17491 |
| 202 | 10683 |
| 203 | 10683 |
| 204 | 1936 |
| 205 | 828 |
| 206 | 9558 |
| 207 | 9558 |
| 208 | 20164 |
| 209 | 969 |
| 210 | 969 |
| 211 | 9910 |
| 212 | 2427 |
| 213 | 19990 |
| 214 | 20605 |
| 215 | 20605 |
| 216 | 10650 |
| 217 | 10650 |
| 218 | 25963 |
| 219 | 25963 |
| 220 | 25562 |
| 221 | 25562 |
| 222 | 3429 |
| 223 | 2725 |
| 224 | 19923 |
| 225 | 20457 |
| 226 | 20457 |
| 227 | 24773 |
| 228 | 24119 |
| 229 | 3908 |
| 230 | 3908 |
| 231 | 8560 |
| 232 | 8560 |
| 233 | 9377 |
| 234 | 9377 |
| 235 | 17618 |
| 236 | 12136 |
| 237 | 17373 |
| 238 | 18577 |
| 239 | 18577 |
| 240 | 3143 |
| 241 | 17737 |
| 242 | 17737 |
| 243 | 20029 |
| 244 | 20029 |
| 245 | 18537 |
| 246 | 18537 |
| 247 | 12102 |
| 248 | 12102 |
| 249 | 8487 |
| 250 | 9252 |
| 251 | 9252 |
| 252 | 25605 |
| 253 | 25605 |
| 254 | 29652 |
| 255 | 10858 |
| 256 | 1261 |
| 257 | 4156 |
| 258 | 4156 |
| 259 | 3452 |
| 260 | 3452 |
| 261 | 2748 |
| 262 | 2046 |
| 263 | 2046 |
| 264 | 2044 |
| 265 | 2044 |
| 266 | 1342 |
| 267 | 1342 |
| 268 | 1326 |
| 269 | 1326 |
| 270 | 9981 |
| 271 | 9981 |
| 272 | 27917 |
| 273 | 8488 |
| 274 | 22793 |
| 275 | 22793 |
| 276 | 26883 |
| 277 | 26883 |
| 278 | 11540 |
| 279 | 17707 |
| 280 | 20649 |
| 281 | 20649 |
| 282 | 24004 |
| 283 | 24004 |
| 284 | 11836 |
| 285 | 11836 |
| 286 | 11836 |
| 287 | 24932 |
| 288 | 19143 |
| 289 | 19143 |
| 290 | 26257 |
| 291 | 26257 |
| 292 | 21239 |
| 293 | 21239 |
| 294 | 16959 |
| 295 | 2568 |
| 296 | 25936 |
| 297 | 25936 |
| 298 | 23041 |
| 299 | 9206 |
| 300 | 25105 |
| 301 | 25105 |
| 302 | 24779 |
| 303 | 22451 |
| 304 | 22451 |
| 305 | 22291 |
| 306 | 22291 |
| 307 | 21143 |
| 308 | 24751 |
| 309 | 24751 |
| 310 | 24294 |
| 311 | 24294 |
| 312 | 24006 |
| 313 | 24006 |
| 314 | 25678 |
| 315 | 25678 |
| 316 | 22027 |
| 317 | 29495 |
| 318 | 29495 |
| 319 | 24577 |
| 320 | 24577 |
| 321 | 24577 |
| 322 | 23527 |
| 323 | 17090 |
| 324 | 25137 |
| 325 | 23772 |
| 326 | 1659 |
| 327 | 8497 |
| 328 | 25272 |
| 329 | 21216 |
| 330 | 21216 |
| 331 | 21216 |
| 332 | 11939 |
| 333 | 11939 |
| 334 | 11939 |
| 335 | 9191 |
| 336 | 3429 |
| 337 | 24588 |
| 338 | 4047 |
| 339 | 28344 |
| 340 | 28344 |
| 341 | 27561 |
| 342 | 3272 |
| 343 | 26735 |
| 344 | 26735 |
| 345 | 24900 |
| 346 | 24900 |
| 347 | 9472 |
| 348 | 9472 |
| 349 | 9979 |
| 350 | 21996 |
| 351 | 22312 |
| 352 | 11327 |
| 353 | 18240 |
| 354 | 18240 |
| 355 | 21922 |
| 356 | 21922 |
| 357 | 22290 |
| 358 | 10390 |
| 359 | 10390 |
| 360 | 2212 |
| 361 | 20213 |
| 362 | 20213 |
| 363 | 24955 |
| 364 | 19574 |
| 365 | 19969 |
| 366 | 8570 |
| 367 | 18519 |
| 368 | 9616 |
| 369 | 9616 |
| 370 | 17459 |
| 371 | 17459 |
| 372 | 25193 |
| 373 | 25193 |
| 374 | 25193 |
| 375 | 25191 |
| 376 | 22566 |
| 377 | 4164 |
| 378 | 4146 |
| 379 | 4072 |
| 380 | 4022 |
| 381 | 3954 |
| 382 | 3838 |
| 383 | 3806 |
| 384 | 3798 |
| 385 | 3792 |
| 386 | 3788 |
| 387 | 3458 |
| 388 | 3194 |
| 389 | 3102 |
| 390 | 25191 |
| 391 | 25191 |
| 392 | 9448 |
| 393 | 9448 |
| 394 | 25224 |
| 395 | 20218 |
| 396 | 3089 |
| 397 | 3089 |
| 398 | 19953 |
| 399 | 19953 |
| 400 | 22362 |
| 401 | 25516 |
| 402 | 25516 |
| 403 | 25757 |
| 404 | 24814 |
| 405 | 21994 |
| 406 | 27117 |
| 407 | 22745 |
| 408 | 24233 |
| 409 | 2001 |
| 410 | 2001 |
| 411 | 2001 |
| 412 | 17147 |
| 413 | 21214 |
| 414 | 21214 |
| 415 | 21214 |
| 416 | 26422 |
| 417 | 21965 |
| 418 | 25541 |
| 419 | 25541 |
| 420 | 18302 |
| 421 | 18302 |
| 422 | 24049 |
| 423 | 24049 |
| 424 | 26326 |
| 425 | 26326 |
| 426 | 2254 |
| 427 | 162502 |
| 428 | 10296 |
| 429 | 20044 |
| 430 | 28806 |
| 431 | 17566 |
| 432 | 17566 |
| 433 | 19005 |
| 434 | 3567 |
| 435 | 159223 |
| 436 | 3567 |
| 437 | 3567 |
| 438 | 458 |
| 439 | 21411 |
| 440 | 22972 |
| 441 | 24853 |
| 442 | 21502 |
| 443 | 18180 |
| 444 | 23918 |
| 445 | 24144 |
| 446 | 19996 |
| 447 | 11528 |
| 448 | 20506 |
| 449 | 20506 |
| 450 | 23833 |
| 451 | 20042 |
| 452 | 20042 |
| 453 | 11646 |
| 454 | 10577 |
| 455 | 10577 |
| 456 | 18556 |
| 457 | 29433 |
| 458 | 28763 |
| 459 | 27450 |
| 460 | 27450 |
| 461 | 27255 |
| 462 | 26550 |
| 463 | 26550 |
| 464 | 26508 |
| 465 | 26334 |
| 466 | 26334 |
| 467 | 26027 |
| 468 | 26027 |
| 469 | 25977 |
| 470 | 25977 |
| 471 | 25965 |
| 472 | 25965 |
| 473 | 25844 |
| 474 | 25844 |
| 475 | 25834 |
| 476 | 25816 |
| 477 | 25746 |
| 478 | 25712 |
| 479 | 25621 |
| 480 | 25621 |
| 481 | 25614 |
| 482 | 25614 |
| 483 | 25603 |
| 484 | 25603 |
| 485 | 25556 |
| 486 | 25556 |
| 487 | 25555 |
| 488 | 25555 |
| 489 | 3094 |
| 490 | 2567 |
| 491 | 1958 |
| 492 | 1680 |
| 493 | 1445 |
| 494 | 1320 |
| 495 | 974 |
| 496 | 652 |
| 497 | 630 |
| 498 | 593 |
| 499 | 256 |

## Claims

1. Use of an agent selected from the group consisting of an antibody, an antisense polynucleotide, and an RNAi molecule based on the DKFZp566I133 polynucleotide or corresponding polypeptide for inhibition of DKFZp566I133 activity for the preparation of a pharmaceutical composition for inhibiting a cancerous phenotype of a breast cell.

2. Use of claim 1, wherein said cancerous phenotype is aberrant cellular proliferation relative to a normal cell.

3. Use of claims 1-2, wherein said cancerous phenotype is loss of contact inhibition of cell growth.

4. Use of claims 1-3, wherein said inhibition is associated with a reduction in a level of DKFZp566I133 protein.

5. Use of claims 1-4, wherein said inhibition is associated with a reduction in a level of DKFZp566I133 RNA.

6. Use of claims 1-5, wherein said inhibition is associated with a reduction in a level of activity of DKFZp566I133 protein.

7. Use of claims 1-6, wherein said pharmaceutical composition is for treating breast cancer.

8. Method for detecting a cancerous breast cell, said method comprising:
detecting a level of DKFZp566I133 or fragment thereof in a test sample obtained from a cell of a subject, and
comparing the level ofDKFZp566I133 to a control level of DKFZp566I133,
wherein the presence of a cancerous breast cell is indicated by detection of said level and comparison to a control level of DKFZp566I133.

9. Method of claim 8, wherein said level of DKFZp566I133 is detected by measuring a level of a DKFZp566I133 gene product.

10. Method of claim 9, wherein said gene product is a polypeptide or a nucleic acid.

11. Method of claims 8-10, wherein said detecting step uses a polymerase chain reaction.

12. Method of claims 8-11, wherein said detecting step uses hybridization.

13. Method of claims 8-12, wherein said sample is a sample of breast tissue.

14. Method of claims 8-13, wherein said level of said DKFZp566I133 or fragment thereof is indicative of the cancerous state of the cell of the test sample.

15. Method for assessing the tumor burden of a subject suspected of or having a breast tumor, said method comprising: detecting a level of DKFZp566I133 in a test sample from a subject, wherein the level of DKFZp566I133 in the test sample is indicative of the tumor burden in the subject.

16. Method for identifying an agent that modulates a biological activity of a gene product differentially expressed in a cancerous breast cell as compared to a normal breast cell, said method comprising:
contacting a candidate agent selected from the group consisting of an antibody, an antisense polynucleotide, and an RNAi molecule with a cancerous cell expressing a DKFZp566I133 gene product; and
detecting modulation of expression of the DKFZp566I133 gene product relative to a level of expression of the DKFZp566I133 gene product in the absence of the candidate agent.

17. Method of claim 16, wherein expression is assessed by detecting a polynucleotide gene product.

18. Method of claim 16, wherein expression is assessed by detecting a polypeptide gene product.

## Patentansprüche

1. Verwendung eines auf dem DKFZp566I133-Polynukleotid oder dem entsprechenden Polypeptid basierenden Mittels zur Inhibierung der DKFZp566I133-Aktivität, das ausgewählt ist aus der Gruppe bestehend aus einem Antikörper, einem Antisense-Polynukleotid und einem RNAi-Molekül, für die Herstellung einer pharmazeutischen Zusammensetzung zur Inhibierung eines kanzerösen Phänotyps einer Brustzelle.

2. Verwendung nach Anspruch 1, wobei der kanzeröse Phänotyp eine anomale zelluläre Proliferation im Verhältnis zu einer normalen Zelle ist.

3. Verwendung nach den Ansprüchen 1 bis 2, wobei der kanzeröse Phänotyp ein Verlust der Kontaktinhibierung des Zellwachstums ist.

4. Verwendung nach den Ansprüchen 1 bis 3, wobei die Inhibierung mit einer Verringerung einer Menge von DKFZp566I133-Protein assoziiert.

5. Verwendung nach den Ansprüchen 1 bis 4, wobei die Inhibierung mit einer Verringerung einer Menge von DKFZp566I133-RNA assoziiert ist.

6. Verwendung nach den Ansprüchen 1 bis 5, wobei die Inhibierung mit einer Verringerung der Menge der Aktivität von DKFZp566I133-Protein assoziiert ist.

7. Verwendung nach den Ansprüchen 1 bis 6, wobei die pharmazeutische Zusammensetzung für die Behandlung von Brustkrebs ist.

8. Verfahren zum Nachweis einer kanzerösen Brustzelle, bei dem man
eine Menge von DKFZp566I133 oder eines Fragments davon in einer Testprobe nachweist, die von einer Zelle eines Subjekts erhalten wurde, und
die Menge von DKFZp566I133 mit der Kontrollmenge von DKFZp566I133 vergleicht,
wobei das Vorhandensein einer kanzerösen Brustzelle durch Nachweis der Menge und durch Vergleich mit einer Kontrollmenge von DKFZp566I133 angezeigt wird.

9. Verfahren nach Anspruch 8, bei dem die Menge von DKFZp566I133 durch Messung der Menge eines DKFZp566I133-Genprodukt nachgewiesen wird.

10. Verfahren nach Anspruch 9, bei dem das Genprodukt ein Polypeptid oder eine Nukleinsäure ist.

11. Verfahren nach den Ansprüchen 8 bis 10, bei dem der Nachweis-Schritt eine Polymerasekettenreaktion verwendet.

12. Verfahren nach den Ansprüchen 8 bis 11, bei dem der Nachweisschritt eine Hybridisierung verwendet.

13. Verfahren nach den Ansprüchen 8 bis 12, bei dem die Probe eine Probe von Brustgewebe ist.

14. Verfahren nach den Ansprüchen 8 bis 13, bei dem die Menge von DKFZp566I133 oder eines Fragments davon indikativ für den kanzerösen Zustand der Zelle der Testprobe ist.

15. Verfahren zur Erfassung der Tumorbelastung eines Subjektes, das einen Brusttumor aufweist oder im Verdacht steht, einen Brusttumor aufzuweisen, bei dem man: die Menge von DKFZp566I133 in einer Testprobe von einem Subjekt nachweist, wobei die Menge von DKFZp566I133 in der Testprobe indikativ für die Tumorbelastung in dem Subjekt ist.

16. Verfahren zur Identifizierung eines Mittels, das eine biologische Aktivität eines Genprodukts moduliert, welches in einer kanzerösen Brustzelle im Vergleich zu einer normalen Brustzelle differentiell exprimiert wird, bei dem man:
ein Kandidatenmittel, das ausgewählt ist aus der Gruppe bestehend aus einem Antikörper, einem Antisense-Polynukleotid und einem RNAi-Molekül mit einer kanzerösen Zelle in Kontakt bringt, die ein DKFZp566I133-Genprodukt exprimiert; und
die Modulation der Expression des DKFZp566I133-Genprodukts relativ zur Menge der Expression des DKFZp566I133-Genprodukts in Abwesenheit der Kandidatensubstanz nachweist.

17. Verfahren nach Anspruch 16, bei dem die Expression durch Nachweis eines Polynukleotid-Genprodukts festgestellt wird.

18. Verfahren nach Anspruch 16, bei dem die Expression durch Nachweis eines Polypeptid-Genprodukts nachgewiesen wird.

## Revendications

1. Utilisation d'un agent choisi dans le groupe constitué par un anticorps, un polynucléotide antisens, et une molécule d'ARNi basés sur le polynucléotide DKFZp566I133 ou le polypeptide correspondant pour l'inhibition de l'activité de DKFZp566I133, pour la préparation d'une composition pharmaceutique destinée à inhiber un phénotype cancéreux d'une cellule du sein.

2. Utilisation selon la revendication 1, dans laquelle ledit phénotype cancéreux est une prolifération cellulaire aberrante par rapport à une cellule normale.

3. Utilisation selon les revendications 1-2, dans laquelle ledit phénotype cancéreux est une perte d'inhibition de contact de la croissance cellulaire.

4. Utilisation selon les revendications 1-3, dans laquelle ladite inhibition est associée à une réduction du niveau de la protéine DKFZp566I133.

5. Utilisation selon les revendications 1-4, dans laquelle ladite inhibition est associée à une réduction du niveau d'ARN de DKFZp566I133.

6. Utilisation selon les revendications 1-5, dans laquelle ladite inhibition est associée à une réduction du niveau d'activité de la protéine DKFZp566I133.

7. Utilisation selon les revendications 1-6, dans laquelle ladite composition pharmaceutique est destinée au traitement du cancer du sein.

8. Procédé de détection d'une cellule du sein cancéreuse, ledit procédé comprenant:
la détection d'un niveau de DKFZp566I133 ou d'un de ses fragments dans un échantillon d'essai obtenu à partir d'une cellule d'un sujet, et
la comparaison du niveau de DKFZp566I133 à un niveau témoin de DKFZp566I133,
la présence d'une cellule du sein cancéreuse étant indiquée par la détection dudit niveau et la comparaison à un niveau témoin de DKFZp566I133.

9. Procédé selon la revendication 8, dans lequel ledit niveau de DKFZp566I133 est détecté par mesure du niveau d'un produit du gène de DKFZp566I133.

10. Procédé selon la revendication 9, dans lequel ledit produit du gène est un polypeptide ou un acide nucléique.

11. Procédé selon les revendications 8-10, dans lequel ladite étape de détection utilise une amplification en chaîne par polymérase.

12. Procédé selon les revendications 8-11, dans lequel ladite étape de détection utilise une hybridation.

13. Procédé selon les revendications 8-12, dans lequel ledit échantillon est un échantillon de tissu du sein.

14. Procédé selon les revendications 8-13, dans lequel ledit niveau dudit DKFZp566I133 ou de son fragment indique l'état cancéreux de la cellule de l'échantillon d'essai.

15. Procédé d'évaluation de la charge tumorale d'un sujet suspecté d'avoir ou ayant une tumeur du sein, ledit procédé comprenant: la détection d'un niveau de DKFZp566I133 dans un échantillon d'essai du sujet, le niveau de DKFZp566I133 dans l'échantillon d'essai indiquant la charge tumorale chez le sujet.

16. Procédé d'identification d'un agent qui module une activité biologique d'un produit de gène exprimé de manière différentielle dans une cellule du sein cancéreuse par comparaison à une cellule du sein normale, ledit procédé comprenant:
la mise en contact d'un agent candidat choisi dans le groupe constitué par un anticorps, un polynucléotide antisens et une molécule d'ARNi avec une cellule cancéreuse exprimant un produit du gène de DKFZp566I133; et
la détection de la modulation de l'expression du produit du gène de DKFZp566I133 par rapport au niveau d'expression du produit du gène de DKFZp566I133 en l'absence de l'agent candidat.

17. Procédé selon la revendication 16, dans lequel l'expression est évaluée par détection d'un produit de gène polynucléotidique.

18. Procédé selon la revendication 16, dans lequel l'expression est évaluée par détection d'un produit de gène polypeptidique.
